# EUROPEAN PATENT APPLICATION

(11) **EP 2 177 523 A1**
(43) Date of publication of application: **21.04.2010**
(21) Application number: 09177761.5
(22) Date of filing: 02.05.2008
(51) Int. Cl.: C07D 487/04, C08H 1/00, A61K 31/407, A61P 31/12

(54) **Novel macrocyclic inhibitors of hepatitis c virus replication**

(30) Priority: 03.05.2007 US 915896 P; 23.08.2007 US 957630 P; 20.12.2007 US 15644 P
(62) Divisional of application: 08755035.6
(71) Applicant: Intermune, Inc., Brisbane, CA 94005 (US); Array Biopharma, Boulder, CO 80301 (US)
(72) Inventor: Blatt, Lawrence M., San Francisco, CA 94121 (US); Pan, Lin, Sunnyvale, CA 94086 (US); Seiwert, Scott, Pacifica, CA 94044 (US); Andrews, Steven W., Longmont, CO 80501 (US); Martin, Pierre, 4310, Rheinfelden (CH); Schumacher, Andreas, 79588, Efringen-Kirchen (DE); Beigelman, Leonid, San Mateo, CA 94402 (US); Liu, Jyanwei, Sunnyvale, CA 94087 (US); Condroski, Kevin, Lafayette, CO 80026 (US); Jiang, Yutong, Longmont, CO 80501 (US); Kaus, Robert, Longmont, CO 80503 (US); Kennedy, April, Denver, CO 80218 (US); Kercher, Timothy, Boulder, CO 80301 (US); Lyon, Michael, Superior, CO 80027 (US); Wang, Bin, Longmont, CO 80501 (US)
(74) Representative: Paemen, Liesbet R.J.

(57) **Abstract**

The embodiments provide macrocyclic compounds of the general Formula I, as well as compositions, including pharmaceutical compositions, comprising a subject compound. The embodiments further provide treatment methods, including methods of treating a hepatitis C virus infection and methods of treating liver fibrosis, the methods generally involving administering to an individual in need thereof an effective amount of a subject compound or composition.

## Description

### BACKGROUND OF THE INVENTION

### Field of the Invention

The present invention relates to compounds, processes for their synthesis, compositions and methods for the treatment of hepatitis C virus (HCV) infection.

### Description of the Related Art

Hepatitis C virus (HCV) infection is the most common chronic blood borne infection in the United States. Although the numbers of new infections have declined, the burden of chronic infection is substantial, with Centers for Disease Control estimates of 3.9 million (1.8%) infected persons in the United States. Chronic liver disease is the tenth leading cause of death among adults in the United States, and accounts for approximately 25,000 deaths annually, or approximately 1% of all deaths. Studies indicate that 40% of chronic liver disease is HCV-related, resulting in an estimated 8,000-10,000 deaths each year. HCV-associated end-stage liver disease is the most frequent indication for liver transplantation among adults.

Antiviral therapy of chronic hepatitis C has evolved rapidly over the last decade, with significant improvements seen in the efficacy of treatment. Nevertheless, even with combination therapy using pegylated IFN-α plus ribavirin, 40% to 50% of patients fail therapy, i.e., are nonresponders or relapsers. These patients currently have no effective therapeutic alternative. In particular, patients who have advanced fibrosis or cirrhosis on liver biopsy are at significant risk of developing complications of advanced liver disease, including ascites, jaundice, variceal bleeding, encephalopathy, and progressive liver failure, as well as a markedly increased risk of hepatocellular carcinoma.

The high prevalence of chronic HCV infection has important public health implications for the future burden of chronic liver disease in the United States. Data derived from the National Health and Nutrition Examination Survey (NHANES III) indicate that a large increase in the rate of new HCV infections occurred from the late 1960s to the early 1980s, particularly among persons between 20 to 40 years of age. It is estimated that the number of persons with long-standing HCV infection of 20 years or longer could more than quadruple from 1990 to 2015, from 750,000 to over 3 million. The proportional increase in persons infected for 30 or 40 years would be even greater. Since the risk of HCV-related chronic liver disease is related to the duration of infection, with the risk of cirrhosis progressively increasing for persons infected for longer than 20 years, this will result in a substantial increase in cirrhosis-related morbidity and mortality among patients infected between the years of 1965-1985.

HCV is an enveloped positive strand RNA virus in the Flaviviridae family. The single strand HCV RNA genome is approximately 9500 nucleotides in lingth and has a single open reading frame (ORF) encoding a single large polyprotein of about 3000 amino acids. In infected cells, this polyprotein is cleaved at multiple sites by cellular and viral proteases to produce the structural and non-structural (NS) proteins of the virus. In the case of HCV, the generation of mature nonstructural proteins (NS2, NS3, NS4, NS4A, NS4B, NS5A, and NS5B) is effected by two viral proteases. The first viral protease cleaves at the NS2-NS3 junction of the polyprotein. The second viral protease is serine protease contained within the N-terminal region of NS3 (herein referred to as "NS3 protease"). NS3 protease mediates all of the subsequent cleavage events at sites downstream relative to the position of NS3 in the polyprotein (i.e., sites located between the C-terminus of NS3 and the C-terminus of the polyprotein). NS3 protease exhibits activity both in cis, at the NS3-NS4 cleavage site, and in trans, for the remaining NS4A-NS4B, NS4B-NS5A, and NS5A-NS5B sites. The NS4A protein is believed to serve multiple functions, acting as a cofactor for the NS3 protease and possibly assisting in the membrane localization of NS3 and other viral replicase components. Apparently, the formation of the complex between NS3 and NS4A is necessary for NS3-mediated processing events and enhances proteolytic efficiency at all sites recognized by NS3. The NS3 protease also exhibits nucleoside triphosphatase and RNA helicase activities. NS5B is an RNA-dependent RNA polymerase involved in the replication of HCV RNA.

### SUMMARY OF THE INVENTION

The present embodiments provide compounds of the general Formula I: 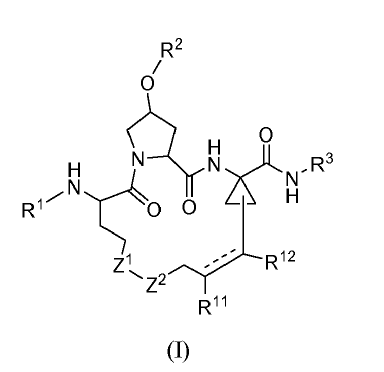 or a pharmaceutically acceptable salt or prodrug thereof wherein:
R¹ is selected from the group consisting of substituted aryl, substituted heteroaryl, - C(O)OR⁴, -C(O)NR⁵R⁶, -C(O)R⁷, and 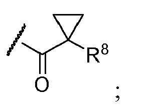
R² is selected from the group consisting of alkyl, -C(O)-alkyl, 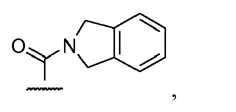 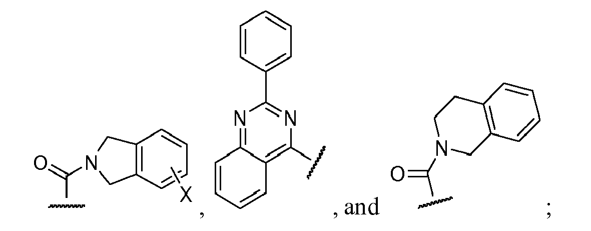
R³ is selected from the group consisting of -OR⁹ and -SO₂R¹⁰;
R⁴ is selected from the group consisting of alkyl, heterocyclyl, and aryl;
R⁵ is alkyl and R⁶ is selected from the group consisting of alkyl and aralkyl, or R⁵ together with R⁶ form an optionally substituted heterocyclyl or optionally substituted heteroaryl;
R⁷ is phenyl substituted one or more times with halogen;
R⁸ is selected from the group consisting of -CF₃ and methyl;
R⁹ is selected from the group consisting of hydrogen, optionally substituted alkyl, optionally substituted aryl, and optionally substituted aralkyl;
R¹⁰ is selected from the group consisting of alkyl optionally substituted with alkoxy or alkenyl, optionally substituted aryl, optionally substituted aralkyl, substituted heteroaryl, and 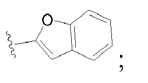
R¹¹ and R¹² are each hydrogen or together with the carbon atoms to which they are attached form an optionally substituted cycloalkyl;
X is halogen and is present 1 to 4 times; and
Z¹ and Z² are independently selected from the group consisting of -CH₂-, -CF₂-, and -O- provided that at least one of Z¹ and Z² is -CH₂-;
provided that if R¹¹ and R¹² are each hydrogen, R² is alkyl, and R³ is -SO₂-cyclopropyl, then R¹ is not -C(O)O-t-butyl;
provided that if R¹¹ and R¹² are each hydrogen and R² is 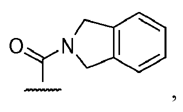 then R³ is -SO₂-phenyl disubstituted with halogen or -SO₂-thiophene disubstituted with halogen, or R¹ is 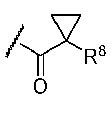 where R⁸ is -CF₃;
provided that if R¹¹ and R¹² are each hydrogen, R² is 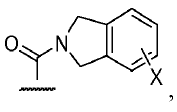 and R³ is - SO₂-cyclopropyl, then R¹ is not -C(O)O-t-butyl, -C(O)O-haloalkyl, or -C(O)O-cyclopentyl;
provided that if R¹¹ and R¹² are each hydrogen, R² is 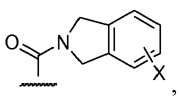 and R³ is substituted -SO₂-heteroaryl, then R¹ is not -C(O)O-cyclopentyl;
provided that if R¹¹ and R¹² are each hydrogen, R² is 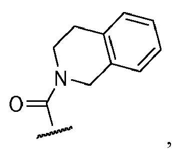 and R³ is - SO₂-cyclopropyl, then R¹ is not -C(O)O-alkyl or -C(O)O-heterocyclyl;
provided that if R¹¹ and R¹² are each hydrogen, R² is 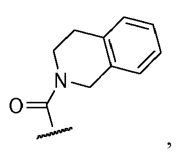 and R³ is - SO₂-alkyl or optionally substituted -SO₂-aryl, then R¹ is not -C(O)O-cycloalkyl;
provided that if R¹¹ and R¹² are each hydrogen, R² is 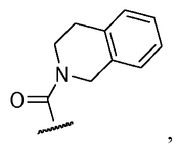 and R³ is optionally substituted -SO₂-phenyl, then R¹ is not -C(O)O-t-butyl;
provided that if R¹¹ and R¹² are each hydrogen, R² is 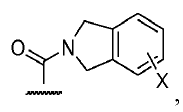 and R³ is - SO₂-alkyl substituted with alkoxy or alkenyl, then R¹ is -C(O)O-t-butyl and X is F; and
provided that the compound of formula (I) is not selected from the group consisting of: 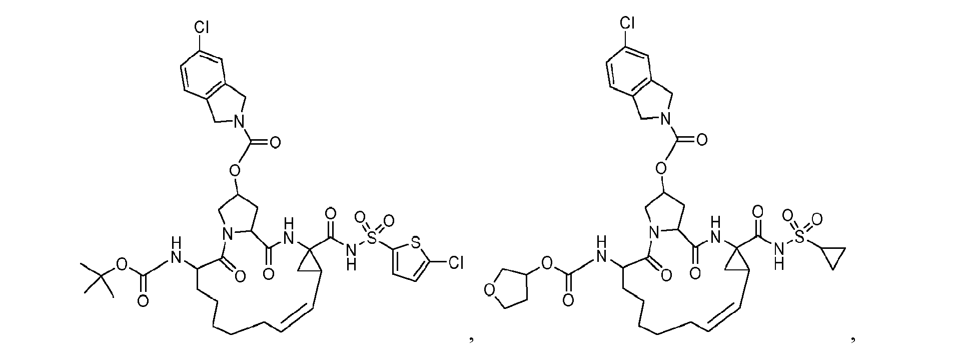 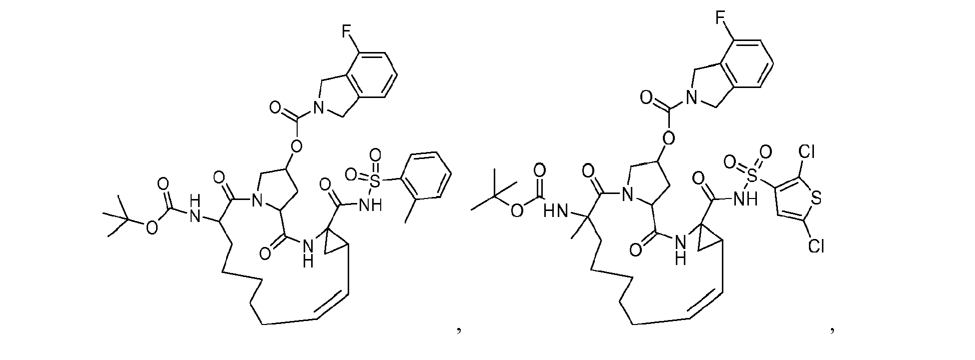 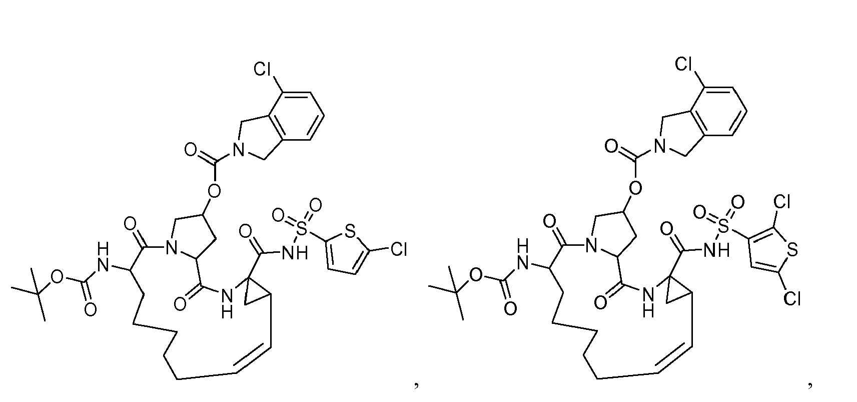 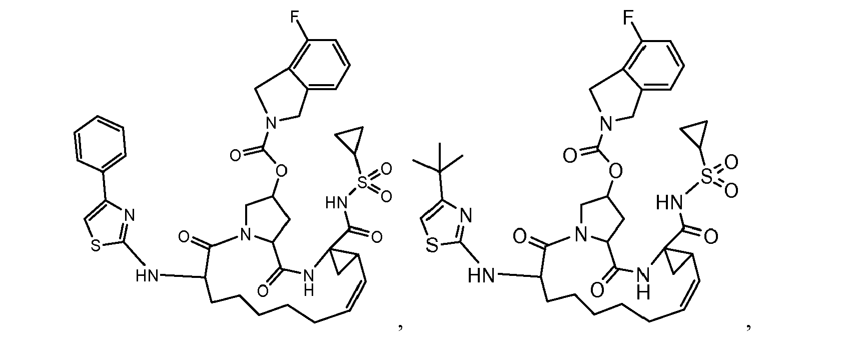 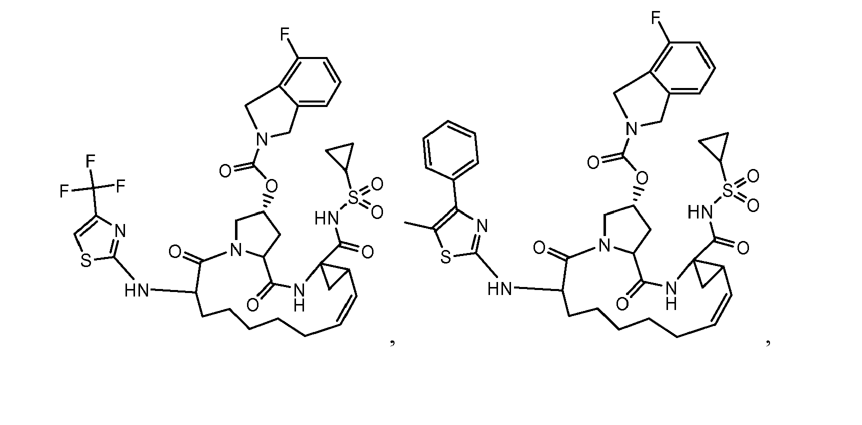 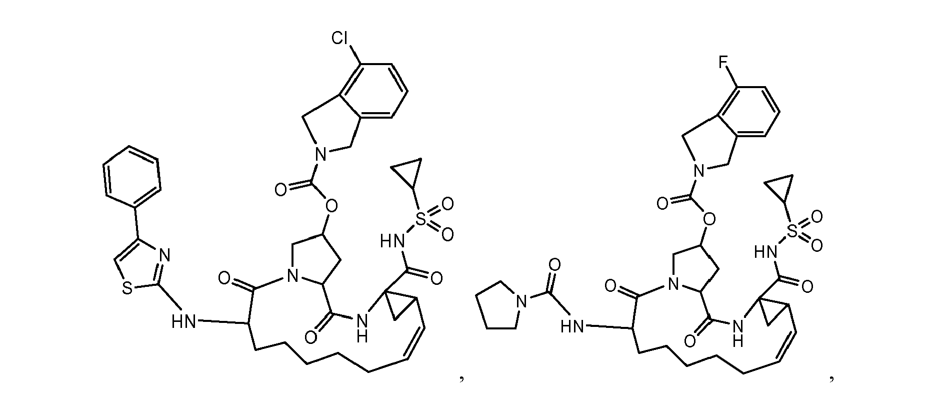 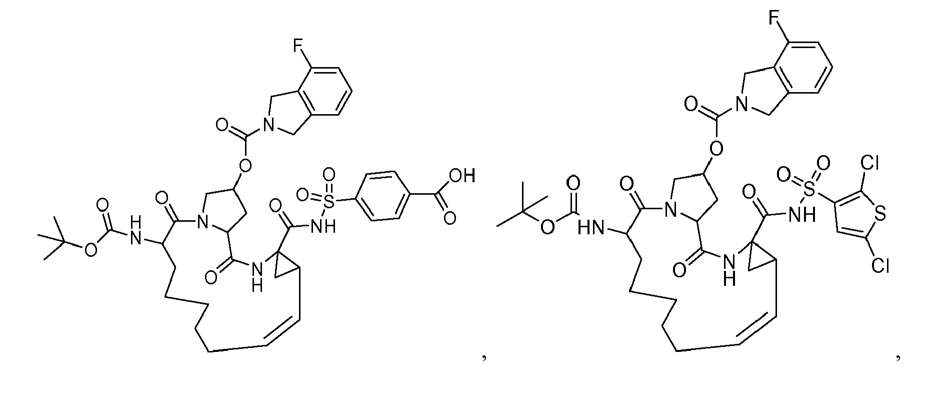 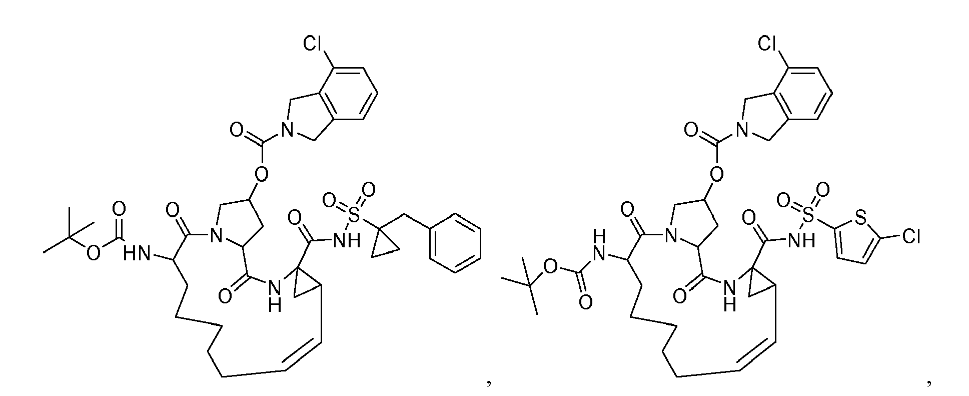 and

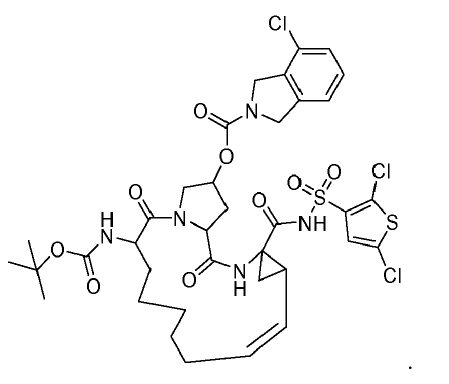

The present embodiments provide for a method of inhibiting NS3/NS4 protease activity comprising contacting a NS3/NS4 protease with a compound disclosed herein.

The present embodiments provide for a method of treating hepatitis by modulating NS3/NS4 protease comprising contacting a NS3/NS4 protease with a compound disclosed herein.

Preferred embodiments provide a pharmaceutical composition comprising: a) a preferred compound; and b) a pharmaceutically acceptable carrier.

Preferred embodiments provide a method of treating a hepatitis C virus infection in an individual, the method comprising administering to the individual an effective amount of a composition comprising a preferred compound.

Preferred embodiments provide a method of treating liver fibrosis in an individual, the method comprising administering to the individual an effective amount of a composition comprising a preferred compound.

Preferred embodiments provide a method of increasing liver function in an individual having a hepatitis C virus infection, the method comprising administering to the individual an effective amount of a composition comprising a preferred compound.

The present embodiments also provide a method of synthesizing a compound having the structure: 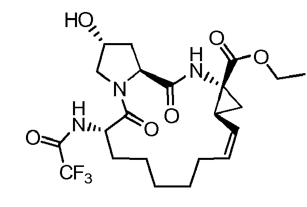 1-A, comprising:
(a) coupling a compound of formula **4-BB** with a compound of formula **5-H** to provide a compound of formula **3-A:** 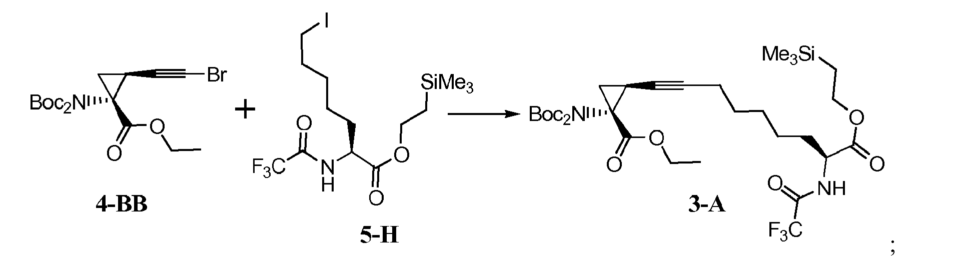
(b) deprotecting a compound of formula **3-A** to provide a compound of formula **3-B:** 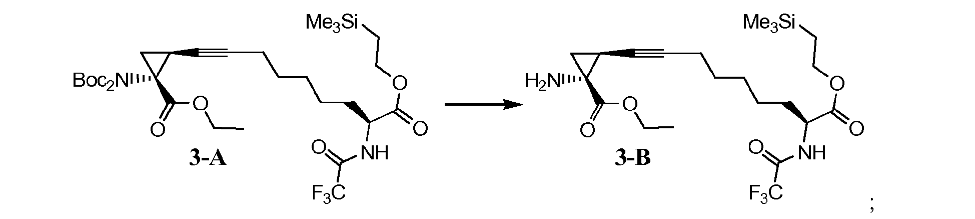
(c) coupling a compound of formula **3-B** with Boc-L-hydroxyproline **(3-D)** to provide a compound of formula **2-A:** 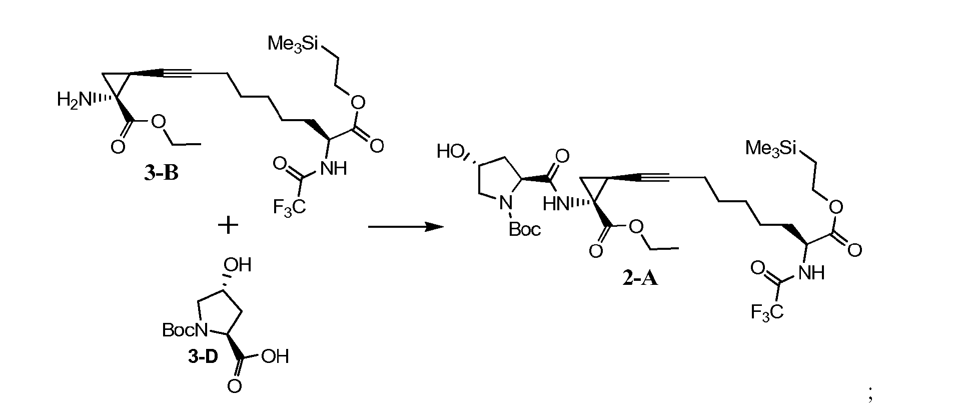
(d) hydrogenating a compound of formula **2-A** to provide a compound a compound of formula **1-D:** 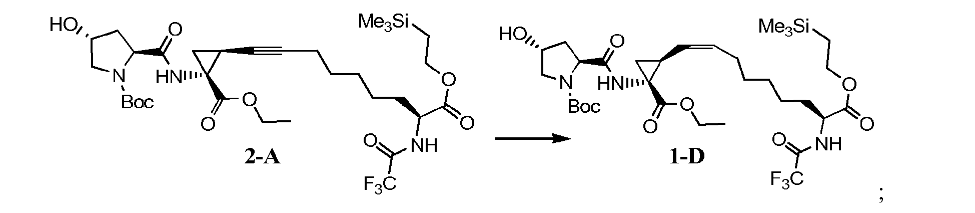
(e) deprotecting a compound of formula **1-D** to provide a compound of formula **1-E:** 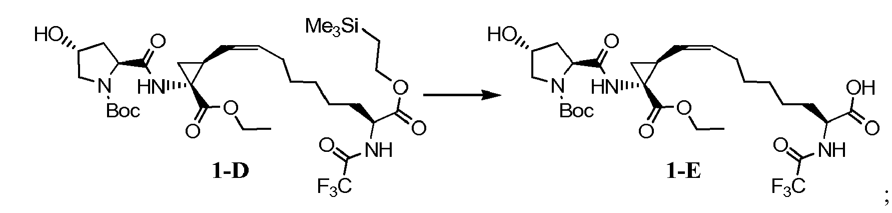 and
(f) transforming a compound of formula **1-E** to provide a compound of formula **1-A:**

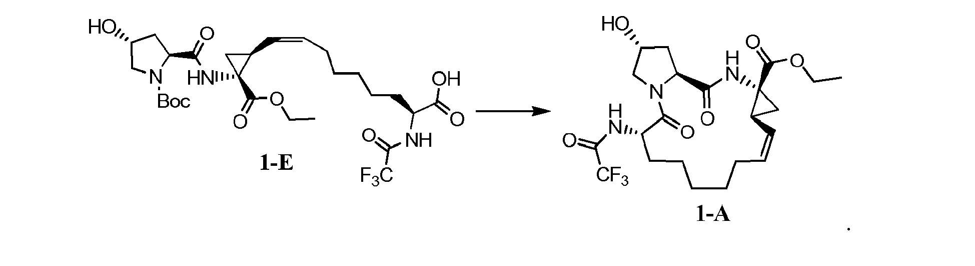

The present embodiments also provide another method of synthesizing compound **1-A,** comprising:
(a) coupling a compound of formula **4-BB** with a compound of formula **5-H** to provide a compound of formula **3-A:** 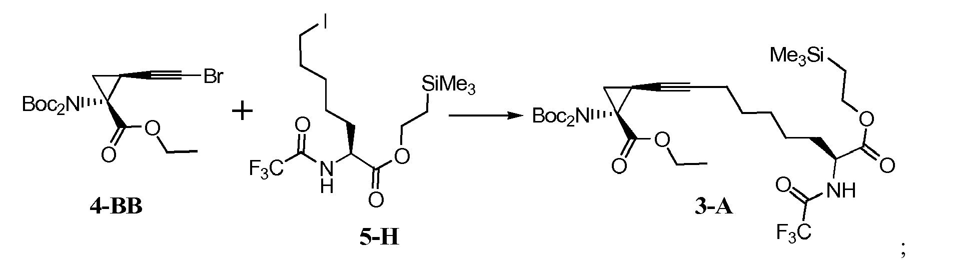
(b) deprotecting a compound of formula **3-A** to provide a compound of formula **3-C:** 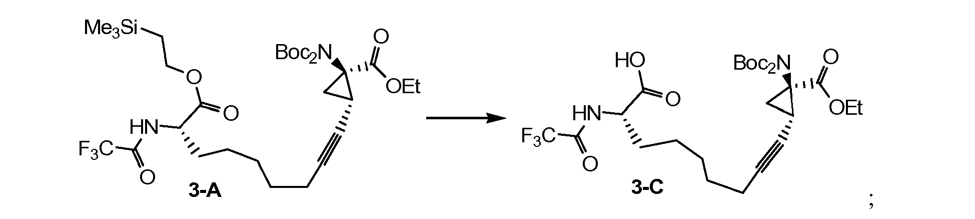
(c) coupling a compound of formula **3-C** with a compound of formula **3-F** to provide a compound of formula **2-B:** 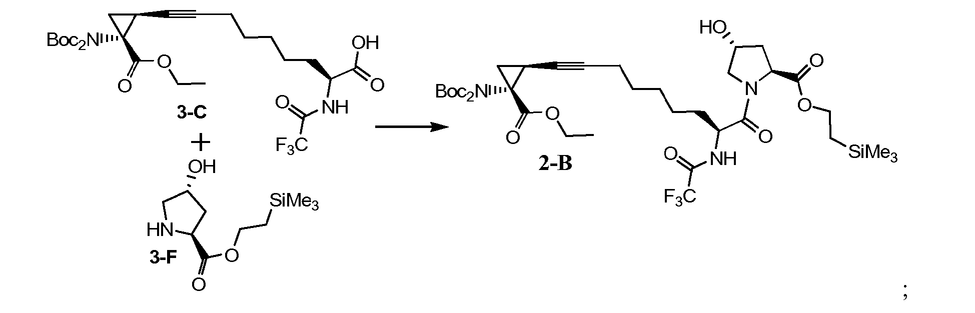
(d) hydrogenating a compound of formula **2-B** to provide a compound a compound of formula **1-H:** 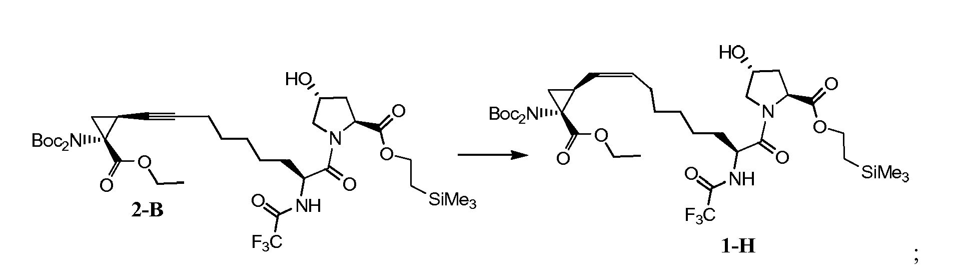
(e) deprotecting a compound of formula **1-H** to provide a compound of formula **1-I:** 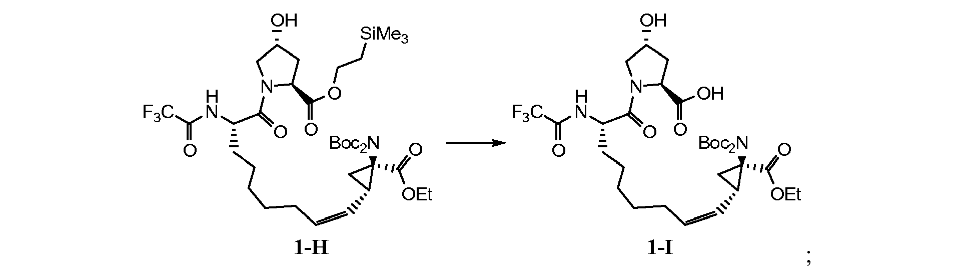
(f) deprotecting a compound of formula **1-I** to provide a compound of formula **1-J:** 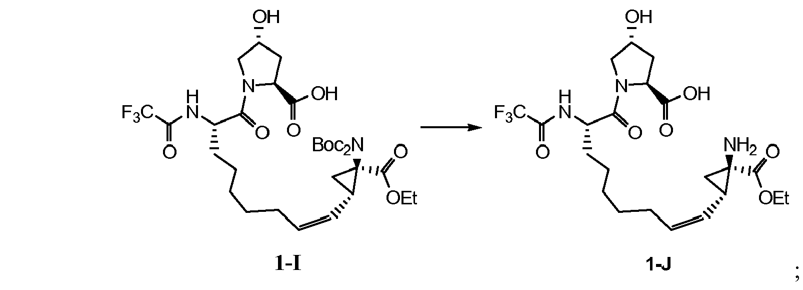 and
(g) cyclizing a compound of formula **1-J** to provide a compound of formula **1-A:**

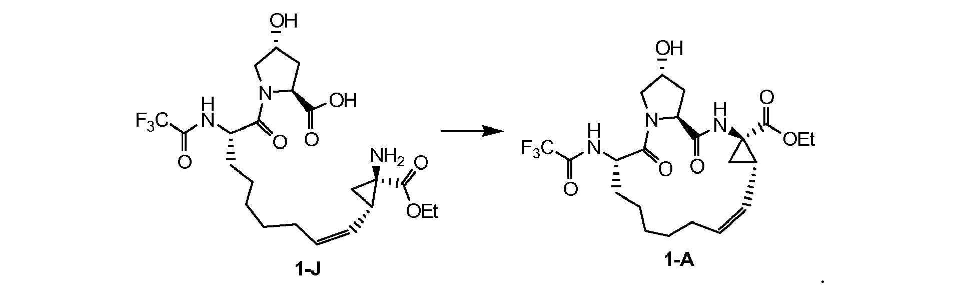

The present embodiments provide a method of synthesizing a compound having the structure: 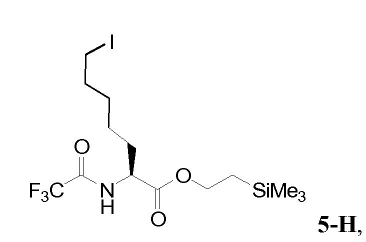 comprising:
(a) saponifying a compound of formula **5-A** to provide a compound of formula **5-B:** 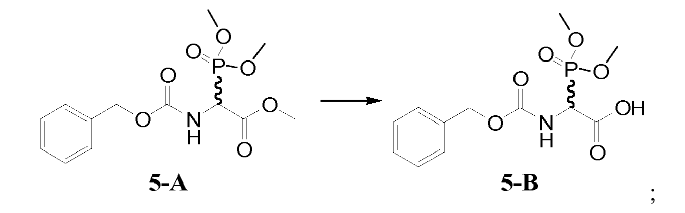
(b) esterifying a compound of formula **5-B** to provide a compound of formula **5-C:** 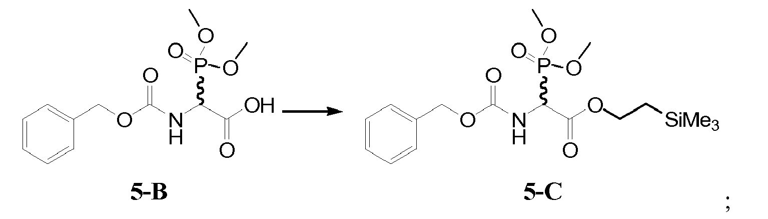
(c) transforming a compound of formula **5-C** to provide a compound of formula **5-E:** 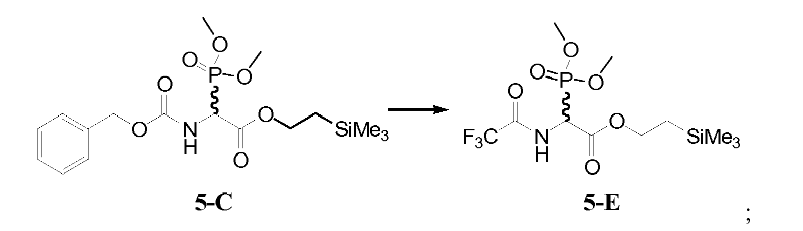
(d) coupling a compound of formula **5-E** with 5-chlorobutanal to provide a compound of formula **5-F:** 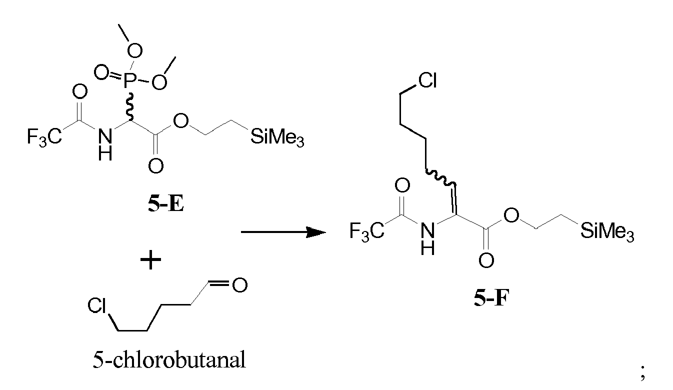
(e) reducing a compound of formula **5-F** to provide a compound of formula **5-G:** 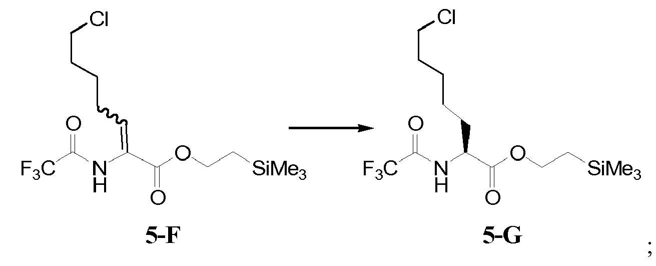 and
(f) transforming a compound of formula **5-G** to provide a compound of formula **5-H:**

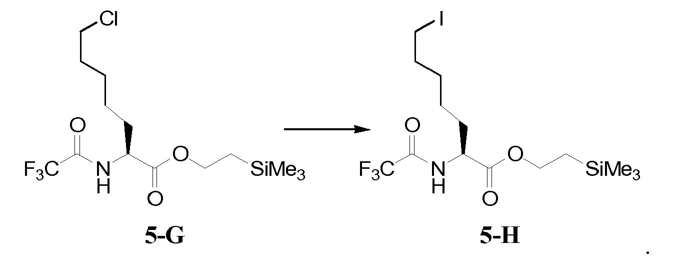

The present embodiments provide a method of synthesizing a compound having the structure: 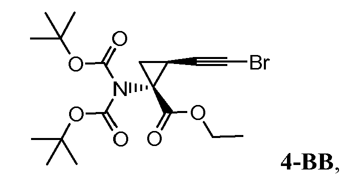 comprising:
(a) protecting a compound of formula **6-A** to provide a compound of formula **6-B:** 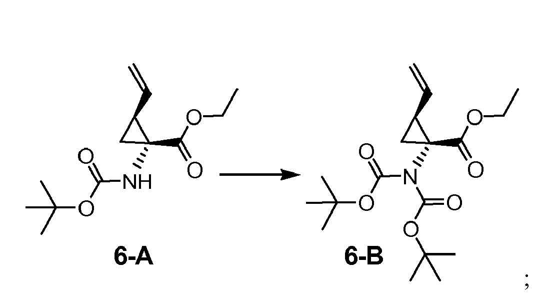
(b) brominating a compound of formula **6-B** to provide a compound of formula **6-C:** 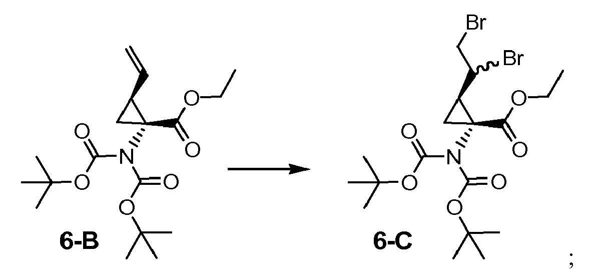
(c) transforming a compound of formula **6-C** to provide a compound of formula **6-D:** 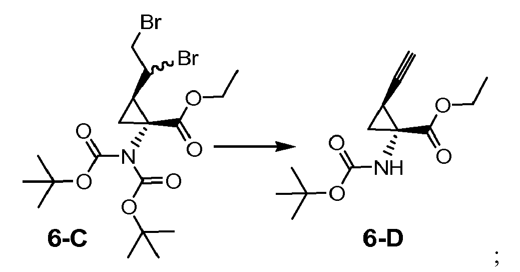
(d) protecting a compound of formula **6-D** to provide a compound of formula **6-E:** 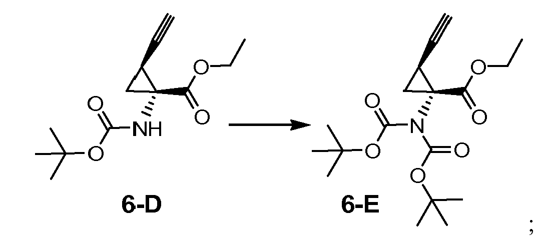 and
(e) brominating a compound of formula **6-E** to provide a compound of formula **4-BB:**

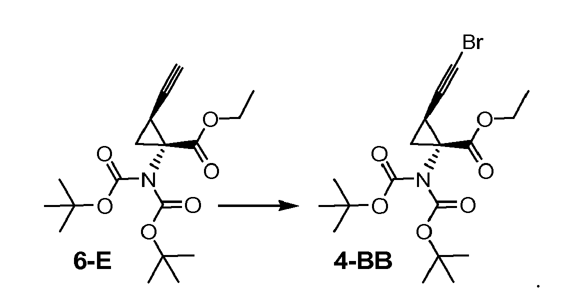

The present embodiments provide a compound selected from the group consisting of: 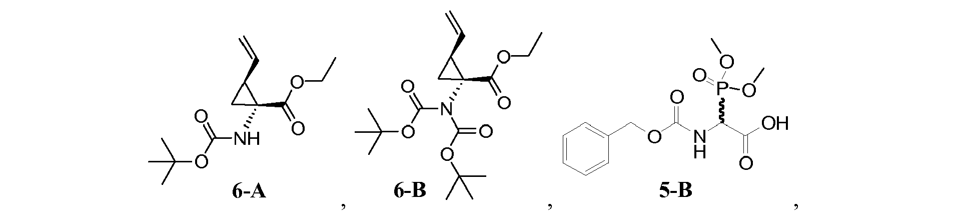 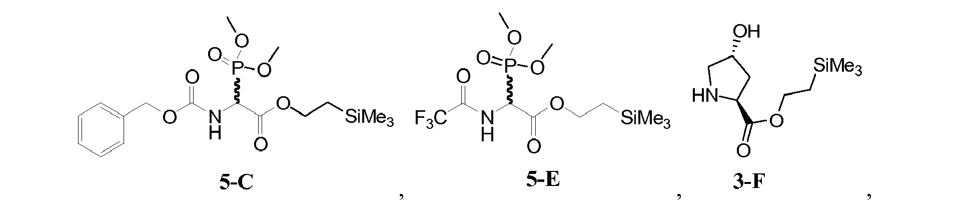 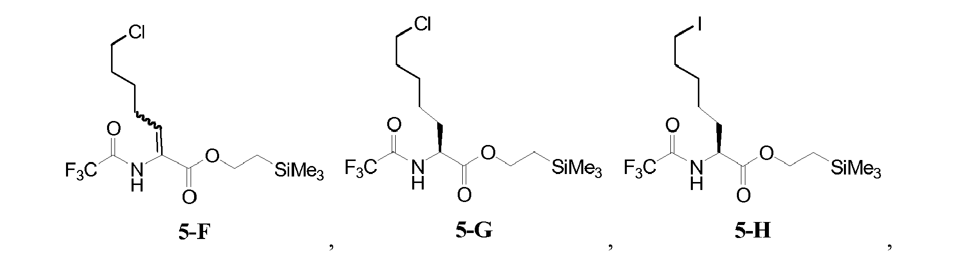 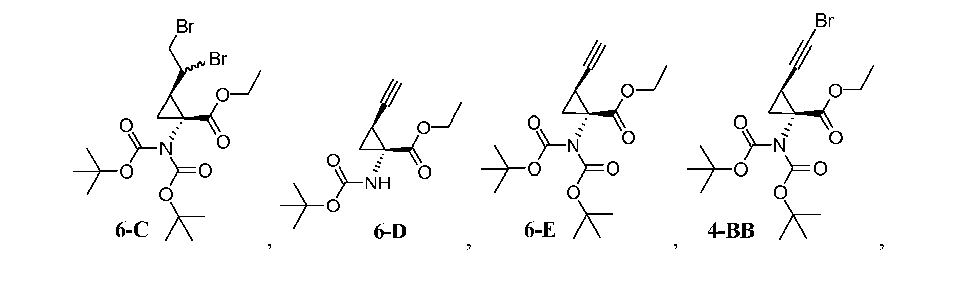 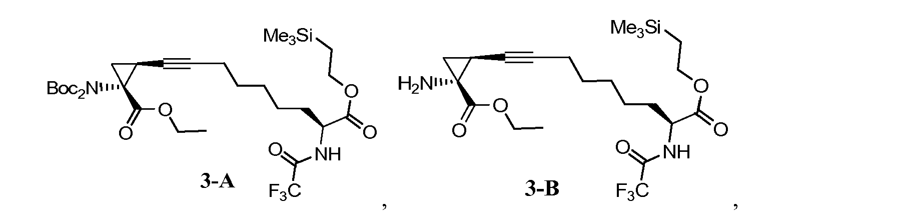 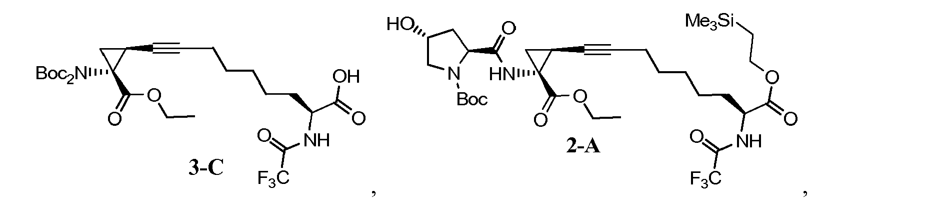 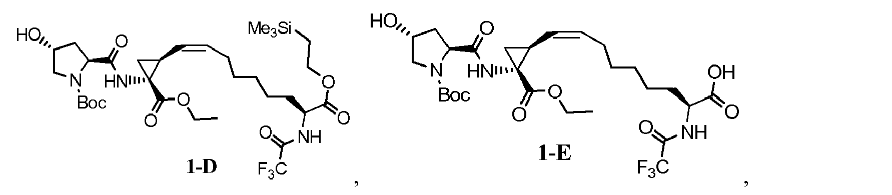 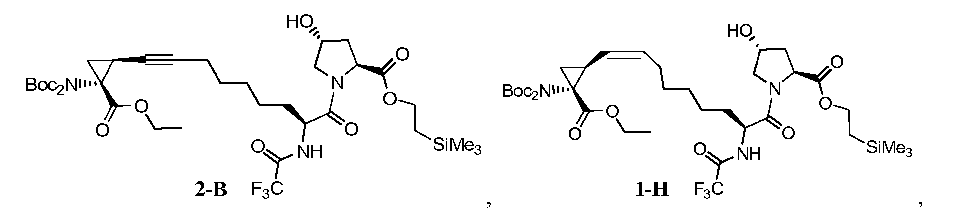 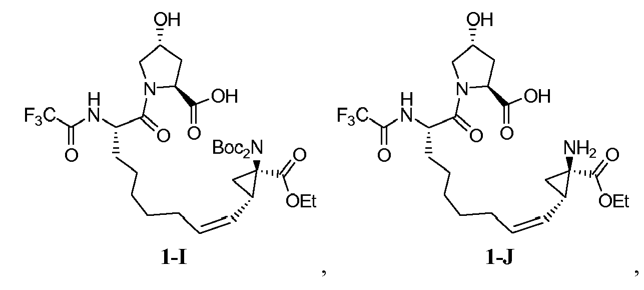 and

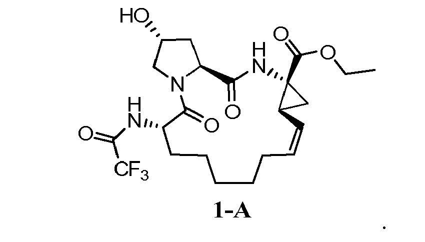

The present embodiments provide a method of administering an inhibitor of hepatitis C virus (HCV) infection, comprising administering to a patient an effective amount of a compound 100, or a pharmaceutically acceptable salt, ester or prodrug thereof, wherein the administering is undertaken in conjunction with the consumption of food by the patient:

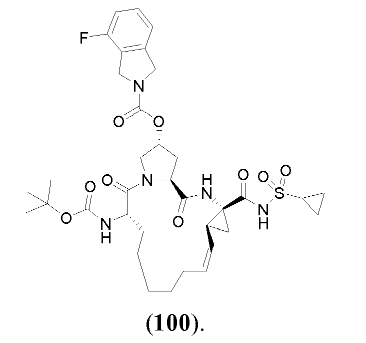

The present embodiments provide a method of administering an inhibitor of hepatitis C virus (HCV) infection comprising administering to a patient an effective amount of a compound 100, or a pharmaceutically acceptable salt, ester or prodrug thereof, and providing information to the patient, which information comprises that the administering of the compound 100, or the pharmaceutically acceptable salt, ester or prodrug thereof, should be accompanied by the consumption of food.

The present embodiments also provide a method of distributing an oral dosage form comprising distributing a pharmaceutical composition, wherein the pharmaceutical composition comprises a compound 100, or a pharmaceutically acceptable salt, ester or prodrug thereof, and concomitantly distributing information, which information comprises that the administering of the pharmaceutical composition should be accompanied by the consumption of food.

### BRIEF DESCRIPTION OF THE DRAWINGS

Figure 1 shows boxplots of the area under the concentration-time curve (AUC_{0-inf}), with individual estimates overlaid, and stratified by fed status.
Figure 2 is a plot of mean AUC_{0-inf} at 100, 200, 400, 800, 1600 mg under fasted condition and 400 and 1600 mg under fed condition.
Figure 3 is a plot of mean maximal drug concentration (Cₘₐₓ) at 100, 200, 400, 800, 1600 mg under fasted condition and 400 and 1600 mg under fed condition.

### DETAILED DESCRIPTION OF THE EMBODIMENTS

### Definitions

As used herein, common organic abbreviations are defined as follows:
- Ac: Acetyl
- Ac₂O: Acetic anhydride
- aq.: Aqueous
- Bn: Benzyl
- Bz: Benzoyl
- BOC or Boc: tert-Butoxycarbonyl
- Bu: n-Butyl
- cat.: Catalytic
- Cbz: Carbobenzyloxy
- CDI: 1,1'-carbonyldiimidazole
- Cy(c-C₆H₁₁: Cyclohexyl
- °C: Temperature in degrees Centigrade
- DBU: 1,8-Diazabicyclo[5.4.0]undec-7-ene
- DCE: 1,2-Dichloroethane
- DCM: methylene chloride
- DIEA: Diisopropylethylamine
- DMA: Dimethylacetamide
- DME: Dimethoxyethane
- DMF: N,N'-Dimethylformamide
- DMSO: Dimethylsulfoxide
- Et: Ethyl
- EtOAc: Ethyl acetate
- g: Gram(s)
- h: Hour (hours)
- HATU: 2-(1*H*-7-azabenzotriazol-1-yl)-1,1,3,3-tetramethyl uronium hexafluorophosphate
- HOBT: N-Hydroxybenzotriazole
- iPr: Isopropyl
- LCMS: Liquid chromatography-mass spectrometry
- LDA: Lithium diisopropylamide
- mCPBA: meta-Chloroperoxybenzoic Acid
- MeOH: Methanol
- MeCN: Acetonitrile
- mL: Milliliter(s)
- MTBE: Methyl tertiary-butyl ether
- NH₄OAc: Ammonium acetate
- PG: Protecting group
- Pd/C: Palladium on activated carbon
- ppt: Precipitate
- RCM: Ring closing metathesis
- rt: Room temperature
- sBuLi: sec-Butylithium
- TEA: Triethylamine
- TCDI: 1,1'-Thiocarbonyl diimidazole
- Tert, t: tertiary
- TFA: Trifluoracetic acid
- THF: Tetrahydrofuran
- TLC: Thin-layer chromatography
- TMEDA: Tetramethylethylenediamine
- µL: Microliter(s)

As used herein, the term "hepatic fibrosis," used interchangeably herein with "liver fibrosis," refers to the growth of scar tissue in the liver that can occur in the context of a chronic hepatitis infection.

The terms "individual," "host," "subject," and "patient" are used interchangeably herein, and refer to a mammal, including, but not limited to, primates, including simians and humans.

As used herein, the term "liver function" refers to a normal function of the liver, including, but not limited to, a synthetic function, including, but not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like.

The term "sustained viral response" (SVR; also referred to as a "sustained response" or a "durable response"), as used herein, refers to the response of an individual to a treatment regimen for HCV infection, in terms of serum HCV titer. Generally, a "sustained viral response" refers to no detectable HCV RNA (e.g., less than about 500, less than about 200, or less than about 100 genome copies per milliliter serum) found in the patient's serum for a period of at least about one month, at least about two months, at least about three months, at least about four months, at least about five months, or at least about six months following cessation of treatment.

"Treatment failure patients" as used herein generally refers to HCV-infected patients who failed to respond to previous therapy for HCV (referred to as "non-responders") or who initially responded to previous therapy, but in whom the therapeutic response was not maintained (referred to as "relapsers"). The previous therapy generally can include treatment with IFN-α monotherapy or IFN-α combination therapy, where the combination therapy may include administration of IFN-α and an antiviral agent such as ribavirin.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment," as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The terms "individual," "host," "subject," and "patient" are used interchangeably herein, and refer to a mammal, including, but not limited to, murines, simians, humans, mammalian farm animals, mammalian sport animals, and mammalian pets.

As used herein, the term "a Type I interferon receptor agonist" refers to any naturally occurring or non-naturally occurring ligand of human Type I interferon receptor, which binds to and causes signal transduction via the receptor. Type I interferon receptor agonists include interferons, including naturally-occurring interferons, modified interferons, synthetic interferons, pegylated interferons, fusion proteins comprising an interferon and a heterologous protein, shuffled interferons; antibody specific for an interferon receptor; non-peptide chemical agonists; and the like.

As used herein, the term "Type II interferon receptor agonist" refers to any naturally occurring or non-naturally occurring ligand of human Type II interferon receptor that binds to and causes signal transduction via the receptor. Type II interferon receptor agonists include native human interferon-γ, recombinant IFN-γ species, glycosylated IFN-γ species, pegylated IFN-γ species, modified or variant IFN-γ species, IFN-γ fusion proteins, antibody agonists specific for the receptor, non-peptide agonists, and the like.

As used herein, the term "a Type III interferon receptor agonist" refers to any naturally occurring or non-naturally occurring ligand of humanIL-28 receptor α ("IL-28R"), the amino acid sequence of which is described by Sheppard, et al., infra., that binds to and causes signal transduction via the receptor.

As used herein, the term "interferon receptor agonist" refers to any Type I interferon receptor agonist, Type II interferon receptor agonist, or Type III interferon receptor agonist.

The term "dosing event" as used herein refers to administration of an antiviral agent to a patient in need thereof, which event may encompass one or more releases of an antiviral agent from a drug dispensing device. Thus, the term "dosing event," as used herein, includes, but is not limited to, installation of a continuous delivery device (e.g., a pump or other controlled release injectible system); and a single subcutaneous injection followed by installation of a continuous delivery system.

"Continuous delivery" as used herein (e.g., in the context of "continuous delivery of a substance to a tissue") is meant to refer to movement of drug to a delivery site, e.g., into a tissue in a fashion that provides for delivery of a desired amount of substance into the tissue over a selected period of time, where about the same quantity of drug is received by the patient each minute during the selected period of time.

"Controlled release" as used herein (e.g., in the context of "controlled drug release") is meant to encompass release of substance (e.g., a Type I or Type III interferon receptor agonist, e.g., IFN-α) at a selected or otherwise controllable rate, interval, and/or amount, which is not substantially influenced by the environment of use. "Controlled release" thus encompasses, but is not necessarily limited to, substantially continuous delivery, and patterned delivery (e.g., intermittent delivery over a period of time that is interrupted by regular or irregular time intervals).

"Patterned" or "temporal" as used in the context of drug delivery is meant delivery of drug in a pattern, generally a substantially regular pattern, over a pre-selected period of time (e.g., other than a period associated with, for example a bolus injection). "Patterned" or "temporal" drug delivery is meant to encompass delivery of drug at an increasing, decreasing, substantially constant, or pulsatile, rate or range of rates (e.g., amount of drug per unit time, or volume of drug formulation for a unit time), and further encompasses delivery that is continuous or substantially continuous, or chronic.

The term "controlled drug delivery device" is meant to encompass any device wherein the release (e.g., rate, timing of release) of a drug or other desired substance contained therein is controlled by or determined by the device itself and not substantially influenced by the environment of use, or releasing at a rate that is reproducible within the environment of use.

By "substantially continuous" as used in, for example, the context of "substantially continuous infusion" or "substantially continuous delivery" is meant to refer to delivery of drug in a manner that is substantially uninterrupted for a pre-selected period of drug delivery, where the quantity of drug received by the patient during any 8 hour interval in the pre-selected period never falls to zero. Furthermore, "substantially continuous" drug delivery can also encompass delivery of drug at a substantially constant, pre-selected rate or range of rates (e.g., amount of drug per unit time, or volume of drug formulation for a unit time) that is substantially uninterrupted for a pre-selected period of drug delivery.

By "substantially steady state" as used in the context of a biological parameter that may vary as a function of time, it is meant that the biological parameter exhibits a substantially constant value over a time course, such that the area under the curve defined by the value of the biological parameter as a function of time for any 8 hour period during the time course (AUC8hr) is no more than about 20% above or about 20% below, and preferably no more than about 15% above or about 15% below, and more preferably no more than about 10% above or about 10% below, the average area under the curve of the biological parameter over an 8 hour period during the time course (AUC8hr average). The AUC8hr average is defined as the quotient (q) of the area under the curve of the biological parameter over the entirety of the time course (AUCtotal) divided by the number of 8 hour intervals in the time course (total/3days), i.e., q = (AUCtotal)/ (total/3days). For example, in the context of a serum concentration of a drug, the serum concentration of the drug is maintained at a substantially steady state during a time course when the area under the curve of serum concentration of the drug over time for any 8 hour period during the time course (AUC8hr) is no more than about 20% above or about 20% below the average area under the curve of serum concentration of the drug over an 8 hour period in the time course (AUC8hr average), i.e., the AUC8hr is no more than 20% above or 20% below the AUC8hr average for the serum concentration of the drug over the time course.

The term "alkyl" as used herein refers to a radical of a fully saturated hydrocarbon, including, but not limited to, methyl, ethyl, n-propyl, isopropyl, n-butyl, isobutyl, tert-butyl, n-hexyl, 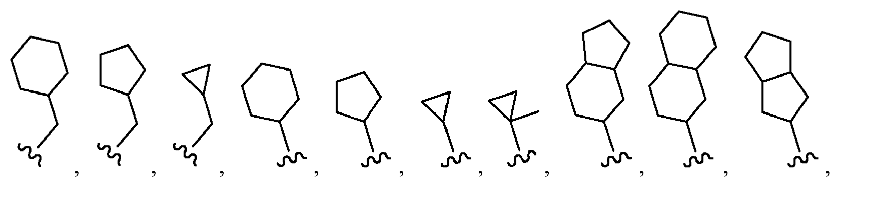 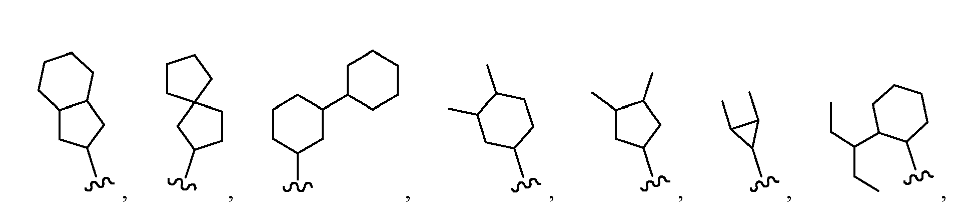 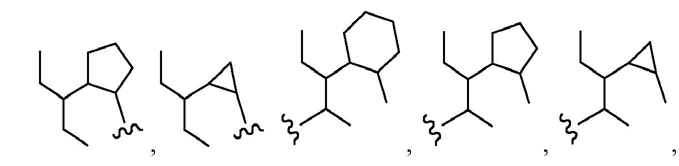 and the like. For example, the term "alkyl" as used herein includes radicals of fully saturated hydrocarbons defined by the following general formula's: the general formula for linear or branched fully saturated hydrocarbons not containing a cyclic structure is CₙH₂ₙ₊₂; the general formula for a fully saturated hydrocarbon containing one ring is CₙH₂ₙ; the general formula for a fully saturated hydrocarbon containing two rings is CₙH₂(ₙ₋₁); the general formula for a saturated hydrocarbon containing three rings is CₙH₂(ₙ₋₂).

The term "halo" used herein refers to fluoro, chloro, bromo, or iodo.

The term "alkoxy" used herein refers to straight or branched chain alkyl radical covalently bonded to the parent molecule through an --O-- linkage. Examples of alkoxy groups include, but are not limited to, methoxy, ethoxy, propoxy, isopropoxy, butoxy, n-butoxy, sec-butoxy, t-butoxy and the like.

The term "alkenyl" used herein refers to a monovalent straight or branched chain radical of from two to twenty carbon atoms containing a carbon double bond including, but not limited to, 1-propenyl, 2-propenyl, 2-methyl-1-propenyl, 1-butenyl, 2-butenyl, and the like.

The term "alkynyl" used herein refers to a monovalent straight or branched chain radical of from two to twenty carbon atoms containing a carbon triple bond including, but not limited to, 1-propynyl, 1-butynyl, 2-butynyl, and the like.

The term "aryl" used herein refers to homocyclic aromatic radical whether one ring or multiple fused rings. Examples of aryl groups include, but are not limited to, phenyl, naphthyl, biphenyl, phenanthrenyl, naphthacenyl, and the like.

The term "cycloalkyl" used herein refers to saturated aliphatic ring system radical having three to twenty carbon atoms including, but not limited to, cyclopropyl, cyclopentyl, cyclohexyl, cycloheptyl, and the like.

The term "cycloalkenyl" used herein refers to aliphatic ring system radical having three to twenty carbon atoms having at least one carbon-carbon double bond in the ring. Examples of cycloalkenyl groups include, but are not limited to, cyclopropenyl, cyclopentenyl, cyclohexenyl, cycloheptenyl, and the like.

The term "polycycloalkyl" used herein refers to saturated aliphatic ring system radical having at least two rings that are fused with or without bridgehead carbons. Examples of polycycloalkyl groups include, but are not limited to, bicyclo[4.4.0]decanyl, bicyclo[2.2.1]heptanyl, adamantyl, norbornyl, and the like.

The term "polycycloalkenyl" used herein refers to aliphatic ring system radical having at least two rings that are fused with or without bridgehead carbons in which at least one of the rings has a carbon-carbon double bond. Examples of polycycloalkenyl groups include, but are not limited to, norbornylenyl, 1,1'-bicyclopentenyl, and the like.

The term "polycyclic hydrocarbon" used herein refers to a ring system radical in which all of the ring members are carbon atoms. Polycyclic hydrocarbons can be aromatic or can contain less than the maximum number of non-cumulative double bonds. Examples of polycyclic hydrocarbon include, but are not limited to, naphthyl, dihydronaphthyl, indenyl, fluorenyl, and the like.

The term "heterocyclic" or "heterocyclyl" used herein refers to cyclic ring system radical having at least one non-aromatic ring in which one or more ring atoms are not carbon, namely heteroatom. Monocyclic "heterocyclic" or "heterocyclyl" moieties are non-aromatic. Bicyclic "heterocyclic" or "heterocyclyl" moieties include one non-aromatic ring wherein at least one heteroatom is present in the non-aromatic ring. Examples of heterocyclic groups include, but are not limited to, morpholinyl, tetrahydrofuranyl, dioxolanyl, pyrolidinyl, oxazolyl, pyranyl, pyrrolyl, isoindoline and the like.

The term "heteroaryl" used herein refers to an aromatic ring system radical in which one or more ring atoms are not carbon, namely heteroatom, whether one ring or multiple fused rings. In fused ring systems, the one or more heteroatoms may be present in only one of the rings. Examples of heteroaryl groups include, but are not limited to, benzothiazyl, benzoxazyl, quinazolinyl, quinolinyl, isoquinolinyl, quinoxalinyl, pyridinyl, pyrrolyl, oxazolyl, indolyl, and the like.

The term "heteroatom" used herein refers to, for example, oxygen, sulfur and nitrogen.

The term "arylalkyl" used herein refers to one or more aryl groups appended to an alkyl radical. Examples of arylalkyl groups include, but are not limited to, benzyl, phenethyl, phenpropyl, phenbutyl, and the like.

The term "cycloalkylalkyl" used herein refers to one or more cycloalkyl groups appended to an alkyl radical. Examples of cycloalkylalkyl include, but are not limited to, cyclohexylmethyl, cyclohexylethyl, cyclopentylmethyl, cyclopentylethyl, and the like.

The term "heteroarylalkyl" used herein refers to one or more heteroaryl groups appended to an alkyl radical. Examples of heteroarylalkyl include, but are not limited to, pyridylmethyl, furanylmethyl, thiopheneylethyl, and the like.

The term "heterocyclylalkyl" used herein refers to one or more heterocyclyl groups appended to an alkyl radical. Examples of heterocyclylalkyl include, but are not limited to, morpholinylmethyl, morpholinylethyl, morpholinylpropyl, tetrahydrofuranylmethyl, pyrrolidinylpropyl, and the like.

he term "aryloxy" used herein refers to an aryl radical covalently bonded to the parent molecule through an --O-- linkage.

The term "alkylthio" used herein refers to straight or branched chain alkyl radical covalently bonded to the parent molecule through an --S-- linkage. Examples of alkylthio groups include, but are not limited to, methanesulfide, ethanesulfide, propanesulfide, isopropanesulfide, butanesulfide, n-butanesulfide, sec-butanesulfide, *tert*-butanesulfide and the like.

The term "arylthio" used herein refers to an aryl radical covalently bonded to the parent molecule through an --S-- linkage.

The term "alkylamino" used herein refers to nitrogen radical with one or more alkyl groups attached thereto. Thus, monoalkylamino refers to nitrogen radical with one alkyl group attached thereto and dialkylamino refers to nitrogen radical with two alkyl groups attached thereto.

The term "cyanoamino" used herein refers to nitrogen radical with nitrile group attached thereto.

The term "carbamyl" used herein refers to RNHCOO--.

The term "keto" and "carbonyl" used herein refers to C=O.

The term "carboxy" used herein refers to -COOH.

The term "sulfamyl" used herein refers to -SO₂NH₂.

The term "sulfonyl" used herein refers to -SO₂-.

The term "sulfinyl" used herein refers to -SO-.

The term "thiocarbonyl" used herein refers to C=S.

The term "thiocarboxy" used herein refers to CSOH.

As used herein, a radical indicates species with a single, unpaired electron such that the species containing the radical can be covalently bonded to another species. Hence, in this context, a radical is not necessarily a free radical. Rather, a radical indicates a specific portion of a larger molecule. The term "radical" can be used interchangeably with the term "group."

As used herein, a substituted group is derived from the unsubstituted parent structure in which there has been an exchange of one or more hydrogen atoms for another atom or group. When substituted, the substituent group(s) is (are) one or more group(s) individually and independently selected from C₁-C₆ alkyl, C₁-C₆ alkenyl, C₁-C₆ alkynyl, C₃-C₆ cycloalkyl (optionally substituted with halo, alkyl, alkoxy, carboxyl, haloalkyl, CN, - SO₂-alkyl, -CF₃, and -OCF₃), C₃-C₆ heterocycloalkyl (e.g., tetrahydrofuryl) (optionally substituted with halo, alkyl, alkoxy, carboxyl, CN, -SO₂-alkyl, -CF₃, and -OCF₃), aryl (optionally substituted with halo, alkyl, alkoxy, carboxyl, CN, -SO₂-alkyl, -CF₃, and -OCF₃) heteroaryl (optionally substituted with halo, alkyl, alkoxy, carboxyl, CN, -SO₂-alkyl, -CF₃, and -OCF₃), halo (e.g., chloro, bromo, iodo and fluoro), cyano, hydroxy, C₁-C₆ alkoxy, aryloxy, sulfhydryl (mercapto), halo(C₁-C₆)alkyl, C₁-C₆ alkylthio, arylthio, mono- and di-(C₁-C₆)alkyl amino, quaternary ammonium salts, amino(C₁-C₆)alkoxy, hydroxy(C₁-C₆)alkylamino, amino(C₁-C₆)alkylthio, cyanoamino, nitro, carbamyl, keto (oxy), carbonyl, carboxy, glycolyl, glycyl, hydrazino, guanyl, sulfamyl, sulfonyl, sulfinyl, thiocarbonyl, thiocarboxy, and combinations thereof. The protecting groups that can form the protective derivatives of the above substituents are known to those of skill in the art and can be found in references such as Greene and Wuts Protective Groups in Organic Synthesis; John Wiley and Sons: New York, 1999. Wherever a substituent is described as "optionally substituted" that substituent can be substituted with the above substituents.

Asymmetric carbon atoms may be present in the compounds described. All such isomers, including diastereomers and enantiomers, as well as the mixtures thereof are intended to be included in the scope of the recited compound. In certain cases, compounds can exist in tautomeric forms. All tautomeric forms are intended to be included in the scope. Likewise, when compounds contain an alkenyl or alkenylene group, there exists the possibility of cis- and trans- isomeric forms of the compounds. Both cis- and trans- isomers, as well as the mixtures of cis- and trans- isomers, are contemplated. Thus, reference herein to a compound includes all of the aforementioned isomeric forms unless the context clearly dictates otherwise.

Various forms are included in the embodiments, including polymorphs, solvates, hydrates, conformers, salts, and prodrug derivatives. A polymorph is a composition having the same chemical formula, but a different structure. A solvate is a composition formed by solvation (the combination of solvent molecules with molecules or ions of the solute). A hydrate is a compound formed by an incorporation of water. A conformer is a structure that is a conformational isomer. Conformational isomerism is the phenomenon of molecules with the same structural formula but different conformations (conformers) of atoms about a rotating bond. Salts of compounds can be prepared by methods known to those skilled in the art. For example, salts of compounds can be prepared by reacting the appropriate base or acid with a stoichiometric equivalent of the compound. A prodrug is a compound that undergoes biotransformation (chemical conversion) before exhibiting its pharmacological effects. For example, a prodrug can thus be viewed as a drug containing specialized protective groups used in a transient manner to alter or to eliminate undesirable properties in the parent molecule. Thus, reference herein to a compound includes all of the aforementioned forms unless the context clearly dictates otherwise.

Where a range of values is provided, it is understood that each intervening value, to the tenth of the unit of the lower limit unless the context clearly dictates otherwise, between the upper and lower limit of that range and any other stated or intervening value in that stated range is encompassed within the embodiments. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the embodiments.

Unless defined otherwise, all technical and scientific terms used herein have the same meaning as commonly understood by one of ordinary skill in the art to which the embodiments belong. Although any methods and materials similar or equivalent to those described herein can also be used in the practice or testing of the embodiments, the preferred methods and materials are now described. All publications mentioned herein are incorporated herein by reference to disclose and describe the methods and/or materials in connection with which the publications are cited.

It must be noted that as used herein and in the appended claims, the singular forms "a," "and," and "the" include plural referents unless the context clearly dictates otherwise. Thus, for example, reference to "a method" includes a plurality of such methods and reference to "a dose" includes reference to one or more doses and equivalents thereof known to those skilled in the art, and so forth.

The present embodiments provide compounds of Formula I, as well as pharmaceutical compositions and formulations comprising any compound of Formula I. A subject compound is useful for treating HCV infection and other disorders, as discussed below. The embodiments provide a compound having the structure of Formula I: 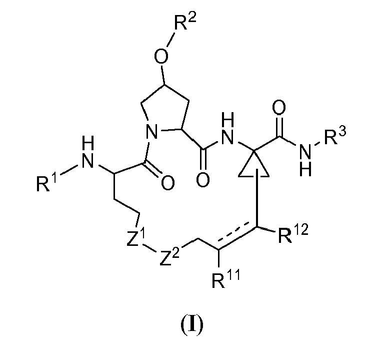 or a pharmaceutically acceptable salt or prodrug thereof wherein:
R¹ is selected from the group consisting of substituted aryl, substituted heteroaryl, - C(O)OR⁴, -C(O)NR⁵R⁶, -C(O)R⁷, and 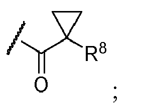
R² is selected from the group consisting of alkyl, -C(O)-alkyl, 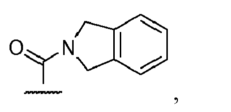 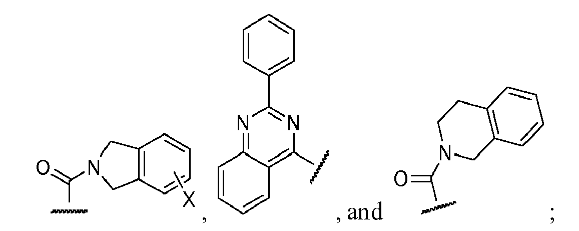
R³ is selected from the group consisting of -OR⁹ and -SO₂R¹⁰;
R⁴ is selected from the group consisting of alkyl, heterocyclyl, and aryl;
R⁵ is alkyl and R⁶ is selected from the group consisting of alkyl and aralkyl, or R⁵ together with R⁶ form an optionally substituted heterocyclyl or optionally substituted heteroaryl;
R⁷ is phenyl substituted one or more times with halogen;
R⁸ is selected from the group consisting of -CF₃ and methyl;
R⁹ is selected from the group consisting of hydrogen, optionally substituted alkyl, optionally substituted aryl, and optionally substituted aralkyl;
R¹⁰ is selected from the group consisting of alkyl optionally substituted with alkoxy or alkenyl, optionally substituted aryl, optionally substituted aralkyl, substituted heteroaryl, and 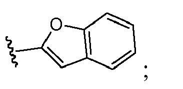
R¹¹ and R¹² are each hydrogen or together with the carbon atoms to which they are attached form an optionally substituted cycloalkyl;
X is halogen and is present 1 to 4 times; and
Z¹ and Z² are independently selected from the group consisting of -CH₂-, -CF₂-, and -O- provided that at least one of Z¹ and Z² is -CH₂-;
provided that if R¹¹ and R¹² are each hydrogen, R² is alkyl, and R³ is -SO₂-cyclopropyl, then R¹ is not -C(O)O-t-butyl;
provided that if R¹¹ and R¹² are each hydrogen and R² is 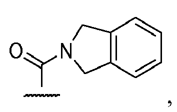 then R³ is -SO₂-phenyl disubstituted with halogen or -SO₂-thiophene disubstituted with halogen, or R¹ is 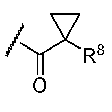 where R⁸ is -CF₃;
provided that if R¹¹ and R¹² are each hydrogen, R² is 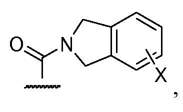 and R³ is - SO₂-cyclopropyl, then R¹ is not -C(O)O-t-butyl, -C(O)O-haloalkyl, or -C(O)O-cyclopentyl;
provided that if R¹¹ and R¹² are each hydrogen, R² is 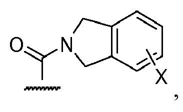 and R³ is substituted -SO₂-heteroaryl, then R¹ is not -C(O)O-cyclopentyl;
provided that if R¹¹ and R¹² are each hydrogen, R² is 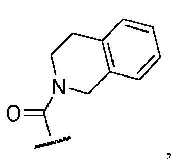 and R³ is - SO₂-cyclopropyl, then R¹ is not -C(O)O-alkyl or -C(O)O-heterocyclyl;
provided that if R¹¹ and R¹² are each hydrogen, R² is 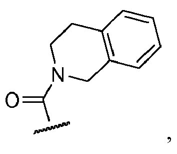 and R³ is - SO₂-alkyl or optionally substituted -SO₂-aryl, then R¹ is not -C(O)O-cycloalkyl;
provided that if R¹¹ and R¹² are each hydrogen, R² is 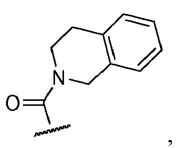 and R³ is optionally substituted -SO₂-phenyl, then R¹ is not -C(O)O-t-butyl;
provided that if R¹¹ and R¹² are each hydrogen, R² is 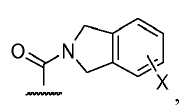 and R³ is - SO₂-alkyl substituted with alkoxy or alkenyl, then R¹ is -C(O)O-t-butyl and X is F; and
provided that the compound of formula (I) is not selected from the group consisting of: 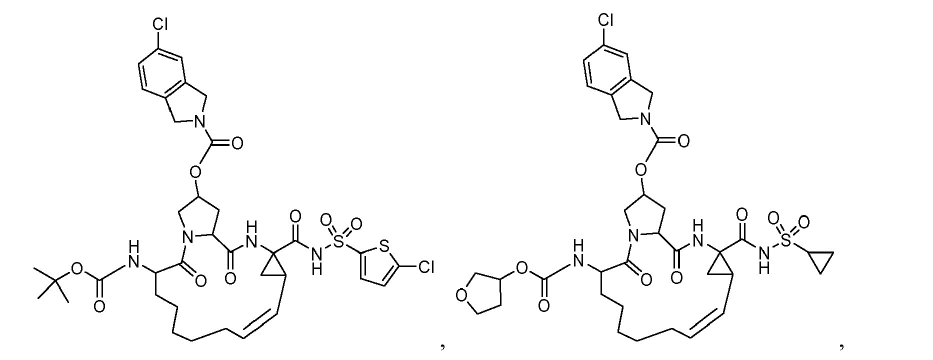 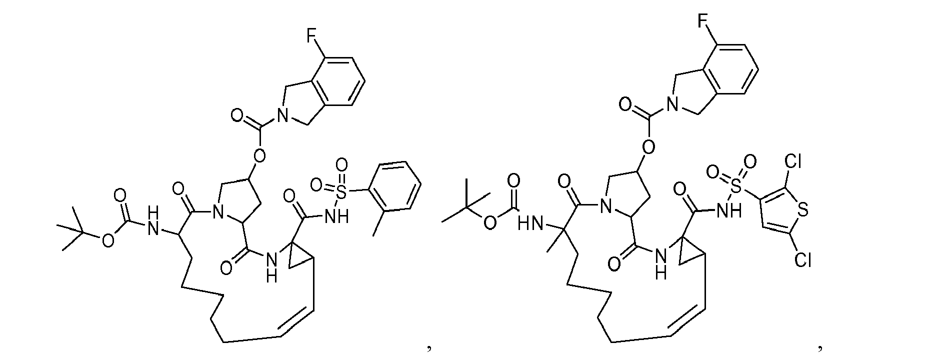 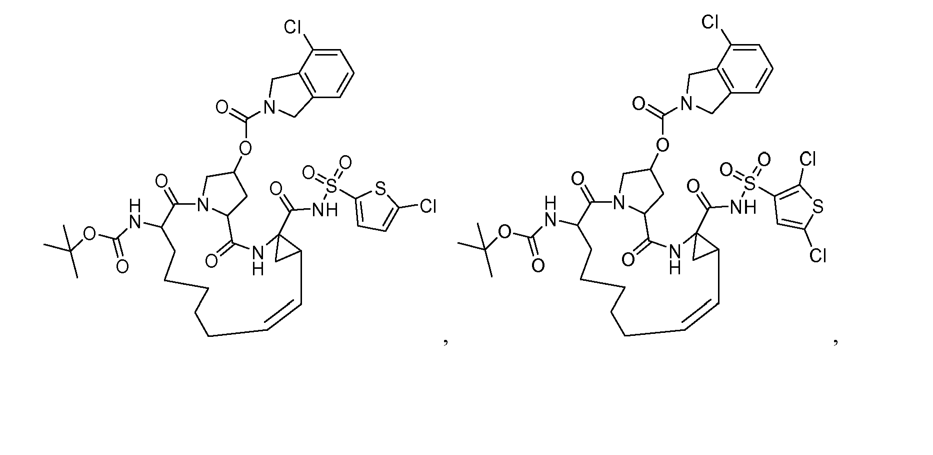 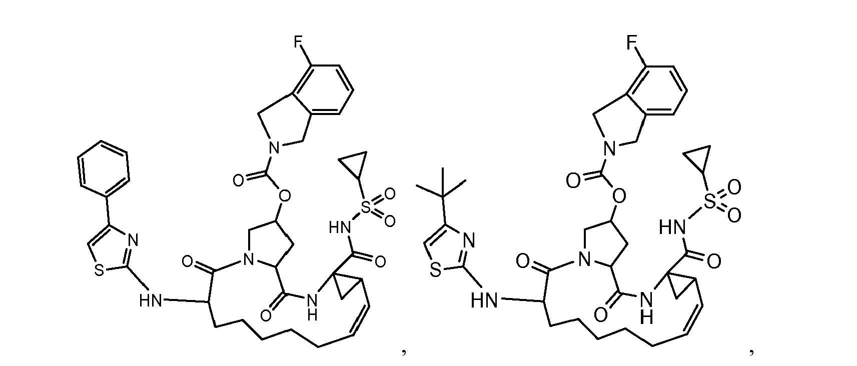 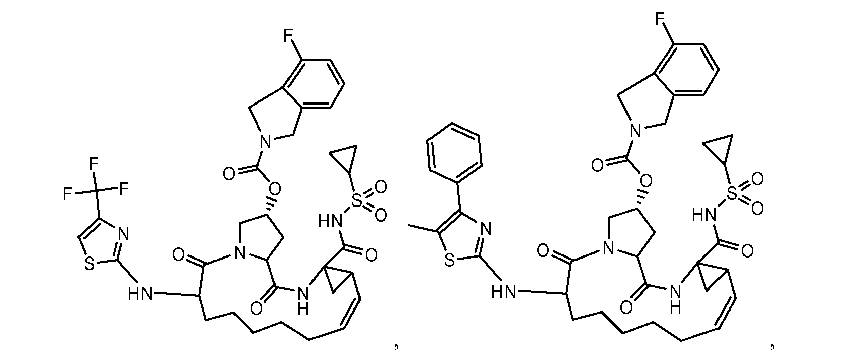 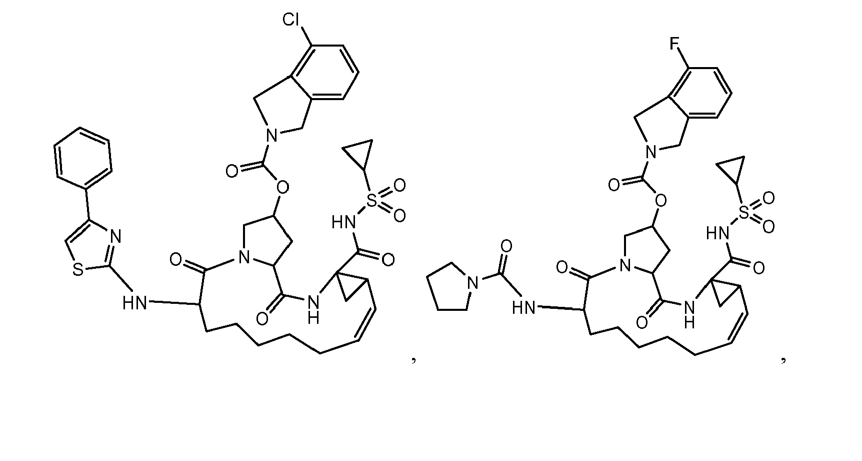 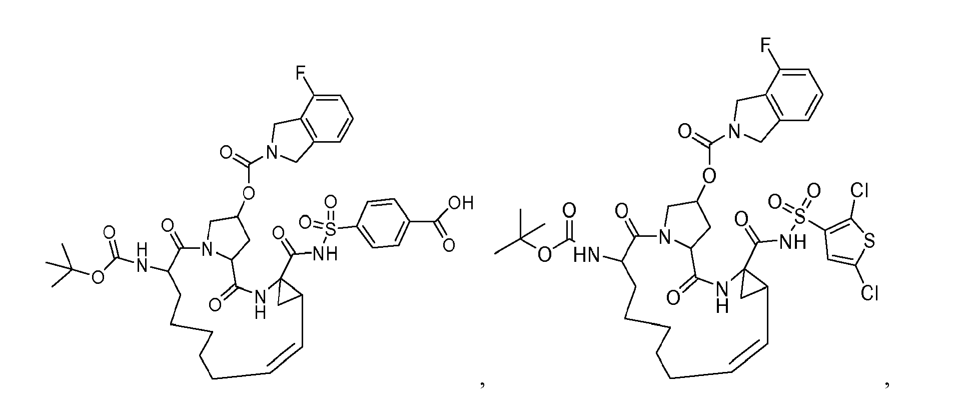 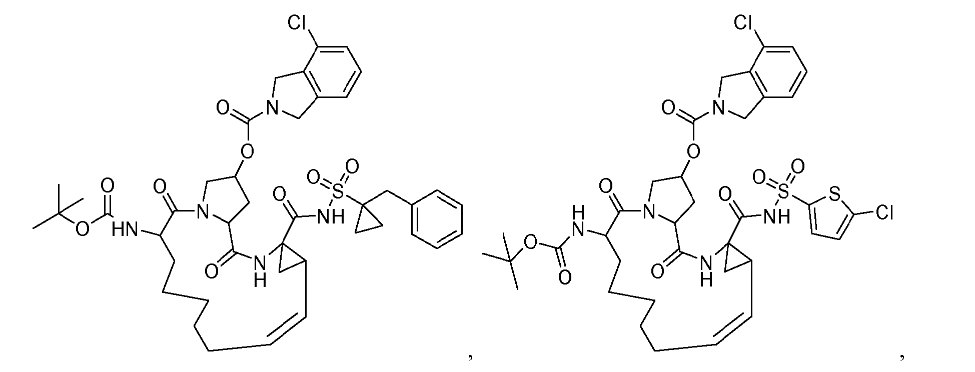 and

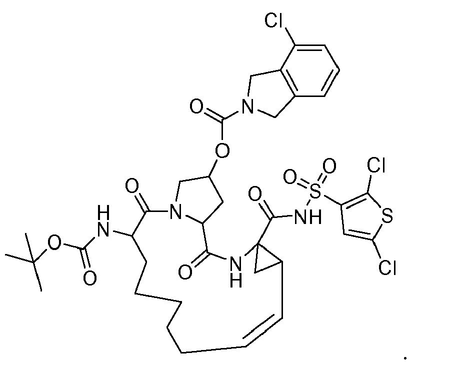

In preferred embodiments, embodiments provide compounds of Formula I, in which R¹ is -C(O)OR⁴.

In preferred embodiments, embodiments provide compounds of Formula I, in which R¹ is -C(O)OR⁴, wherein R⁴ is selected from the group consisting of alkyl, tetrahydrofuran, tetrahydropyran, and phenyl.

In preferred embodiments, embodiments provide compounds of Formula I, in which R¹ is -C(O)OR⁴, wherein R⁴ is tert-butyl.

In preferred embodiments, embodiments provide compounds of Formula I, in which R¹ has the structure: 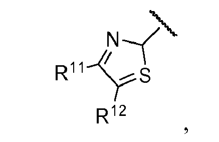 wherein R¹¹ and R¹² are independently selected from the group consisting of hydrogen, optionally substituted alkyl, optionally substituted aryl, and optionally substituted heteroaryl, or R¹¹ and R¹² together form a cycloalkyl, provided that at least one of R¹¹ and R¹² is not hydrogen. For example, R¹¹ and R¹² can be independently selected from the group consisting of hydrogen; alkyl; phenyl optionally substituted with one or more of halogen, - CN, -SO₂CH₃, -CF₃, and -OCF₃; pyridine optionally substituted with one or more halogen; and benzothiazole; or R¹¹ and R¹² can together form a cyclopentyl, provided that at least one of R¹¹ and R¹² is not hydrogen.

In preferred embodiments, embodiments provide compounds of Formula I, in which R¹ is -C(O)NR⁵R⁶. For example, in some embodiments, R⁵ can be methyl and R⁶ can be alkyl or benzyl. In some embodiments, R⁵ together with R⁶ can form an optionally substituted heterocyclyl or optionally substituted heteroaryl selected from the group consisting of N-morphlino, N-heterocyclyl optionally substituted with one or more halogen, and N-isoindolinyl.

In preferred embodiments, embodiments provide compounds of Formula I, in which R¹ is 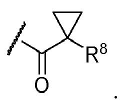 For example, in some embodiments, R⁸ can be -CF₃ or methyl.

In preferred embodiments, embodiments provide compounds of Formula I, in which R¹ is phenyl substituted with one or more halogen.

In preferred embodiments, embodiments provide compounds of Formula I, in which R¹ is -C(O)R⁷. For example,in some embodiments, R⁷ can be selected from the group consisting of phenyl substituted with one or more halogen.

In preferred embodiments, embodiments provide compounds of Formula I, in which R³ is -OR⁹. For example,in some embodiments, R⁹ can be selected from the group consisting of hydrogen, alkyl optionally substituted with hydroxy, phenyl, and benzyl optionally substituted with -CF₃.

In preferred embodiments, embodiments provide compounds of Formula I, in which R³ is -SO₂R¹⁰. For example, in some embodiments, R¹⁰ is selected from the group consisting of alkyl; phenyl optionally substituted with one or more of methyl, halogen, carboxy, CF₃, and alkoxy; and thiophene substituted with one or more of alkyl and halogen. In some embodiments, R¹⁰ can be cyclopropyl.

### Compositions

The present embodiments further provide compositions, including pharmaceutical compositions, comprising compounds of the general Formula I.

A subject pharmaceutical composition comprises a subject compound; and a pharmaceutically acceptable excipient. A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A.H. Kibbe et al., eds., 3rd ed. Amer. Pharmaceutical Assoc.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

The present embodiments provide for a method of inhibiting NS3/NS4 protease activity comprising contacting a NS3/NS4 protease with a compound disclosed herein.

The present embodiments provide for a method of treating hepatitis by modulating NS3/NS4 protease comprising contacting a NS3/NS4 protease with a compound disclosed herein.

Exemplary compounds of Formula I are set forth in Tables 1-7 and compounds therein below.

Preferred compounds of Formula I include Compound Numbers 101-907.

Preferred embodiments provide a method of treating a hepatitis C virus infection in an individual, the method comprising administering to the individual an effective amount of a composition comprising a preferred compound.

Preferred embodiments provide a method of treating liver fibrosis in an individual, the method comprising administering to the individual an effective amount of a composition comprising a preferred compound.

Preferred embodiments provide a method of increasing liver function in an individual having a hepatitis C virus infection, the method comprising administering to the individual an effective amount of a composition comprising a preferred compound.

In many embodiments, a subject compound inhibits the enzymatic activity of a hepatitis virus C (HCV) NS3 protease. Whether a subject compound inhibits HCV NS3 protease can be readily determined using any known method. Typical methods involve a determination of whether an HCV polyprotein or other polypeptide comprising an NS3 recognition site is cleaved by NS3 in the presence of the agent. In many embodiments, a subject compound inhibits NS3 enzymatic activity by at least about 10%, at least about 15%, at least about 20%, at least about 25%, at least about 30%, at least about 40%, at least about 50%, at least about 60%, at least about 70%, at least about 80%, or at least about 90%, or more, compared to the enzymatic activity of NS3 in the absence of the compound.

In many embodiments, a subject compound inhibits enzymatic activity of an HCV NS3 protease with an IC₅₀ of less than about 50 µM, e.g., a subject compound inhibits an HCV NS3 protease with an IC₅₀ of less than about 40 µM, less than about 25 µM, less than about 10 µM, less than about 1 µM, less than about 100 nM, less than about 80 nM, less than about 60 nM, less than about 50 nM, less than about 25 nM, less than about 10 nM, or less than about 1 nM, or less.

In many embodiments, a subject compound inhibits the enzymatic activity of a hepatitis virus C (HCV) NS3 helicase. Whether a subject compound inhibits HCV NS3 helicase can be readily determined using any known method. In many embodiments, a subject compound inhibits NS3 enzymatic activity by at least about 10%, at least about 15%, at least about 20%, at least about 25%, at least about 30%, at least about 40%, at least about 50%, at least about 60%, at least about 70%, at least about 80%, or at least about 90%, or more, compared to the enzymatic activity of NS3 in the absence of the compound.

In many embodiments, a subject compound inhibits HCV viral replication. For example, a subject compound inhibits HCV viral replication by at least about 10%, at least about 15%, at least about 20%, at least about 25%, at least about 30%, at least about 40%, at least about 50%, at least about 60%, at least about 70%, at least about 80%, or at least about 90%, or more, compared to HCV viral replication in the absence of the compound. Whether a subject compound inhibits HCV viral replication can be determined using methods known in the art, including an in vitro viral replication assay.

### Treating a hepatitis virus infection

The methods and compositions described herein are generally useful in treatment of an of HCV infection.

Whether a subject method is effective in treating an HCV infection can be determined by a reduction in viral load, a reduction in time to seroconversion (virus undetectable in patient serum), an increase in the rate of sustained viral response to therapy, a reduction of morbidity or mortality in clinical outcomes, or other indicator of disease response.

In general, an effective amount of a compound of Formula I, and optionally one or more additional antiviral agents, is an amount that is effective to reduce viral load or achieve a sustained viral response to therapy.

Whether a subject method is effective in treating an HCV infection can be determined by measuring viral load, or by measuring a parameter associated with HCV infection, including, but not limited to, liver fibrosis, elevations in serum transaminase levels, and necroinflammatory activity in the liver. Indicators of liver fibrosis are discussed in detail below.

The method involves administering an effective amount of a compound of Formula I, optionally in combination with an effective amount of one or more additional antiviral agents. In some embodiments, an effective amount of a compound of Formula I, and optionally one or more additional antiviral agents, is an amount that is effective to reduce viral titers to undetectable levels, e.g., to about 1000 to about 5000, to about 500 to about 1000, or to about 100 to about 500 genome copies/mL serum. In some embodiments, an effective amount of a compound of Formula I, and optionally one or more additional antiviral agents, is an amount that is effective to reduce viral load to lower than 100 genome copies/mL serum.

In some embodiments, an effective amount of a compound of Formula I, and optionally one or more additional antiviral agents, is an amount that is effective to achieve a 1.5-log, a 2-log, a 2.5-log, a 3-log, a 3.5-log, a 4-log, a 4.5-log, or a 5-log reduction in viral titer in the serum of the individual.

In many embodiments, an effective amount of a compound of Formula I, and optionally one or more additional antiviral agents, is an amount that is effective to achieve a sustained viral response, e.g., non-detectable or substantially non-detectable HCV RNA (e.g., less than about 500, less than about 400, less than about 200, or less than about 100 genome copies per milliliter serum) is found in the patient's serum for a period of at least about one month, at least about two months, at least about three months, at least about four months, at least about five months, or at least about six months following cessation of therapy.

As noted above, whether a subject method is effective in treating an HCV infection can be determined by measuring a parameter associated with HCV infection, such as liver fibrosis. Methods of determining the extent of liver fibrosis are discussed in detail below. In some embodiments, the level of a serum marker of liver fibrosis indicates the degree of liver fibrosis.

As one non-limiting example, levels of serum alanine aminotransferase (ALT) are measured, using standard assays. In general, an ALT level of less than about 45 international units is considered normal. In some embodiments, an effective amount of a compound of Formula I, and optionally one or more additional antiviral agents, is an amount effective to reduce ALT levels to less than about 45 IU/mL serum.

A therapeutically effective amount of a compound of Formula I, and optionally one or more additional antiviral agents, is an amount that is effective to reduce a serum level of a marker of liver fibrosis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or to a placebo-treated individual. Methods of measuring serum markers include immunological-based methods, e.g., enzyme-linked immunosorbent assays (ELISA), radioimmunoassays, and the like, using antibody specific for a given serum marker.

In many embodiments, an effective amount of a compound of Formula I and an additional antiviral agent is a synergistic amount. As used herein, a "synergistic combination" or a "synergistic amount" of a compound of Formula I and an additional antiviral agent is a combined dosage that is more effective in the therapeutic or prophylactic treatment of an HCV infection than the incremental improvement in treatment outcome that could be predicted or expected from a merely additive combination of (i) the therapeutic or prophylactic benefit of the compound of Formula I when administered at that same dosage as a monotherapy and (ii) the therapeutic or prophylactic benefit of the additional antiviral agent when administered at the same dosage as a monotherapy.

In some embodiments, a selected amount of a compound of Formula I and a selected amount of an additional antiviral agent are effective when used in combination therapy for a disease, but the selected amount of the compound of Formula I and/or the selected amount of the additional antiviral agent is ineffective when used in monotherapy for the disease. Thus, the embodiments encompass (1) regimens in which a selected amount of the additional antiviral agent enhances the therapeutic benefit of a selected amount of the compound of Formula I when used in combination therapy for a disease, where the selected amount of the additional antiviral agent provides no therapeutic benefit when used in monotherapy for the disease (2) regimens in which a selected amount of the compound of Formula I enhances the therapeutic benefit of a selected amount of the additional antiviral agent when used in combination therapy for a disease, where the selected amount of the compound of Formula I provides no therapeutic benefit when used in monotherapy for the disease and (3) regimens in which a selected amount of the compound of Formula I and a selected amount of the additional antiviral agent provide a therapeutic benefit when used in combination therapy for a disease, where each of the selected amounts of the compound of Formula I and the additional antiviral agent, respectively, provides no therapeutic benefit when used in monotherapy for the disease. As used herein, a "synergistically effective amount" of a compound of Formula I and an additional antiviral agent, and its grammatical equivalents, shall be understood to include any regimen encompassed by any of (1)-(3) above.

### Fibrosis

The embodiments provides methods for treating liver fibrosis (including forms of liver fibrosis resulting from, or associated with, HCV infection), generally involving administering a therapeutic amount of a compound of Formula I, and optionally one or more additional antiviral agents. Effective amounts of compounds of Formula I, with and without one or more additional antiviral agents, as well as dosing regimens, are as discussed below.

Whether treatment with a compound of Formula I, and optionally one or more additional antiviral agents, is effective in reducing liver fibrosis is determined by any of a number of well-established techniques for measuring liver fibrosis and liver function. Liver fibrosis reduction is determined by analyzing a liver biopsy sample. An analysis of a liver biopsy comprises assessments of two major components: necroinflammation assessed by "grade" as a measure of the severity and ongoing disease activity, and the lesions of fibrosis and parenchymal or vascular remodeling as assessed by "stage" as being reflective of long-term disease progression. See, e.g., Brunt (2000) Hepatol. 31:241-246; and METAVIR (1994) Hepatology 20:15-20. Based on analysis of the liver biopsy, a score is assigned. A number of standardized scoring systems exist which provide a quantitative assessment of the degree and severity of fibrosis. These include the METAVIR, Knodell, Scheuer, Ludwig, and Ishak scoring systems.

The METAVIR scoring system is based on an analysis of various features of a liver biopsy, including fibrosis (portal fibrosis, centrilobular fibrosis, and cirrhosis); necrosis (piecemeal and lobular necrosis, acidophilic retraction, and ballooning degeneration); inflammation (portal tract inflammation, portal lymphoid aggregates, and distribution of portal inflammation); bile duct changes; and the Knodell index (scores of periportal necrosis, lobular necrosis, portal inflammation, fibrosis, and overall disease activity). The definitions of each stage in the METAVIR system are as follows: score: 0, no fibrosis; score: 1, stellate enlargement of portal tract but without septa formation; score: 2, enlargement of portal tract with rare septa formation; score: 3, numerous septa without cirrhosis; and score: 4, cirrhosis.

Knodell's scoring system, also called the Hepatitis Activity Index, classifies specimens based on scores in four categories of histologic features: I. Periportal and/or bridging necrosis; II. Intralobular degeneration and focal necrosis; III. Portal inflammation; and IV. Fibrosis. In the Knodell staging system, scores are as follows: score: 0, no fibrosis; score: 1, mild fibrosis (fibrous portal expansion); score: 2, moderate fibrosis; score: 3, severe fibrosis (bridging fibrosis); and score: 4, cirrhosis. The higher the score, the more severe the liver tissue damage. Knodell (1981) Hepatol. 1:431.

In the Scheuer scoring system scores are as follows: score: 0, no fibrosis; score: 1, enlarged, fibrotic portal tracts; score: 2, periportal or portal-portal septa, but intact architecture; score: 3, fibrosis with architectural distortion, but no obvious cirrhosis; score: 4, probable or definite cirrhosis. Scheuer (1991) J. Hepatol. 13:372.

The Ishak scoring system is described in Ishak (1995) J. Hepatol. 22:696-699. Stage 0, No fibrosis; Stage 1, Fibrous expansion of some portal areas, with or without short fibrous septa; stage 2, Fibrous expansion of most portal areas, with or without short fibrous septa; stage 3, Fibrous expansion of most portal areas with occasional portal to portal (P-P) bridging; stage 4, Fibrous expansion of portal areas with marked bridging (P-P) as well as portal-central (P-C); stage 5, Marked bridging (P-P and/or P-C) with occasional nodules (incomplete cirrhosis); stage 6, Cirrhosis, probable or definite.

The benefit of anti-fibrotic therapy can also be measured and assessed by using the Child-Pugh scoring system which comprises a multicomponent point system based upon abnormalities in serum bilirubin level, serum albumin level, prothrombin time, the presence and severity of ascites, and the presence and severity of encephalopathy. Based upon the presence and severity of abnormality of these parameters, patients may be placed in one of three categories of increasing severity of clinical disease: A, B, or C.

In some embodiments, a therapeutically effective amount of a compound of Formula I, and optionally one or more additional antiviral agents, is an amount that effects a change of one unit or more in the fibrosis stage based on pre- and post-therapy liver biopsies. In particular embodiments, a therapeutically effective amount of a compound of Formula I, and optionally one or more additional antiviral agents, reduces liver fibrosis by at least one unit in the METAVIR, the Knodell, the Scheuer, the Ludwig, or the Ishak scoring system.

Secondary, or indirect, indices of liver function can also be used to evaluate the efficacy of treatment with a compound of Formula I. Morphometric computerized semi-automated assessment of the quantitative degree of liver fibrosis based upon specific staining of collagen and/or serum markers of liver fibrosis can also be measured as an indication of the efficacy of a subject treatment method. Secondary indices of liver function include, but are not limited to, serum transaminase levels, prothrombin time, bilirubin, platelet count, portal pressure, albumin level, and assessment of the Child-Pugh score.

An effective amount of a compound of Formula I, and optionally one or more additional antiviral agents, is an amount that is effective to increase an index of liver function by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the index of liver function in an untreated individual, or to a placebo-treated individual. Those skilled in the art can readily measure such indices of liver function, using standard assay methods, many of which are commercially available, and are used routinely in clinical settings.

Serum markers of liver fibrosis can also be measured as an indication of the efficacy of a subject treatment method. Serum markers of liver fibrosis include, but are not limited to, hyaluronate, N-terminal procollagen III peptide, 7S domain of type IV collagen, C-terminal procollagen I peptide, and laminin. Additional biochemical markers of liver fibrosis include α-2-macroglobulin, haptoglobin, gamma globulin, apolipoprotein A, and gamma glutamyl transpeptidase.

A therapeutically effective amount of a compound of Formula I, and optionally one or more additional antiviral agents, is an amount that is effective to reduce a serum level of a marker of liver fibrosis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or to a placebo-treated individual. Those skilled in the art can readily measure such serum markers of liver fibrosis, using standard assay methods, many of which are commercially available, and are used routinely in clinical settings. Methods of measuring serum markers include immunological-based methods, e.g., enzyme-linked immunosorbent assays (ELISA), radioimmunoassays, and the like, using antibody specific for a given serum marker.

Quantitative tests of functional liver reserve can also be used to assess the efficacy of treatment with an interferon receptor agonist and pirfenidone (or a pirfenidone analog). These include: indocyanine green clearance (ICG), galactose elimination capacity (GEC), aminopyrine breath test (ABT), antipyrine clearance, monoethylglycine-xylidide (MEG-X) clearance, and caffeine clearance.

As used herein, a "complication associated with cirrhosis of the liver" refers to a disorder that is a sequellae of decompensated liver disease, i.e., or occurs subsequently to and as a result of development of liver fibrosis, and includes, but it not limited to, development of ascites, variceal bleeding, portal hypertension, jaundice, progressive liver insufficiency, encephalopathy, hepatocellular carcinoma, liver failure requiring liver transplantation, and liver-related mortality.

A therapeutically effective amount of a compound of Formula I, and optionally one or more additional antiviral agents, is an amount that is effective in reducing the incidence (e.g., the likelihood that an individual will develop) of a disorder associated with cirrhosis of the liver by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to an untreated individual, or to a placebo-treated individual.

Whether treatment with a compound of Formula I, and optionally one or more additional antiviral agents, is effective in reducing the incidence of a disorder associated with cirrhosis of the liver can readily be determined by those skilled in the art.

Reduction in liver fibrosis increases liver function. Thus, the embodiments provide methods for increasing liver function, generally involving administering a therapeutically effective amount of a compound of Formula I, and optionally one or more additional antiviral agents. Liver functions include, but are not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like.

Whether a liver function is increased is readily ascertainable by those skilled in the art, using well-established tests of liver function. Thus, synthesis of markers of liver function such as albumin, alkaline phosphatase, alanine transaminase, aspartate transaminase, bilirubin, and the like, can be assessed by measuring the level of these markers in the serum, using standard immunological and enzymatic assays. Splanchnic circulation and portal hemodynamics can be measured by portal wedge pressure and/or resistance using standard methods. Metabolic functions can be measured by measuring the level of ammonia in the serum.

Whether serum proteins normally secreted by the liver are in the normal range can be determined by measuring the levels of such proteins, using standard immunological and enzymatic assays. Those skilled in the art know the normal ranges for such serum proteins. The following are non-limiting examples. The normal level of alanine transaminase is about 45 IU per milliliter of serum. The normal range of aspartate transaminase is from about 5 to about 40 units per liter of serum. Bilirubin is measured using standard assays. Normal bilirubin levels are usually less than about 1.2 mg/dL. Serum albumin levels are measured using standard assays. Normal levels of serum albumin are in the range of from about 35 to about 55 g/L. Prolongation of prothrombin time is measured using standard assays. Normal prothrombin time is less than about 4 seconds longer than control.

A therapeutically effective amount of a compound of Formula I, and optionally one or more additional antiviral agents, is one that is effective to increase liver function by at least about 10%, at least about 20%, at least about 30%, at least about 40%, at least about 50%, at least about 60%, at least about 70%, at least about 80%, or more. For example, a therapeutically effective amount of a compound of Formula I, and optionally one or more additional antiviral agents, is an amount effective to reduce an elevated level of a serum marker of liver function by at least about 10%, at least about 20%, at least about 30%, at least about 40%, at least about 50%, at least about 60%, at least about 70%, at least about 80%, or more, or to reduce the level of the serum marker of liver function to within a normal range. A therapeutically effective amount of a compound of Formula I, and optionally one or more additional antiviral agents, is also an amount effective to increase a reduced level of a serum marker of liver function by at least about 10%, at least about 20%, at least about 30%, at least about 40%, at least about 50%, at least about 60%, at least about 70%, at least about 80%, or more, or to increase the level of the serum marker of liver function to within a normal range.

### Dosages, Formulations, and Routes of Administration

In the subject methods, the active agent(s) (e.g., compound of Formula I, and optionally one or more additional antiviral agents) may be administered to the host using any convenient means capable of resulting in the desired therapeutic effect. Thus, the agent can be incorporated into a variety of formulations for therapeutic administration. More particularly, the agents of the embodiments can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols.

### Formulations

The above-discussed active agent(s) can be formulated using well-known reagents and methods. Compositions are provided in formulation with a pharmaceutically acceptable excipient(s). A wide variety of pharmaceutically acceptable excipients is known in the art and need not be discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H.C. Ansel et al., eds., 7th ed., Lippincott, Williams, & Wilkins; and Handbook of Pharmaceutical Excipients (2000) A.H. Kibbe et al., eds., 3rd ed. Amer. Pharmaceutical Assoc.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

In some embodiments, an agent is formulated in an aqueous buffer. Suitable aqueous buffers include, but are not limited to, acetate, succinate, citrate, and phosphate buffers varying in strengths from about 5mM to about 100mM. In some embodiments, the aqueous buffer includes reagents that provide for an isotonic solution. Such reagents include, but are not limited to, sodium chloride; and sugars e.g., mannitol, dextrose, sucrose, and the like. In some embodiments, the aqueous buffer further includes a non-ionic surfactant such as polysorbate 20 or 80. Optionally the formulations may further include a preservative. Suitable preservatives include, but are not limited to, a benzyl alcohol, phenol, chlorobutanol, benzalkonium chloride, and the like. In many cases, the formulation is stored at about 4°C. Formulations may also be lyophilized, in which case they generally include cryoprotectants such as sucrose, trehalose, lactose, maltose, mannitol, and the like. Lyophilized formulations can be stored over extended periods of time, even at ambient temperatures.

As such, administration of the agents can be achieved in various ways, including oral, buccal, rectal, parenteral, intraperitoneal, intradermal, subcutaneous, intramuscular, transdermal, intratracheal, etc., administration. In many embodiments, administration is by bolus injection, e.g., subcutaneous bolus injection, intramuscular bolus injection, and the like.

The pharmaceutical compositions of the embodiments can be administered orally, parenterally or via an implanted reservoir. Oral administration or administration by injection is preferred.

Subcutaneous administration of a pharmaceutical composition of the embodiments is accomplished using standard methods and devices, e.g., needle and syringe, a subcutaneous injection port delivery system, and the like. See, e.g., U.S. Patent Nos. 3,547,119; 4,755,173; 4,531,937; 4,311,137; and 6,017,328. A combination of a subcutaneous injection port and a device for administration of a pharmaceutical composition of the embodiments to a patient through the port is referred to herein as "a subcutaneous injection port delivery system." In many embodiments, subcutaneous administration is achieved by bolus delivery by needle and syringe.

In pharmaceutical dosage forms, the agents may be administered in the form of their pharmaceutically acceptable salts, or they may also be used alone or in appropriate association, as well as in combination, with other pharmaceutically active compounds. The following methods and excipients are merely exemplary and are in no way limiting.

For oral preparations, the agents can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

The agents can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Furthermore, the agents can be made into suppositories by mixing with a variety of bases such as emulsifying bases or water-soluble bases. The compounds of the embodiments can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more inhibitors. Similarly, unit dosage forms for injection or intravenous administration may comprise the inhibitor(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of compounds of the embodiments calculated in an amount sufficient to produce the desired effect in association with a pharmaceutically acceptable diluent, carrier or vehicle. The specifications for the novel unit dosage forms of the embodiments depend on the particular compound employed and the effect to be achieved, and the pharmacodynamics associated with each compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

### Other antiviral or antifibrotic agents

As discussed above, a subject method will in some embodiments be carried out by administering an NS3 inhibitor that is a compound of Formula I, and optionally one or more additional antiviral agent(s).

In some embodiments, the method further includes administration of one or more interferon receptor agonist(s). Interferon receptor agonists are described herein.

In other embodiments, the method further includes administration of pirfenidone or a pirfenidone analog. Pirfenidone and pirfenidone analogs are described herein.

Additional antiviral agents that are suitable for use in combination therapy include, but are not limited to, nucleotide and nucleoside analogs. Non-limiting examples include azidothymidine (AZT) (zidovudine), and analogs and derivatives thereof; 2',3'-dideoxyinosine (DDI) (didanosine), and analogs and derivatives thereof; 2',3'-dideoxycytidine (DDC) (dideoxycytidine), and analogs and derivatives thereof; 2',3'-didehydro-2',3'-dideoxythymidine (D4T) (stavudine), and analogs and derivatives thereof; combivir; abacavir; adefovir dipoxil; cidofovir; ribavirin; ribavirin analogs; and the like.

In some embodiments, the method further includes administration of ribavirin. Ribavirin, 1-β-D-ribofuranosyl-1*H*-1,2,4-triazole-3-carboxamide, available from ICN Pharmaceuticals, Inc., Costa Mesa, Calif., is described in the Merck Index, compound No. 8199, Eleventh Edition. Its manufacture and formulation is described in U.S. Pat. No. 4,211,771. Some embodiments also involve use of derivatives of ribavirin (see, e.g., U.S. Pat. No. 6,277,830). The ribavirin may be administered orally in capsule or tablet form, or in the same or different administration form and in the same or different route as the NS-3 inhibitor compound. Of course, other types of administration of both medicaments, as they become available are contemplated, such as by nasal spray, transdermally, intravenously, by suppository, by sustained release dosage form, etc. Any form of administration will work so long as the proper dosages are delivered without destroying the active ingredient.

In some embodiments, the method further includes administration of ritonavir. Ritonavir, 10-hydroxy-2-methyl-5-(1-methylethyl)-1-[2-(1-methylethyl)-4-thiazolyl]-3,6-dioxo-8,11-bis(phenylmethyl)-2,4,7,12-tetraazatridecan-13-oic acid, 5-thiazolylmethyl ester [5*S*-(5*R**,8*R**,10*R**,11*R**)], available from Abbott Laboratories, is an inhibitor of the protease of the human immunodeficiency virus and also of the cytochrome P450 3A and P450 2D6 liver enzymes frequently involved in hepatic metabolism of therapeutic molecules in man. Because of its strong inhibitory effect on cytochrome P450 3A and the inhibitory effect on cytochrome P450 2D6, ritonavir at doses below the normal therapeutic dosage may be combined with other protease inhibitors to achieve therapeutic levels of the second protease inhibitor while reducing the number of dosage units required, the dosing frequency, or both.

Coadministration of low-dose ritonavir may also be used to compensate for drug interactions that tend to decrease levels of a protease inhibitor metabolized by CYP3A. Its structure, synthesis, manufacture and formulation are described in U.S. Pat. No. 5,541,206 U.S. Pat. No. 5,635,523 U.S. Pat. No. 5,648,497 U.S. Pat. No. 5,846,987 and U.S. Pat. No. 6,232,333. The ritonavir may be administered orally in capsule or tablet or oral solution form, or in the same or different administration form and in the same or different route as the NS-3 inhibitor compound. Of course, other types of administration of both medicaments, as they become available are contemplated, such as by nasal spray, transdermally, intravenously, by suppository, by sustained release dosage form, etc. Any form of administration will work so long as the proper dosages are delivered without destroying the active ingredient.

In some embodiments, an additional antiviral agent is administered during the entire course of NS3 inhibitor compound treatment. In other embodiments, an additional antiviral agent is administered for a period of time that is overlapping with that of the NS3 inhibitor compound treatment, e.g., the additional antiviral agent treatment can begin before the NS3 inhibitor compound treatment begins and end before the NS3 inhibitor compound treatment ends; the additional antiviral agent treatment can begin after the NS3 inhibitor compound treatment begins and end after the NS3 inhibitor compound treatment ends; the additional antiviral agent treatment can begin after the NS3 inhibitor compound treatment begins and end before the NS3 inhibitor compound treatment ends; or the additional antiviral agent treatment can begin before the NS3 inhibitor compound treatment begins and end after the NS3 inhibitor compound treatment ends.

### Methods of Treatment

### Monotherapies

The NS3 inhibitor compounds described herein may be used in acute or chronic therapy for HCV disease. In many embodiments, the NS3 inhibitor compound is administered for a period of about 1 day to about 7 days, or about 1 week to about 2 weeks, or about 2 weeks to about 3 weeks, or about 3 weeks to about 4 weeks, or about 1 month to about 2 months, or about 3 months to about 4 months, or about 4 months to about 6 months, or about 6 months to about 8 months, or about 8 months to about 12 months, or at least one year, and may be administered over longer periods of time. The NS3 inhibitor compound can be administered 5 times per day, 4 times per day, tid, bid, qd, qod, biw, tiw, qw, qow, three times per month, or once monthly. In other embodiments, the NS3 inhibitor compound is administered as a continuous infusion.

In many embodiments, an NS3 inhibitor compound of the embodiments is administered orally.

In connection with the above-described methods for the treatment of HCV disease in a patient, an NS3 inhibitor compound as described herein may be administered to the patient at a dosage from about 0.01 mg to about 100 mg/kg patient bodyweight per day, in 1 to 5 divided doses per day. In some embodiments, the NS3 inhibitor compound is administered at a dosage of about 0.5 mg to about 75 mg/kg patient bodyweight per day, in 1 to 5 divided doses per day.

The amount of active ingredient that may be combined with carrier materials to produce a dosage form can vary depending on the host to be treated and the particular mode of administration. A typical pharmaceutical preparation can contain from about 5% to about 95% active ingredient (w/w). In other embodiments, the pharmaceutical preparation can contain from about 20% to about 80% active ingredient.

Those of skill will readily appreciate that dose levels can vary as a function of the specific NS3 inhibitor compound, the severity of the symptoms and the susceptibility of the subject to side effects. Preferred dosages for a given NS3 inhibitor compound are readily determinable by those of skill in the art by a variety of means. A preferred means is to measure the physiological potency of a given interferon receptor agonist.

In many embodiments, multiple doses of NS3 inhibitor compound are administered. For example, an NS3 inhibitor compound is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid), over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

### Combination therapies with ribavirin

In some embodiments, the methods provide for combination therapy comprising administering an NS3 inhibitor compound as described above, and an effective amount of ribavirin. Ribavirin can be administered in dosages of about 400 mg, about 800 mg, about 1000 mg, or about 1200 mg per day.

One embodiment provides any of the above-described methods modified to include co-administering to the patient a therapeutically effective amount of ribavirin for the duration of the desired course of NS3 inhibitor compound treatment.

Another embodiment provides any of the above-described methods modified to include co-administering to the patient about 800 mg to about 1200 mg ribavirin orally per day for the duration of the desired course of NS3 inhibitor compound treatment. In another embodiment, any of the above-described methods may be modified to include co-administering to the patient (a) 1000 mg ribavirin orally per day if the patient has a body weight less than 75 kg or (b) 1200 mg ribavirin orally per day if the patient has a body weight greater than or equal to 75 kg, where the daily dosage of ribavirin is optionally divided into to 2 doses for the duration of the desired course of NS3 inhibitor compound treatment.

### Combination therapies with levovirin

In some embodiments, the methods provide for combination therapy comprising administering an NS3 inhibitor compound as described above, and an effective amount of levovirin. Levovirin is generally administered in an amount ranging from about 30 mg to about 60 mg, from about 60 mg to about 125 mg, from about 125 mg to about 200 mg, from about 200 mg to about 300 gm, from about 300 mg to about 400 mg, from about 400 mg to about 1200 mg, from about 600 mg to about 1000 mg, or from about 700 to about 900 mg per day, or about 10 mg/kg body weight per day. In some embodiments, levovirin is administered orally in dosages of about 400, about 800, about 1000, or about 1200 mg per day for the desired course of NS3 inhibitor compound treatment.

### Combination therapies with viramidine

In some embodiments, the methods provide for combination therapy comprising administering an NS3 inhibitor compound as described above, and an effective amount of viramidine. Viramidine is generally administered in an amount ranging from about 30 mg to about 60 mg, from about 60 mg to about 125 mg, from about 125 mg to about 200 mg, from about 200 mg to about 300 gm, from about 300 mg to about 400 mg, from about 400 mg to about 1200 mg, from about 600 mg to about 1000 mg, or from about 700 to about 900 mg per day, or about 10 mg/kg body weight per day. In some embodiments, viramidine is administered orally in dosages of about 800, or about 1600 mg per day for the desired course of NS3 inhibitor compound treatment.

### Combination therapies with ritonavir

In some embodiments, the methods provide for combination therapy comprising administering an NS3 inhibitor compound as described above, and an effective amount of ritonavir. Ritonavir is generally administered in an amount ranging from about 50 mg to about 100 mg, from about 100 mg to about 200 mg, from about 200 mg to about 300 mg, from about 300 mg to about 400 mg, from about 400 mg to about 500 mg, or from about 500 mg to about 600 mg, twice per day. In some embodiments, ritonavir is administered orally in dosages of about 300 mg, or about 400 mg, or about 600 mg twice per day for the desired course of NS3 inhibitor compound treatment.

### Combination therapies with alpha-glucosidase inhibitors

Suitable α-glucosidase inhibitors include any of the above-described imino-sugars, including long-alkyl chain derivatives of imino sugars as disclosed in U.S. Patent Publication No. 2004/0110795; inhibitors of endoplasmic reticulum-associated α-glucosidases; inhibitors of membrane bound α-glucosidase; miglitol (Glyset®), and active derivatives, and analogs thereof; and acarbose (Precose®), and active derivatives, and analogs thereof.

In many embodiments, the methods provide for combination therapy comprising administering an NS3 inhibitor compound as described above, and an effective amount of an α-glucosidase inhibitor administered for a period of about 1 day to about 7 days, or about 1 week to about 2 weeks, or about 2 weeks to about 3 weeks, or about 3 weeks to about 4 weeks, or about 1 month to about 2 months, or about 3 months to about 4 months, or about 4 months to about 6 months, or about 6 months to about 8 months, or about 8 months to about 12 months, or at least one year, and may be administered over longer periods of time.

An α-glucosidase inhibitor can be administered 5 times per day, 4 times per day, tid (three times daily), bid, qd, qod, biw, tiw, qw, qow, three times per month, or once monthly. In other embodiments, an α-glucosidase inhibitor is administered as a continuous infusion.

In many embodiments, an α-glucosidase inhibitor is administered orally.

In connection with the above-described methods for the treatment of a flavivirus infection, treatment of HCV infection, and treatment of liver fibrosis that occurs as a result of an HCV infection, the methods provide for combination therapy comprising administering an NS3 inhibitor compound as described above, and an effective amount of α-glucosidase inhibitor administered to the patient at a dosage of from about 10 mg per day to about 600 mg per day in divided doses, e.g., from about 10 mg per day to about 30 mg per day, from about 30 mg per day to about 60 mg per day, from about 60 mg per day to about 75 mg per day, from about 75 mg per day to about 90 mg per day, from about 90 mg per day to about 120 mg per day, from about 120 mg per day to about 150 mg per day, from about 150 mg per day to about 180 mg per day, from about 180 mg per day to about 210 mg per day, from about 210 mg per day to about 240 mg per day, from about 240 mg per day to about 270 mg per day, from about 270 mg per day to about 300 mg per day, from about 300 mg per day to about 360 mg per day, from about 360 mg per day to about 420 mg per day, from about 420 mg per day to about 480 mg per day, or from about 480 mg to about 600 mg per day.

In some embodiments, the methods provide for combination therapy comprising administering an NS3 inhibitor compound as described above, and an effective amount of α-glucosidase inhibitor administered in a dosage of about 10 mg three times daily. In some embodiments, an α-glucosidase inhibitor is administered in a dosage of about 15 mg three times daily. In some embodiments, an α-glucosidase inhibitor is administered in a dosage of about 20 mg three times daily. In some embodiments, an α-glucosidase inhibitor is administered in a dosage of about 25 mg three times daily. In some embodiments, an α-glucosidase inhibitor is administered in a dosage of about 30 mg three times daily. In some embodiments, an α-glucosidase inhibitor is administered in a dosage of about 40 mg three times daily. In some embodiments, an α-glucosidase inhibitor is administered in a dosage of about 50 mg three times daily. In some embodiments, an α-glucosidase inhibitor is administered in a dosage of about 100 mg three times daily. In some embodiments, an α-glucosidase inhibitor is administered in a dosage of about 75 mg per day to about 150 mg per day in two or three divided doses, where the individual weighs 60 kg or less. In some embodiments, an α-glucosidase inhibitor is administered in a dosage of about 75 mg per day to about 300 mg per day in two or three divided doses, where the individual weighs 60 kg or more.

The amount of active ingredient (e.g., α-glucosidase inhibitor) that may be combined with carrier materials to produce a dosage form can vary depending on the host to be treated and the particular mode of administration. A typical pharmaceutical preparation can contain from about 5% to about 95% active ingredient (w/w). In other embodiments, the pharmaceutical preparation can contain from about 20% to about 80% active ingredient.

Those of skill will readily appreciate that dose levels can vary as a function of the specific α-glucosidase inhibitor, the severity of the symptoms and the susceptibility of the subject to side effects. Preferred dosages for a given α-glucosidase inhibitor are readily determinable by those of skill in the art by a variety of means. A typical means is to measure the physiological potency of a given active agent.

In many embodiments, multiple doses of an α-glucosidase inhibitor are administered. For example, the methods provide for combination therapy comprising administering an NS3 inhibitor compound as described above, and an effective amount of α-glucosidase inhibitor administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid), over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

### Combination therapies with thymosin-α

In some embodiments, the methods provide for combination therapy comprising administering an NS3 inhibitor compound as described above, and an effective amount of thymosin-α. Thymosin-α (Zadaxin™) is generally administered by subcutaneous injection. Thymosin-α can be administered tid, bid, qd, qod, biw, tiw, qw, qow, three times per month, once monthly, substantially continuously, or continuously for the desired course of NS3 inhibitor compound treatment. In many embodiments, thymosin-α is administered twice per week for the desired course of NS3 inhibitor compound treatment. Effective dosages of thymosin-α range from about 0.5 mg to about 5 mg, e.g., from about 0.5 mg to about 1.0 mg, from about 1.0 mg to about 1.5 mg, from about 1.5 mg to about 2.0 mg, from about 2.0 mg to about 2.5 mg, from about 2.5 mg to about 3.0 mg, from about 3.0 mg to about 3.5 mg, from about 3.5 mg to about 4.0 mg, from about 4.0 mg to about 4.5 mg, or from about 4.5 mg to about 5.0 mg. In particular embodiments, thymosin-α is administered in dosages containing an amount of 1.0 mg or 1.6 mg.

Thymosin-α can be administered over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more. In one emobidment, thymosin-α is administered for the desired course of NS3 inhibitor compound treatment.

### Combination therapies with interferon(s)

In many embodiments, the methods provide for combination therapy comprising administering an NS3 inhibitor compound as described above, and an effective amount of an interferon receptor agonist. In some embodiments, a compound of Formula I and a Type I or III interferon receptor agonist are co-administered in the treatment methods described herein. Type I interferon receptor agonists suitable for use herein include any interferon-α (IFN-α). In certain embodiments, the interferon-α is a PEGylated interferon-α. In certain other embodiments, the interferon-α is a consensus interferon, such as INFERGEN® interferon alfacon-1. In still other embodiments, the interferon-α is a monoPEG (30 kD, linear)-ylated consensus interferon.

Effective dosages of an IFN-α range from about 3 µg to about 27 µg, from about 3 MU to about 10 MU, from about 90 µg to about 180 µg, or from about 18 µg to about 90 µg. Effective dosages of Infergen® consensus IFN-α include about 3 µg, about 6 µg, about 9 µg, about 12 µg, about 15 µg, about 18 µg, about 21 µg, about 24 µg, about 27 µg, or about 30 µg, of drug per dose. Effective dosages of IFN-α2a and IFN-α2b range from 3 million Units (MU) to 10 MU per dose. Effective dosages of PEGASYS®PEGylated IFN-α2a contain an amount of about 90 µg to 270 µg, or about 180 µg, of drug per dose. Effective dosages of PEG-INTRON®PEGylated IFN-α2b contain an amount of about 0.5 µg to 3.0 µg of drug per kg of body weight per dose. Effective dosages of PEGylated consensus interferon (PEG-CIFN) contain an amount of about 18 µg to about 90 µg, or from about 27 µg to about 60 µg, or about 45 µg, of CIFN amino acid weight per dose of PEG-CIFN. Effective dosages of monoPEG (30 kD, linear)-ylated CIFN contain an amount of about 45 µg to about 270 µg, or about 60 µg to about 180 µg, or about 90 µg to about 120 µg, of drug per dose. IFN-α can be administered daily, every other day, once a week, three times a week, every other week, three times per month, once monthly, substantially continuously or continuously.

In many embodiments, the Type I or Type III interferon receptor agonist and/or the Type II interferon receptor agonist is administered for a period of about 1 day to about 7 days, or about 1 week to about 2 weeks, or about 2 weeks to about 3 weeks, or about 3 weeks to about 4 weeks, or about 1 month to about 2 months, or about 3 months to about 4 months, or about 4 months to about 6 months, or about 6 months to about 8 months, or about 8 months to about 12 months, or at least one year, and may be administered over longer periods of time. Dosage regimens can include tid, bid, qd, qod, biw, tiw, qw, qow, three times per month, or monthly administrations. Some embodiments provide any of the above-described methods in which the desired dosage of IFN-α is administered subcutaneously to the patient by bolus delivery qd, qod, tiw, biw, qw, qow, three times per month, or monthly, or is administered subcutaneously to the patient per day by substantially continuous or continuous delivery, for the desired treatment duration. In other embodiments, any of the above-described methods may be practiced in which the desired dosage of PEGylated IFN-α (PEG-IFN-α) is administered subcutaneously to the patient by bolus delivery qw, qow, three times per month, or monthly for the desired treatment duration.

In other embodiments, an NS3 inhibitor compound and a Type II interferon receptor agonist are co-administered in the treatment methods of the embodiments. Type II interferon receptor agonists suitable for use herein include any interferon-γ (IFN-γ).

Effective dosages of IFN-γ can range from about 0.5 µg/m² to about 500 µg/m², usually from about 1.5 µg/m² to 200 µg/m², depending on the size of the patient. This activity is based on 10⁶ international units (U) per 50 µg of protein. IFN-γ can be administered daily, every other day, three times a week, or substantially continuously or continuously.

In specific embodiments of interest, IFN-γ is administered to an individual in a unit dosage form of from about 25 µg to about 500 µg, from about 50 µg to about 400 µg, or from about 100 µg to about 300 µg. In particular embodiments of interest, the dose is about 200 µg IFN-γ. In many embodiments of interest, IFN-γ1b is administered.

Where the dosage is 200 µg IFN-γ per dose, the amount of IFN-γ per body weight (assuming a range of body weights of from about 45 kg to about 135 kg) is in the range of from about 4.4 µg IFN-γ per kg body weight to about 1.48 µg IFN-γ per kg body weight.

The body surface area of subject individuals generally ranges from about 1.33 m² to about 2.50 m². Thus, in many embodiments, an IFN-γ dosage ranges from about 150 µg/m² to about 20 µg/m². For example, an IFN-γ dosage ranges from about 20 µg/m² to about 30 µg/m², from about 30 µg/m² to about 40 µg/m², from about 40 µg/m² to about 50 µg/m², from about 50 µg/m² to about 60 µg/m², from about 60 µg/m² to about 70 µg/m², from about 70 µg/m² to about 80 µg/m², from about 80 µg/m² to about 90 µg/m², from about 90 µg/m² to about 100 µg/m², from about 100 µg/m² to about 110 µg/m², from about 110 µg/m² to about 120 µg/m², from about 120 µg/m² to about 130 µg/m², from about 130 µg/m² to about 140 µg/m², or from about 140 µg/m² to about 150 µg/m². In some embodiments, the dosage groups range from about 25 µg/m² to about 100 µg/m². In other embodiments, the dosage groups range from about 25 µg/m² to about 50 µg/m².

In some embodiments, a Type I or a Type III interferon receptor agonist is administered in a first dosing regimen, followed by a second dosing regimen. The first dosing regimen of Type I or a Type III interferon receptor agonist (also referred to as "the induction regimen") generally involves administration of a higher dosage of the Type I or Type III interferon receptor agonist. For example, in the case of Infergen® consensus IFN-α (CIFN), the first dosing regimen comprises administering CIFN at about 9 µg, about 15 µg, about 18 µg, or about 27 µg. The first dosing regimen can encompass a single dosing event, or at least two or more dosing events. The first dosing regimen of the Type I or Type III interferon receptor agonist can be administered daily, every other day, three times a week, every other week, three times per month, once monthly, substantially continuously or continuously.

The first dosing regimen of the Type I or Type III interferon receptor agonist is administered for a first period of time, which time period can be at least about 4 weeks, at least about 8 weeks, or at least about 12 weeks.

The second dosing regimen of the Type I or Type III interferon receptor agonist (also referred to as "the maintenance dose") generally involves administration of a lower amount of the Type I or Type III interferon receptor agonist. For example, in the case of CIFN, the second dosing regimen comprises administering CIFN at a dose of at least about 3 µg, at least about 9 µg, at least about 15 µg, or at least about 18 µg. The second dosing regimen can encompass a single dosing event, or at least two or more dosing events.

The second dosing regimen of the Type I or Type III interferon receptor agonist can be administered daily, every other day, three times a week, every other week, three times per month, once monthly, substantially continuously or continuously.

In some embodiments, where an "induction"/"maintenance" dosing regimen of a Type I or a Type III interferon receptor agonist is administered, a "priming" dose of a Type II interferon receptor agonist (e.g., IFN-γ) is included. In these embodiments, IFN-γ is administered for a period of time from about 1 day to about 14 days, from about 2 days to about 10 days, or from about 3 days to about 7 days, before the beginning of treatment with the Type I or Type III interferon receptor agonist. This period of time is referred to as the "priming" phase.

In some of these embodiments, the Type II interferon receptor agonist treatment is continued throughout the entire period of treatment with the Type I or Type III interferon receptor agonist. In other embodiments, the Type II interferon receptor agonist treatment is discontinued before the end of treatment with the Type I or Type III interferon receptor agonist. In these embodiments, the total time of treatment with Type II interferon receptor agonist (including the "priming" phase) is from about 2 days to about 30 days, from about 4 days to about 25 days, from about 8 days to about 20 days, from about 10 days to about 18 days, or from about 12 days to about 16 days. In still other embodiments, the Type II interferon receptor agonist treatment is discontinued once Type I or a Type III interferon receptor agonist treatment begins.

In other embodiments, the Type I or Type III interferon receptor agonist is administered in single dosing regimen. For example, in the case of CIFN, the dose of CIFN is generally in a range of from about 3 µg to about 15 µg, or from about 9 µg to about 15 µg. The dose of Type I or a Type III interferon receptor agonist is generally administered daily, every other day, three times a week, every other week, three times per month, once monthly, or substantially continuously. The dose of the Type I or Type III interferon receptor agonist is administered for a period of time, which period can be, for example, from at least about 24 weeks to at least about 48 weeks, or longer.

In some embodiments, where a single dosing regimen of a Type I or a Type III interferon receptor agonist is administered, a "priming" dose of a Type II interferon receptor agonist (e.g., IFN-γ) is included. In these embodiments, IFN-γ is administered for a period of time from about 1 day to about 14 days, from about 2 days to about 10 days, or from about 3 days to about 7 days, before the beginning of treatment with the Type I or Type III interferon receptor agonist. This period of time is referred to as the "priming" phase. In some of these embodiments, the Type II interferon receptor agonist treatment is continued throughout the entire period of treatment with the Type I or Type III interferon receptor agonist. In other embodiments, the Type II interferon receptor agonist treatment is discontinued before the end of treatment with the Type I or Type III interferon receptor agonist. In these embodiments, the total time of treatment with the Type II interferon receptor agonist (including the "priming" phase) is from about 2 days to about 30 days, from about 4 days to about 25 days, from about 8 days to about 20 days, from about 10 days to about 18 days, or from about 12 days to about 16 days. In still other embodiments, Type II interferon receptor agonist treatment is discontinued once Type I or a Type III interferon receptor agonist treatment begins.

In additional embodiments, an NS3 inhibitor compound, a Type I or III interferon receptor agonist, and a Type II interferon receptor agonist are co-administered for the desired duration of treatment in the methods described herein. In some embodiments, an NS3 inhibitor compound, an interferon-α, and an interferon-γ are co-administered for the desired duration of treatment in the methods described herein.

In some embodiments, the invention provides methods using an amount of a Type I or Type III interferon receptor agonist, a Type II interferon receptor agonist, and an NS3 inhibitor compound, effective for the treatment of HCV infection in a patient. Some embodiments provide methods using an effective amount of an IFN-α, IFN-γ, and an NS3 inhibitor compound in the treatment of HCV infection in a patient. One embodiment provides a method using an effective amount of a consensus IFN-α, IFN-γ and an NS3 inhibitor compound in the treatment of HCV infection in a patient.

In general, an effective amount of a consensus interferon (CIFN) and IFN-γ suitable for use in the methods of the embodiments is provided by a dosage ratio of 1 µg CIFN: 10 µg IFN-γ, where both CIFN and IFN-γ are unPEGylated and unglycosylated species.

In one embodiment, the invention provides any of the above-described methods modified to use an effective amount of INFERGEN®consensus IFN-α and IFN-γ in the treatment of HCV infection in a patient comprising administering to the patient a dosage of INFERGEN® containing an amount of about 1 µg to about 30 µg, of drug per dose of INFERGEN®, subcutaneously qd, qod, tiw, biw, qw, qow, three times per month, once monthly, or per day substantially continuously or continuously, in combination with a dosage of IFN-γ containing an amount of about 10 µg to about 300 µg of drug per dose of IFN-γ, subcutaneously qd, qod, tiw, biw, qw, qow, three times per month, once monthly, or per day substantially continuously or continuously, for the desired duration of treatment with an NS3 inhibitor compound.

Another embodiment provides any of the above-described methods modified to use an effective amount of INFERGEN®consensus IFN-α and IFN-γ in the treatment of virus infection in a patient comprising administering to the patient a dosage of INFERGEN® containing an amount of about 1 µg to about 9 µg, of drug per dose of INFERGEN®, subcutaneously qd, qod, tiw, biw, qw, qow, three times per month, once monthly, or per day substantially continuously or continuously, in combination with a dosage of IFN-γ containing an amount of about 10 µg to about 100 µg of drug per dose of IFN-γ, subcutaneously qd, qod, tiw, biw, qw, qow, three times per month, once monthly, or per day substantially continuously or continuously, for the desired duration of treatment with an NS3 inhibitor compound.

Another embodiment provides any of the above-described methods modified to use an effective amount of INFERGEN®consensus IFN-α and IFN-γ in the treatment of virus infection in a patient comprising administering to the patient a dosage of INFERGEN® containing an amount of about 1 µg of drug per dose of INFERGEN®, subcutaneously qd, qod, tiw, biw, qw, qow, three times per month, once monthly, or per day substantially continuously or continuously, in combination with a dosage of IFN-γ containing an amount of about 10 µg to about 50 µg of drug per dose of IFN-γ, subcutaneously qd, qod, tiw, biw, qw, qow, three times per month, once monthly, or per day substantially continuously or continuously, for the desired duration of treatment with an NS3 inhibitor compound.

Another embodiment provides any of the above-described methods modified to use an effective amount of INFERGEN®consensus IFN-α and IFN-γ in the treatment of a virus infection in a patient comprising administering to the patient a dosage of INFERGEN® containing an amount of about 9 µg of drug per dose of INFERGEN®, subcutaneously qd, qod, tiw, biw, qw, qow, three times per month, once monthly, or per day substantially continuously or continuously, in combination with a dosage of IFN-γ containing an amount of about 90 µg to about 100 µg of drug per dose of IFN-γ, subcutaneously qd, qod, tiw, biw, qw, qow, three times per month, once monthly, or per day substantially continuously or continuously, for the desired duration of treatment with an NS3 inhibitor compound.

Another embodiment provides any of the above-described methods modified to use an effective amount of INFERGEN®consensus IFN-α and IFN-γ in the treatment of a virus infection in a patient comprising administering to the patient a dosage of INFERGEN® containing an amount of about 30 µg of drug per dose of INFERGEN®, subcutaneously qd, qod, tiw, biw, qw, qow, three times per month, once monthly, or per day substantially continuously or continuously, in combination with a dosage of IFN-γ containing an amount of about 200 µg to about 300 µg of drug per dose of IFN-γ, subcutaneously qd, qod, tiw, biw, qw, qow, three times per month, once monthly, or per day substantially continuously or continuously, for the desired duration of treatment with an NS3 inhibitor compound.

Another embodiment provides any of the above-described methods modified to use an effective amount of PEGylated consensus IFN-α and IFN-γ in the treatment of a virus infection in a patient comprising administering to the patient a dosage of PEGylated consensus IFN-α (PEG-CIFN) containing an amount of about 4 µg to about 60 µg of CIFN amino acid weight per dose of PEG-CIFN, subcutaneously qw, qow, three times per month, or monthly, in combination with a total weekly dosage of IFN-γ containing an amount of about 30 µg to about 1,000 µg of drug per week in divided doses administered subcutaneously qd, qod, tiw, biw, or administered substantially continuously or continuously, for the desired duration of treatment with an NS3 inhibitor compound.

Another embodiment provides any of the above-described methods modified to use an effective amount of PEGylated consensus IFN-α and IFN-γ in the treatment of a virus infection in a patient comprising administering to the patient a dosage of PEGylated consensus IFN-α (PEG-CIFN) containing an amount of about 18 µg to about 24 µg of CIFN amino acid weight per dose of PEG-CIFN, subcutaneously qw, qow, three times per month, or monthly, in combination with a total weekly dosage of IFN-γ containing an amount of about 100 µg to about 300 µg of drug per week in divided doses administered subcutaneously qd, qod, tiw, biw, or substantially continuously or continuously, for the desired duration of treatment with an NS3 inhibitor compound.

In general, an effective amount of IFN-α 2a or 2b or 2c and IFN-γ suitable for use in the methods of the embodiments is provided by a dosage ratio of 1 million Units (MU) IFN-α 2a or 2b or 2c : 30 µg IFN-γ, where both IFN-α 2a or 2b or 2c and IFN-γ are unPEGylated and unglycosylated species.

Another embodiment provides any of the above-described methods modified to use an effective amount of IFN-α 2a or 2b or 2c and IFN-γ in the treatment of a virus infection in a patient comprising administering to the patient a dosage of IFN-α 2a, 2b or 2c containing an amount of about 1 MU to about 20 MU of drug per dose of IFN-α 2a, 2b or 2c subcutaneously qd, qod, tiw, biw, or per day substantially continuously or continuously, in combination with a dosage of IFN-γ containing an amount of about 30 µg to about 600 µg of drug per dose of IFN-γ, subcutaneously qd, qod, tiw, biw, or per day substantially continuously or continuously, for the desired duration of treatment with an NS3 inhibitor compound.

Another embodiment provides any of the above-described methods modified to use an effective amount of IFN-α 2a or 2b or 2c and IFN-γ in the treatment of a virus infection in a patient comprising administering to the patient a dosage of IFN-α 2a, 2b or 2c containing an amount of about 3 MU of drug per dose of IFN-α 2a, 2b or 2c subcutaneously qd, qod, tiw, biw, or per day substantially continuously or continuously, in combination with a dosage of IFN-γ containing an amount of about 100 µg of drug per dose of IFN-γ, subcutaneously qd, qod, tiw, biw, or per day substantially continuously or continuously, for the desired duration of treatment with an NS3 inhibitor compound.

Another embodiment provides any of the above-described methods modified to use an effective amount of IFN-α 2a or 2b or 2c and IFN-γ in the treatment of a virus infection in a patient comprising administering to the patient a dosage of IFN-α 2a, 2b or 2c containing an amount of about 10 MU of drug per dose of IFN-α 2a, 2b or 2c subcutaneously qd, qod, tiw, biw, or per day substantially continuously or continuously, in combination with a dosage of IFN-γ containing an amount of about 300 µg of drug per dose of IFN-γ, subcutaneously qd, qod, tiw, biw, or per day substantially continuously or continuously, for the desired duration of treatment with an NS3 inhibitor compound.

Another embodiment provides any of the above-described methods modified to use an effective amount of PEGASYS®PEGylated IFN-α2a and IFN-γ in the treatment of a virus infection in a patient comprising administering to the patient a dosage of PEGASYS® containing an amount of about 90 µg to about 360 µg, of drug per dose of PEGASYS®, subcutaneously qw, qow, three times per month, or monthly, in combination with a total weekly dosage of IFN-γ containing an amount of about 30 µg to about 1,000 µg, of drug per week administered in divided doses subcutaneously qd, qod, tiw, or biw, or administered substantially continuously or continuously, for the desired duration of treatment with an NS3 inhibitor compound.

Another embodiment provides any of the above-described methods modified to use an effective amount of PEGASYS®PEGylated IFN-α2a and IFN-γ in the treatment of a virus infection in a patient comprising administering to the patient a dosage of PEGASYS® containing an amount of about 180 µg of drug per dose of PEGASYS®, subcutaneously qw, qow, three times per month, or monthly, in combination with a total weekly dosage of IFN-γ containing an amount of about 100 µg to about 300 µg, of drug per week administered in divided doses subcutaneously qd, qod, tiw, or biw, or administered substantially continuously or continuously, for the desired duration of treatment with an NS3 inhibitor compound.

Another embodiment provides any of the above-described methods modified to use an effective amount of PEG-INTRON®PEGylated IFN-α2b and IFN-γ in the treatment of a virus infection in a patient comprising administering to the patient a dosage of PEG-INTRON® containing an amount of about 0.75 µg to about 3.0 µg of drug per kilogram of body weight per dose of PEG-INTRON®, subcutaneously qw, qow, three times per month, or monthly, in combination with a total weekly dosage of IFN-γ containing an amount of about 30 µg to about 1,000 µg of drug per week administered in divided doses subcutaneously qd, qod, tiw, or biw, or administered substantially continuously or continuously, for the desired duration of treatment with an NS3 inhibitor compound.

Another embodiment provides any of the above-described methods modified to use an effective amount of PEG-INTRON®PEGylated IFN-α2b and IFN-γ in the treatment of a virus infection in a patient comprising administering to the patient a dosage of PEG-INTRON® containing an amount of about 1.5 µg of drug per kilogram of body weight per dose of PEG-INTRON®, subcutaneously qw, qow, three times per month, or monthly, in combination with a total weekly dosage of IFN-γ containing an amount of about 100 µg to about 300 µg of drug per week administered in divided doses subcutaneously qd, qod, tiw, or biw, or administered substantially continuously or continuously, for the desired duration of treatment with an NS3 inhibitor compound.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw, and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; 50 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; 100 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; and 50 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; and 100 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; 25 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an

NS3 inhibitor; and a regimen of 9 µg INFERGEN® consensus IFN-α administered subcutaneously qd or tiw; 200 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; and 25 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 9 µg INFERGEN® consensus IFN-α administered subcutaneously qd or tiw; and 200 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 100 µg monoPEG(30 kD, linear)-ylated consensus IFN-α administered subcutaneously every 10 days or qw, and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 100 µg monoPEG(30 kD, linear)-ylated consensus IFN-α administered subcutaneously every 10 days or qw; 50 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 100 µg monoPEG(30 kD, linear)-ylated consensus IFN-α administered subcutaneously every 10 days or qw; 100 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 100 µg monoPEG(30 kD, linear)-ylated consensus IFN-α administered subcutaneously every 10 days or qw; and 50 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 100 µg monoPEG(30 kD, linear)-ylated consensus IFN-α administered subcutaneously every 10 days or qw; and 100 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 150 µg monoPEG(30 kD, linear)-ylated consensus IFN-α administered subcutaneously every 10 days or qw, and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 150 µg monoPEG(30 kD, linear)-ylated consensus IFN-α administered subcutaneously every 10 days or qw; 50 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 150 µg monoPEG(30 kD, linear)-ylated consensus IFN-α administered subcutaneously every 10 days or qw; 100 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 150 µg monoPEG(30 kD, linear)-ylated consensus IFN-α administered subcutaneously every 10 days or qw; and 50 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 150 µg monoPEG(30 kD, linear)-ylated consensus IFN-α administered subcutaneously every 10 days or qw; and 100 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 200 µg monoPEG(30 kD, linear)-ylated consensus IFN-α administered subcutaneously every 10 days or qw, and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 200 µg monoPEG(30 kD, linear)-ylated consensus IFN-α administered subcutaneously every 10 days or qw; 50 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 200 µg monoPEG(30 kD, linear)-ylated consensus IFN-α administered subcutaneously every 10 days or qw; 100 µg Actimmune® human IFN-γ1b administered subcutaneously tiw; and ribavirin administered orally qd, where the duration of therapy is 48 weeks. In this embodiment, ribavirin is administered in an amount of 1000 mg for individuals weighing less than 75 kg, and 1200 mg for individuals weighing 75 kg or more.

One embodiment provides any of the above-described methods modified to comprise administering to an individual having an HCV infection an effective amount of an NS3 inhibitor; and a regimen of 200 µg monoPEG(30 kD, linear)-ylated consensus IFN-α administered subcutaneously every 10 days or qw; and 50 µg Actimmune® human IFN-γ1b administered subcutaneously tiw, where the duration of therapy is 48 weeks.

One embodiment provides any of the above-described methods modified to comprise administering to an individual having an HCV infection an effective amount of an NS3 inhibitor; and a regimen of 200 µg monoPEG(30 kD, linear)-ylated consensus IFN-α administered subcutaneously every 10 days or qw; and 100 µg Actimmune® human IFN-γ1b administered subcutaneously tiw, where the duration of therapy is 48 weeks.

Any of the above-described methods involving administering an NS3 inhibitor, a Type I interferon receptor agonist (e.g., an IFN-α), and a Type II interferon receptor agonist (e.g., an IFN-γ), can be augmented by administration of an effective amount of a TNF-α antagonist (e.g., a TNF-α antagonist other than pirfenidone or a pirfenidone analog). Exemplary, non-limiting TNF-α antagonists that are suitable for use in such combination therapies include ENBREL®, REMICADE®, and HUMIRA™.

One embodiment provides a method using an effective amount of ENBREL®; an effective amount of IFN-α; an effective amount of IFN-γ; and an effective amount of an NS3 inhibitor in the treatment of an HCV infection in a patient, comprising administering to the patient a dosage ENBREL® containing an amount of from about 0.1 µg to about 23 mg per dose, from about 0.1 µg to about 1 µg, from about 1 µg to about 10 µg, from about 10 µg to about 100 µg, from about 100 µg to about 1 mg, from about 1 mg to about 5 mg, from about 5 mg to about 10 mg, from about 10 mg to about 15 mg, from about 15 mg to about 20 mg, or from about 20 mg to about 23 mg of ENBREL®, subcutaneously qd, qod, tiw, biw, qw, qow, three times per month, once monthly, or once every other month, or per day substantially continuously or continuously, for the desired duration of treatment.

One embodiment provides a method using an effective amount of REMICADE®, an effective amount of IFN-α; an effective amount of IFN-γ; and an effective amount of an NS3 inhibitor in the treatment of an HCV infection in a patient, comprising administering to the patient a dosage of REMICADE® containing an amount of from about 0.1 mg/kg to about 4.5 mg/kg, from about 0.1 mg/kg to about 0.5 mg/kg, from about 0.5 mg/kg to about 1.0 mg/kg, from about 1.0 mg/kg to about 1.5 mg/kg, from about 1.5 mg/kg to about 2.0 mg/kg, from about 2.0 mg/kg to about 2.5 mg/kg, from about 2.5 mg/kg to about 3.0 mg/kg, from about 3.0 mg/kg to about 3.5 mg/kg, from about 3.5 mg/kg to about 4.0 mg/kg, or from about 4.0 mg/kg to about 4.5 mg/kg per dose of REMICADE®, intravenously qd, qod, tiw, biw, qw, qow, three times per month, once monthly, or once every other month, or per day substantially continuously or continuously, for the desired duration of treatment.

One embodiment provides a method using an effective amount of HUMIRA™, an effective amount of IFN-α; an effective amount of IFN-γ; and an effective amount of an NS3 inhibitor in the treatment of an HCV infection in a patient, comprising administering to the patient a dosage of HUMIRA™ containing an amount of from about 0.1 µg to about 35 mg, from about 0.1 µg to about 1 µg, from about 1 µg to about 10 µg, from about 10 µg to about 100 µg, from about 100 µg to about 1 mg, from about 1 mg to about 5 mg, from about 5 mg to about 10 mg, from about 10 mg to about 15 mg, from about 15 mg to about 20 mg, from about 20 mg to about 25 mg, from about 25 mg to about 30 mg, or from about 30 mg to about 35 mg per dose of a HUMIRA™, subcutaneously qd, qod, tiw, biw, qw, qow, three times per month, once monthly, or once every other month, or per day substantially continuously or continuously, for the desired duration of treatment.

### Combination therapies with pirfenidone

In many embodiments, the methods provide for combination therapy comprising administering an NS3 inhibitor compound as described above, and an effective amount of pirfenidone or a pirfenidone analog. In some embodiments, an NS3 inhibitor compound, one or more interferon receptor agonist(s), and pirfenidone or pirfenidone analog are co-administered in the treatment methods of the embodiments. In certain embodiments, an NS3 inhibitor compound, a Type I interferon receptor agonist, and pirfenidone (or a pirfenidone analog) are co-administered. In other embodiments, an NS3 inhibitor compound, a Type I interferon receptor agonist, a Type II interferon receptor agonist, and pirfenidone (or a pirfenidone analog) are co-administered. Type I interferon receptor agonists suitable for use herein include any IFN-α, such as interferon alfa-2a, interferon alfa-2b, interferon alfacon-1, and PEGylated IFN-α's, such as peginterferon alfa-2a, peginterferon alfa-2b, and PEGylated consensus interferons, such as monoPEG (30 kD, linear)-ylated consensus interferon. Type II interferon receptor agonists suitable for use herein include any interferon-γ.

Pirfenidone or a pirfenidone analog can be administered once per month, twice per month, three times per month, once per week, twice per week, three times per week, four times per week, five times per week, six times per week, daily, or in divided daily doses ranging from once daily to 5 times daily over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

Effective dosages of pirfenidone or a specific pirfenidone analog include a weight-based dosage in the range from about 5 mg/kg/day to about 125 mg/kg/day, or a fixed dosage of about 400 mg to about 3600 mg per day, or about 800 mg to about 2400 mg per day, or about 1000 mg to about 1800 mg per day, or about 1200 mg to about 1600 mg per day, administered orally in one to five divided doses per day. Other doses and formulations of pirfenidone and specific pirfenidone analogs suitable for use in the treatment of fibrotic diseases are described in U.S. Pat. Nos., 5,310,562; 5,518,729; 5,716,632; and 6,090,822.

One embodiment provides any of the above-described methods modified to include co-administering to the patient a therapeutically effective amount of pirfenidone or a pirfenidone analog for the duration of the desired course of NS3 inhibitor compound treatment.

### Combination therapies with TNF-α antagonists

In many embodiments, the methods provide for combination therapy comprising administering an effective amount of an NS3 inhibitor compound as described above, and an effective amount of TNF-α antagonist, in combination therapy for treatment of an HCV infection.

Effective dosages of a TNF-α antagonist range from 0.1 µg to 40 mg per dose, e.g., from about 0.1 µg to about 0.5 µg per dose, from about 0.5 µg to about 1.0 µg per dose, from about 1.0 µg per dose to about 5.0 µg per dose, from about 5.0 µg to about 10 µg per dose, from about 10 µg to about 20 µg per dose, from about 20 µg per dose to about 30 µg per dose, from about 30 µg per dose to about 40 µg per dose, from about 40 µg per dose to about 50 µg per dose, from about 50 µg per dose to about 60 µg per dose, from about 60 µg per dose to about 70 µg per dose, from about 70 µg to about 80 µg per dose, from about 80 µg per dose to about 100 µg per dose, from about 100 µg to about 150 µg per dose, from about 150 µg to about 200 µg per dose, from about 200 µg per dose to about 250 µg per dose, from about 250 µg to about 300 µg per dose, from about 300 µg to about 400 µg per dose, from about 400 µg to about 500 µg per dose, from about 500 µg to about 600 µg per dose, from about 600 µg to about 700 µg per dose, from about 700 µg to about 800 µg per dose, from about 800 µg to about 900 µg per dose, from about 900 µg to about 1000 µg per dose, from about 1 mg to about 10 mg per dose, from about 10 mg to about 15 mg per dose, from about 15 mg to about 20 mg per dose, from about 20 mg to about 25 mg per dose, from about 25 mg to about 30 mg per dose, from about 30 mg to about 35 mg per dose, or from about 35 mg to about 40 mg per dose.

In some embodiments, effective dosages of a TNF-α antagonist are expressed as mg/kg body weight. In these embodiments, effective dosages of a TNF-α antagonist are from about 0.1 mg/kg body weight to about 10 mg/kg body weight, e.g., from about 0.1 mg/kg body weight to about 0.5 mg/kg body weight, from about 0.5 mg/kg body weight to about 1.0 mg/kg body weight, from about 1.0 mg/kg body weight to about 2.5 mg/kg body weight, from about 2.5 mg/kg body weight to about 5.0 mg/kg body weight, from about 5.0 mg/kg body weight to about 7.5 mg/kg body weight, or from about 7.5 mg/kg body weight to about 10 mg/kg body weight.

In many embodiments, a TNF-α antagonist is administered for a period of about 1 day to about 7 days, or about 1 week to about 2 weeks, or about 2 weeks to about 3 weeks, or about 3 weeks to about 4 weeks, or about 1 month to about 2 months, or about 3 months to about 4 months, or about 4 months to about 6 months, or about 6 months to about 8 months, or about 8 months to about 12 months, or at least one year, and may be administered over longer periods of time. The TNF-α antagonist can be administered tid, bid, qd, qod, biw, tiw, qw, qow, three times per month, once monthly, substantially continuously, or continuously.

In many embodiments, multiple doses of a TNF-α antagonist are administered. For example, a TNF-α antagonist is administered once per month, twice per month, three times per month, every other week (qow), once per week (qw), twice per week (biw), three times per week (tiw), four times per week, five times per week, six times per week, every other day (qod), daily (qd), twice a day (bid), or three times a day (tid), substantially continuously, or continuously, over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

A TNF-α antagonist and an NS3 inhibitor are generally administered in separate formulations. A TNF-α antagonist and an NS3 inhibitor may be administered substantially simultaneously, or within about 30 minutes, about 1 hour, about 2 hours, about 4 hours, about 8 hours, about 16 hours, about 24 hours, about 36 hours, about 72 hours, about 4 days, about 7 days, or about 2 weeks of one another.

One embodiment provides a method using an effective amount of a TNF-α antagonist and an effective amount of an NS3 inhibitor in the treatment of an HCV infection in a patient, comprising administering to the patient a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

One embodiment provides a method using an effective amount of ENBREL® and an effective amount of an NS3 inhibitor in the treatment of an HCV infection in a patient, comprising administering to the patient a dosage ENBREL® containing an amount of from about 0.1 µg to about 23 mg per dose, from about 0.1 µg to about 1 µg, from about 1 µg to about 10 µg, from about 10 µg to about 100 µg, from about 100 µg to about 1 mg, from about 1 mg to about 5 mg, from about 5 mg to about 10 mg, from about 10 mg to about 15 mg, from about 15 mg to about 20 mg, or from about 20 mg to about 23 mg of ENBREL®, subcutaneously qd, qod, tiw, biw, qw, qow, three times per month, once monthly, or once every other month, or per day substantially continuously or continuously, for the desired duration of treatment with an NS3 inhibitor compound.

One embodiment provides a method using an effective amount of REMICADE® and an effective amount of an NS3 inhibitor in the treatment of an HCV infection in a patient, comprising administering to the patient a dosage of REMICADE® containing an amount of from about 0.1 mg/kg to about 4.5 mg/kg, from about 0.1 mg/kg to about 0.5 mg/kg, from about 0.5 mg/kg to about 1.0 mg/kg, from about 1.0 mg/kg to about 1.5 mg/kg, from about 1.5 mg/kg to about 2.0 mg/kg, from about 2.0 mg/kg to about 2.5 mg/kg, from about 2.5 mg/kg to about 3.0 mg/kg, from about 3.0 mg/kg to about 3.5 mg/kg, from about 3.5 mg/kg to about 4.0 mg/kg, or from about 4.0 mg/kg to about 4.5 mg/kg per dose of REMICADE®, intravenously qd, qod, tiw, biw, qw, qow, three times per month, once monthly, or once every other month, or per day substantially continuously or continuously, for the desired duration of treatment with an NS3 inhibitor compound.

One embodiment provides a method using an effective amount of HUMIRA™ and an effective amount of an NS3 inhibitor in the treatment of an HCV infection in a patient, comprising administering to the patient a dosage of HUMIRA™ containing an amount of from about 0.1 µg to about 35 mg, from about 0.1 µg to about 1 µg, from about 1 µg to about 10 µg, from about 10 µg to about 100 µg, from about 100 µg to about 1 mg, from about 1 mg to about 5 mg, from about 5 mg to about 10 mg, from about 10 mg to about 15 mg, from about 15 mg to about 20 mg, from about 20 mg to about 25 mg, from about 25 mg to about 30 mg, or from about 30 mg to about 35 mg per dose of a HUMIRA™, subcutaneously qd, qod, tiw, biw, qw, qow, three times per month, once monthly, or once every other month, or per day substantially continuously or continuously, for the desired duration of treatment with an NS3 inhibitor compound.

### Combination therapies with thymosin-α

In many embodiments, the methods provide for combination therapy comprising administering an effective amount of an NS3 inhibitor compound as described above, and an effective amount of thymosin-α, in combination therapy for treatment of an HCV infection.

Effective dosages of thymosin-α range from about 0.5 mg to about 5 mg, e.g., from about 0.5 mg to about 1.0 mg, from about 1.0 mg to about 1.5 mg, from about 1.5 mg to about 2.0 mg, from about 2.0 mg to about 2.5 mg, from about 2.5 mg to about 3.0 mg, from about 3.0 mg to about 3.5 mg, from about 3.5 mg to about 4.0 mg, from about 4.0 mg to about 4.5 mg, or from about 4.5 mg to about 5.0 mg. In particular embodiments, thymosin-α is administered in dosages containing an amount of 1.0 mg or 1.6 mg.

One embodiment provides a method using an effective amount of ZADAXIN™ thymosin-α and an effective amount of an NS3 inhibitor in the treatment of an HCV infection in a patient, comprising administering to the patient a dosage of ZADAXIN™ containing an amount of from about 1.0 mg to about 1.6 mg per dose, subcutaneously twice per week for the desired duration of treatment with the NS3 inhibitor compound.

### Combination therapies with a TNF-α antagonist and an interferon

Some embodiments provide a method of treating an HCV infection in an individual having an HCV infection, the method comprising administering an effective amount of an NS3 inhibitor, and effective amount of a TNF-α antagonist, and an effective amount of one or more interferons.

One embodiment provides any of the above-described methods modified to use an effective amount of IFN-γ and an effective amount of a TNF-α antagonist in the treatment of HCV infection in a patient comprising administering to the patient a dosage of IFN-γ containing an amount of about 10 µg to about 300 µg of drug per dose of IFN-γ, subcutaneously qd, qod, tiw, biw, qw, qow, three times per month, once monthly, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

One embodiment provides any of the above-described methods modified to use an effective amount of IFN-γ and an effective amount of a TNF-α antagonist in the treatment of HCV infection in a patient comprising administering to the patient a dosage of IFN-γ containing an amount of about 10 µg to about 100 µg of drug per dose of IFN-γ, subcutaneously qd, qod, tiw, biw, qw, qow, three times per month, once monthly, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of IFN-γ and an effective amount of a TNF-α antagonist in the treatment of a virus infection in a patient comprising administering to the patient a total weekly dosage of IFN-γ containing an amount of about 30 µg to about 1,000 µg of drug per week in divided doses administered subcutaneously qd, qod, tiw, biw, or administered substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of IFN-γ and an effective amount of a TNF-α antagonist in the treatment of a virus infection in a patient comprising administering to the patient a total weekly dosage of IFN-γ containing an amount of about 100 µg to about 300 µg of drug per week in divided doses administered subcutaneously qd, qod, tiw, biw, or administered substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

One embodiment provides any of the above-described methods modified to use an effective amount of INFERGEN® consensus IFN-α and a TNF-α antagonist in the treatment of HCV infection in a patient comprising administering to the patient a dosage of INFERGEN® containing an amount of about 1 µg to about 30 µg, of drug per dose of INFERGEN®, subcutaneously qd, qod, tiw, biw, qw, qow, three times per month, once monthly, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

One embodiment provides any of the above-described methods modified to use an effective amount of INFERGEN® consensus IFN-α and a TNF-α antagonist in the treatment of HCV infection in a patient comprising administering to the patient a dosage of INFERGEN® containing an amount of about 1 µg to about 9 µg, of drug per dose of INFERGEN®, subcutaneously qd, qod, tiw, biw, qw, qow, three times per month, once monthly, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEGylated consensus IFN-α and an effective amount of a TNF-α antagonist in the treatment of a virus infection in a patient comprising administering to the patient a dosage of PEGylated consensus IFN-α (PEG-CIFN) containing an amount of about 4 µg to about 60 µg of CIFN amino acid weight per dose of PEG-CIFN, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEGylated consensus IFN-α and an effective amount of a TNF-α antagonist in the treatment of a virus infection in a patient comprising administering to the patient a dosage of PEGylated consensus IFN-α (PEG-CIFN) containing an amount of about 18 µg to about 24 µg of CIFN amino acid weight per dose of PEG-CIFN, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of IFN-α 2a or 2b or 2c and an effective amount of a TNF-α antagonist in the treatment of a virus infection in a patient comprising administering to the patient a dosage of IFN-α 2a, 2b or 2c containing an amount of about 1 MU to about 20 MU of drug per dose of IFN-α 2a, 2b or 2c subcutaneously qd, qod, tiw, biw, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of IFN-α 2a or 2b or 2c and an effective amount of a TNF-α antagonist in the treatment of a virus infection in a patient comprising administering to the patient a dosage of IFN-α 2a, 2b or 2c containing an amount of about 3 MU of drug per dose of IFN-α 2a, 2b or 2c subcutaneously qd, qod, tiw, biw, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of IFN-α 2a or 2b or 2c and an effective amount of a TNF-α antagonist in the treatment of a virus infection in a patient comprising administering to the patient a dosage of IFN-α 2a, 2b or 2c containing an amount of about 10 MU of drug per dose of IFN-α 2a, 2b or 2c subcutaneously qd, qod, tiw, biw, or per day substantially continuously or continuously, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEGASYS®PEGylated IFN-α2a and an effective amount of a TNF-α antagonist in the treatment of a virus infection in a patient comprising administering to the patient a dosage of PEGASYS® containing an amount of about 90 µg to about 360 µg, of drug per dose of PEGASYS®, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEGASYS®PEGylated IFN-α2a and an effective amount of a TNF-α antagonist in the treatment of a virus infection in a patient comprising administering to the patient a dosage of PEGASYS® containing an amount of about 180 µg, of drug per dose of PEGASYS®, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEG-INTRON®PEGylated IFN-α2b and an effective amount of a TNF-α antagonist in the treatment of a virus infection in a patient comprising administering to the patient a dosage of PEG-INTRON® containing an amount of about 0.75 µg to about 3.0 µg of drug per kilogram of body weight per dose of PEG-INTRON®, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

Another embodiment provides any of the above-described methods modified to use an effective amount of PEG-INTRON®PEGylated IFN-α2b and an effective amount of a TNF-α antagonist in the treatment of a virus infection in a patient comprising administering to the patient a dosage of PEG-INTRON® containing an amount of about 1.5 µg of drug per kilogram of body weight per dose of PEG-INTRON®, subcutaneously qw, qow, three times per month, or monthly, in combination with a dosage of a TNF-α antagonist containing an amount of from about 0.1 µg to about 40 mg per dose of a TNF-α antagonist, subcutaneously qd, qod, tiw, or biw, or per day substantially continuously or continuously, for the desired duration of treatment with an NS3 inhibitor compound.

### Combination therapies with other antiviral agents

Other agents such as inhibitors of HCV NS3 helicase are also attractive drugs for combinational therapy, and are contemplated for use in combination therapies described herein. Ribozymes such as Heptazyme^{™} and phosphorothioate oligonucleotides which are complementary to HCV protein sequences and which inhibit the expression of viral core proteins are also suitable for use in combination therapies described herein.

In some embodiments, the additional antiviral agent(s) is administered during the entire course of treatment with the NS3 inhibitor compound described herein, and the beginning and end of the treatment periods coincide. In other embodiments, the additional antiviral agent(s) is administered for a period of time that is overlapping with that of the NS3 inhibitor compound treatment, e.g., treatment with the additional antiviral agent(s) begins before the NS3 inhibitor compound treatment begins and ends before the NS3 inhibitor compound treatment ends; treatment with the additional antiviral agent(s) begins after the NS3 inhibitor compound treatment begins and ends after the NS3 inhibitor compound treatment ends; treatment with the additional antiviral agent(s) begins after the NS3 inhibitor compound treatment begins and ends before the NS3 inhibitor compound treatment ends; or treatment with the additional antiviral agent(s) begins before the NS3 inhibitor compound treatment begins and ends after the NS3 inhibitor compound treatment ends.

The NS3 inhibitor compound can be administered together with (i.e., simultaneously in separate formulations; simultaneously in the same formulation; administered in separate formulations and within about 48 hours, within about 36 hours, within about 24 hours, within about 16 hours, within about 12 hours, within about 8 hours, within about 4 hours, within about 2 hours, within about 1 hour, within about 30 minutes, or within about 15 minutes or less) one or more additional antiviral agents.

As non-limiting examples, any of the above-described methods featuring an IFN-α regimen can be modified to replace the subject IFN-α regimen with a regimen of monoPEG (30 kD, linear)-ylated consensus IFN-α comprising administering a dosage of monoPEG (30 kD, linear)-ylated consensus IFN-α containing an amount of 100 µg of drug per dose, subcutaneously once weekly, once every 8 days, or once every 10 days for the desired treatment duration with an NS3 inhibitor compound.

As non-limiting examples, any of the above-described methods featuring an IFN-α regimen can be modified to replace the subject IFN-α regimen with a regimen of monoPEG (30 kD, linear)-ylated consensus IFN-α comprising administering a dosage of monoPEG (30 kD, linear)-ylated consensus IFN-α containing an amount of 150 µg of drug per dose, subcutaneously once weekly, once every 8 days, or once every 10 days for the desired treatment duration with an NS3 inhibitor compound.

As non-limiting examples, any of the above-described methods featuring an IFN-α regimen can be modified to replace the subject IFN-α regimen with a regimen of monoPEG (30 kD, linear)-ylated consensus IFN-α comprising administering a dosage of monoPEG (30 kD, linear)-ylated consensus IFN-α containing an amount of 200 µg of drug per dose, subcutaneously once weekly, once every 8 days, or once every 10 days for the desired treatment duration with an NS3 inhibitor compound.

As non-limiting examples, any of the above-described methods featuring an IFN-α regimen can be modified to replace the subject IFN-α regimen with a regimen of INFERGEN® interferon alfacon-1 comprising administering a dosage of INFERGEN® interferon alfacon-1 containing an amount of 9 µg of drug per dose, subcutaneously once daily or three times per week for the desired treatment duration with an NS3 inhibitor compound.

As non-limiting examples, any of the above-described methods featuring an IFN-α regimen can be modified to replace the subject IFN-α regimen with a regimen of INFERGEN® interferon alfacon-1 comprising administering a dosage of INFERGEN® interferon alfacon-1 containing an amount of 15 µg of drug per dose, subcutaneously once daily or three times per week for the desired treatment duration with an NS3 inhibitor compound.

As non-limiting examples, any of the above-described methods featuring an IFN-γ regimen can be modified to replace the subject IFN-γ regimen with a regimen of IFN-γ comprising administering a dosage of IFN-γ containing an amount of 25 µg of drug per dose, subcutaneously three times per week for the desired treatment duration with an NS3 inhibitor compound.

As non-limiting examples, any of the above-described methods featuring an IFN-γ regimen can be modified to replace the subject IFN-γ regimen with a regimen of IFN-γ comprising administering a dosage of IFN-γ containing an amount of 50 µg of drug per dose, subcutaneously three times per week for the desired treatment duration with an NS3 inhibitor compound.

As non-limiting examples, any of the above-described methods featuring an IFN-γ regimen can be modified to replace the subject IFN-γ regimen with a regimen of IFN-γ comprising administering a dosage of IFN-γ containing an amount of 100 µg of drug per dose, subcutaneously three times per week for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 100 µg of drug per dose, subcutaneously once weekly, once every 8 days, or once every 10 days; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring a TNF antagonist regimen can be modified to replace the subject TNF antagonist regimen with a TNF antagonist regimen comprising administering a dosage of a TNF antagonist selected from the group of: (a) etanercept in an amount of 25 mg of drug per dose subcutaneously twice per week, (b) infliximab in an amount of 3 mg of drug per kilogram of body weight per dose intravenously at weeks 0, 2 and 6, and every 8 weeks thereafter, or (c) adalimumab in an amount of 40 mg of drug per dose subcutaneously once weekly or once every 2 weeks; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 100 µg of drug per dose, subcutaneously once weekly, once every 8 days, or once every 10 days; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 150 µg of drug per dose, subcutaneously once weekly, once every 8 days, or once every 10 days; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 150 µg of drug per dose, subcutaneously once weekly, once every 8 days, or once every 10 days; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 200 µg of drug per dose, subcutaneously once weekly, once every 8 days, or once every 10 days; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of monoPEG (30 kD, linear)-ylated consensus IFN-α containing an amount of 200 µg of drug per dose, subcutaneously once weekly, once every 8 days, or once every 10 days; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously three times per week; and (b) administering a dosage of IFN-γ containing an amount of 25 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously three times per week; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously three times per week; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously once daily; and (b) administering a dosage of IFN-γ containing an amount of 25 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously once daily; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 9 µg of drug per dose, subcutaneously once daily; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously three times per week; and (b) administering a dosage of IFN-γ containing an amount of 25 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously three times per week; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously three times per week; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously once daily; and (b) administering a dosage of IFN-γ containing an amount of 25 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously once daily; and (b) administering a dosage of IFN-γ containing an amount of 50 µg of drug per dose, subcutaneously three times per week; for the desired treatment duration with an NS3 inhibitor compound.

As non-limiting examples, any of the above-described methods featuring an IFN-α and IFN-γ combination regimen can be modified to replace the subject IFN-α and IFN-γ combination regimen with an IFN-α and IFN-γ combination regimen comprising: (a) administering a dosage of INFERGEN® interferon alfacon-1 containing an amount of 15 µg of drug per dose, subcutaneously once daily; and (b) administering a dosage of IFN-γ containing an amount of 100 µg of drug per dose, subcutaneously three times per week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 100 µg of drug per dose, subcutaneously once weekly, once every 8 days, or once every 10 days; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 100 µg of drug per dose, subcutaneously once weekly, once every 8 days, or once every 10 days; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 150 µg of drug per dose, subcutaneously once weekly, once every 8 days, or once every 10 days; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 150 µg of drug per dose, subcutaneously once weekly, once every 8 days, or once every 10 days; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 200 µg of drug per dose, subcutaneously once weekly, once every 8 days, or once every 10 days; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of monoPEG (30 kD, linear)-ylated consensus IFN-α containing an amount of 200 µg of drug per dose, subcutaneously once weekly, once every 8 days, or once every 10 days; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously three times per week; (b) administering a dosage of IFN-γ containing an amount of 25 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously three times per week; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously three times per week; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously once daily; (b) administering a dosage of IFN-γ containing an amount of 25 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously once daily; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 9 µg of drug per dose, subcutaneously once daily; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously three times per week; (b) administering a dosage of IFN-γ containing an amount of 25 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously three times per week; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously three times per week; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously once daily; (b) administering a dosage of IFN-γ containing an amount of 25 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-α, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously once daily; (b) administering a dosage of IFN-γ containing an amount of 50 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α, IFN-γ and TNF antagonist combination regimen can be modified to replace the subject IFN-a, IFN-γ and TNF antagonist combination regimen with an IFN-α, IFN-γ and TNF antagonist combination regimen comprising: (a) administering a dosage of INFERGEN® interferon alfacon-1 containing an amount of 15 µg of drug per dose, subcutaneously once daily; (b) administering a dosage of IFN-γ containing an amount of 100 µg of drug per dose, subcutaneously three times per week; and (c) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α and TNF antagonist combination regimen can be modified to replace the subject IFN-α and TNF antagonist combination regimen with an IFN-α and TNF antagonist combination regimen comprising: (a) administering a dosage of monoPEG (30 kD, linear)-ylated consensus IFN-α containing an amount of 100 µg of drug per dose, subcutaneously once weekly, once every 8 days, or once every 10 days; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α and TNF antagonist combination regimen can be modified to replace the subject IFN-α and TNF antagonist combination regimen with an IFN-α and TNF antagonist combination regimen comprising: (a) administering a dosage of monoPEG (30 kD, linear)-ylated consensus IFN-α containing an amount of 150 µg of drug per dose, subcutaneously once weekly, once every 8 days, or once every 10 days; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α and TNF antagonist combination regimen can be modified to replace the subject IFN-α and TNF antagonist combination regimen with an IFN-α and TNF antagonist combination regimen comprising: (a) administering a dosage of monoPEG (30 kD, linear)-ylated consensus IFN-α containing an amount of 200 µg of drug per dose, subcutaneously once weekly, once every 8 days, or once every 10 days; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α and TNF antagonist combination regimen can be modified to replace the subject IFN-α and TNF antagonist combination regimen with an IFN-α and TNF antagonist combination regimen comprising: (a) administering a dosage of INFERGEN® interferon alfacon-1 containing an amount of 9 µg of drug per dose, subcutaneously once daily or three times per week; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-α and TNF antagonist combination regimen can be modified to replace the subject IFN-α and TNF antagonist combination regimen with an IFN-α and TNF antagonist combination regimen comprising: (a) administering a dosage of INFERGEN® interferon alfacon-1 containing an amount of 15 µg of drug per dose, subcutaneously once daily or three times per week; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-γ and TNF antagonist combination regimen can be modified to replace the subject IFN-γ and TNF antagonist combination regimen with an IFN-γ and TNF antagonist combination regimen comprising: (a) administering a dosage of IFN-γ containing an amount of 25 µg of drug per dose, subcutaneously three times per week; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-γ and TNF antagonist combination regimen can be modified to replace the subject IFN-γ and TNF antagonist combination regimen with an IFN-γ and TNF antagonist combination regimen comprising: (a) administering a dosage of IFN-γ containing an amount of 50 µg of drug per dose, subcutaneously three times per week; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods featuring an IFN-γ and TNF antagonist combination regimen can be modified to replace the subject IFN-γ and TNF antagonist combination regimen with an IFN-γ and TNF antagonist combination regimen comprising: (a) administering a dosage of IFN-γ containing an amount of 100 µg of drug per dose, subcutaneously three times per week; and (b) administering a dosage of a TNF antagonist selected from (i) etanercept in an amount of 25 mg subcutaneously twice per week, (ii) infliximab in an amount of 3 mg of drug per kilogram of body weight intravenously at weeks 0, 2 and 6, and every 8 weeks thereafter or (iii) adalimumab in an amount of 40 mg subcutaneously once weekly or once every other week; for the desired treatment duration with an NS3 inhibitor compound.

As non-limiting examples, any of the above-described methods that includes a regimen of monoPEG (30 kD, linear)-ylated consensus IFN-α can be modified to replace the regimen of monoPEG (30 kD, linear)-ylated consensus IFN-α with a regimen of peginterferon alfa-2a comprising administering a dosage of peginterferon alfa-2a containing an amount of 180 µg of drug per dose, subcutaneously once weekly for the desired treatment duration with an NS3 inhibitor compound.

As non-limiting examples, any of the above-described methods that includes a regimen of monoPEG (30 kD, linear)-ylated consensus IFN-α can be modified to replace the regimen of monoPEG (30 kD, linear)-ylated consensus IFN-α with a regimen of peginterferon alfa-2b comprising administering a dosage of peginterferon alfa-2b containing an amount of 1.0 µg to 1.5 µg of drug per kilogram of body weight per dose, subcutaneously once or twice weekly for the desired treatment duration with an NS3 inhibitor compound.

As non-limiting examples, any of the above-described methods can be modified to include administering a dosage of ribavirin containing an amount of 400 mg, 800 mg, 1000 mg or 1200 mg of drug orally per day, optionally in two or more divided doses per day, for the desired treatment duration with an NS3 inhibitor compound.

As non-limiting examples, any of the above-described methods can be modified to include administering a dosage of ribavirin containing (i) an amount of 1000 mg of drug orally per day for patients having a body weight of less than 75 kg or (ii) an amount of 1200 mg of drug orally per day for patients having a body weight of greater than or equal to 75 kg, optionally in two or more divided doses per day, for the desired treatment duration with an NS3 inhibitor compound.

As non-limiting examples, any of the above-described methods can be modified to replace the subject NS3 inhibitor regimen with an NS3 inhibitor regimen comprising administering a dosage of 0.01 mg to 0.1 mg of drug per kilogram of body weight orally daily, optionally in two or more divided doses per day, for the desired treatment duration with the NS3 inhibitor compound.

As non-limiting examples, any of the above-described methods can be modified to replace the subject NS3 inhibitor regimen with an NS3 inhibitor regimen comprising administering a dosage of 0.1 mg to 1 mg of drug per kilogram of body weight orally daily, optionally in two or more divided doses per day, for the desired treatment duration with the NS3 inhibitor compound.

As non-limiting examples, any of the above-described methods can be modified to replace the subject NS3 inhibitor regimen with an NS3 inhibitor regimen comprising administering a dosage of 1 mg to 10 mg of drug per kilogram of body weight orally daily, optionally in two or more divided doses per day, for the desired treatment duration with the NS3 inhibitor compound.

As non-limiting examples, any of the above-described methods can be modified to replace the subject NS3 inhibitor regimen with an NS3 inhibitor regimen comprising administering a dosage of 10 mg to 100 mg of drug per kilogram of body weight orally daily, optionally in two or more divided doses per day, for the desired treatment duration with the NS3 inhibitor compound.

As non-limiting examples, any of the above-described methods featuring an NS5B inhibitor regimen can be modified to replace the subject NS5B inhibitor regimen with an NS5B inhibitor regimen comprising administering a dosage of 0.01 mg to 0.1 mg of drug per kilogram of body weight orally daily, optionally in two or more divided doses per day, for the desired treatment duration with an NS3 inhibitor compound.

As non-limiting examples, any of the above-described methods featuring an NS5B inhibitor regimen can be modified to replace the subject NS5B inhibitor regimen with an NS5B inhibitor regimen comprising administering a dosage of 0.1 mg to 1 mg of drug per kilogram of body weight orally daily, optionally in two or more divided doses per day, for the desired treatment duration with an NS3 inhibitor compound.

As non-limiting examples, any of the above-described methods featuring an NS5B inhibitor regimen can be modified to replace the subject NS5B inhibitor regimen with an NS5B inhibitor regimen comprising administering a dosage of 1 mg to 10 mg of drug per kilogram of body weight orally daily, optionally in two or more divided doses per day, for the desired treatment duration with an NS3 inhibitor compound.

As non-limiting examples, any of the above-described methods featuring an NS5B inhibitor regimen can be modified to replace the subject NS5B inhibitor regimen with an NS5B inhibitor regimen comprising administering a dosage of 10 mg to 100 mg of drug per kilogram of body weight orally daily, optionally in two or more divided doses per day, for the desired treatment duration with an NS3 inhibitor compound.

### Patient Identification

In certain embodiments, the specific regimen of drug therapy used in treatment of the HCV patient is selected according to certain disease parameters exhibited by the patient, such as the initial viral load, genotype of the HCV infection in the patient, liver histology and/or stage of liver fibrosis in the patient.

Thus, some embodiments provide any of the above-described methods for the treatment of HCV infection in which the subject method is modified to treat a treatment failure patient for a duration of 48 weeks.

Other embodiments provide any of the above-described methods for HCV in which the subject method is modified to treat a non-responder patient, where the patient receives a 48 week course of therapy.

Other embodiments provide any of the above-described methods for the treatment of HCV infection in which the subject method is modified to treat a relapser patient, where the patient receives a 48 week course of therapy.

Other embodiments provide any of the above-described methods for the treatment of HCV infection in which the subject method is modified to treat a naive patient infected with HCV genotype 1, where the patient receives a 48 week course of therapy.

Other embodiments provide any of the above-described methods for the treatment of HCV infection in which the subject method is modified to treat a naive patient infected with HCV genotype 4, where the patient receives a 48 week course of therapy.

Other embodiments provide any of the above-described methods for the treatment of HCV infection in which the subject method is modified to treat a naive patient infected with HCV genotype 1, where the patient has a high viral load (HVL), where "HVL" refers to an HCV viral load of greater than 2 x 10⁶ HCV genome copies per mL serum, and where the patient receives a 48 week course of therapy.

One embodiment provides any of the above-described methods for the treatment of an HCV infection, where the subject method is modified to include the steps of (1) identifying a patient having advanced or severe stage liver fibrosis as measured by a Knodell score of 3 or 4 and then (2) administering to the patient the drug therapy of the subject method for a time period of about 24 weeks to about 60 weeks, or about 30 weeks to about one year, or about 36 weeks to about 50 weeks, or about 40 weeks to about 48 weeks, or at least about 24 weeks, or at least about 30 weeks, or at least about 36 weeks, or at least about 40 weeks, or at least about 48 weeks, or at least about 60 weeks.

Another embodiment provides any of the above-described methods for the treatment of an HCV infection, where the subject method is modified to include the steps of (1) identifying a patient having advanced or severe stage liver fibrosis as measured by a Knodell score of 3 or 4 and then (2) administering to the patient the drug therapy of the subject method for a time period of about 40 weeks to about 50 weeks, or about 48 weeks.

Another embodiment provides any of the above-described methods for the treatment of an HCV infection, where the subject method is modified to include the steps of (1) identifying a patient having an HCV genotype 1 infection and an initial viral load of greater than 2 million viral genome copies per mL of patient serum and then (2) administering to the patient the drug therapy of the subject method for a time period of about 24 weeks to about 60 weeks, or about 30 weeks to about one year, or about 36 weeks to about 50 weeks, or about 40 weeks to about 48 weeks, or at least about 24 weeks, or at least about 30 weeks, or at least about 36 weeks, or at least about 40 weeks, or at least about 48 weeks, or at least about 60 weeks.

Another embodiment provides any of the above-described methods for the treatment of an HCV infection, where the subject method is modified to include the steps of (1) identifying a patient having an HCV genotype 1 infection and an initial viral load of greater than 2 million viral genome copies per mL of patient serum and then (2) administering to the patient the drug therapy of the subject method for a time period of about 40 weeks to about 50 weeks, or about 48 weeks.

Another embodiment provides any of the above-described methods for the treatment of an HCV infection, where the subject method is modified to include the steps of (1) identifying a patient having an HCV genotype 1 infection and an initial viral load of greater than 2 million viral genome copies per mL of patient serum and no or early stage liver fibrosis as measured by a Knodell score of 0, 1, or 2 and then (2) administering to the patient the drug therapy of the subject method for a time period of about 24 weeks to about 60 weeks, or about 30 weeks to about one year, or about 36 weeks to about 50 weeks, or about 40 weeks to about 48 weeks, or at least about 24 weeks, or at least about 30 weeks, or at least about 36 weeks, or at least about 40 weeks, or at least about 48 weeks, or at least about 60 weeks.

Another embodiment provides any of the above-described methods for the treatment of an HCV infection, where the subject method is modified to include the steps of (1) identifying a patient having an HCV genotype 1 infection and an initial viral load of greater than 2 million viral genome copies per mL of patient serum and no or early stage liver fibrosis as measured by a Knodell score of 0, 1, or 2 and then (2) administering to the patient the drug therapy of the subject method for a time period of about 40 weeks to about 50 weeks, or about 48 weeks.

Another embodiment provides any of the above-described methods for the treatment of an HCV infection, where the subject method is modified to include the steps of (1) identifying a patient having an HCV genotype 1 infection and an initial viral load of less than or equal to 2 million viral genome copies per mL of patient serum and then (2) administering to the patient the drug therapy of the subject method for a time period of about 20 weeks to about 50 weeks, or about 24 weeks to about 48 weeks, or about 30 weeks to about 40 weeks, or up to about 20 weeks, or up to about 24 weeks, or up to about 30 weeks, or up to about 36 weeks, or up to about 48 weeks.

Another embodiment provides any of the above-described methods for the treatment of an HCV infection, where the subject method is modified to include the steps of (1) identifying a patient having an HCV genotype 1 infection and an initial viral load of less than or equal to 2 million viral genome copies per mL of patient serum and then (2) administering to the patient the drug therapy of the subject method for a time period of about 20 weeks to about 24 weeks.

Another embodiment provides any of the above-described methods for the treatment of an HCV infection, where the subject method is modified to include the steps of (1) identifying a patient having an HCV genotype 1 infection and an initial viral load of less than or equal to 2 million viral genome copies per mL of patient serum and then (2) administering to the patient the drug therapy of the subject method for a time period of about 24 weeks to about 48 weeks.

Another embodiment provides any of the above-described methods for the treatment of an HCV infection, where the subject method is modified to include the steps of (1) identifying a patient having an HCV genotype 2 or 3 infection and then (2) administering to the patient the drug therapy of the subject method for a time period of about 24 weeks to about 60 weeks, or about 30 weeks to about one year, or about 36 weeks to about 50 weeks, or about 40 weeks to about 48 weeks, or at least about 24 weeks, or at least about 30 weeks, or at least about 36 weeks, or at least about 40 weeks, or at least about 48 weeks, or at least about 60 weeks.

Another embodiment provides any of the above-described methods for the treatment of an HCV infection, where the subject method is modified to include the steps of (1) identifying a patient having an HCV genotype 2 or 3 infection and then (2) administering to the patient the drug therapy of the subject method for a time period of about 20 weeks to about 50 weeks, or about 24 weeks to about 48 weeks, or about 30 weeks to about 40 weeks, or up to about 20 weeks, or up to about 24 weeks, or up to about 30 weeks, or up to about 36 weeks, or up to about 48 weeks.

Another embodiment provides any of the above-described methods for the treatment of an HCV infection, where the subject method is modified to include the steps of (1) identifying a patient having an HCV genotype 2 or 3 infection and then (2) administering to the patient the drug therapy of the subject method for a time period of about 20 weeks to about 24 weeks.

Another embodiment provides any of the above-described methods for the treatment of an HCV infection, where the subject method is modified to include the steps of (1) identifying a patient having an HCV genotype 2 or 3 infection and then (2) administering to the patient the drug therapy of the subject method for a time period of at least about 24 weeks.

Another embodiment provides any of the above-described methods for the treatment of an HCV infection, where the subject method is modified to include the steps of (1) identifying a patient having an HCV genotype 1 or 4 infection and then (2) administering to the patient the drug therapy of the subject method for a time period of about 24 weeks to about 60 weeks, or about 30 weeks to about one year, or about 36 weeks to about 50 weeks, or about 40 weeks to about 48 weeks, or at least about 24 weeks, or at least about 30 weeks, or at least about 36 weeks, or at least about 40 weeks, or at least about 48 weeks, or at least about 60 weeks.

Another embodiment provides any of the above-described methods for the treatment of an HCV infection, where the subject method is modified to include the steps of (1) identifying a patient having an HCV infection characterized by any of HCV genotypes 5, 6, 7, 8 and 9 and then (2) administering to the patient the drug therapy of the subject method for a time period of about 20 weeks to about 50 weeks.

Another embodiment provides any of the above-described methods for the treatment of an HCV infection, where the subject method is modified to include the steps of (1) identifying a patient having an HCV infection characterized by any of HCV genotypes 5, 6, 7, 8 and 9 and then (2) administering to the patient the drug therapy of the subject method for a time period of at least about 24 weeks and up to about 48 weeks.

### Subjects Suitable for Treatment

Any of the above treatment regimens can be administered to individuals who have been diagnosed with an HCV infection. Any of the above treatment regimens can be administered to individuals who have failed previous treatment for HCV infection ("treatment failure patients," including non-responders and relapsers).

Individuals who have been clinically diagnosed as infected with HCV are of particular interest in many embodiments. Individuals who are infected with HCV are identified as having HCV RNA in their blood, and/or having anti-HCV antibody in their serum. Such individuals include anti-HCV ELISA-positive individuals, and individuals with a positive recombinant immunoblot assay (RIBA). Such individuals may also, but need not, have elevated serum ALT levels.

Individuals who are clinically diagnosed as infected with HCV include naive individuals (e.g., individuals not previously treated for HCV, particularly those who have not previously received IFN-α-based and/or ribavirin-based therapy) and individuals who have failed prior treatment for HCV ("treatment failure" patients). Treatment failure patients include non-responders (i.e., individuals in whom the HCV titer was not significantly or sufficiently reduced by a previous treatment for HCV, e.g., a previous IFN-α monotherapy, a previous IFN-α and ribavirin combination therapy, or a previous pegylated IFN-α and ribavirin combination therapy); and relapsers (i.e., individuals who were previously treated for HCV, e.g., who received a previous IFN-α monotherapy, a previous IFN-α and ribavirin combination therapy, or a previous pegylated IFN-α and ribavirin combination therapy, whose HCV titer decreased, and subsequently increased).

In particular embodiments of interest, individuals have an HCV titer of at least about 10⁵, at least about 5 x 10⁵, or at least about 10⁶, or at least about 2 x 10⁶, genome copies of HCV per milliliter of serum. The patient may be infected with any HCV genotype (genotype 1, including 1a and 1b, 2, 3, 4, 6, etc. and subtypes (e.g., 2a, 2b, 3a, etc.)), particularly a difficult to treat genotype such as HCV genotype 1 and particular HCV subtypes and quasispecies.

Also of interest are HCV-positive individuals (as described above) who exhibit severe fibrosis or early cirrhosis (non-decompensated, Child's-Pugh class A or less), or more advanced cirrhosis (decompensated, Child's-Pugh class B or C) due to chronic HCV infection and who are viremic despite prior anti-viral treatment with IFN-α-based therapies or who cannot tolerate IFN-α-based therapies, or who have a contraindication to such therapies. In particular embodiments of interest, HCV-positive individuals with stage 3 or 4 liver fibrosis according to the METAVIR scoring system are suitable for treatment with the methods described herein. In other embodiments, individuals suitable for treatment with the methods of the embodiments are patients with decompensated cirrhosis with clinical manifestations, including patients with far-advanced liver cirrhosis, including those awaiting liver transplantation. In still other embodiments, individuals suitable for treatment with the methods described herein include patients with milder degrees of fibrosis including those with early fibrosis (stages 1 and 2 in the METAVIR, Ludwig, and Scheuer scoring systems; or stages 1, 2, or 3 in the Ishak scoring system.).

### Compound 100 and Precursor Compound

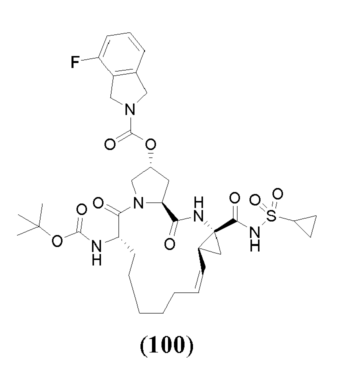

The compound **100** can be used in treating a hepatitis C infection. The compound **100** can reduce viral load, increase the rate of sustained viral response to therapy, and reduce morbidity or mortality in clinical outcomes. The compound **100** can be used in treating liver fibrosis (including forms of liver fibrosis resulting from, or associated with, HCV infection), generally involving administering a therapeutic amount of a compound **100,** and optionally one or more additional antiviral agents. The compound **100** can be used to treat a hepatitis C infection and to treat liver fibrosis in combination with ribavirin, levovirin, viramidine, ritonavir, alpha-glucosidase inhibitors, thymosin-α, interferon(s), pirfenidone, TNF-α antagonists, TNF-α antagonist and an interferon, and other antiviral agents.

The compound **100** is a synthetic macrocyclic molecule. Obtaining the compound **100** is dependent on developing efficient synthetic methods. Accordingly, some embodiments include novel methods for synthesizing the compound **100.**

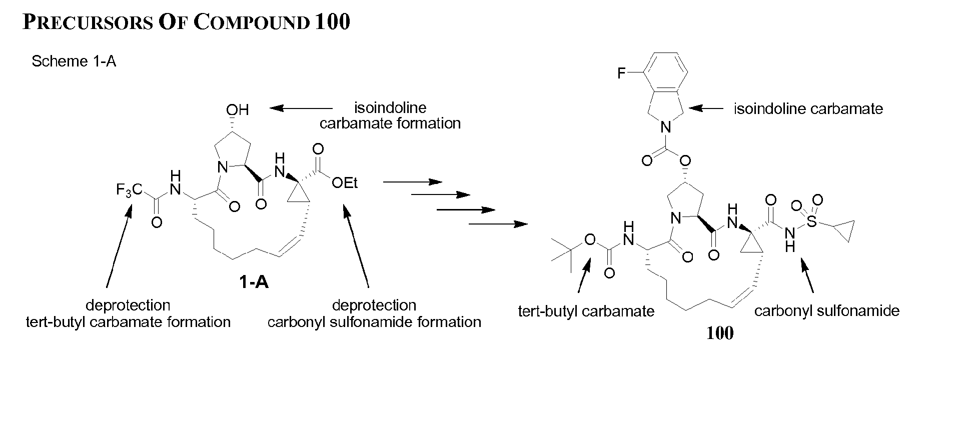

The macrocycle **1-A** can be considered as an important precursor to the compound 100 as shown in **Scheme 1-A.** The isoindoline carbamate formation, tert-butyl carbamate transformation and carbonyl sulfonamide transformation and be accomplished in any order to afford the compound 100. In some embodiments, the isoindoline carbamate can be formed first in the synthetic sequence. For example the isoindoline carbamate can be incorporated followed by tert-butyl carbamate incorporation and finally carbonyl sulfonamide incorporation. In some embodiments, the tert-butyl carbamate can be formed first in the synthetic sequence. For example, the tert-butyl carbamate can be incorporated followed by isoindoline carbamate formation and finally carbonyl sulfonamide incorporation. The formation of the tert-butyl carbamate and the carbonyl sulfonamide can be respectively accomplished after removal of the trifluoroacetyl protecting group and the hydrolysis of the ethyl ester under appropriate conditions. For example, the trifluoroacetyl can be removed by treatment of compound **1-A** with a stoichiometric amount of ethoxide in anhydrous ethanol to provide the free amine. Subsequent BOC-protection of the amine can provide an intermediate from which the compound 100 can be obtained following the method disclosed in U.S. Pat. App. No. 11/093884 filed March 29, 2005 incorporated herein in its entitety.

The macrocyclic ring of compound **1-A** can be formed by a macrolactamization reaction. The amide bond of the lactam can be potentially formed in two different places in compound **1-A,** position A and position B as indicated by the arrows in Scheme **1-B.**

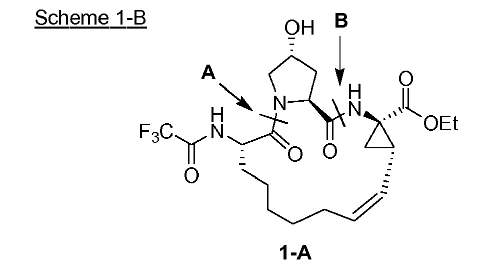

The formation of the macrocyclic ring of the compound **1-A** can be envisioned as being formed by a coupling between a carboxylic acid and as amine, as shown in **Scheme 1-C.** It can be envisioned, that the carboxylic acid of compound **1-B** can be activated by a coupling agent and subsequently the activated carboxylic acid can then self condense with the cyclic secondary amine to afford the compound **1-A**. In an analogous manner, the carboxylic acid of compound **1-C** can be activated by a coupling agent and subsequently the activated carboxylic acid can then self condense with the primary amine to afford the compound of formula **1-A**. For practicality, the carboxylic acid may be activated with the amine protected or unprotected, if protected the amine can be deprotected in situ and the reaction between the activated carboxylic acid and the free amine can then be allowed to proceed to afford the desired macrolactam of formula **1-A**.

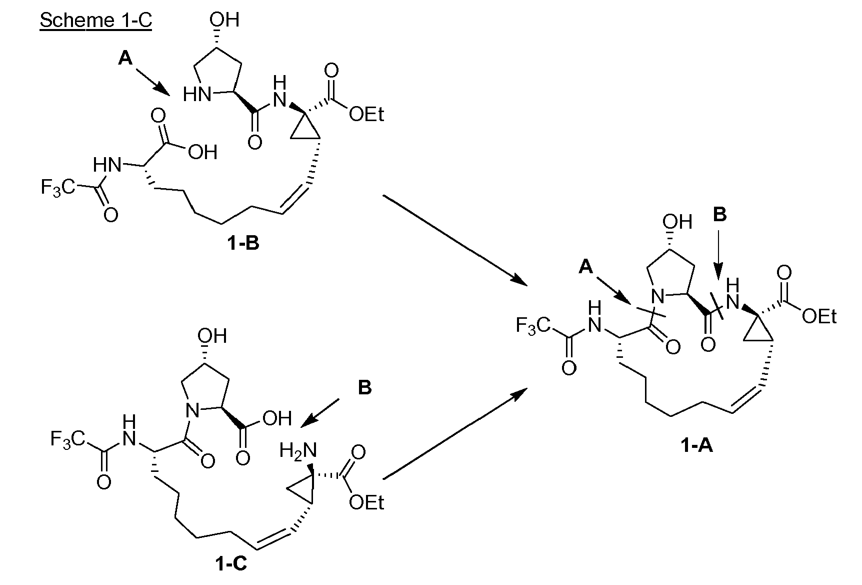

The carboxylic acid **1-E,** related to compound **1-B,** can be synthesized from the trimethylsilylethyl ester **1-D** by cleavage of the silyl protecting group, as shown in **Scheme 1-D.** For example, the silyl protecting group can be cleaved with TBAF to afford carboxylic acid **1-E.**

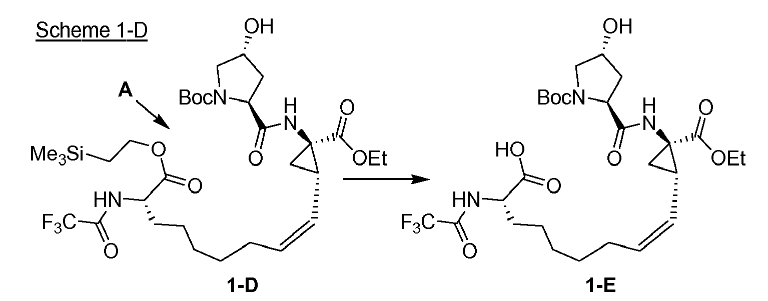

The carboxylic acid **1-E** can be converted to the macrocycle **1-A** by two routes, as shown in **Scheme 1-E**.

### Route 1:

In a typical embodiment, the carboxylic acid **1-E** can be converted to an active ester, such as a PFP ester **1-F.** Subsequently, the Boc protecting group can be removed and cyclization conditions can be applied to afford the macrocycle **1-A**. Alternatively, the carboxylic acid **1-E** can be converted to an acid chloride, mixed anhydride, acyl carbonate, and the like. Subsequently, the Boc protecting group can be removed and cyclization conditions can be applied to afford the macrocycle **1-A**.

### Route 2:

In a typical embodiment, the Boc protecting group of **1-E** can be removed under acidic conditions and subsequently a coupling reagent can eb added under appropriate conditions to afford the macrocycle **1-A**. For example, the Boc protecting group can be removed with hydrochloric acid in an ethereal solvent (e.g. HCl-dioxane) to afford the amine **1-G** as a hydrochloride salt, subsequently a coupling agent can be added under the appropriate conditions to afford the macrocycle **1-A**. For example, the HCl salt of **1-G** can be dissolved in a polar aprotic solvent, such as DMF, and then treated with a coupling agent, such as TBTU, HATU, HBTU, PyBOP, PyBrOP, and the like, to afford the macrocycle **1-A**. It will be appreciated that other coupling conditions may also be applicable to this system. For example, coupling agents such as chloroformates (e.g. isopropyl chloroformate), acid chlorides (e.g. Pivaloyl chloride), phosgene equivalents (e.g. CDI), carbodiimides (e.g. EDAC), and carbodiimides with HOBT, N-Hydroxysuccinimide, PFP and the like can be used in place of HATU.

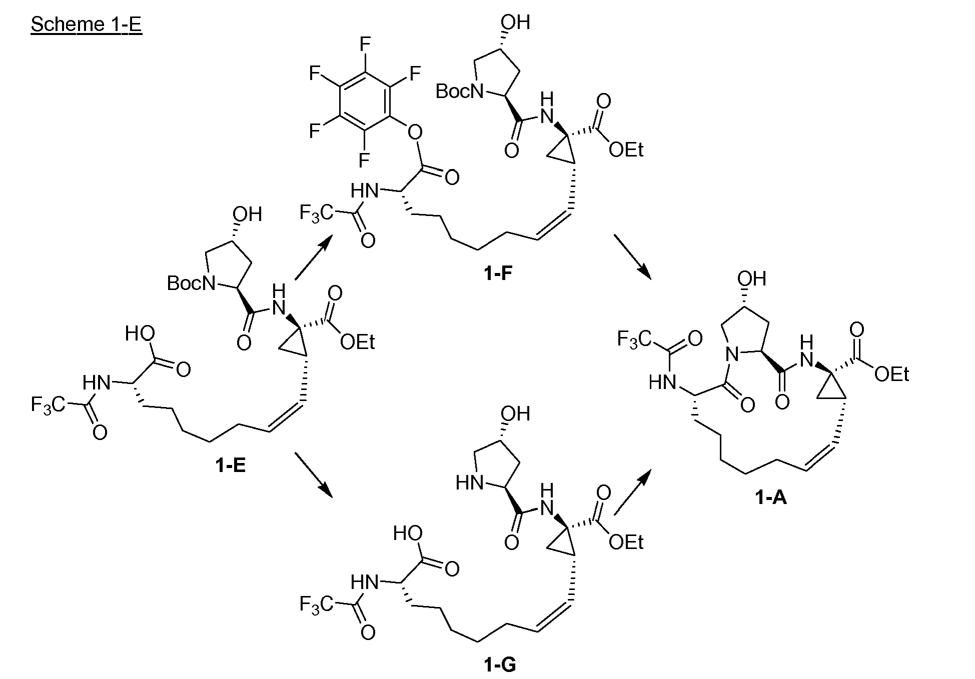

In some embodiments, alternative protecting group strategies can also be envisioned to afford compounds related to the compound of formula **1-A**. For example, a compound of the formula **1-A'** can be synthesized from the compound of the formula **1-E'** in a two step synthetic protocol, as shown in **Scheme 1-F.** The silyl protecting groups can be cleaved with TBAF to afford the carboxylic acid **1-G',** subsequently a coupling agent can be added under the appropriate conditions to afford the macrocycle **1-A'**. For example, the amine **1-G'** can be dissolved in a polar aprotic solvent, such as DMF, and then treated with a coupling agent, such as TBTU, HATU, HBTU, PyBOP, PyBrOP, EDAC-HCl with HOBT, DCC with HOBT and the like, to afford the macrocycle **1-A'**.

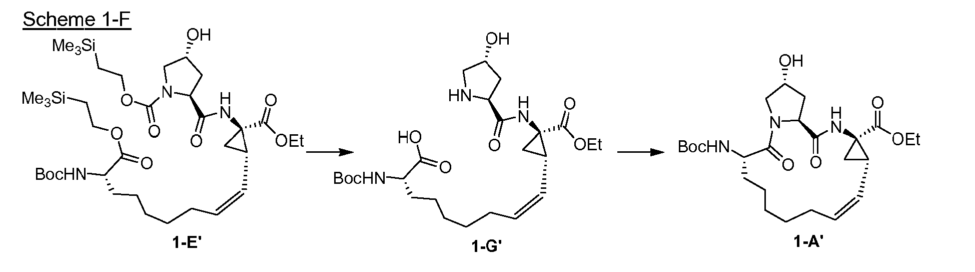

In some embodiments, the compound formula **1-A** can be synthesized from the BisBoc protected compound of formula **1-H,** related to compound **1-C,** in a two step protocol, as shown in **Scheme 1-G.** For example, in one embodiment, the trimethylsilylethyl ester in the compound of formula **1-H** can be cleaved with TBAF to afford the carboxylic acid of formula **1-I**. Subsequently, the Boc protecting groups can be removed to afford the compound of formula **1-J.** For example, in some embodiments, the Boc protecting groups can be removed with hydrochloric acid in an ethereal solvent (e.g. HCl-dioxane) to afford the HCl salt of the amine **1-J.** The compound of formula **1-J** can be treated with the appropriate coupling agents to afford the compound of formula **1-A**. For example, the HCl salt of **1-J** can be dissolved in a polar aprotic solvent, such as DMF, and then treated with a coupling agent, such as TBTU, HATU, HBTU, PyBOP, PyBrOP, and the like, to afford the macrocycle **1-A**. It will be appreciated that other coupling conditions may also be applicable to this system. For example, coupling agents such as chloroformates (e.g. isopropyl chloroformate), acid chlorides (e.g. Pivaloyl chloride), phosgene equivalents (e.g. CDI), carbodiimides (e.g. EDAC-HCl), and carbodiimides with HOBT, N-Hydroxysuccinimide, PFP and the like can be used in place of HATU.

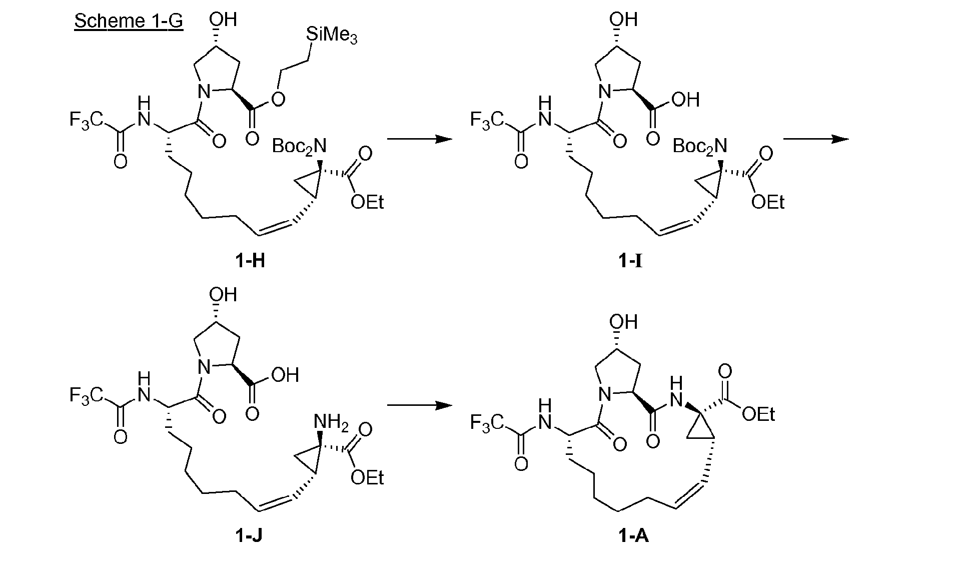

In some embodiments, alternative protecting group strategies can also be envisioned to afford compounds related to the compound **1-A**. For example, a compound of the formula **1-A'** can be synthesized from the compound of the formula **1-H'** in a two step synthetic protocol, as shown in **Scheme 1-I**. The silyl protecting groups can be cleaved with TBAF to afford the carboxylic acid **1-I'**, subsequently a coupling agent can be added under the appropriate conditions to afford the macrocycle **1-A'**. For example, the amine **1-I'** can be dissolved in a polar aprotic solvent, such as DMF, and then treated with a coupling agent, such as TBTU, HATU, HBTU, PyBOP, PyBrOP, EDAC-HCl with HOBT, DCC with HOBT and the like, to afford the macrocycle **1-A'**.

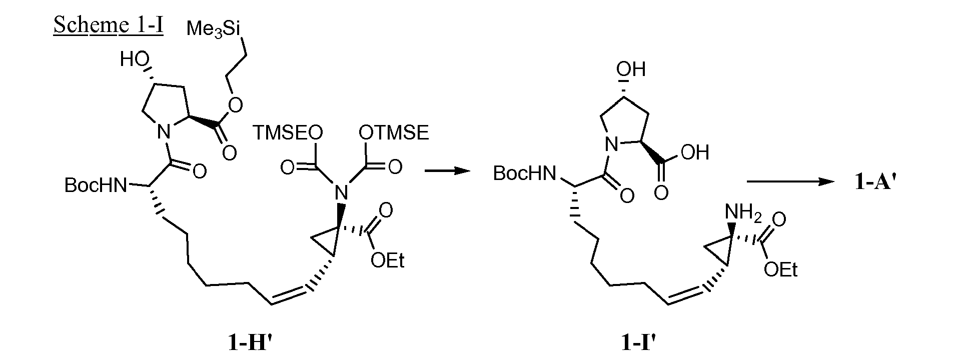

In some embodiments, the compound **1-D** and the compound **1-H** can be synthesized by the reduction of a carbon-carbon triple bond, as shown in **Scheme 2-A.**

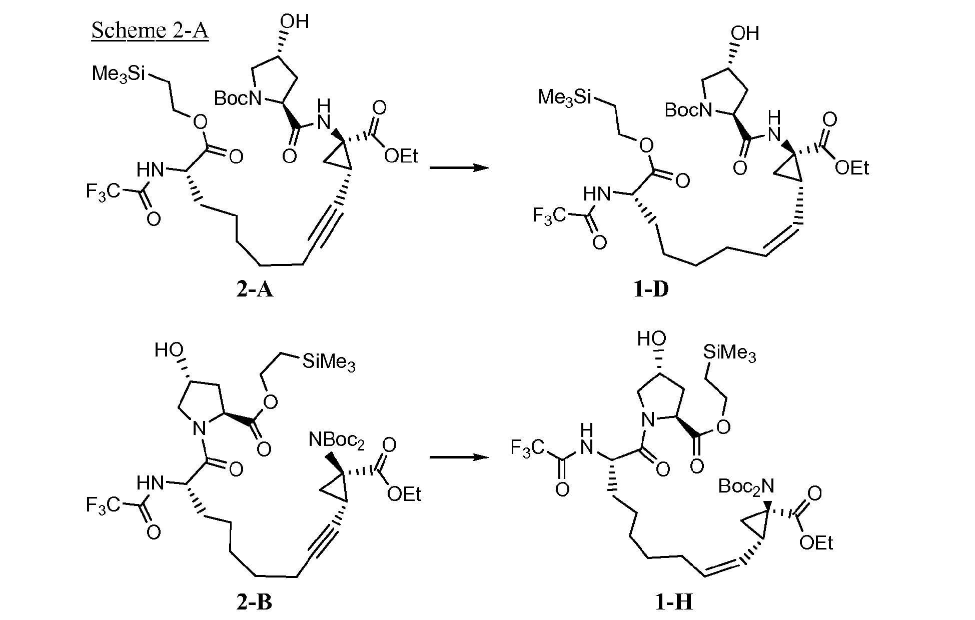

In an exemplary embodiment, the triple bond in the compound of formula **2-A** can be reduced with hydrogen gas over a catalyst. Typical catalysts for the selective hydrogenation of triple bonds to double bonds are Lindlar catalyst (Pd/CaCO₃:Pb poisoned), Lindlar catalyst with quinoline, Pd/CaCO₃ with quinoline, Pd/BaSO₄ with quinoline, Pd/CaCO₃ with pyridine, Pd/BaSO₄ with pyridine, and the like. For example, in a typical embodiment, the triple bond in the compound **2-A** can be reduced with hydrogen gas over Pd/BaSO₄ in the presence of quinoline.

In an exemplary embodiment, the triple bond in the compound **2-B** can be reduced with hydrogen gas over a catalyst. Typical catalysts for the selective hydrogenation of triple bonds to double bonds are Lindlar catalyst (Pd/CaCO₃:Pb poisoned), Lindlar catalyst with quinoline, Pd/CaCO₃ with quinoline, Pd/BaSO₄ with quinoline, Pd/CaCO₃ with pyridine, Pd/BaSO₄ with pyridine, and the like. For example, in a typical embodiment, the triple bond in the compound **2-B** can be reduced with hydrogen gas over Pd/BaSO₄ in the presence of quinoline.

In some embodiments, the common intermediate **3-A** can be used to synthesize the tripeptide of formula **2-A** and the tripeptide formula **2-B** in a two step procedure.

In an exemplary embodiment, the tripeptide of formula **2-A** can be synthesized from the orthogonally protected dipeptide of formula **3-A.** In one embodiment, the Boc protecting groups can be cleaved to afford the compound **3-B.** For example, in a typical embodiment, the Boc protecting groups can be removed with hydrochloric acid in an ethereal solvent (e.g. HCl-dioxane) to afford the HCl salt of the amine **3-B.** The HCl salt of the amine **3-B** can be treated with *N*-Boc 4-Hydroxyproline under the appropriate conditions to afford the compound **2-A.** For example, the HCl salt of **3-B** can be dissolved in a polar aprotic solvent, such as DMF, and then treated with *N*-Boc 4-Hydroxyproline and a coupling agent, such as TBTU, HATU, HBTU, PyBOP, PyBrOP, and the like, to afford the trmacrocycle **1-A**. In a typical embodiment, **3-B** can be dissolved in DMF, and then treated with *N*-Boc 4-Hydroxyproline and HATU in the presence of DIEA. It will be appreciated that other coupling conditions may also be applicable to this system. For example, coupling agents such as chloroformates (e.g. isopropyl chloroformate), acid chlorides (e.g. Pivaloyl chloride), phosgene equivalents (e.g. CDI), carbodiimides (e.g. EDAC-HCl), and carbodiimides with HOBT, N-Hydroxysuccinimide, PFP and the like can be used in place of HATU. Additionally, the order of addition of the coupling agents and substrates can be varied for optimization of yield.

In an exemplary embodiment, the tripeptide of formula **2-B** can be synthesized from the orthogonally protected dipeptide of formula **3-A.** In one embodiment, the silyl protecting group can be cleaved to afford the compound of formula **3-C.** For example, in a typical embodiment, the silyl protecting group can be removed with TBAF in an ethereal solvent (e.g. TBAF in THF) to afford the carboxylic acid **3-C.** The carboxylic acid **3-C** can be reacted with silyl protected 4-Hydroxyproline **(3-E)** under the appropriate conditions to afford the compound **2-B.** For example, the HCl salt of **3-E** can be treated with the carboxylic acid **3-C** and a coupling agent, such as TBTU, HATU, HBTU, PyBOP, PyBrOP, and the like, with DMF as the solvent to afford tripeptide **2-B.** In a typical embodiment, the HCl salt of **3-E** can be dissolved in DMF, and then treated with the carboxylic acid **3-C** and HATU in the presence of DIEA. It will be appreciated that other coupling conditions may also be applicable to this system. For example, coupling agents such as chloroformates (e.g. isopropyl chloroformate), acid chlorides (e.g. Pivaloyl chloride), phosgene equivalents (e.g. CDI), carbodiimides (e.g. EDAC-HCl), and carbodiimides with HOBT, N-Hydroxysuccinimide, PFP and the like can be used in place of HATU. Additionally, the order of addition of the coupling agents and substrates can be varied for optimization of yield.

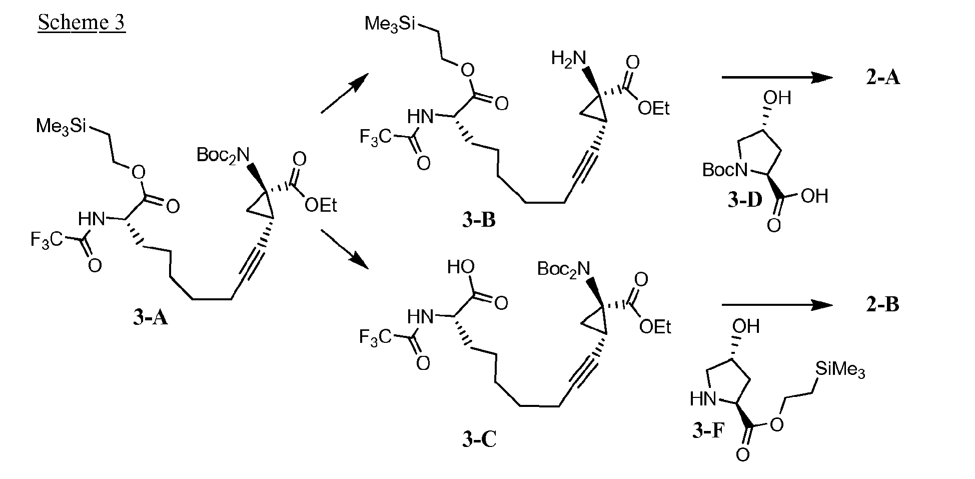

In some embodiments, as shown in **Scheme 3-A,** the compound of formula **3-F** can be synthesized from the compound of formula **3-E.** For example, the compound of formula **3-E** can be treated with acid in an ethereal solvent to afford the compound of formula **3-F.** In a typical embodiment, the compound of formula **3-E** can be treated with hydrochloric acid in dioxane to afford the compound of formula **3-F**

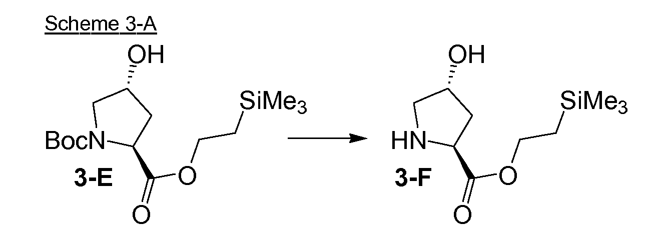

In some embodiments, the compound **3-A** can be synthesized using an alkyl halide of formula **4-A** and an alkyne of formula **4-B,** as seen in **Scheme 4.** In some embodiments, the alkyl halide **4-A** can be converted to an organometallic compound and then coupled with the alkyne **4-B** in the presence of catalyst. In a typical embodiment, the alkyl halide **4-A,** where **X** is I, can be converted to an organozinc compound and then coupled with the alkyne **4-B,** where **R¹** is Bromine, in the presence of CuLi-complex. For example, alkyl halide **4-A,** where **X** is I, can be added to zinc dust in THF to afford an organozinc intermediate, the organozinc intermediate can be then transferred to a solution of CuLi-complex (formed from CuCN and LiCl in THF). The organozinc-copper complex can be treated with the alkyne **4-B,** where **R¹** is Bromine, at reduced temperature and the reaction can be allowed to proceed until complete, upon completion of the reaction to compound of formula **3-A** can be isolated.

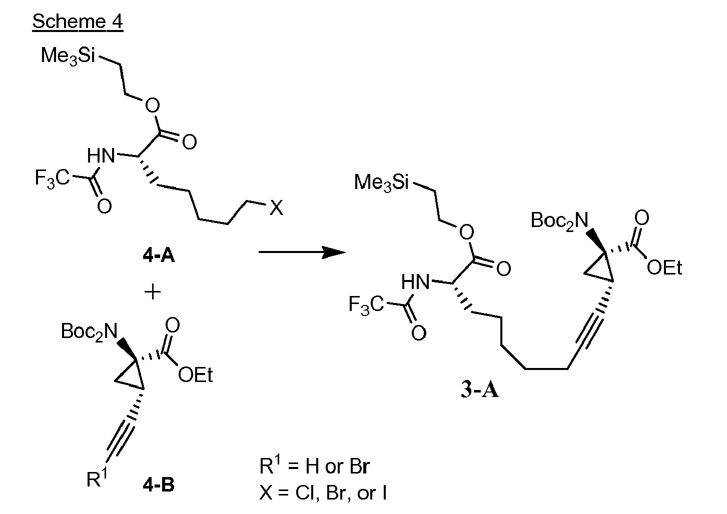

In some embodiments, the terminal hydrogen of the alkyne **4-B,** where **R¹** is Hydrogen, can be deprotonated with a base and then alkylated the alkyl halide **4-A** under appropriate conditions. For example, the base can be K₂CO₃, Cs₂CO₃ LDA, LiHMDS, KHMDS iPrMgX, EtMgX, PhMgX, Et₂Zn, BuLi, sec-BuLi, NaH, KH and the like. Additionally, in some embodiments, Palladium catalyzed alkylation of the alkyne **4-B,** where **R¹** is Hydrogen, can be accomplished using conditions developed by the Fu group (Eckhardt and Fu "The First Applications of Carbene Ligands in Cross-Couplings of Alkyl Electrophiles: Sonogashira Reactions of Unactivated Alkyl Bromides and Iodides" J. Am. Chem. Soc., 2003, 125, 13642-13643), which is incorporated herein by reference in its entirety.

In some embodiments, the alkyhalide **4-A** can be synthesized from (±)-Trimethyl Cbz-α-phosphonoglycinate **5-A** in a six step procedure, as shown in **Scheme 5.**

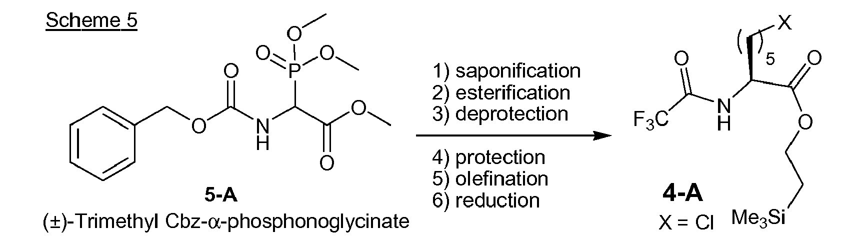

In a typical embodiment, the six step synthesis, as shown in **Scheme 5-A,** can provide the desired alkyhalide **4-A,** where **X** is chloride, in good overall yield and in high enantiomeric excess. The carboxylic acid ester **5-A** can be saponified using aqueous base. For example, in a typical embodiment, the carboxylic acid ester **5-A** can be saponified using aqueous NaOH in MeOH and acidified to afford the carboxylic acid **5-B.** The carboxylic acid **5-B** can be converted to the silyl ester **5-C** using 2-(trimethylsilyl)ethanol and appropriate coupling conditions. For example, in a typical embodiment, the carboxylic acid **5-B** can be converted to the silyl ester **5-C** using 2-(trimethylsilyl)ethanol with EDAC and DMAP in methylene chloride. The Cbz protecting group in the silyl ester 5-C can then be removed to afford the amino ester **5-D.** In a typical embodiment, the Cbz protecting group in the silyl ester **5-C** can be removed by hydrogenolysis. For example, the hydrogenolysis can be accomplished using 10% Pd/C with methanol as the solvent in the presence of TEA under a hydrogen atmosphere. The free amine of the amino ester **5-D** can then be protected to afford the trifluoroacetyl amide **5-E.** In a typical embodiment, the free amine can be converted to the trifluoroacetyl amide using TFAA in methylene chloride in the presence of DIEA. The trifluoroacetyl amide **5-E** can then be condensed with 5-chloropentanal to afford the α,β-unsaturated amino ester **5-F.** In a typical embodiment, the trifluoroacetyl amide **5-E** can be treated with NaH in THF and then 5-chloropentanal in toluene, prepared by reduction of methyl 5-chloropentanoate with DIBALH, can be carefully added to afford the α,β-unsaturated amino ester **5-F.** The stereogenic center of alkyl halide **5-G** can be generated by homogenous hydrogenation. In one embodiment, the stereogenic center of alkyl halide **5-G** can be generated by hydrogenation using Rh-(*S,S*)-Me-Duphos as the catalyst and methanol as the solvent. For example, the alkyl halide **5-G** can be isolated in 99 % yield and an ee of 99 % by hydrogenation of **5-F,** using Rh(NBD)₂BF₄ as the rhodium source and (*S,S*)-Me-Duphos as the chiral ligand in methanol. In some embodiments, alternative cationic and neutral rhodium catalysts can be used in the hydrogenation reaction. For example, Rh(COD)₂BF₄, Rh(COD)₂SO₃CF₃, Rh(NBD)₂SO₃CF₃, [Rh(COD)Cl]₂, [Rh(NBD)Cl]₂, and the like. In some embodiments, cationic and neutral Iridium catalysts can be used in the hydrogenation reaction. For example, Ir(COD)₂BF₄, Ir(COD)₂SO₃CF₃, Ir(NBD)₂SO₃CF₃, [Ir(COD)Cl]₂, [Ir(NBD)Cl]₂, and the like. In some embodiments, alternative chiral ligands can be used in the hydrogenation reaction. For example, the chiral ligands can be (*S,S,R,R*)-TANGPHOS, BINAP, (*R,R*)-DiPAMP, (*S,S*)-DiPAMP, DIOP, (R)-MeO-BIPHEP, (*R,R*)-Et-BPE, (*S,S*)-Et-BPE, (*R,R*)-Me-BPE, (*S,S*)-Me-BPE, SEGPHOS, and the like. Additional ligands and metal catalysts not listed above that are well know in the art can be used in the asymmetric hydrogenation reaction.

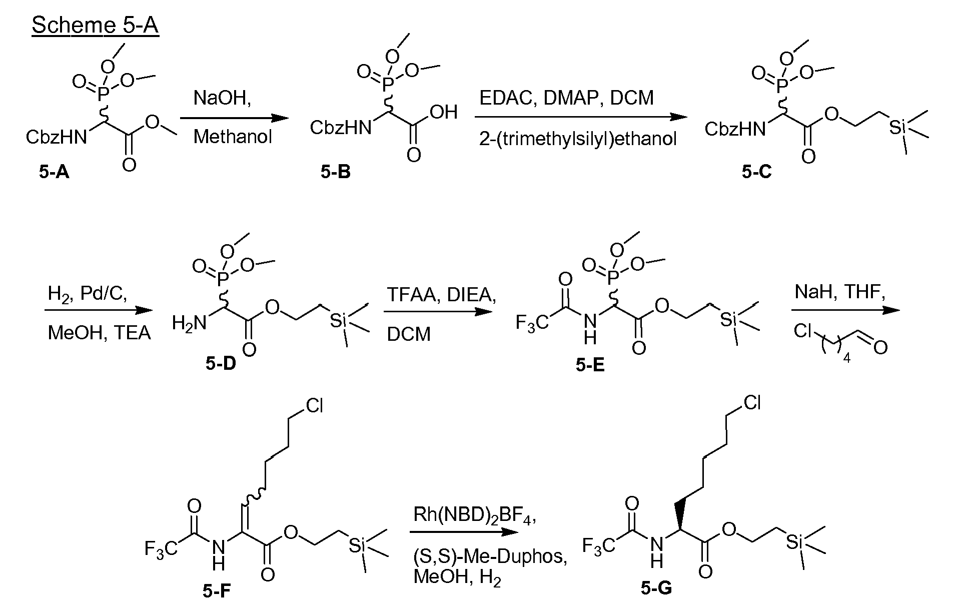

In some embodiments, the alkyl halide **5-G (4-A** where **X** is Cl) can be converted to the alkyl halide **5-H (4-A** where **X** is I) using a Finkelstein reaction. For example, the alkyl halide **5-G** can be heated with NaI in acetone to afford the alkyl halide **5-H** (Corey and Helal, "An Efficient Catalytic Stereoselective Route to a Key Intermediate for the Synthesis of the Long-Lived PGI2 Analog ZK 96480 (Cicaprost™)", Tetrahedron Lett., 1997, 43, 7511-7514), which is incorporated herein by reference in its entirety.

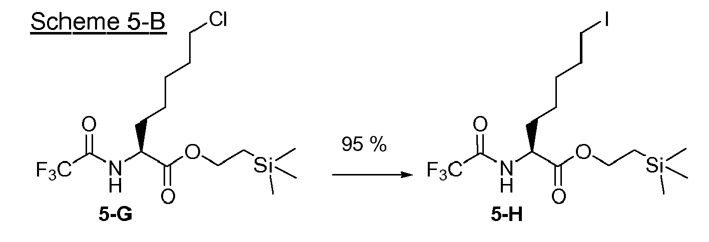

The intermediate **4-A,** where R¹ is hydrogen, can be synthesized from **6-A,** prepared according to the method of Beaulieu *et al*. (Beaulieu et al., "Synthesis of (1R,2S)-1-Amino-2-vinylcyclopropanecarboxylic Acid Vinyl-ACCA) Derivatives: Key Intermediates for the Preparation of Inhibitors of the Hepatitis C Virus NS3 Protease," J. Org. Chem. 2005, 70(15), 5869-5879 incorporated herein in its entirety, in a four step protocol, as shown in

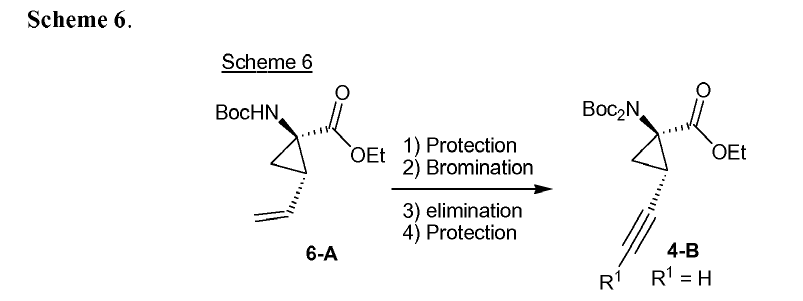

In a typical embodiment, the four step synthesis, as shown in **Scheme 6-A,** can provide the desired alkyne **6-E (4-B,** where **R¹** is hydrogen), in good overall yield. In one embodiment, the mono-Boc protected amino ester **6-A** can be converted to the bis-Boc protected amino ester **6-B.** In a typical embodiment, the mono-Boc protected amino ester **6-A** in MeCN can be treated with Boc₂O and DMAP to afford in the bis-Boc protected amino ester **6-B.** For example, Boc₂O (3 equiv.) and DMAP (0.3 equiv.) can be added to a solution of **6-A** in MeCN to afford the bis-Boc protected amino ester **6-B.** In some embodiments, the bis-Boc protected amino ester **6-B** can be treated with Br₂ afford the dibromide **6-C.** In a typical embodiment, the bis-Boc protected amino ester **6-B** can be treated with Br₂ in CCl₄ to afford the dibromide **6-C.** In some embodiments, the dibromide **6-C** can be treated with base to afford the mono-Boc protected alkyne **6-D.** For example, the base can be tert-BuOK. In a typical embodiment, a solution of the dibromide **6-C** in THF can be treated with be tert-BuOK (4 equiv. in THF) to afford the mono-Boc protected alkyne **6-D.** In a typical embodiment, the mono-Boc protected alkyne **6-D** dissolved in MeCN can be treated with Boc₂O and DMAP to afford in the bis-Boc protected alkyne **6-E.** For example, Boc₂O (3 equiv.) and DMAP (0.2 equiv.) can be added to a solution of **6-A** in MeCN to afford the bis-Boc protected alkyne **6-E (4-B,** where **R¹** is hydrogen) in high yield.

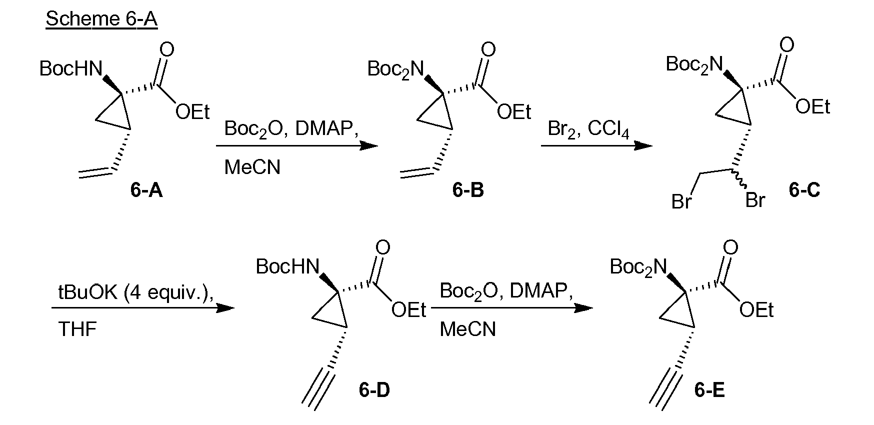

In some embodiments, the terminal alkyne **6-E** can be further converted to the alkynyl bromide **6-F (4-B** where **R¹** is Bromine). In typical embodiment, as shown in **Scheme 6-B,** NBS and AgNO₃ can be added to a solution of **6-E** in acetone to afford the alkynyl bromide **6-F (4-B** where **R¹** is Bromine), (Yoo et al., "Rhodium-Catalyzed Intramolecular [4 + 2] Cycloadditions of Alkynyl Halides," Org. Lett., 2005, 7 (26), 5853 - 5856), which is incorporated herein by reference in its entirety.

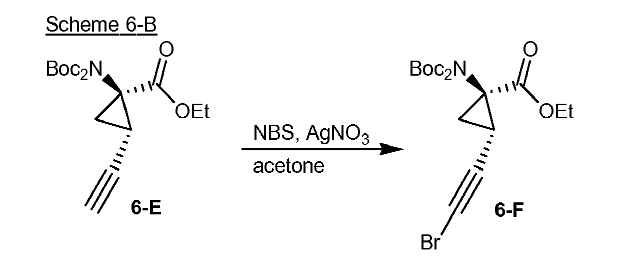

### Methods of Administering an Inhibitor of hepatitis C virus (HCV) Infection

A single ascending dose (SAD) study of the compound **100** was conducted in healthy volunteers. The compound 100 was administered as monotherapy both with and without food. The safety and pharmacokinetic profile of the single dose of the compound 100 was assessed.

Plasma levels of the compound 100 were observed in all dose groups and all doses were well tolerated following administration of the single dose. A higher than anticipated exposure was observed when the compound 100 was administered with food, as compared to when it was administered without food.

U.S. Patent Publication No. 2005-0267018-A1, published December 1, 2005, is hereby incorporated herein by reference, and particularly for the purpose of providing descriptions of various terms used herein.

One embodiment provides a method of administering an inhibitor of hepatitis C virus (HCV) infection, comprising administering to a patient an effective amount of a compound 100, or a pharmaceutically acceptable salt, ester or prodrug thereof, wherein the administering is undertaken in conjunction with the consumption of food by the patient.

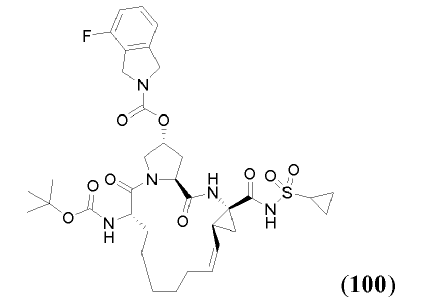

In some embodiments, the compound **100,** or the pharmaceutically acceptable salt, ester or prodrug thereof, may be administered to a patient by orally administering a pharmaceutical composition comprising the compound **100,** or the pharmaceutically acceptable salt, ester or prodrug thereof. In some embodiments, the pharmaceutical composition comprises a pharmaceutically acceptable salt of the compound 100. In some embodiments, the pharmaceutically acceptable salt of compound **100** is a sodium salt.

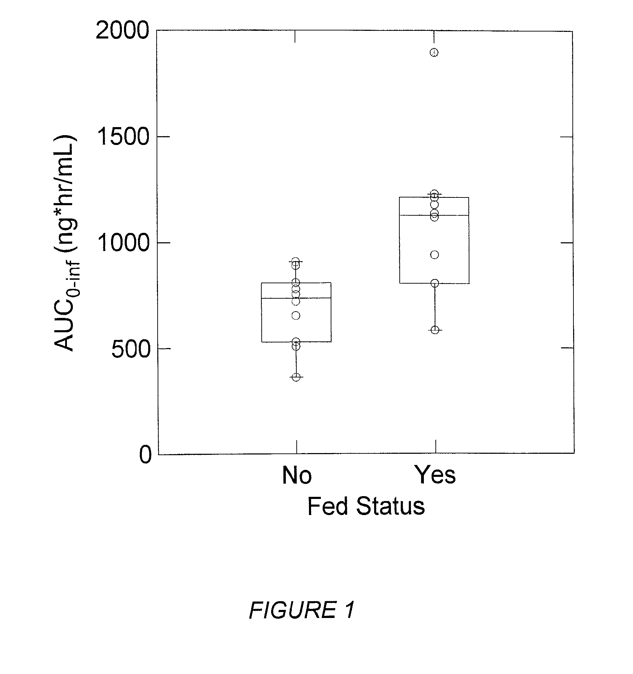

As shown in Figure 1, when the administering of compound 100, or the pharmaceutically acceptable salt, ester or prodrug thereof, is undertaken in conjunction with the consumption of food by the patient, the area under the plasma concentration-time curve (AUC_{0-inf} after a single dose or AUC₀₋₂₄ at steady-state) for the compound 100, or active metabolite thereof is increased. The consumption of food by the patient is effective to provide AUC_{0-inf} or AUC₀₋₂₄ that is greater than when the administering is not undertaken in conjunction with the consumption of food by the patient. In some embodiments, the consumption of food by the patient is undertaken substantially simultaneously with the administration of the compound 100, or the pharmaceutically acceptable salt, ester or prodrug thereof.

Another embodiment provides a method of administering an inhibitor of hepatitis C virus (HCV) infection, comprising administering to a patient an effective amount of a compound 100, or a pharmaceutically acceptable salt, ester or prodrug thereof; and providing information to the patient indicating that the administering of the compound 100, or the pharmaceutically acceptable salt, ester or prodrug thereof, should be accompanied by the consumption of food. In some embodiment, the compound 100, or a pharmaceutically acceptable salt, ester or prodrug thereof, is administered to a patient by orally administering a pharmaceutical composition comprising the compound 100, or a pharmaceutically acceptable salt, ester or prodrug thereof. In some embodiments, the pharmaceutical composition comprises a pharmaceutically acceptable salt of the compound 100. In some embodiments, the pharmaceutically acceptable salt may be a sodium salt.

Another embodiment provides a method of distributing an oral dosage form, comprising distributing a pharmaceutical composition, wherein the pharmaceutical composition comprises a compound 100, or a pharmaceutically acceptable salt, ester or prodrug thereof; and concomitantly distributing information that the administering of the pharmaceutical composition should be accompanied by the consumption of food.

Another embodiment provides a compound having the structure of Formula I: 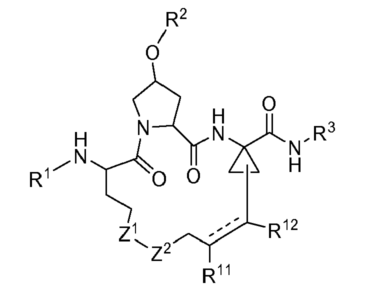 or a pharmaceutically acceptable salt or prodrug thereof wherein:
R¹ is selected from the group consisting of substituted aryl, substituted heteroaryl, - C(O)OR⁴, -C(O)NR⁵R⁶, -C(O)R⁷, and 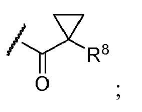
R² is selected from the group consisting of alkyl, -C(O)-alkyl, 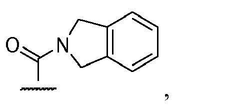 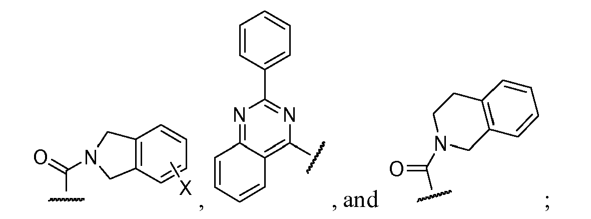
R³ is selected from the group consisting of -OR⁹ and -SO₂R¹⁰;
R⁴ is selected from the group consisting of alkyl, heterocyclyl, and aryl;
R⁵ is alkyl and R⁶ is selected from the group consisting of alkyl and aralkyl, or R⁵ together with R⁶ form an optionally substituted heterocyclyl or optionally substituted heteroaryl;
R⁷ is phenyl substituted one or more times with halogen;
R⁸ is selected from the group consisting of -CF₃ and methyl;
R⁹ is selected from the group consisting of hydrogen, optionally substituted alkyl, optionally substituted aryl, and optionally substituted aralkyl;
R¹⁰ is selected from the group consisting of alkyl optionally substituted with alkoxy or alkenyl, optionally substituted aryl, optionally substituted aralkyl, substituted heteroaryl, and 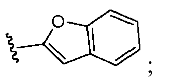
R¹¹ and R¹² are each hydrogen or together with the carbon atoms to which they are attached form an optionally substituted cycloalkyl;
X is halogen and is present 1 to 4 times; and
Z¹ and Z² are independently selected from the group consisting of -CH₂-, -CF₂-, and -O-provided that at least one of Z¹ and Z² is -CH₂-;
provided that if R¹¹ and R¹² are each hydrogen, R² is alkyl, and R³ is -SO₂-cyclopropyl, then R¹ is not -C(O)O-t-butyl;
provided that if R¹¹ and R¹² are each hydrogen and R² is 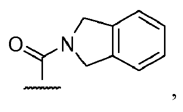 then R³ is -SO₂-phenyl disubstituted with halogen or -SO₂-thiophene disubstituted with halogen, or R¹ is 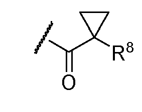 where R⁸ is -CF₃;
provided that if R¹¹ and R¹² are each hydrogen, R² is 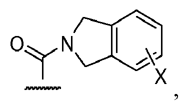 and R³ is -SO₂-cyclopropyl, then R¹ is not -C(O)O-t-butyl, -C(O)O-haloalkyl, or -C(O)O-cyclopentyl;
provided that if R¹¹ and R¹² are each hydrogen, R² is 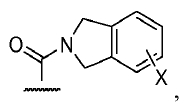 and R³ is substituted -SO₂-heteroaryl, then R¹ is not -C(O)O-cyclopentyl;
provided that if R¹¹ and R¹² are each hydrogen, R² is 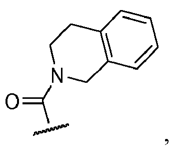 and R³ is -SO₂-cyclopropyl, then R¹ is not -C(O)O-alkyl or -C(O)O-heterocyclyl;
provided that if R¹¹ and R¹² are each hydrogen, R² is 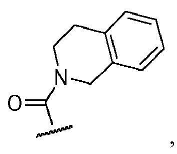 and R³ is -SO₂-alkyl or optionally substituted -SO₂-aryl, then R¹ is not -C(O)O-cycloalkyl;
provided that if R¹¹ and R¹² are each hydrogen, R² is 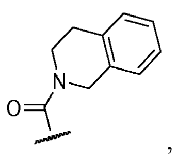 and R³ is optionally substituted -SO₂-phenyl, then R¹ is not -C(O)O-t-butyl;
provided that if R¹¹ and R¹² are each hydrogen, R² is 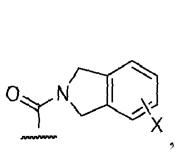 and R³ is -SO₂-alkyl substituted with alkoxy or alkenyl, then R¹ is -C(O)O-t-butyl and X is F; and
provided that the compound of formula (I) is not selected from the group consisting of:

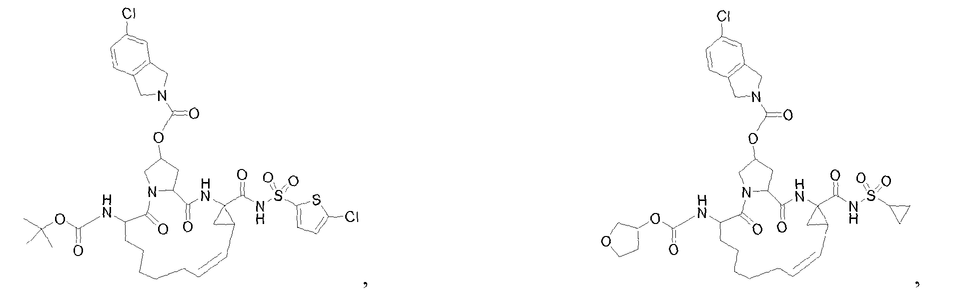

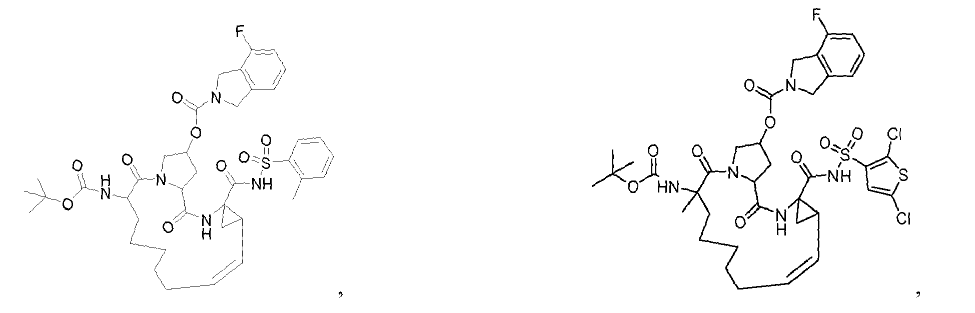

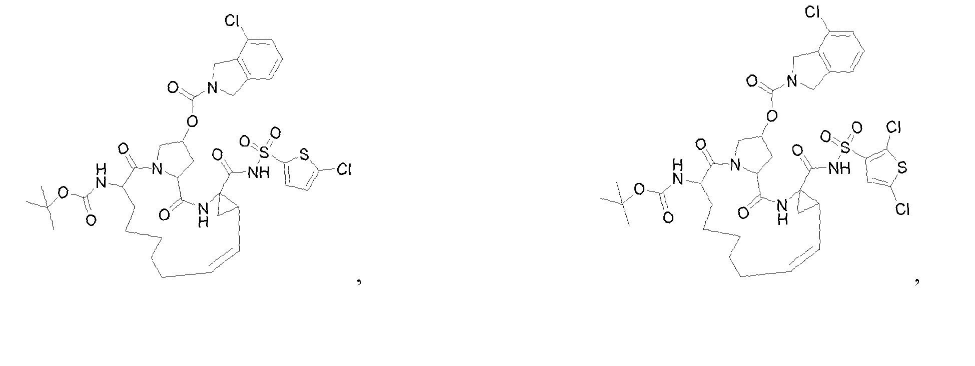

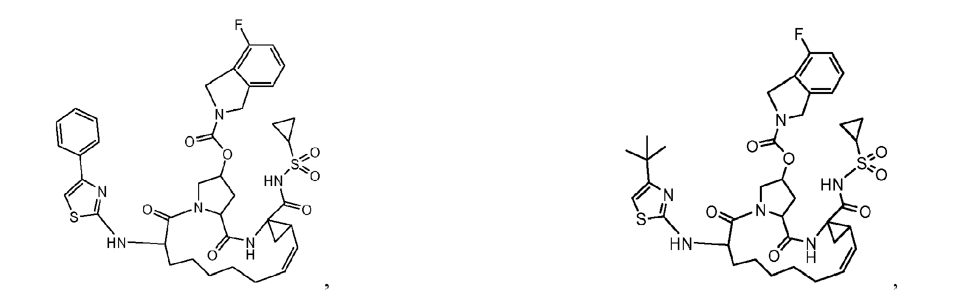

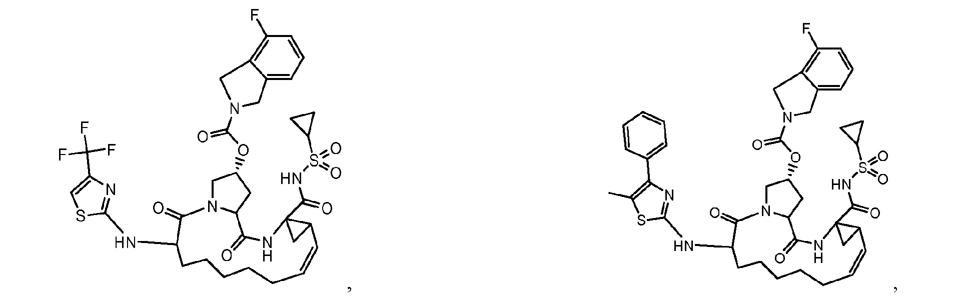

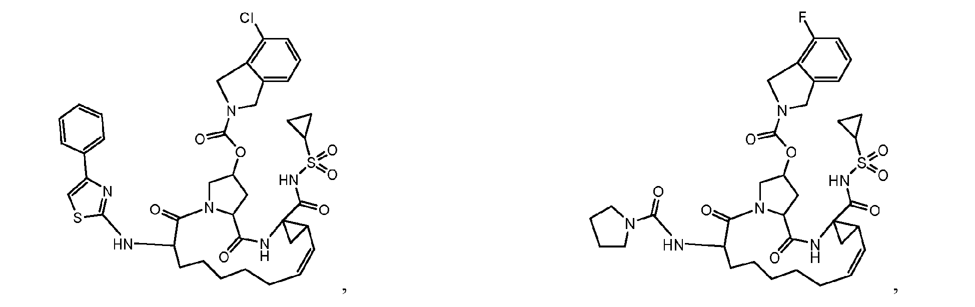

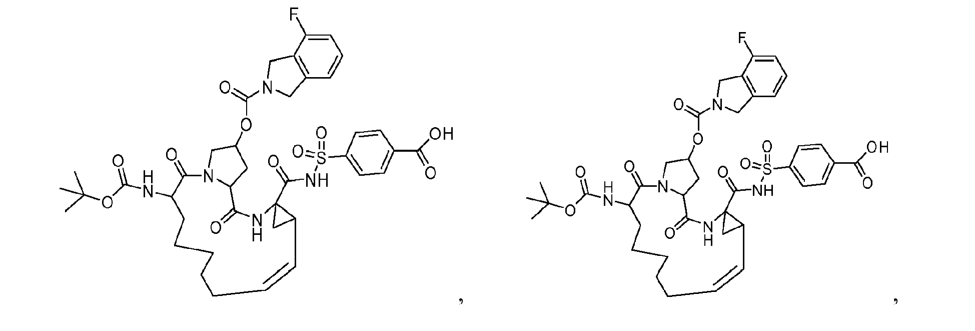

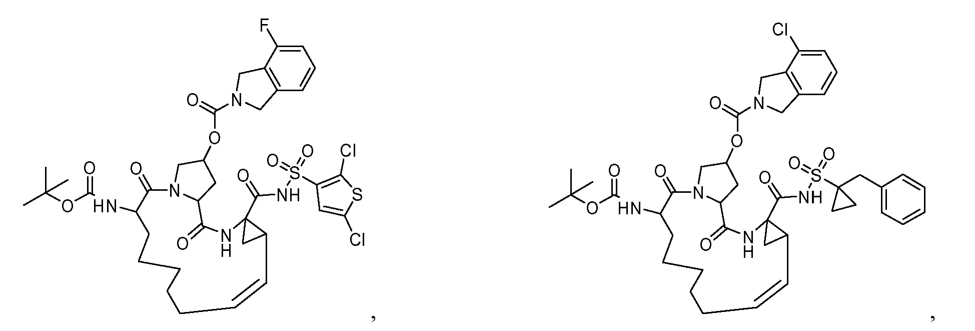

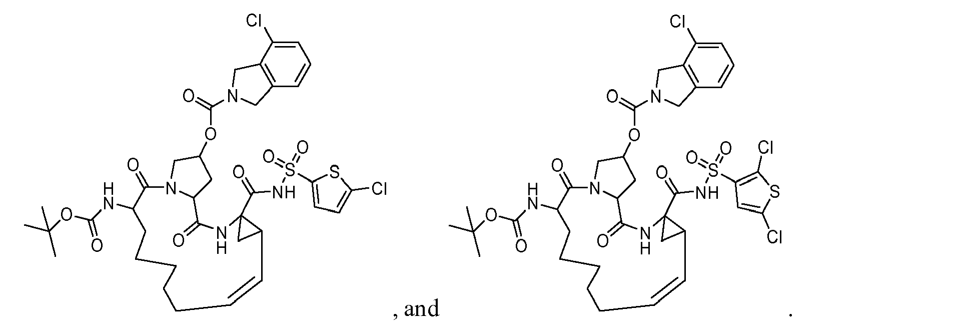

More preferably, the present invention relates to a compound according to the present invention wherein R¹ is -C(O)OR⁴. More preferably R⁴ is selected from the group consisting of alkyl, tetrahydrofuranyl, tetrahydropyranyl, and phenyl. According to another embodiment the present invention relates to a compound according to the present invention wherein R⁴ is tert-butyl.

According to another embodiment, the present invention relates to a compound according to the present invention wherein R¹ has the structure: 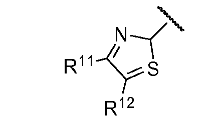 wherein R¹¹ and R¹² are independently selected from the group consisting of hydrogen, optionally substituted alkyl, optionally substituted aryl, and optionally substituted heteroaryl, or R¹¹ and R¹² together form a cycloalkyl, provided that at least one of R¹¹ and R¹² is not hydrogen. More preferably R¹¹ and R¹² are independently selected from the group consisting of hydrogen; alkyl; phenyl optionally substituted with one or more of halogen, - CN, -SO₂CH₃, -CF₃, and -OCF₃; pyridine optionally substituted with one or more halogen; and benzothiazole; or R¹¹ and R¹² together form a cyclopentyl, provided that at least one of R¹¹ and R¹² is not hydrogen.

According to another embodiment, the present invention relates to a compound according to the present invention wherein R¹ is -C(O)NR⁵R⁶. More preferably R⁵ is methyl and R⁶ is alkyl or benzyl. According to another embodiment the present invention relates to a compound according to the present invention wherein R⁵ together with R⁶ form an optionally substituted heterocyclyl or optionally substituted heteroaryl selected from the group consisting of N-morphlino, N-heterocyclyl optionally substituted with one or more halogen, and N-isoindolinyl.

According to another embodiment, the present invention relates to a compound according to the present invention wherein R¹ is 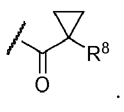 More preferably R⁸ is -CF₃ or methyl.

According to another embodiment, the present invention relates to a compound according to the present invention wherein R¹ is phenyl substituted with one or more halogen. According to another embodiment, the present invention relates to a compound according to the present invention wherein R¹ is -C(O)R⁷. More preferably wherein R⁷ is selected from the group consisting of phenyl substituted with one or more halogen.

According to another embodiment, the present invention relates to any compound according to the present invention wherein R³ is -OR⁹. More preferably R⁹ is selected from the group consisting of hydrogen, alkyl optionally substituted with hydroxy, phenyl, and benzyl optionally substituted with -CF₃.

According to another embodiment, the present invention relates to any compound according to the present invention wherein R³ is -SO₂R¹⁰. More preferably R¹⁰ is selected from the group consisting of alkyl; phenyl optionally substituted with one or more of methyl, halogen, carboxy, CF₃, and alkoxy; and thiophene substituted with one or more of alkyl and halogen. And more preferably R¹⁰ is cyclopropyl.

According to another embodiment, the present invention relates to any compound according to the present invention wherein:
R¹⁰ is selected from the group consisting of alkyl, optionally substituted aryl, optionally substituted aralkyl, and substituted heteroaryl;
R¹¹ and R¹² are each hydrogen; and
Z² is -CH₂-.

According to another embodiment, the present invention relates to any of the compounds having a formula selected from the group consisting of the formulas of compound numbers 101-907. More preferably, the present invention relates to a compound having a formula 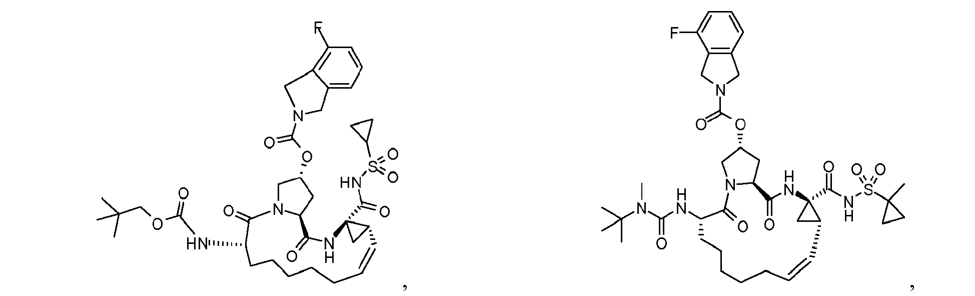 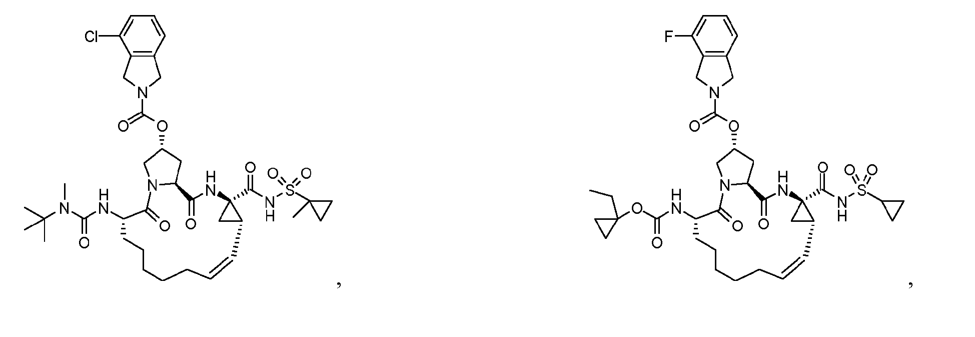 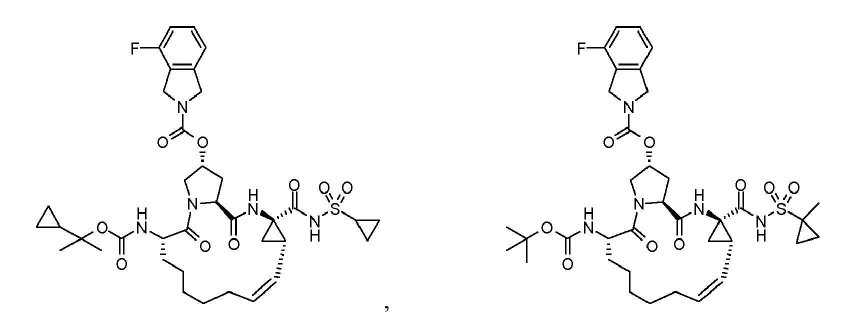 or

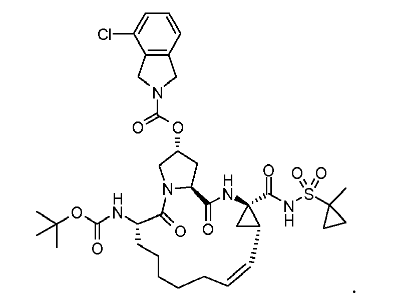

According to another embodiment, the present invention relates to a pharmaceutical composition comprising a pharmaceutically acceptable excipient and a compound according to the present invention.

According to another embodiment, the present invention relates to a method of inhibiting NS3/NS4 protease activity comprising contacting a NS3/NS4 protease with a compound or pharmaceutical composition according to the present invention. More preferably said contacting is conducted in vivo. The method according to the present invention preferably further comprises the step of identifying a subject suffering from a hepatitis C infection and administering the compound to the subject in an amount effective to treat the infection. More preferably the method according to the present invention further comprises administering to the individual an effective amount of a nucleoside analog, wherein preferably said nucleoside analog is selected from ribavirin, levovirin, viramidine, an L-nucleoside, and isatoribine. According to another embodiment, the method further comprises administering to the individual an effective amount of a human immunodeficiency virus 1 protease inhibitor, wherein preferably said protease inhibitor is ritonavir. According to another embodiment, the method further comprises administering to the individual an effective amount of an NS5B RNA-dependent RNA polymerase inhibitor. Preferably the method further comprises administering to the individual an effective amount of interferon-gamma (IFN-γ) said IFN-γ being preferably administered subcutaneously in an amount of from about 10 µg to about 300 µg. According to another embodiment, the method further comprises administering to the individual an effective amount of interferon-alpha (IFN-α). More preferably the IFN-α is monoPEG-ylated consensus IFN-α administered at a dosing interval of every 8 days to every 14 days. According to another embodiment, the IFN-α is monoPEG-ylated consensus IFN-α administered at a dosing interval of once every 7 days. Preferably said IFN-α is INFERGEN consensus IFN-α.

According to another embodiment, the method of the present invention further comprises the step of administering an effective amount of an agent selected from 3'-azidothymidine, 2',3'-dideoxyinosine, 2',3'-dideoxycytidine, 2',3'-didehydro-2',3'-dideoxythymidine, combivir, abacavir, adefovir dipoxil, cidofovir, and an inosine monophosphate dehydrogenase inhibitor. According to another embodiment, the method provides that a sustained viral response is achieved. According to yet another embodiment, the method of the present invention provides that the contacting is conducted ex vivo.

According to another embodiment, the present invention relates to a method of treating liver fibrosis in an individual, the method comprising administering to the individual an effective amount of a compound or pharmaceutical composition according to the present invention. More preferably the method further comprises administering to the individual an effective amount of a nucleoside analog, wherein the nucleoside analog is preferably selected from ribavirin, levovirin, viramidine, an L-nucleoside, and isatoribine. According to another embodiment the method further comprises administering to the individual an effective amount of a human immunodeficiency virus 1 protease inhibitor wherein the protease inhibitor is preferably ritonavir. According to another embodiment the method further comprises administering to the individual an effective amount of an NS5B RNA-dependent RNA polymerase inhibitor. According to another embodiment wherein the method further comprises administering to the individual an effective amount of interferon-gamma (IFN-γ). Said IFN-γ is preferably administered subcutaneously in an amount of from about 10 µg to about 300 µg. According to another embodiment the method further comprises administering to the individual an effective amount of interferon-alpha (IFN-α). Said IFN-α is preferably monoPEG-ylated consensus IFN-α administered at a dosing interval of every 8 days to every 14 days. According to another embodiment, said IFN-α is monoPEG-ylated consensus IFN-α administered at a dosing interval of once every 7 days. Preferably said IFN-α is INFERGEN consensus IFN-α. According to another embodiment, the method further comprises administering an effective amount of an agent selected from 3'-azidothymidine, 2',3'-dideoxyinosine, 2',3'-dideoxycytidine, 2',3'-didehydro-2',3'-dideoxythymidine, combivir, abacavir, adefovir dipoxil, cidofovir, and an inosine monophosphate dehydrogenase inhibitor.

According to another embodiment, the present invention relates to a method of increasing liver function in an individual having a hepatitis C virus infection, the method comprising administering to the individual an effective amount of a compound compound or pharmaceutical composition according to the present invention. More preferably the method further comprises administering to the individual an effective amount of a nucleoside analog, wherein the nucleoside analog is preferably selected from ribavirin, levovirin, viramidine, an L-nucleoside, and isatoribine. According to another embodiment wherein the method further comprises administering to the individual an effective amount of a human immunodeficiency virus 1 protease inhibitor wherein the protease inhibitor is preferably ritonavir. According to another embodiment the method further comprises administering to the individual an effective amount of an NS5B RNA-dependent RNA polymerase inhibitor. According to another embodiment wherein the method further comprises administering to the individual an effective amount of interferon-gamma (IFN-γ), preferably subcutaneously in an amount of from about 10 µg to about 300 µg. According to another embodiment wherein the method further comprises administering to the individual an effective amount of interferon-alpha (IFN-α). Said IFN-α is preferably monoPEG-ylated consensus IFN-α administered at a dosing interval of every 8 days to every 14 days. According to another embodiment, said IFN-α is monoPEG-ylated consensus IFN-α administered at a dosing interval of once every 7 days. Preferably said IFN-α is INFERGEN consensus IFN-α. According to another embodiment, the method further comprises comprising administering an effective amount of an agent selected from 3'-azidothymidine, 2',3'-dideoxyinosine, 2',3'-dideoxycytidine, 2',3'-didehydro-2',3'-dideoxythymidine, combivir, abacavir, adefovir dipoxil, cidofovir, and an inosine monophosphate dehydrogenase inhibitor.

According to yet another embodiment, the present invention relates to a method of synthesizing a compound having the structure: 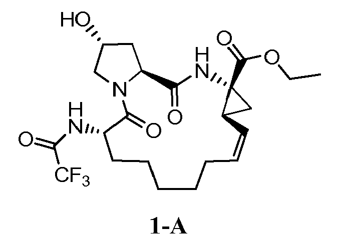 comprising:
(a) coupling a compound of formula **4-BB** with a compound of formula **5-H** to provide a compound of formula **3-A:** 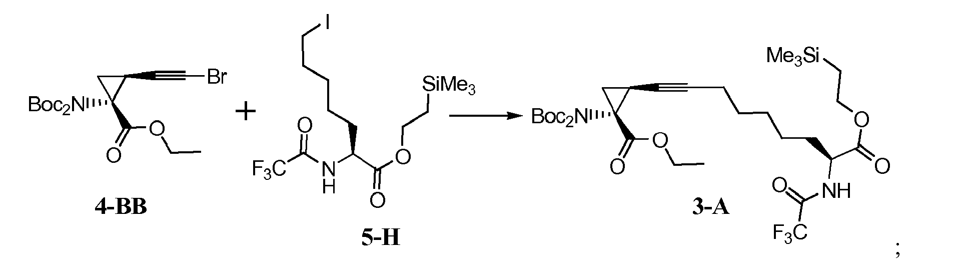
(b) deprotecting a compound of formula **3-A** to provide a compound of formula **3-B:** 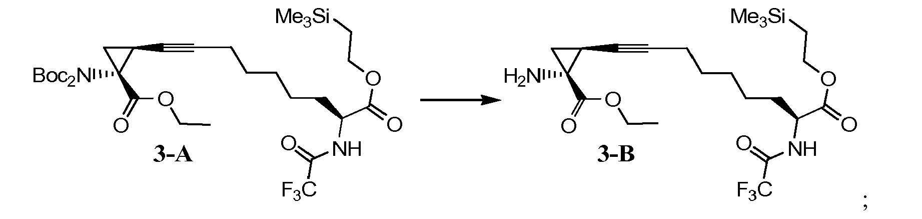
(c) coupling a compound of formula **3-B** with Boc-L-hydroxyproline **(3-D)** to provide a compound of formula **2-A:** 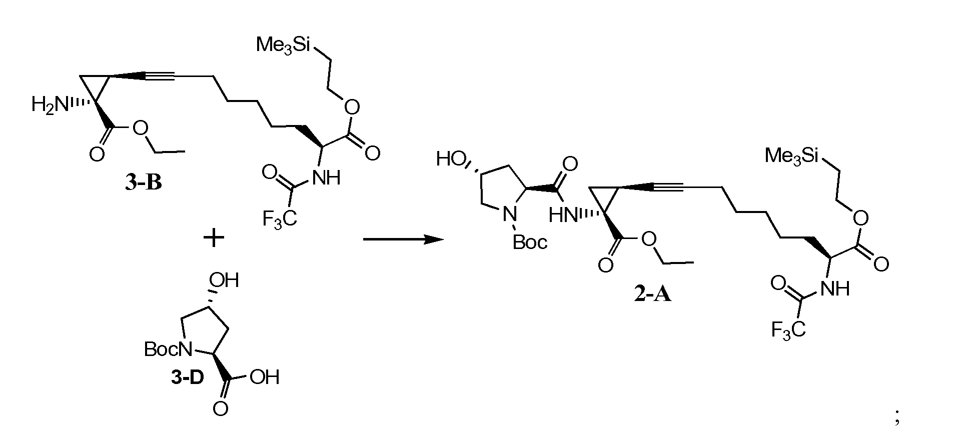
(d) hydrogenating a compound of formula **2-A** to provide a compound a compound of formula **1-D:** 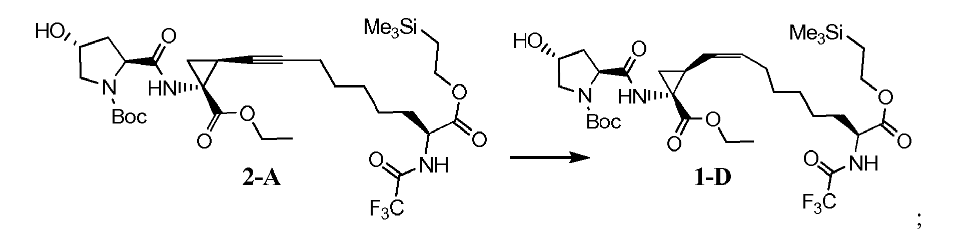
(e) deprotecting a compound of formula **1-D** to provide a compound of formula **1-E:** 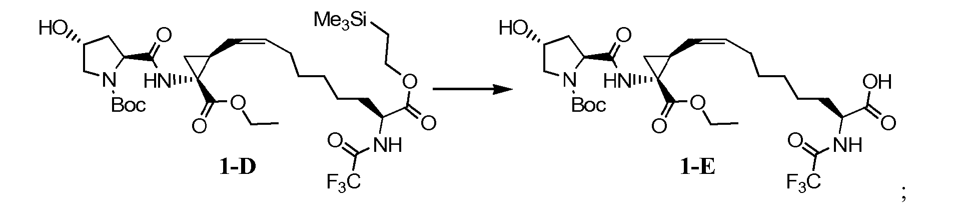 and
(f) transforming a compound of formula **1-E** to provide a compound of formula **1-A:**

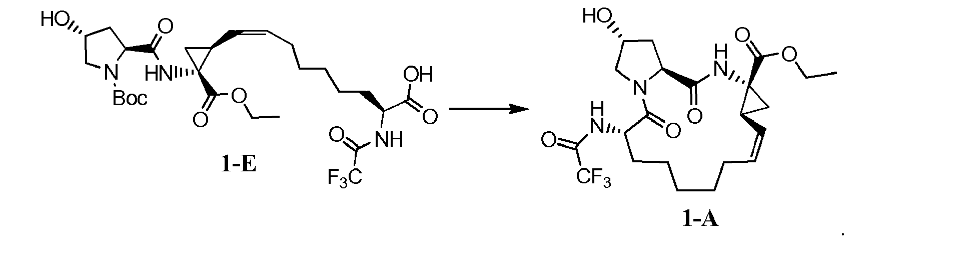

According to another embodiment, the present invention relates to a method of synthesizing a compound having the structure: 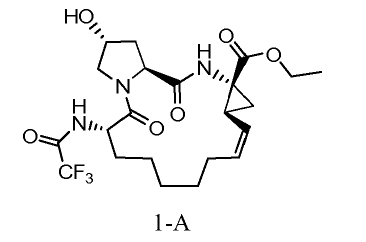 comprising:
(a) coupling a compound of formula **4-BB** with a compound of formula **5-H** to provide a compound of formula **3-A:** 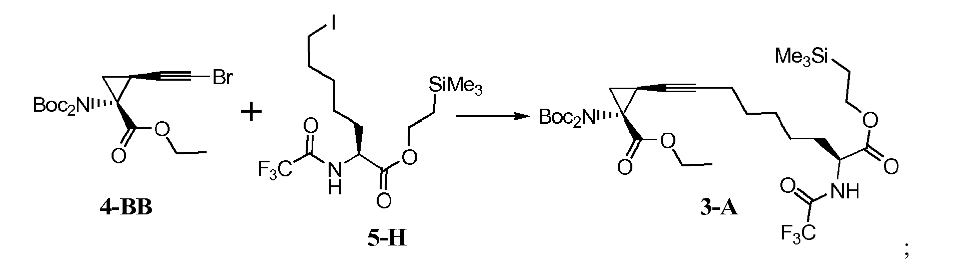
(b) deprotecting a compound of formula **3-A** to provide a compound of formula **3-C:** 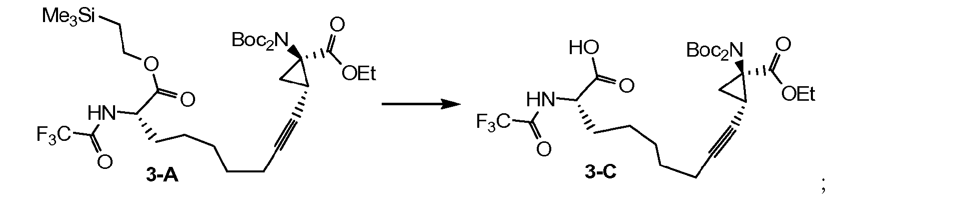
(c) coupling a compound of formula **3-C** with a compound of formula **3-F** to provide a compound of formula **2-B:** 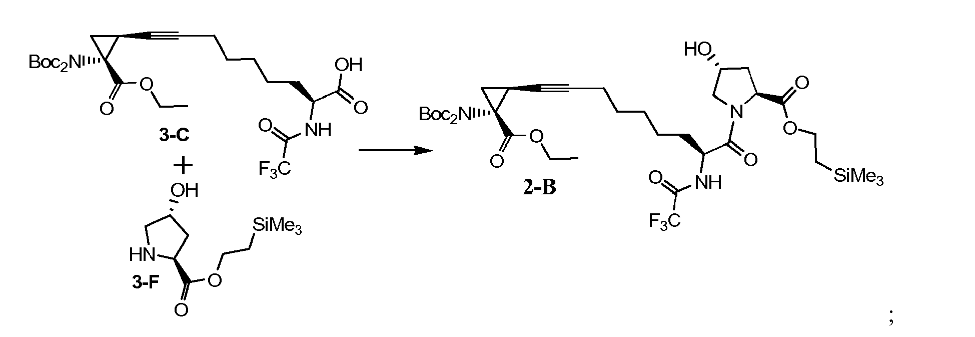
(d) hydrogenating a compound of formula **2-B** to provide a compound a compound of formula **1-H:** 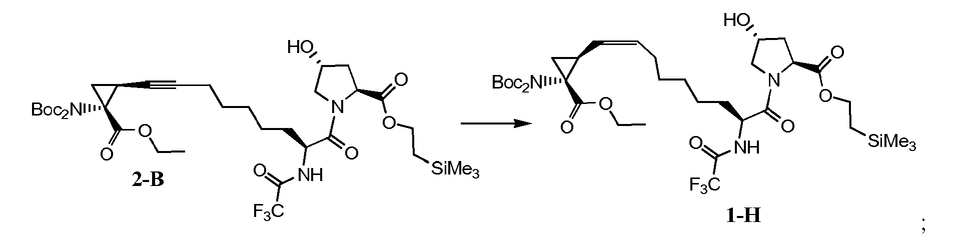
(e) deprotecting a compound of formula **1-H** to provide a compound of formula **1-I:** 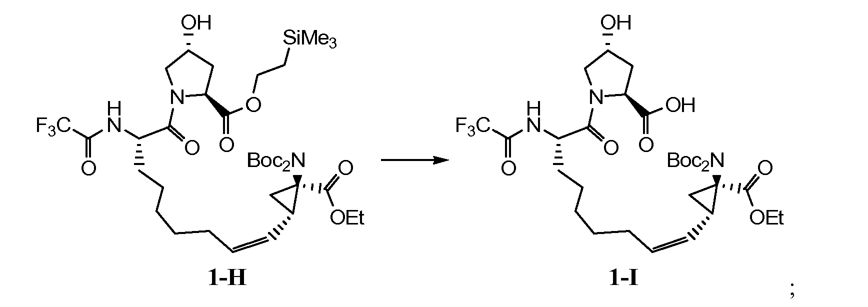
(f) deprotecting a compound of formula **1-I** to provide a compound of formula **1-J:** 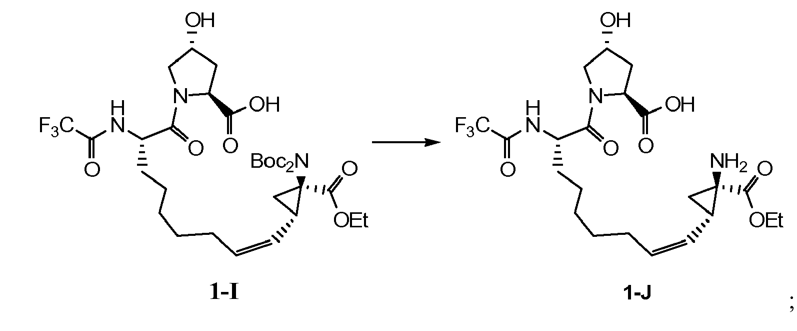 and
(g) cyclizing a compound of formula **1-J** to provide a compound of formula **1-A:**

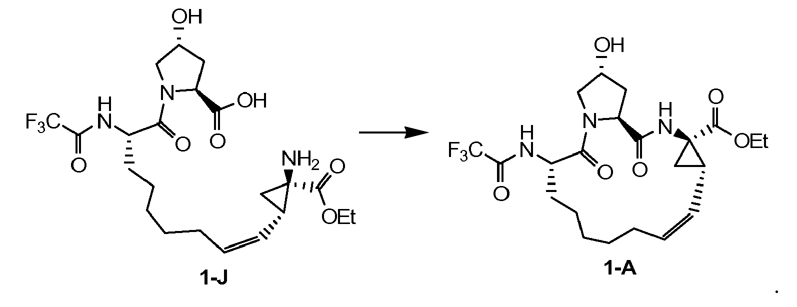

According to another embodiment, the present invention relates to a method of synthesizing a compound having the structure: 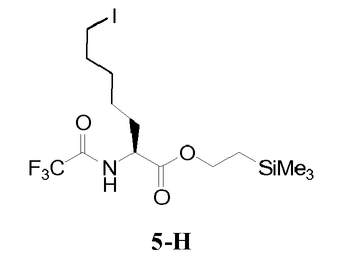 comprising:
(a) saponifying a compound of formula **5-A** to provide a compound of formula **5-B:** 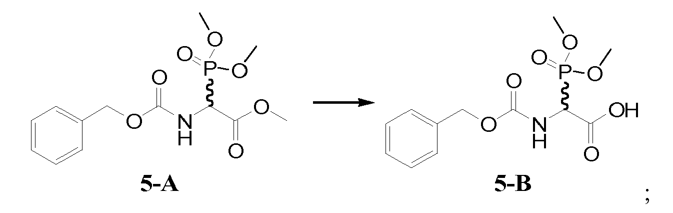
(b) esterifying a compound of formula **5-B** to provide a compound of formula **5-C:** 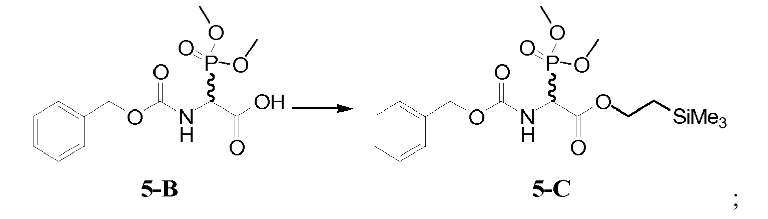
(c) transforming a compound of formula **5-C** to provide a compound of formula **5-E:** 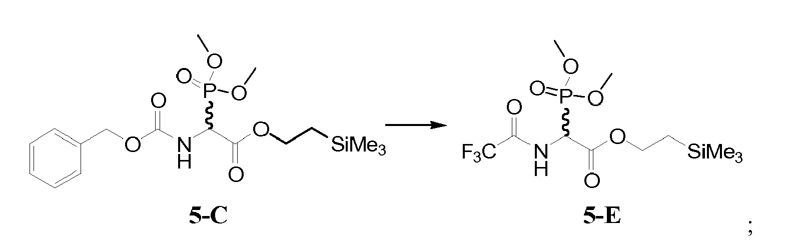
(d) coupling a compound of formula **5-E** with 5-chlorobutanal to provide a compound of formula **5-F:** 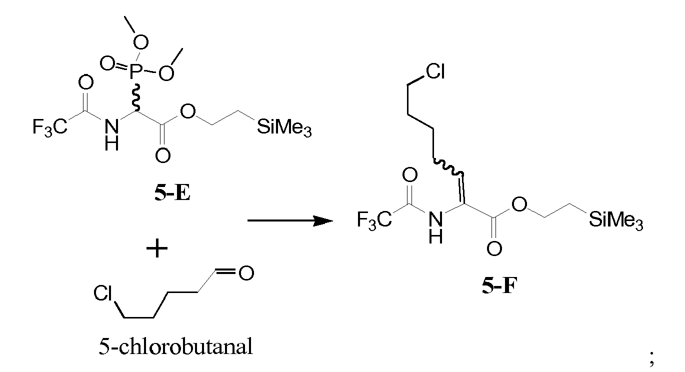
(e) reducing a compound of formula **5-F** to provide a compound of formula **5-G:** 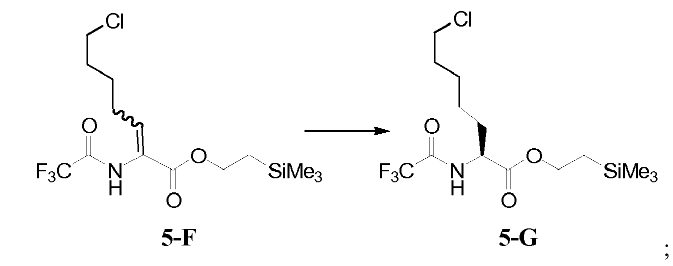 and
(f) transforming a compound of formula **5-G** to provide a compound of formula **5-H:**

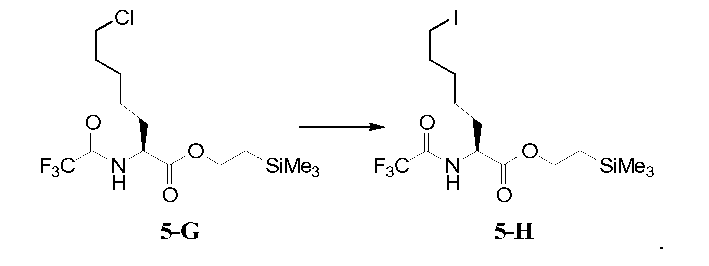

According to another embodiment, the present invention relates to a method of synthesizing a compound having the structure: 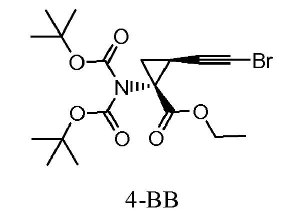 comprising:
(a) protecting a compound of formula **6-A** to provide a compound of formula **6-B:** 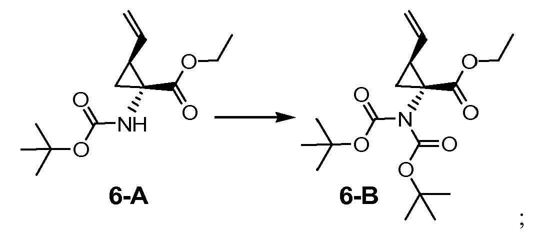
(b) brominating a compound of formula **6-B** to provide a compound of formula **6-C:** 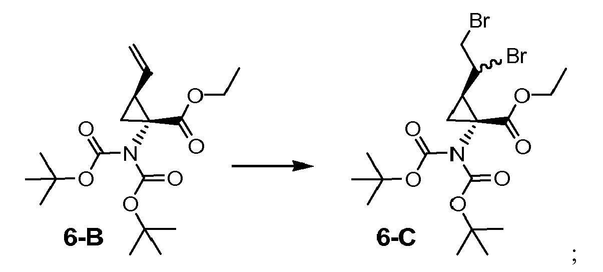
(c) transforming a compound of formula **6-C** to provide a compound of formula **6-D:** 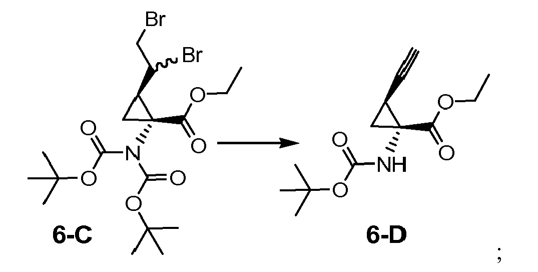
(d) protecting a compound of formula **6-D** to provide a compound of formula **6-E:** 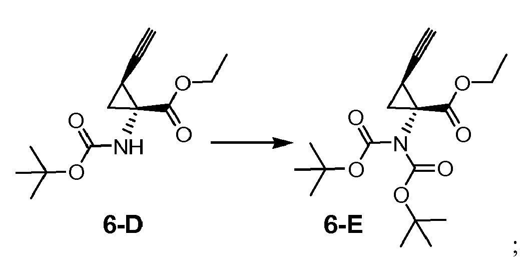 and
(e) brominating a compound of formula **6-E** to provide a compound of formula **4-BB:**

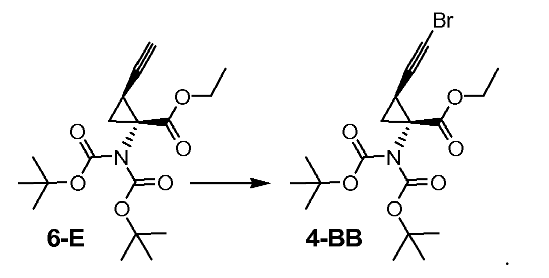

According to another embodiment, the present invention relates to a compound selected from the group consisting of: 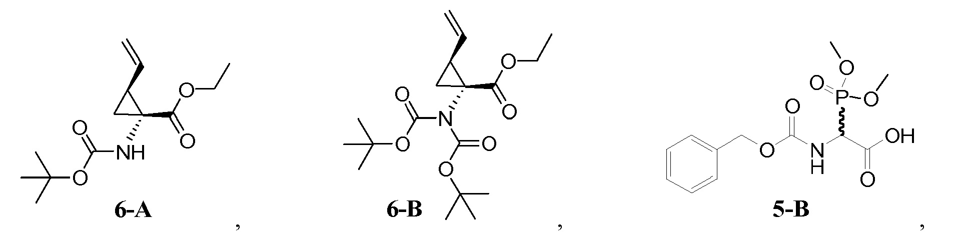 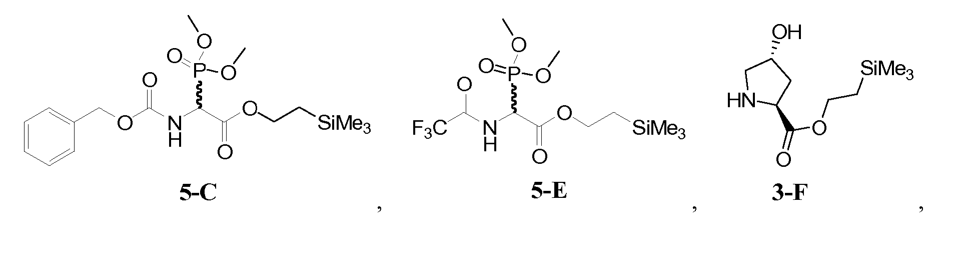 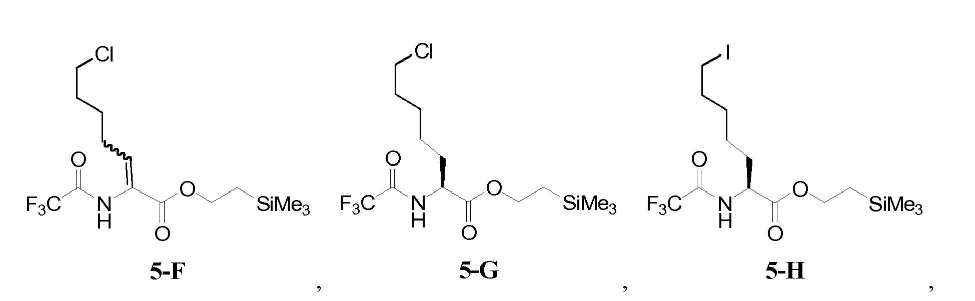 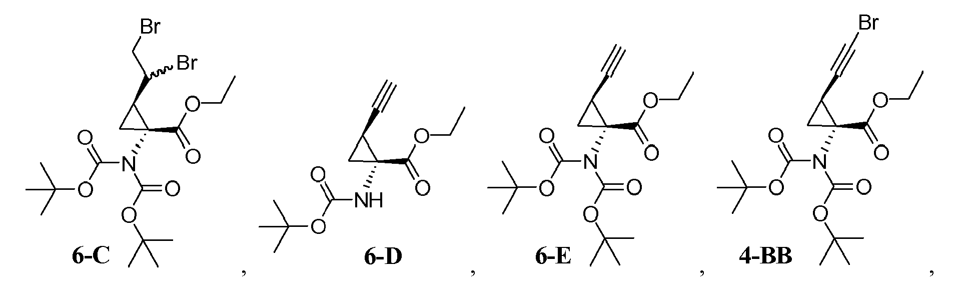 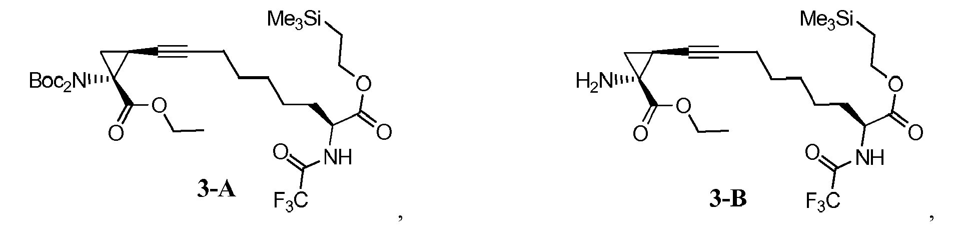 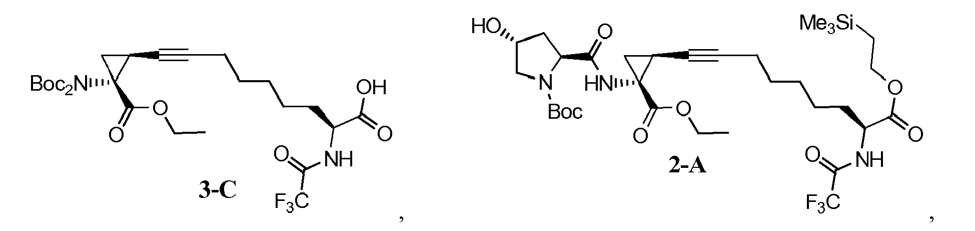 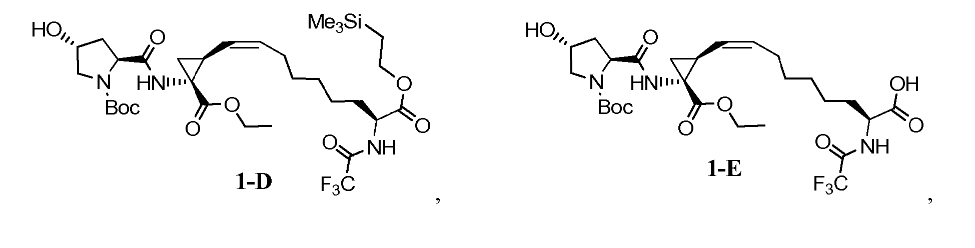 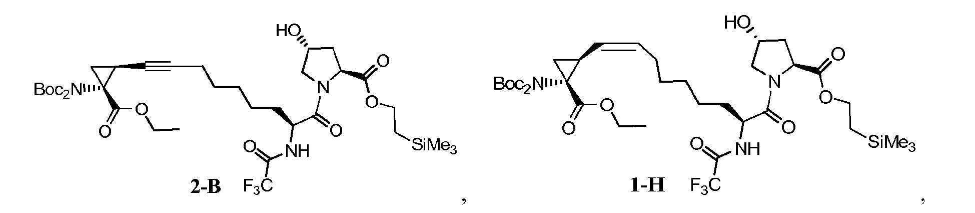 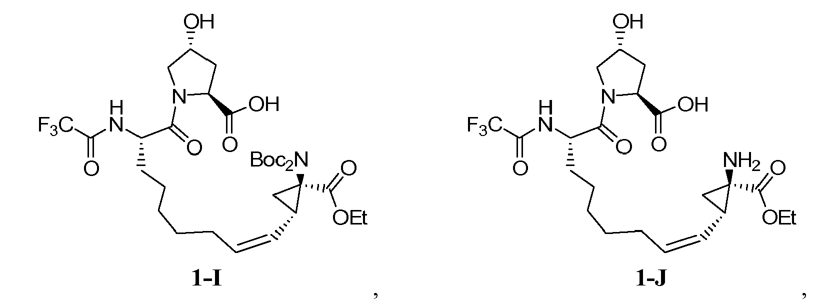 and/or

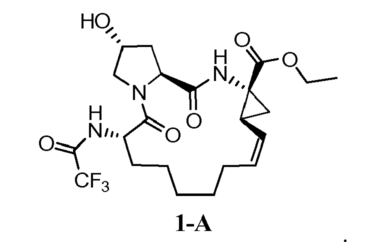

According to yet another embodiment, the present invention relates to a compound selected from the group consisting of: 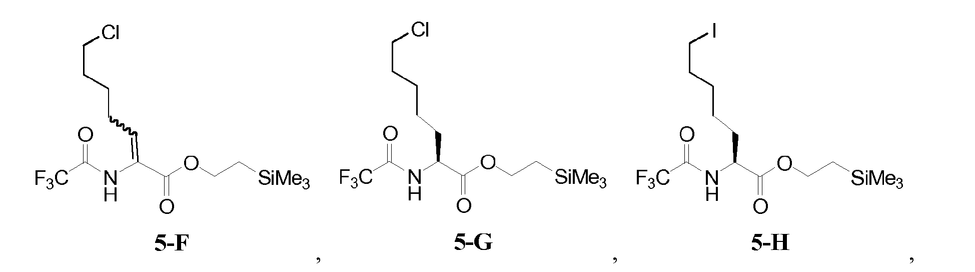 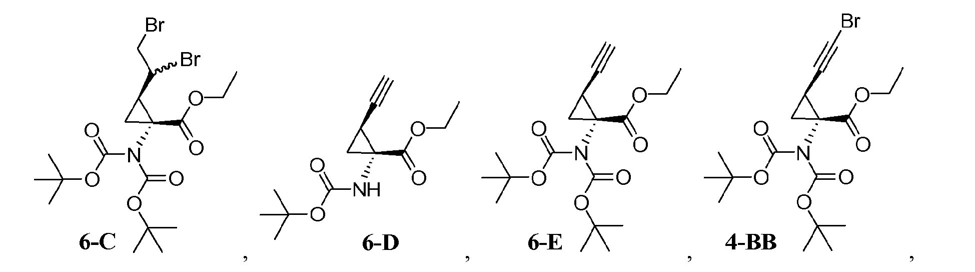 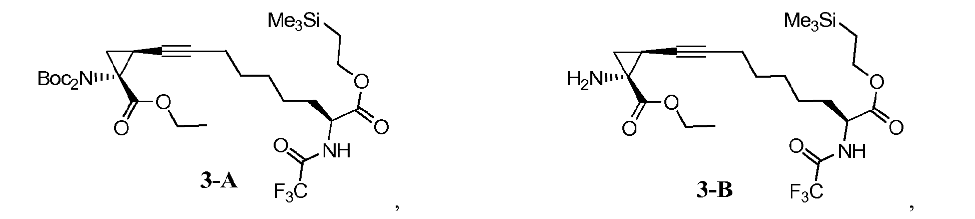 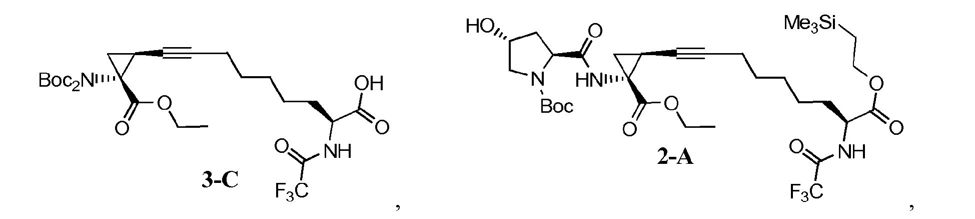 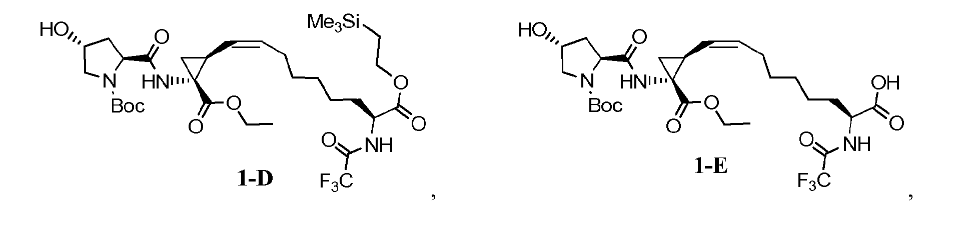 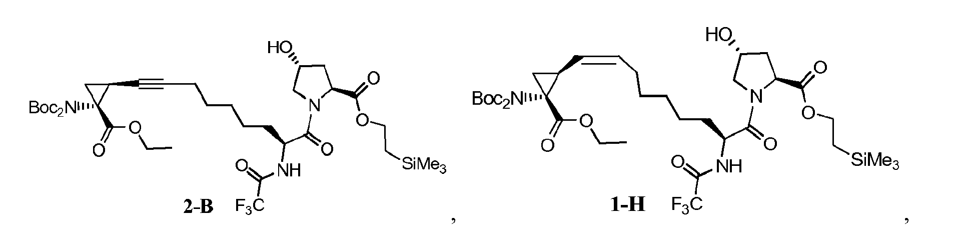 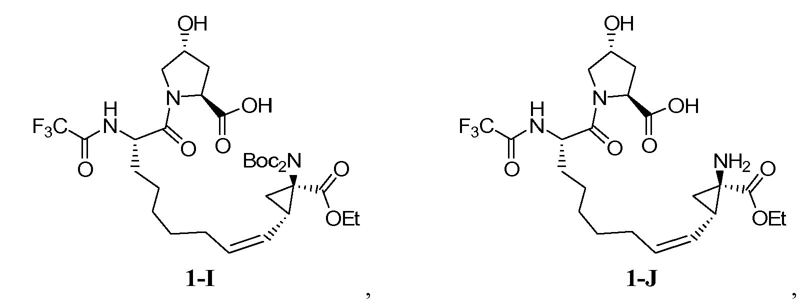 and/or

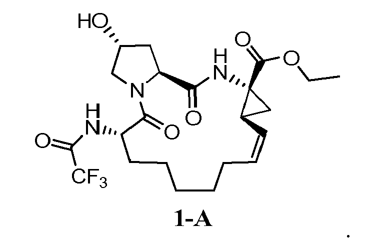

According to yet another embodiment, the present invention relates to a compound selected from the group consisting of: 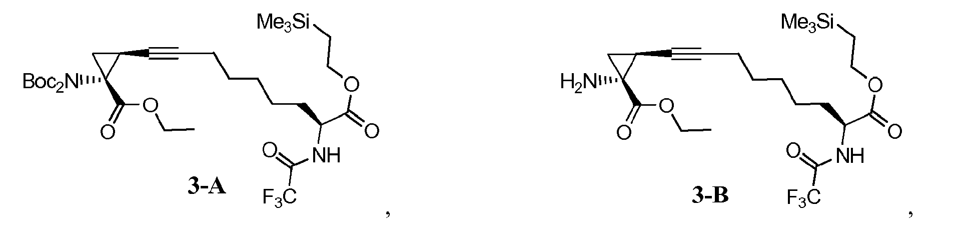 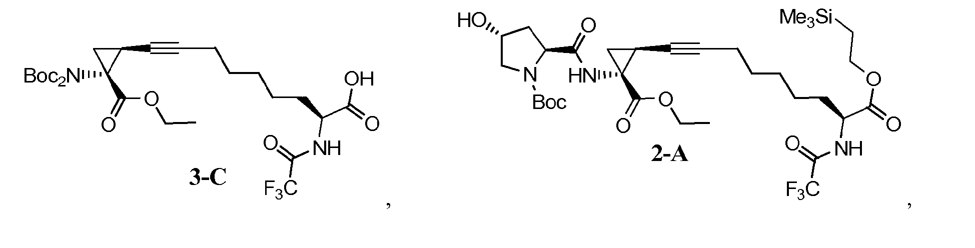 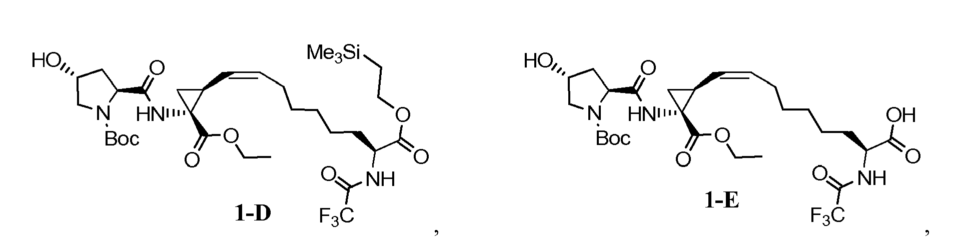 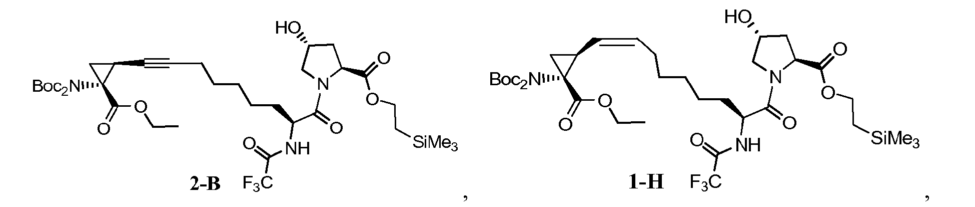 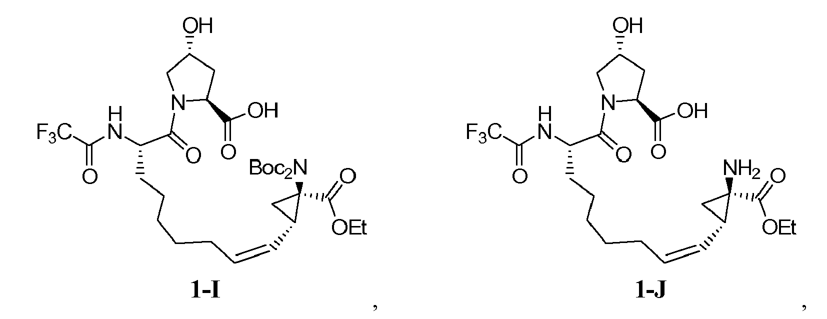 and/or

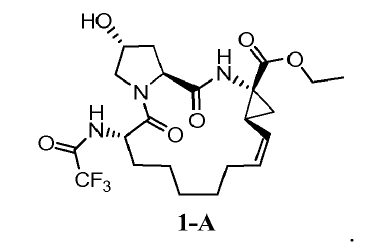

According to yet another embodiment, the present invention relates to a method of chemical synthesis comprising hydrogenating a compound of formula **2-A** to provide a compound of formula **1-D:**

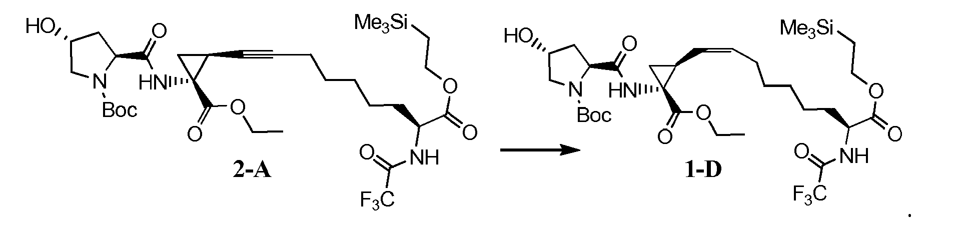

According to yet another embodiment, the present invention relates to a method of chemical synthesis comprising hydrogenating a compound of formula **2-B** to provide a compound of formula **1-H:**

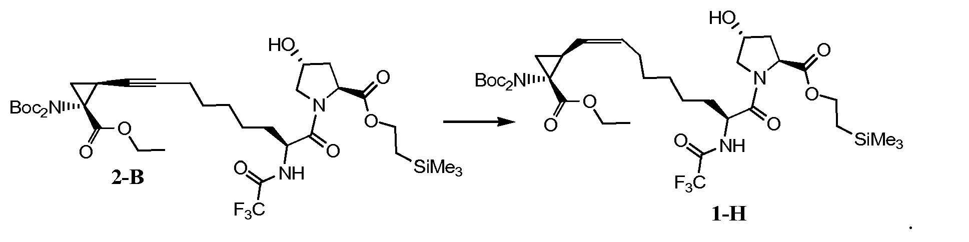

According to yet another embodiment, the present invention relates to a method of chemical synthesis comprising transforming a compound of formula **1-E** to provide a compound of formula **1-A:**

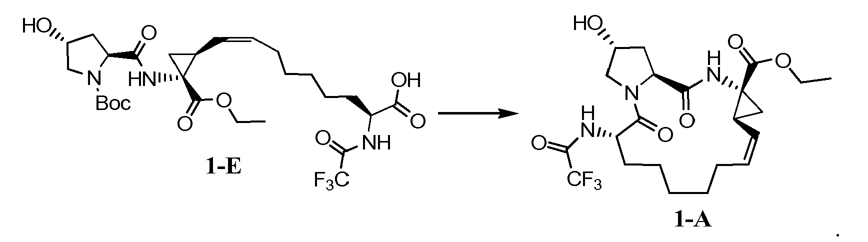

According to yet another embodiment, the present invention relates to a method of chemical synthesis comprising cyclizing a compound of formula **1-J** to provide a compound of formula **1-A:**

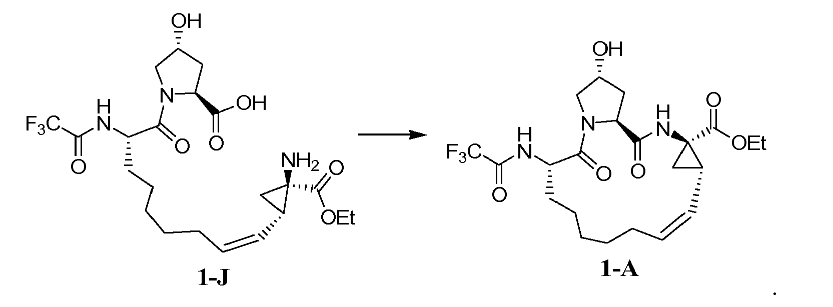

According to yet another embodiment, the present invention relates to a method of administering an inhibitor of hepatitis C virus (HCV) infection, comprising administering to a patient an effective amount of compound 100, or a pharmaceutically acceptable salt, ester or prodrug thereof, wherein the administering is undertaken in conjunction with the consumption of food by the patient: 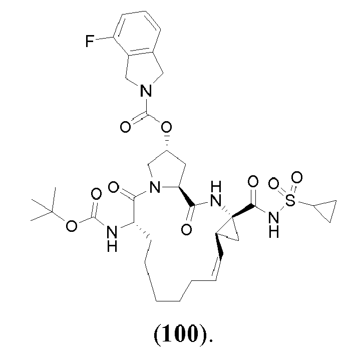 More preferably the method provides that the administration of compound 100, or the pharmaceutically acceptable salt, ester or prodrug thereof, comprises orally administering a pharmaceutical composition to the patient, wherein the pharmaceutical composition comprises compound 100, or the pharmaceutically acceptable salt, ester or prodrug thereof. More preferably the consumption of food by the patient is effective to provide an area under the plasma concentration-time curve (AUC_{0-inf} after a single dose or AUC₀₋₂₄ at steady-state) for compound 100, or active metabolite thereof, that is greater than when the administering is not undertaken in conjunction with the consumption of food by the patient. Furthermore, the consumption of food by the patient is preferably undertaken substantially simultaneously with the administration of compound 100, or the pharmaceutically acceptable salt, ester or prodrug thereof and more preferably a sodium salt of compound 100.

According to yet another embodiment, the present invention relates to a method of administering an inhibitor of hepatitis C virus (HCV) infection, comprising:
administering to a patient an effective amount of compound 100, or a pharmaceutically acceptable salt, ester or prodrug thereof: 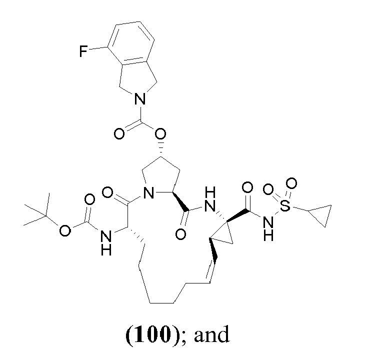
providing information to the patient, which information comprises that the administering of compound 100, or the pharmaceutically acceptable salt, ester or prodrug thereof, should be accompanied by the consumption of food. More preferably the administration of compound 100, or the pharmaceutically acceptable salt, ester or prodrug thereof, comprises orally administering a pharmaceutical composition to the patient, wherein the pharmaceutical composition comprises compound 100, or the pharmaceutically acceptable salt, ester or prodrug thereof. Furthermore the pharmaceutical composition comprises preferably a pharmaceutically acceptable salt of compound 100 and more preferably a sodium salt of compound 100.

According to yet another embodiment, the present invention relates to a method of distributing an oral dosage form, comprising:
distributing a pharmaceutical composition, wherein the pharmaceutical composition comprises compound 100, or a pharmaceutically acceptable salt, ester or prodrug thereof: 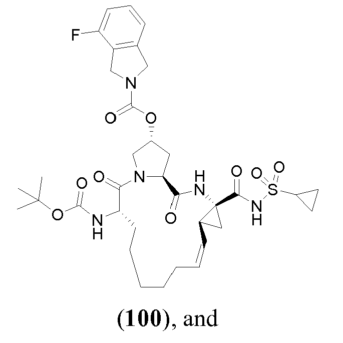
concomitantly distributing information, which information comprises that the administering of the pharmaceutical composition should be accompanied by the consumption of food.

More preferably the administration of compound 100, or the pharmaceutically acceptable salt, ester or prodrug thereof, comprises orally administering a pharmaceutical composition to the patient, wherein the pharmaceutical composition comprises compound 100, or the pharmaceutically acceptable salt, ester or prodrug thereof. Furthermore the pharmaceutical composition comprises a pharmaceutically acceptable salt of compound 100 and more preferably a sodium salt of compound 100.

### EXAMPLES

### PREPARATION OF NS3 INHIBITORS

The HCV protease inhibitors in the following sections can be prepared according to the procedures and schemes shown in each section. The numberings in each of the following Preparation of NS3 Inhibitor sections are meant for that specific section only, and should not be construed or confused with the same numberings in other sections.

### Example 1

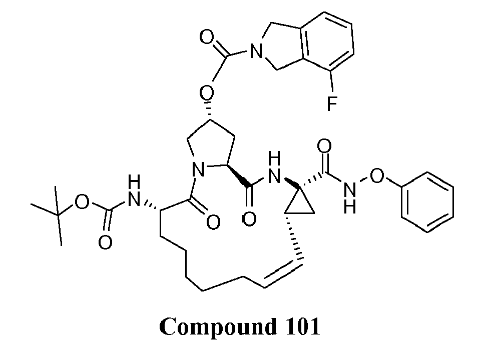

(2*R*,6*S*,13a*S*, 14a*R*, 16a*S*,Z)-6-(tert-butoxycarbonylamino)-5,16-dioxo-14a-(phenoxycarbamoyl)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 101,** was prepared as shown in the following scheme:

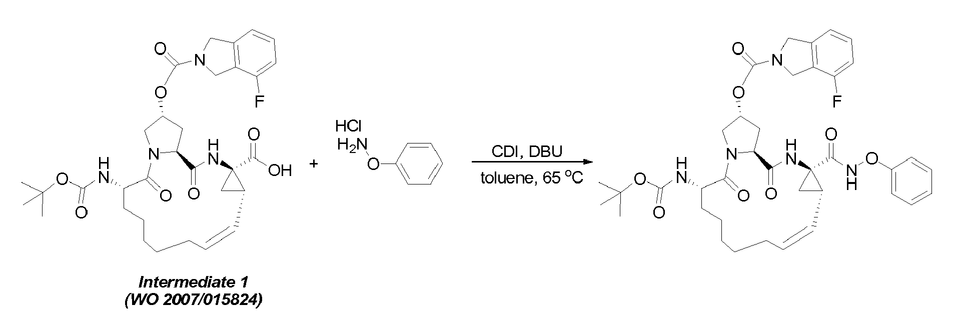

**Intermediate 1** (0.050 g, 0.078 mmol), 1,1'-carbonyldiimidazole (0.0181 g, 0.111 mmol) in toluene was stirred at 65 °C for 2 hours. 1,8-diazabicyclo[5.4.0]undec-7-ene (0.036 mL, 0.24 mmol) and O-phenylhydroxylamine hydrochloride (0.017 g, 0.12 mmol) were added and the reaction was stirred at 65 °C for 18 hours then water (5 mL) was added and the reaction was acidified with saturated KHSO₄ until pH reached 3-4. The mixture was extracted with EtOAc (20 mL), washed with brine and dried over sodium sulfate. After removal of solvent, the residue was purified by chromatography to provide (2*R*,6*S*,13a*S*, 14a*R*, 16a*S*,Z)-6-(tert-butoxycarbonylamino)-5,16-dioxo-14a-(phenoxycarbamoyl)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 101**, (0.027 g, 47%) as white solid. MS: Calcd.: 719.3; Found: [M+H]⁺720.1. ¹H NMR (400 MHz, DMSO-*d*⁶) 11.52 (s, 1H), 8.78 & 8.76 (s, 1H), 7.17 (m, 1H), 7.13 (m, 2H), 7.08-7.10 (m, 3H), 6.99 (m, 3H), 5.45 (m, 1H), 5.28 (s, 1H), 5.18 (m, 1H), 4.62 (s, 4H), 4.41 (m, 1H), 4.25 (m, 1H), 3.92 (m, 1H), 3.84 (m, 1H), 2.77 (m, 1H), 2.35 (m, 2H), 2.11 (m, 1H), 1.02-1.73 (m, 20H).

### Example 2

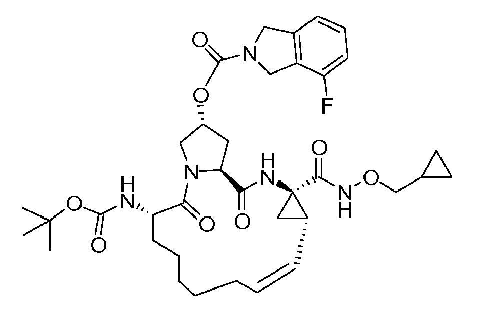

### Compound 102

(2*R*,6*S*, 13a*S*, 14a*R*, 16a*S*,Z)-6-(tert-butoxycarbonylamino)-14a-(cyclopropylmethoxycarbamoyl)-5,16-dioxo-1,2,3,5,6,7, 8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 102**, was prepared as shown in the following scheme:

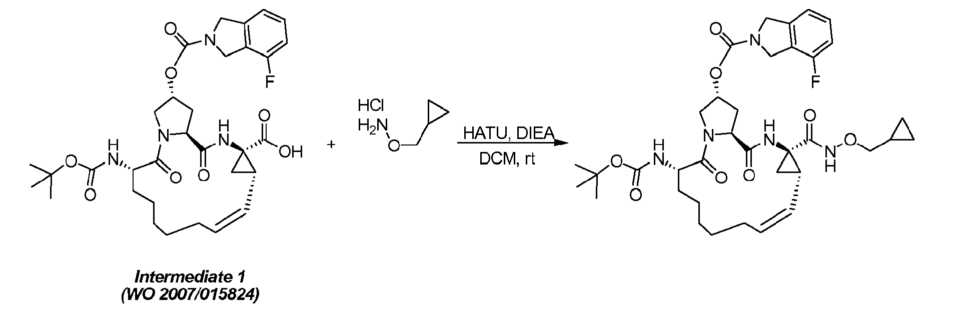

**Intermediate 1** (0.075 g, 0.119 mmol), O-(cyclopropyhnethyl)hydroxylamine hydrochloride (0.016 g, 0.18 mmol) and 2-(1H-7-azabenzotriazol-1-yl)-1,1,3,3-tetramethyl uronium hexafluorophosphate (0.0454 g, 0.119 mmol) in DMF (3 mL) was added diisopropylethylamine (d = 0.742 g/mL) (0.052 mL, 0.30 mmol) at room temperature. The reaction was stirred at room temperature for 2 hours the H₂O (5 mL) was added and acidified with saturated KHSO₄ until pH=3∼4. The mixture was extracted with ethyl ether (20 mL), washed with brine and dried over sodium sulfate. After removal of solvent, the residue was purified by chromatography to give (2*R*,6*S*,13a*S*,14a*R*,16a*S*,Z)-6-(tert-butoxycarbonylamino)-14a-(cyclopropylmethoxycarbamoyl)-5,16-dioxo-1,2,3,5,6,7,8,9,10,11,13a,14,14a,15,16,16a-hexadecahydrocyclopropa[e]pyrrolo[l,2-a][1,4]diazacyclopentadecin-2-yl 4-fluoroisoindoline-2-carboxylate, **Compound 102,** (0.042 g, 61%) as white solid. MS: Calcd.: 697.4; Found: [M+H]⁺ 698.1. ¹H NMR (400 MHz, DMSO-*d*⁶) 10.62 (s, 1H), 8.63 & 8.61 (s, 1H), 7.35 (m, 1H), 7.02-7.18 (m, 3H), 5.47 (m, 1H), 5.27 (s, 1H), 5.18 (m, 1H), 4.62 & 4.61 (s, 4H), 4.40 (m, 1H), 4.28 (m, 1H), 3.89 (m, 1H), 3.65 (m, 1H), 3.54 (m, 2H), 2.64 (m, 1H), 2.32 (m, 2H), 2.10 (m, 1H), 1.00-1.68 (m, 21H), 0.44 (m, 2H), 0.20 (m, 2H).

### Example 3

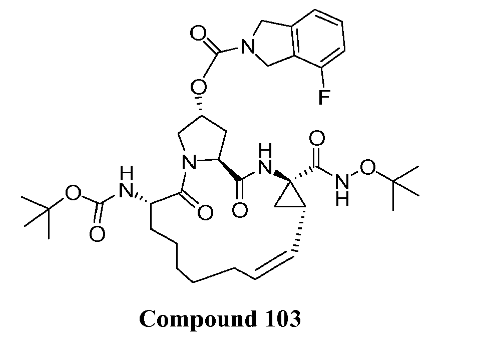

(2*R*,6*S*, 13a*S*, 14a*R*, 16a*S*,Z)-14a-(tert-butoxycarbamoyl)-6-(tert-butoxycarbonylamino)-5,16-dioxo-1,2,3,5,6,7,8,9,10,11,13a, 14,14a, 15,16,16a-hexadecahydrocyclopropa[e]pyrrolo[1,2-a][1,4]diazacyclopentadecin-2-yl 4-fluoroisoindoline-2-carboxylate, **Compound 103,** was prepared in a similar fashion as described for **Compound 102** in **Example 2**, except that O-tert-butyl-hydroxylamine hydrochlorde was used in lieu of O-(cyclopropylmethyl)hydroxylamine hydrochloride. MS: Calcd.: 699.4; Found: [M-H]⁺ 698.4. ¹H NMR (400 MHz, DMSO-*d*⁶) 10.03 (s, 1H), 8.87 & 8.84 (s, 1H), 7.37 (m, 1H), 7.10-7.20 (m, 3H), 5.49 (m, 1H), 5.29 (s, 1H), 5.29 (m, 1H), 4.67 (s, 4H), 4.46 (m, 1H), 4.30 (m, 1H), 3.92 (m, 1H), 3.71 (m, 1H), 2.62 (m, 1H), 2.27 (m, 2H), 2.09 (m, 1H), 1.04-1.68 (m, 29H).

### Example 4

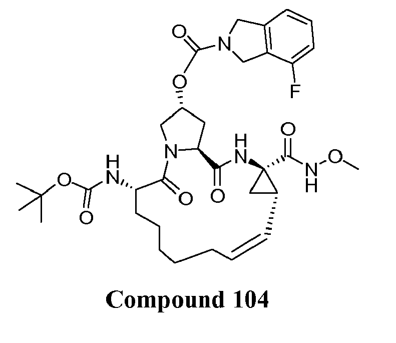

(2*R*,6*S*, 13a*S*, 14a*R*, 16a*S*,Z)-6-(tert-butoxycarbonylamino)-14a-(methoxycarbamoyl)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 104,** was prepared in a similar fashion as described for **Compound 102** in **Example 2,** except that O-methylhydroxylamine hydrochlorde was used in lieu of O-(cyclopropylmethyl)hydroxylamine hydrochloride. MS: Calcd.: 657.3; Found: [M-H]⁺ 656.3. ¹H NMR (400 MHz, DMSO-*d*⁶) 10.75 (s, 1H), 8.63 & 8.61 (s, 1H), 7.35 (m, 1H), 7.09-7.20 (m, 3H), 5.46 (m, 1H), 5.29 (s, 1H), 5.23 (m, 1H), 4.67 & 4.66 (s, 4H), 4.40 (m, 1H), 4.29 (m, 1H), 3.92 (m, 1H), 3.67 (m, 1H), 3.53 (s, 3H), 2.64 (m,1H), 2.31 (m, 2H), 2.10 (m, 1H), 1.07-1.69 (m, 20H).

### Example 5

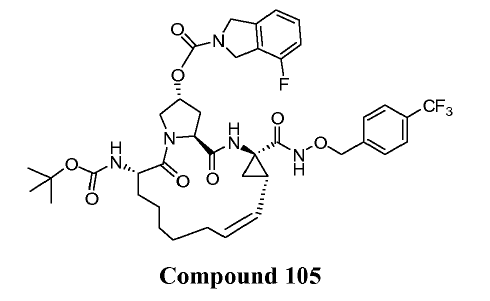

(2*R*,6*S*, 13a*S*, 14a*R*, 16a*S*,Z)-6-(tert-butoxycarbonylamino)-5,16-dioxo- 14a-(4-(trifluotomethyl)benzyloxycarbamoyl)-1,2,3,5,6,7,8,9,10,11,13a, 14,14a, 15,16,16a-hexadecahydrocyclopropa[e]pyrrolo[1,2-a][1,4]diazacyclopentadecin-2-yl 4-fluoroisoindoline-2-carboxylate, **Compound 105,** was prepared in a similar fashion as described for **Compound 102** in **Example 3_{,}** except that O-(4-(trifluoromethyl)benzyl)hydroxylamine was used in lieu of O-(cyclopropylmethyl)hydroxylamine hydrochloride. ¹H NMR (400 MHz, DMSO-*d*⁶) 10.86 (s, 1H), 8.69 & 8.67 (s, 1H), 7.73 (m, 2H), 7.62 (m, 2H), 7.35 (m, 1H), 7.09-7.21 (m, 3H), 5.47 (m, 1H), 5.28 (s, 1H), 5.20 (m, 1H), 4.84 (s, 2H), 4.67 & 4.66 (s, 4H), 4.39 (m, 1H), 4.27 (m, 1H), 3.91 (m, 1H), 3.68 (m, 1H), 2.62 (m, 1H), 2.27 (m, 2H), 2.11 (m, 1H), 1.07-1.66 (m, 20H).

**Table 1: Additional examples of compound prepared using Example procedures 1-3**

| Compound | Structure | Mass spectra data | Example Procedure Used |
|---|---|---|---|
| 106 | 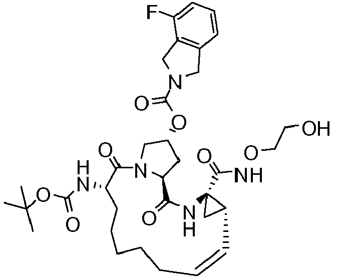 | (APCI+) m/z 688 (M+1) | 3 |
| 107 | 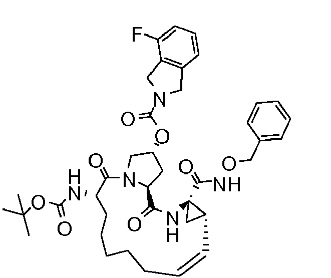 | (APCI+) m/z 734 (M+1) | 3 |
| 108 | 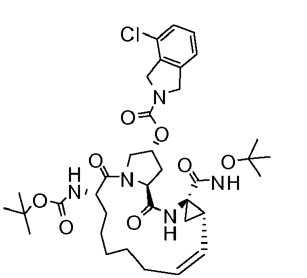 | (APCI+) m/z 717 (M+1) | 1 |
| 109 | 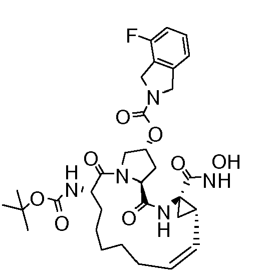 | (APCI+) m/z 632 (M-1) | 1 |
| 110 | 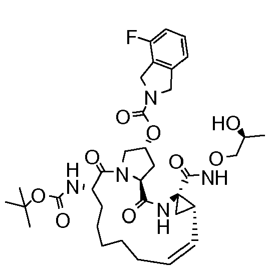 | (APCI+) m/z 702 (M+1) | 3 |

### Example 6

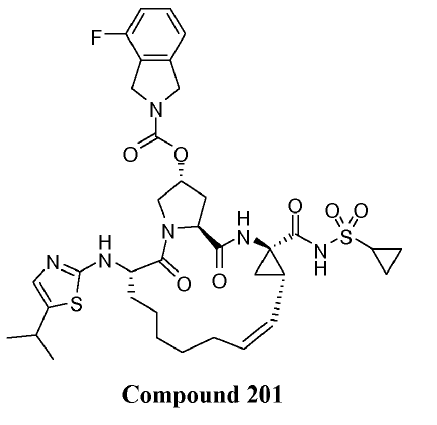

(2*R*,6*S*, 13a*S*, 14a*R*, 16a*S*,Z)-14a-(cyclopropylsulfonylcarbamoyl)-6-(5-isopropylthiazol-2-ylamino)-5,16-dioxo-1,2,3,5,6,7,8,9,10,11,13a, 14,14a, 15,16,16a-hexadecahydrocyclopropa[e]pyrrolo[1,2-a][1,4]diazacyclopentadecin-2-yl 4-fluoroisoindoline-2-carboxylate, **Compound 201,** was prepared as follows:

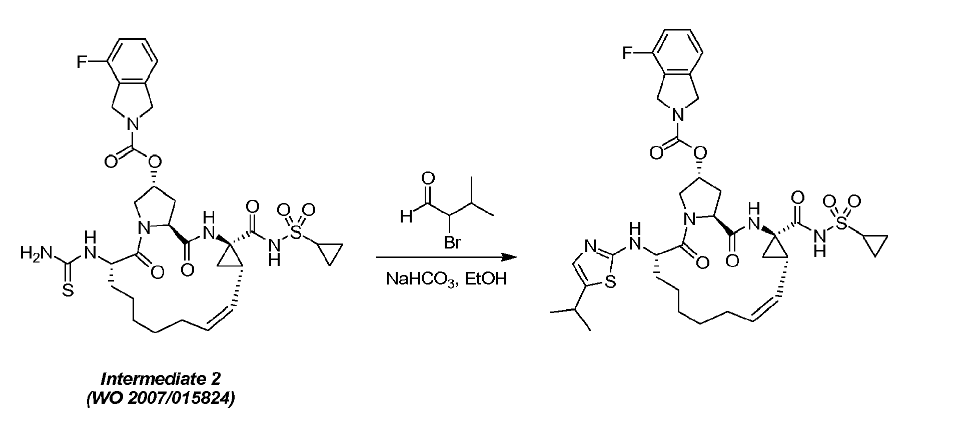

2-bromo-3-methylbutanal (110mg, 0.434 mmol) and **Intermediate 2** (150 mg, 0.217 mmol) and NaHCO₃ (182 mg, 2.17 mmol) were mixed in 1 mL EtOH in 4 mL vial equipped with a stirbar. Sealed and heated to 100 °C for 20 minutes with stirring. The reaction was then cooled to room temperature and recharged with more 2-bromo-3-methylbutanal (110 mg, 0.434 mmol), sealed and heated to 100 °C for 10 minutes then was concentrated *in vacuo* and purified by reverse phase chromatography (Biotage SP4). The resultant white solid was then triturated in hexanes and filtered to provide (2*R*,6*S*,13a*S*,14a*R*,16a*S*,Z)-14a-(cyclopropylsulfonylcarbamoyl)-6-(5-isopropylthiazol-2-ylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 201,** (98 mg, 0.13 mmol, 60% yield) as a white solid. LCMS (APCI-) m/z 755.3 (MH⁻).

### Example 7

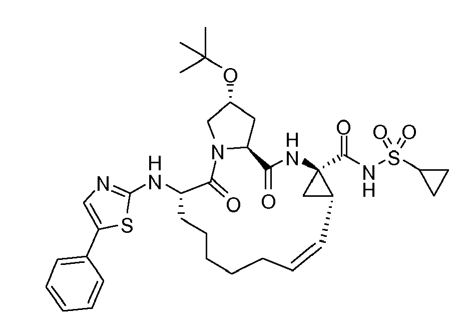

### Compound 202

(2*R*,6*S*,13a*S*, 14a*R*, 16a*S*,Z)-2-tert-butoxy-N-(cyclopropylsulfonyl)-5,16-dioxo-6-(5-phenylthiazol-2-ylamino)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, **Compound 202,** was prepared as follows:

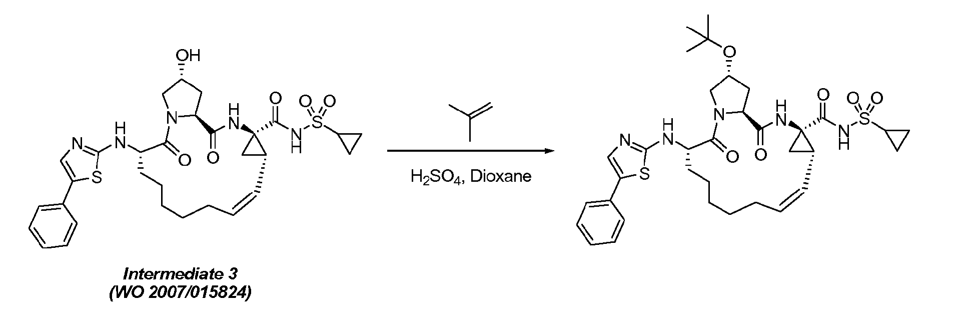

To **Intermediate 3** (22 mg, 0.035 mmol) in dioxane (0.5 mL) was added H₂SO₄ (0.0004 mL, 0.007 mmol) in a 2 mL high pressure reaction vessel with stirbar. The result mixture was cooled to -78 °C and 2-methylprop-1-ene was blown into the vial until approximately 0.1 mL 2-methylprop-1-ene had precipitated in the vial. The reaction was sealed and stirred at room temperature for 48 hours then cooled back to -78 °C and unsealed allowing 2-methylprop-1-ene to boil off. After conentrating *in vacuo* the reactrion was purified by reverse phase chromatography to provide (2*R*,6*S*, 13a*S*,14a*R*,16a*S*,Z)-2-tert-butoxy-N-(cyclopropylsulfonyl)-5,16-dioxo-6-(5-phenylthiazol-2-ylamino)-1,2,3,5,6,7,8,9,10,11,13a,14,14a,15,16,16a-hexadecahydrocyclopropa[e]pyrrolo[1,2-a][1,4]diazacyclopentadecine-14a-carboxamide, **Compound 202**, (2 mg, 0.0029 mmol, 8.3% yield) as a white solid. LCMS (APCI-) m/z 682.4 (MH⁻).

### Example 8

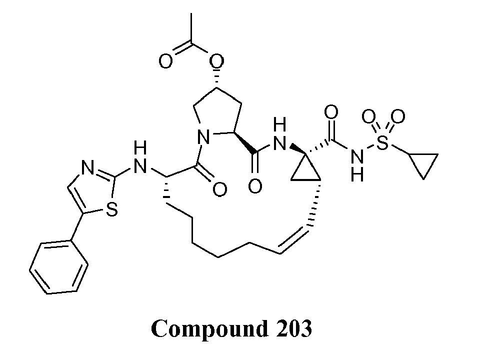

The titled compound, (2*R*,6*S*,13a*S*,14a*R*,16a*S*,Z)-14a-(cyclopropylsulfonylcarbamoyl)-5,16-dioxo-6-(5-phenylthiazol-2-ylamino)-1,2,3,5,6,7,8,9,10,11,13a,14,14a,15,16,16a-hexadecahydrocyclopropa[e]pyrrolo[1,2-a][1,4]diazacyclopentadecin-2-yl acetate, **Compound 203,** was prepared as follows: 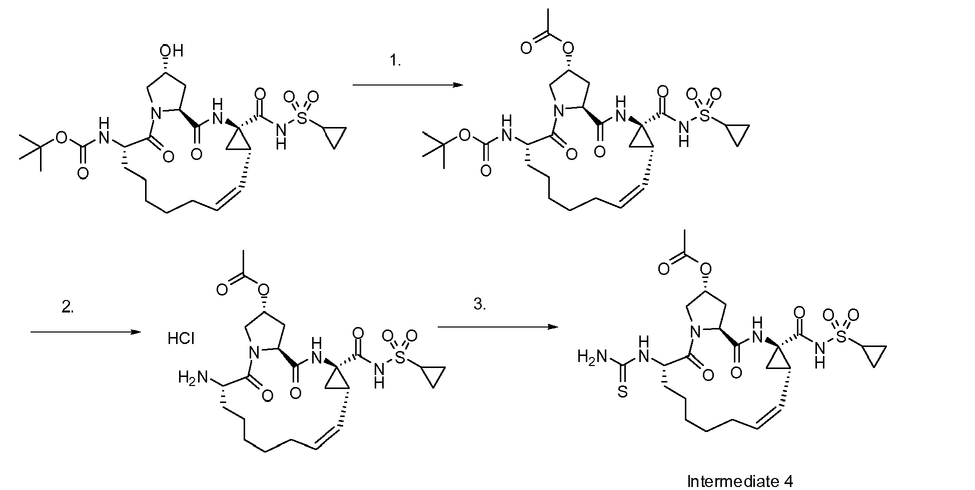 1. Acetyl chloride (1.2 eq), pyridine (15 eq), in THF, room temperature 3 hours, 59%. 2. 4M HCl/dioxane (15 eq), 30 minutes, 100% 3. (a) triethyl amine (5 eq), Thiocarbonyldiimidazole (1.5 eq), in THF, 1 hours, room temperature (b) ammonia (∼10 eq bubbled), 1hr, room temperature, 65%.

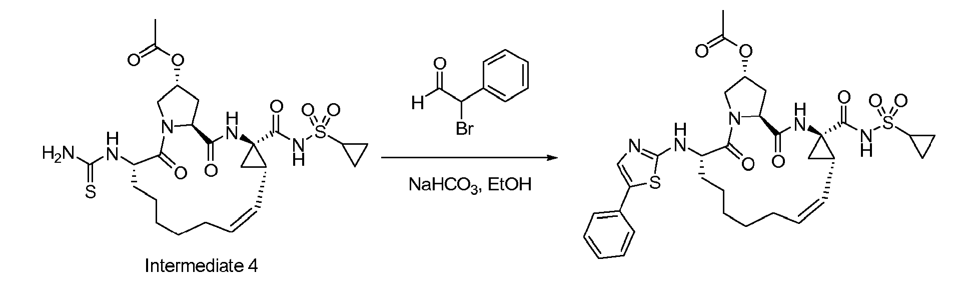

**Intermediate** 4 (70 mg, 0.12 mmol) was disolved in 1 mL EtOH and to this solution was added NaHCO₃ (103 mg, 1.23 mmol) and 2-bromo-2-phenylacetaldehyde (61 mg, 0.31 mmol) and the resulting mixture was heated to 100° C for 10 minutes. The reaction was concentrated *in vacuo* and purified by reverse phase chromatography to provide (2*R*,6*S*,13a*S*,14a*R*,16a*S*,Z)-14a-(cyclopropylsulfonylcarbamoyl)-5,16-dioxo-6-(5-phenylthiazol-2-ylamino)-1,2,3,5,6,7,8,9,10,11,13a, 14,14a, 15,16,16a-hexadecahydrocyclopropa[e]pyrrolo[1,2-a][1,4]diazacyclopentadecin-2-yl acetate, **Compound 203,** (50 mg, 0.075 mmol, 61% yield) as a white solid. LCMS (APCI-) m/z 668.4 (MH⁻).

### Example 9

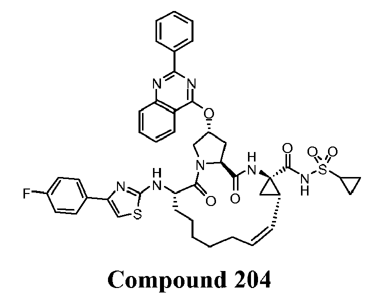

(2*R*,6*S*, 13a*S*, 14a*R*, 16a*S*,Z)-N-(cyclopropylsulfonyl)-6-(4-(4-fluorophenyl)thiazol-2-ylamino)-5,16-dioxo-2-(2-phenylquinazolin-4-yloxy)-1,2,3,5,6,7,8,9,10,11,13a, 14,14a,15,16,16a-hexadecahydrocyclopropa[e]pyrrolo[1,2-a][1,4]diazacyclopentadecine-14a-carboxamide, **Compound 204,** was prepared as follows:

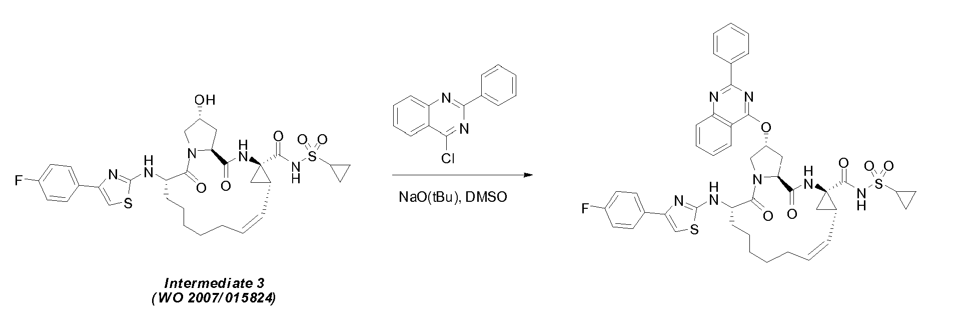

To a dimethyl sulfoxide (0.16 mL) solution of **Intermediate 3** (10 mg, 0.0155 mmol) was added tBuONa (4.46 mg, 0.0465 mmol) at room temperature in one portion. The reaction was stirred for 30 minutes the 4-chloro-2-phenylquinazoline (4.03 mg, 0.0163 mmol) was added and the reaction was stirred at room temperature for overnight. The reaction was then quenched with iced citric acid (10%, aq) and extracted with EtOAc. The combined organic layers were combined and washed with citric acid and brine, then dried (Na₂SO₄). The crude material was purified by reverse-phase column chromatography (25 to 95% MeCN/water), yielding the product as a white solid. LCMS (APCI+) m/z 850.2 (MH⁺).

### Example 10

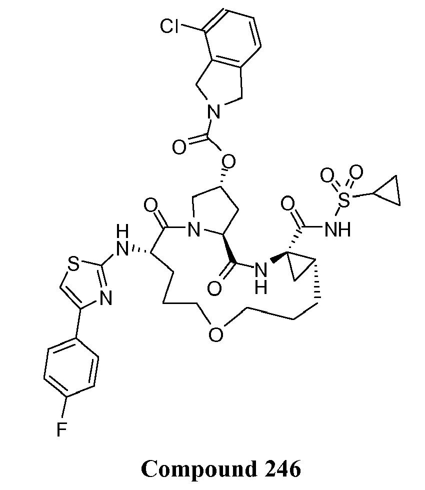

The titled compound, (2*R*,6*S*,13a*R*,14a*R*,16a*S*)-14a-(cyclopropylsulfonylcarbamoyl)-6-(4-(4-fluorophenyl)thiazol-2-ylamino)-5,16-dioxohexadecahydro-1*H*-cyclopropa[e]pyrrolo[2,1-i][1,7,10]oxadiazacyclopentadecin-2-yl 4-fluoroisoindoline-2-carboxylate, **Compound 246,** was prepared as shown in the following scheme:

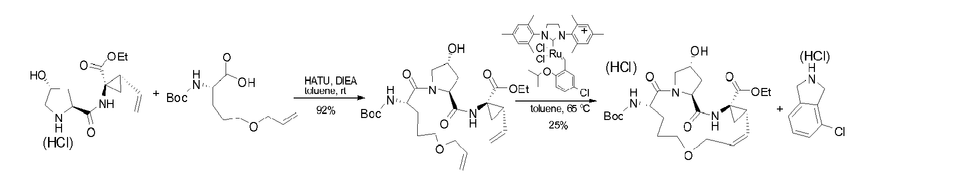

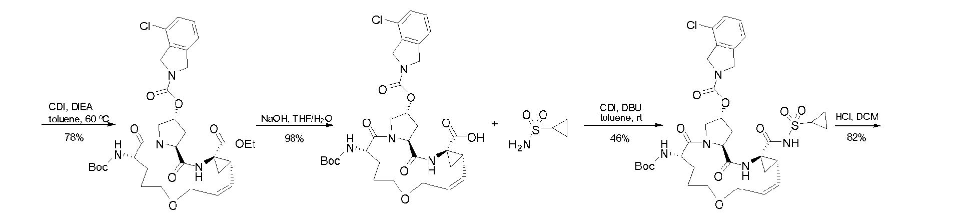

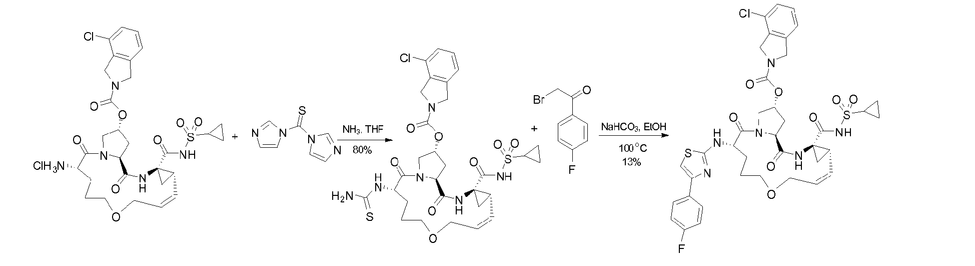

### Step 1: Synthesis of (1R,2S)-ethyl 1-((2S,4R)-1-((S)-5-(allyloxy)-2-(tert-butoxycarbonylamino)pentanoyl)-4-hydroxypyrrolidine-2-carboxamido)-2-vinylcyclopropanecarboxylate

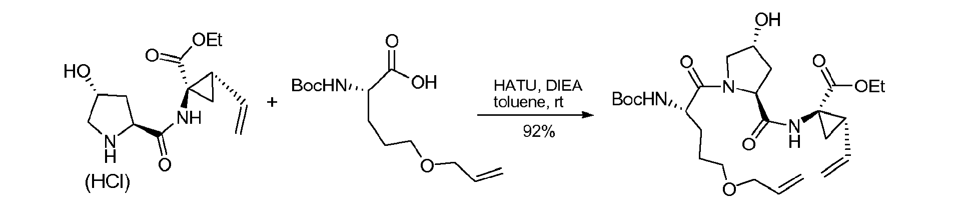

(1*R*,2*S*)-ethyl-1-((2*S*,4*R*)-4-hydroxypyrrolidine-2-carboxamido)-2-vinylcyclopropanecarboxylate hydrochloride salt (WO2005095403) (2.44 g, 7.76 mmol), (*S*)-5-(allyloxy)-2-(*tert*-butoxycarbonylamino)-pentanoic acid (WO2004094452) (2.02 g, 7.39 mmol) and 2-(1*H*-7-azabenzotriazol-1-yl)-1,1,3,3-tetramethyl uronium hexafluorophosphate (3.09 g, 8.13 mmol) in toluene (36 mL) and MeCN (4 mL) was added diisopropylethylamine (2.58 mL, 14.78 mmol) at 0 °C. The reaction warmed to room temperature and stirred at room temperature for 1 hours. Ethyl acetate (30 mL) and water (20 mL) was added. The organic layer was separated and washed with brine, dried over sodium sulfate. After removal of solvent, the residue was purified by chromatography (Ethyl acetate) to give (1*R*,2*S*)-ethyl 1-((2*S*,4*R*)-1-((*S*)-5-(allyloxy)-2-(*tert-*butoxycarbonylamino)pentanoyl)-4-hydroxypyrrolidine-2-carboxamido)-2-vinylcyclopropanecarboxylate as white wax solid (3.55 g, 92%). MS: Calcd.: 523; Found: [M+H]⁺ 524.

### Step 2: Synthesis of (2R,6S,13aS,14aR,16aS,Z)-ethyl 6-(tert-butoxycarbonylamino)-2-hydroxy-5,16-dioxo-2,3,5,6,7,8,9,11,13a,14,14a,15,16,16a-tetradecahydro-1H-cyclopropa(e)pyrrolo[2,1-i][1,7,10]oxadiazacyclopentadecine-14a-carboxylate

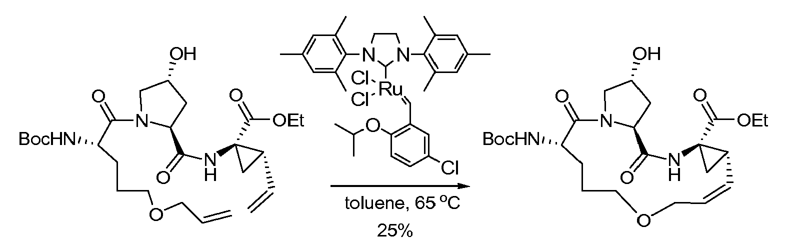

(1*R*,2*S*)-ethyl 1-((2*S*,4*R*)-1-((*S*)-5-(allyloxy)-2-(*tert-*butoxycarbonylamino)pentanoyl)-4-hydroxypyrrolidine-2-carboxamido)-2-vinylcyclopropanecarboxylate (3.55 g, 6.78 mmol) in toluene (750 mL) was degassed by bubbling a stream of nitrogen through the reaction for 1 hours at room temperature. (5-chloro-2-isopropoxybenzylidene)(1,3-dimesitylimidazolidin-2-yl)ruthenium(V) chloride (0.090 g, 0.14 mmol) was added to the mixture and the mixture was heated to 68 °C (oil bath) and stirred at this temperature for 4 hours. After removal of solvent, the residue was purified by chromatography (Ethyl acetate:MeOH=40:1) to give (2*R*,6*S*,13a*S*,14a*R*,16a*S*,*Z*)-ethyl 6-(*tert*-butoxycarbonylamino)-2-hydroxy-5,16-dioxo-2,3,5,6,7,8,9,11,13a,14,14a,15,16,16a-tetradecahydro-1*H*-cyclopropa(*e*)pyrrolo[2,1-i][1,7,10]oxadiazacyclopentadecine-14a-carboxylate as off white solid (0.84 g, 25%). MS: Calcd.: 495; Found: [M+H]⁺ 496. ¹H NMR (400 MHz, DMSO-*d*⁶) δ 8.42 (s, 1H), 6.89 (d, *J*=7.6 Hz, 1H), 5.48-5.60 (m, 2H), 5.10 (d, *J*=3.6 Hz, 1H), 4.41 (s, 1H), 4.27 (m, 2H), 4.17 (m, 1H), 4.02 (m, 2H), 3.72 (m, 2H), 3.62 (m, 1H), 3.35 (m, 1H), 3.28 (m, 1H), 2.42 (m, 1H), 1.98 (m, 2H), 1.78 (m, 1H), 1.62 (m, 1H), 1.52 (m, 2H), 1.42 (m, 2H), 1.36 (s, 9 H), 1.13 (t, *J*=7.2 Hz, 3H).

### Step 3: Synthesis of (2R,6S,13aS,14aR,16aS,Z)-ethyl 6-(tert-butoxycarbonylamino)-2-(4-chloroisoindoline-2-carbonyloxy)-5,16-dioxo-2,3,5,6,7,8,9,11,13a,14,14a,15,16,16a-tetradecahydro-1H-cyclopropa(e)pyrrolo[2,1-i][1,7,10] oxadiazacyclopentadecine-14a-carboxylate

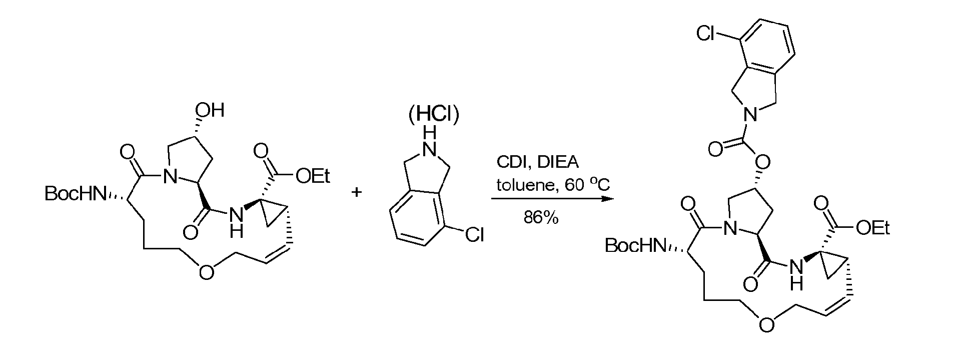

(2*R*,6*S*,13a*S*,14a*R*,16a*S*,*Z*)-ethyl 6-(*tert*-butoxycarbonylamino)-2-hydroxy-5,16-dioxo-2,3,5,6,7,8,9,11,13a,14,14a,15,16,16a-tetradecahydro-1*H*-cyclopropa(*e*)pyrrolo[2,1-*i*][1,7,10]oxadiazacyclopentadecine-14a-carboxylate (0.30 g, 0.61 mmol) in toluene (5 mL) was added 1,1'-carbonyldiimidazole (0.12 g, 0.73 mmol) in one portion. The reaction was stirred at room temperature for 3 hours. To the reaction was then added the N-ethyl-N-isopropylpropan-2-amine (0.53 mL, 3.0 mmol), followed by 4-chloroisoindoline hydrochloride salts (0.15 g, 0.79 mmol). The reaction was stirred at 60 °C for 3 hours. The solvent was removed. The residue was partitioned between ethyl acetate (20 mL) and saturated sodium bicarbonate solution. The organic layer was separated and dried over sodium sulfate. After removal of solvent, the residue was purified by chromatography (Hexane:Ethyl acetate=1:3) to give (2*R*,6*S*,13a*S*,14a*R*,16a*S*,*Z*)-ethyl 6-(*tert-*butoxycarbonylamino)-2-(4-chloroisoindoline-2-carbonyloxy)-5,16-dioxo-2,3,5,6,7,8,9,11,13a,14,14a,15,16,16a-tetradecahydro-1*H*-cyclopropa(*e*)pyrrolo[2,1-*i*][1,7,10]oxadiazacyclopentadecine-14a-carboxylate as white solid (0.18 g, 86%). MS: Calcd.: 674.3; Found: [M+H]⁺ 675.1.

### Step 4: Synthesis of (2R,6S,13aS,14aR,16aS,Z)-6-(tert-butoxycarbonylamino)-2-(4-chloroisoindoline-2-carbonyloxy)-5, 16-dioxo-2,3,5,6,7,8,9,11,13a,14,14a,15,16,16a-tetradecahydro-1H-cyclopropa(e)pyrrolo[2,1-i][1,7,10] oxadiazacyclopentadecine-14a-carboxylic acid

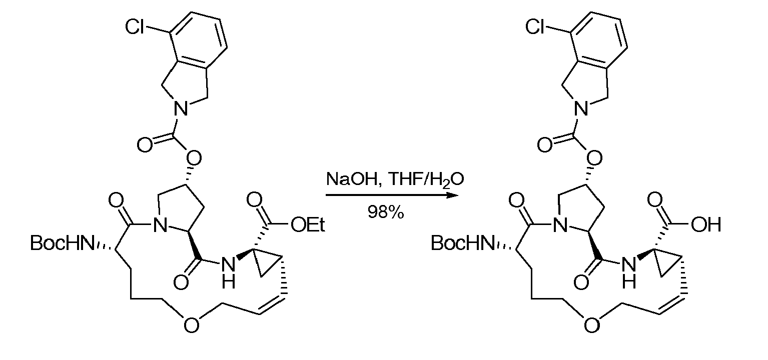

(2*R*,6*S*, 13a*S*, 14a*R*, 16a*S*,*Z*)-ethyl 6-(*tert*-butoxycarbonylamino)-2-(4-chloroisoindoline-2-carbonyloxy)-5, 16-dioxo-2,3,5,6,7,8,9,11,13a, 14,14a, 15,16,16a-tetradecahydro-1*H*-cyclopropa(*e*)pyrrolo[2,1-*i*][1,7,10]oxadiazacyclopentadecine-14a-carboxylate (0.32 g, 0.47 mmol) in THF (6 mL) was added 0.4 N NaOH solution (2.96 mL, 1.18 mmol). The reaction was stirred at room temperature for 3 days. Water (5 mL) and ether (15 mL) was added. The aqueous layer was separated and acidified by saturated potassium hydrogen sulfate solution to pH=2∼3. The aqueous layer was extracted with EtOAc (2x15 mL), washed with brine and dried over sodium sulfate. After removal of solvent, it gave (2*R*,6*S*,13a*S*,14a*R*,16a*S*,*Z*)-6-(*tert*-butoxycarbonylamino)-2-(4-chloroisoindoline-2-carbonyloxy)-5,16-dioxo-2,3,5,6,7,8,9,11,13a, 14,14a, 15,16,16a-tetradecahydro-1*H*-cyclopropa(*e*)pyrrolo[2, 1-*i*][1,7,10]oxadiazacyclopentadecine-14a-carboxylic acid as white solid (0.30 g, 98%). MS: Calcd.: 646.2; Found: [M+H]⁺ 647.1.

### Step 5: Synthesis of (2R,6S,13aS,14aR,16aS,Z)-6-(tert-butoxycarbonylamino)-14a-(cyclopropylsulfonylcarbamoyl)-5, 16-dioxo-2,3,5,6,7,8,9,11,13a,14,14a,15,16,16a-tetradecahydro-1H-cyclopropa(e)pyrrolo[2,1-i][1,7,10]oxadiazacyclopentadecin-2-yl 4-chloroisoindoline-2-carboxylate

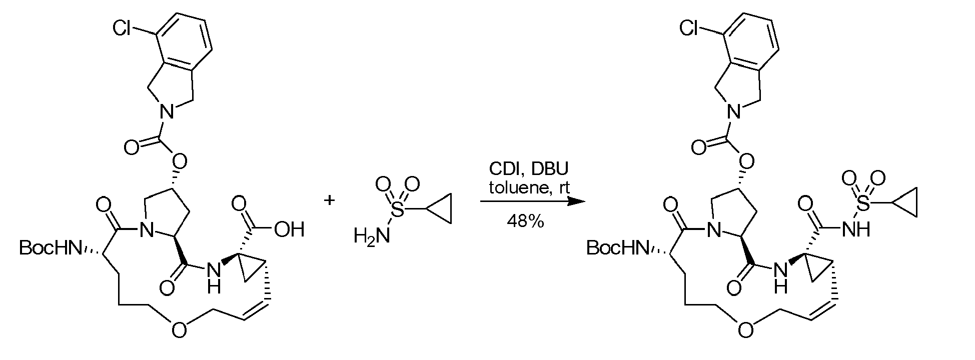

(2*R*,6*S*,13a*S*,14a*R*,16a*S*,*Z*)-6-(*tert*-butoxycarbonylamino)-2-(4-chloroisoindoline-2-carbonyloxy)-5,16-dioxo-2,3,5,6,7,8,9,11,13a,14,14a,15,16,16a-tetradecahydro-1*H-*cyclopropa(*e*)pyrrolo[2,1-*i*][1,7,10]oxadiazacyclopentadecine-14a-carboxylic acid (0.30 g, 0.46 mmol) in toluene (3 mL) was added 1,1'-carbonyldiimidazole (0.097 g, 0.60 mmol) in room temperature. The reaction was stirred at 60 °C for 3 hours. Cyclopropanesulfonamide (0.084 g, 0.69 mmol) was added, followed by addition of 1,8-diazabicyclo[5.4.0]undec-7-ene (0.14 mL, 0.92 mmol). The reaction was then stirred at room temperature for 17 hours. Water (5 mL) was added and acidified with saturated potassium hydrogen sulfate until pH=2∼3. The mixture was extracted with ethyl acetate (20 mL), washed with brine and dried over sodium sulfate. After removal of solvent, the residue was purified by chromatography (Ethyl acetate) to give (2*R*,6*S*,13a*S*,14a*R*,16a*S*,*Z*)-6-(*tert-*butoxycarbonylamino)-14a-(cyclopropylsulfonylcarbamoyl)-5, 16-dioxo-2,3,5,6,7,8,9,11,13a,14,14a,15,16,16a-tetradecahydro-1*H*-cyclopropa(*e*)pyrrolo[2,1-*i*][1,7,10]oxadiazacyclopentadecin-2-yl 4-chloroisoindoline-2-carboxylate as white solid (0.16 g, 46%). MS: Calcd.: 749.3; Found: [M+H]⁺750.0.

### Step 6: Synthesis of (2R,6S,13aS,14aR,16aS,Z)-6-amino-14a-(cyclopropylsulfonylcarbamoyl)-5,16-dioxo-2,3,5,6,7,8,9,11,13a,14,14a,15,16,16a-tetradecahydro-1H-cyclopropa[e]pyrrolo[2,1-i][1,7,10]oxadiazacyclopentadecin-2-yl 4-chloroisoindoline-2-carboxylate hydrochloride

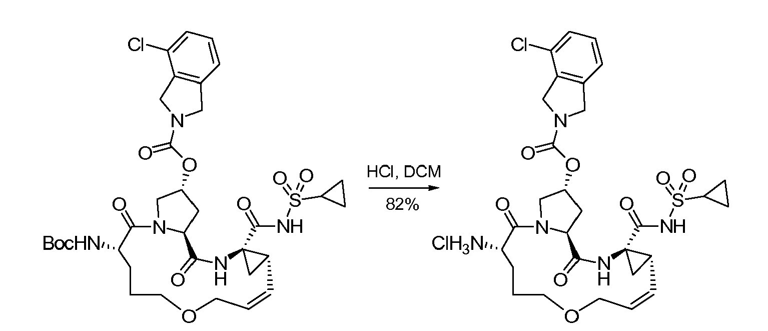

(2*R*,6*S*,13a*S*,14a*R*,16a*S*,*Z*)-6-(*tert*-butoxycarbonylamino)-14a-(cyclopropylsulfonylcarbamoyl)-5,16-dioxo-2,3,5,6,7,8,9,11,13a, 14,14a, 15,16,16a-tetradecahydro-1H-cyclopropa(*e*)pyrrolo[2,1-*i*][1,7,10]oxadiazacyclopentadecin-2-yl 4-chloroisoindoline-2-carboxylate (0.12 g, 0.16 mmol) in DCM (5 mL) was added HCl (0.24 mL, 0.96 mmol) in dioxane. The reaction mixture was stirred at room temperature for 3 days. The solvent was removed to give the (2*R*,6*S*,13a*S*,14a*R*,16a*S*,*Z*)-6-amino-14a-(cyclopropylsulfonylcarbamoyl)-5,16-dioxo-2,3,5,6,7,8,9,11,13a,14,14a,15,16,16a-tetradecahydro-1H-cyclopropa[e]pyrrolo[2,1-i][1,7,10]oxadiazacyclopentadecin-2-yl 4-chloroisoindoline-2-carboxylate hydrochloride as white solid (0.090 g, 82%).

### Step 7: Synthesis of (2R,6S,13aS,14aR,16aS,Z)-14a-(cyclopropylsulfonylcarbamoyl)-5,16-dioxo-6-thioureido-2,3,5,6,7,8,9,11,13a,14,14a,15,16,16a-tetradecahydro-1H-cyclopropa[e]pyrrolo[2,1-i][1,7,10]oxadiazacyclopentadecin-2-yl 4-chloroisoindoline-2-carboxylate

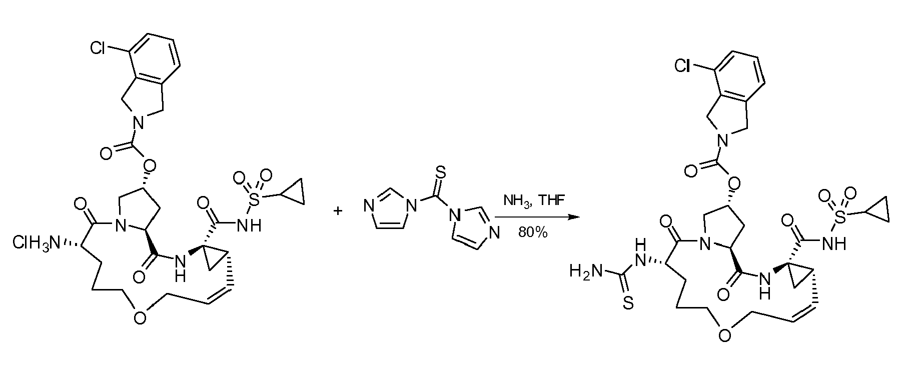

(2*R*,6*S*,13a*S*, 14a*R*, 16a*S*,*Z*)-6-amino-14a-(cyclopropylsulfonylcarbamoyl)-5,16-dioxo-2,3,5,6,7,8,9,11,13a,14,14a,15,16,16a-tetradecahydro-1*H*-cyclopropa[*e*]pyrrolo[2,1-*i*][1,7,10]oxadiazacyclopentadecin-2-yl 4-chloroisoindoline-2-carboxylate hydrochloride (0.090 g, 0.13 mmol) and triethyl amine (d = 0.726 g/mL) (0.037 mL, 0.26 mmol) in THF (10 mL) was added di(1*H*-imidazol-1-yl)methanethione (0.035 g, 0.20 mmol). The reaction was stirred at room temperature for 3 hours. Bubbled NH₃ for 15 minutes and sealed. It was stirred at room temperature for 40 mins. Water (5 mL) was added and acidified with saturated potassium hydrogen sulfate until pH=2∼3. The mixture was extracted with ethyl acetate (20 mL), washed with brine and dried over sodium sulfate. After removal of solvent provided (2*R*,6*S*,13a*S*,14a*R*,16a*S*,*Z*)-14a-(cyclopropylsulfonylcarbamoyl)-5,16-dioxo-6-thioureido-2,3,5,6,7,8,9,11,13a, 14,14a, 15,16,16a-tetradecahydro-1*H-*cyclopropa[e]pyrrolo[2,1-*i*][1,7,10]oxadiazacyclopentadecin-2-yl 4-chloroisoindoline-2-carboxylate as white solid (0.074 g, 80%).

### Step 8: Synthesis of (2R,6S,13aS,14aR,16aS,Z)-14a-(cyclopropylsulfonylcarbamoyl)-6-(4-(4-fluorophenyl)thiazol-2-ylamino)-5,16-dioxo-2,3,5,6,7,8,9,11,13a,14,14a,15,16,16a-tetradecahydro-1H-cyclopropa[e]pyrrolo[2,1-i][1,7,10]oxadiazacyclopentadecin-2-yl 4-chloroisoindoline-2-carboxylate

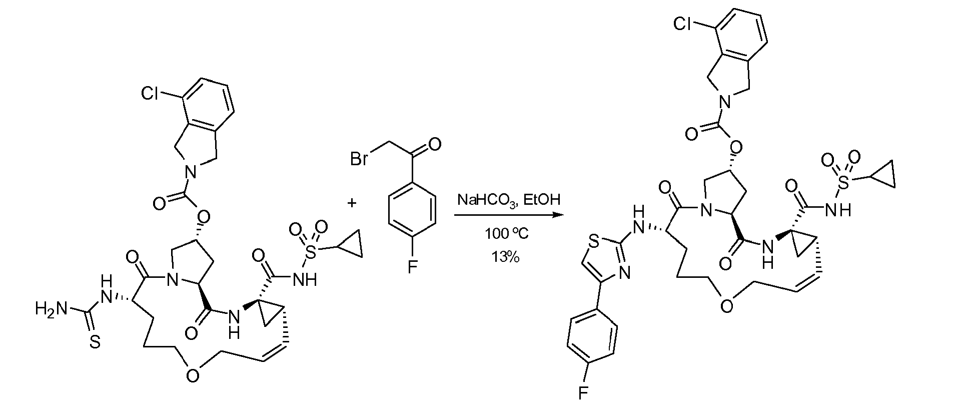

(2*R*,6*S*,13a*S*, 14a*R*, 16a*S*,*Z*)-14a-(cyclopropylsulfonylcarbamoyl)-5,16-dioxo-6-thioureido-2,3,5,6,7,8,9,11,13a, 14,14a, 15,16,16a-tetradecahydro-1*H-*cyclopropa[e]pyrrolo[2,1-*i*][1,7,10]oxadiazacyclopentadecin-2-yl 4-chloroisoindoline-2-carboxylate (0.074 g, 0.10 mmol), NaHCO₃ (0.044 g, 0.52 mmol) and 2-bromo-1-(4-fluorophenyl)ethanone (0.034 g, 0.16 mmol) in EtOH (2 mL) was sealed and heated to 100°C for 5 minutes. The solvent was removed. Water (5 mL) was added and acidified with saturated potassium hydrogen sulfate until pH=2∼3. The mixture was extracted with ethyl acetate (20 mL), washed with brine and dried over sodium sulfate. After removal of solvent, the residue was purified by chromatography (Ethyl acetate) to give (2*R*,6*S*, 13a*S*, 14a*R*, 16a*S*,*Z*)-14a-(cyclopropylsulfonylcarbamoyl)-6-(4-(4-fchlorophenyl)thiazol-2-ylamino)-5,16-dioxo-2,3,5,6,7,8,9,11,13a,14,14a,15,16,16a-tetradecahydro-1H-cyclopropa[e]pyrrolo[2,1-*i*][1,7,10]oxadiazacyclopentadecin-2-yl 4-chloroisoindoline-2-carboxylate, **Compound 246,** as white solid (0.011 g, 13%). MS: Calcd.: 826.2; Found: [M+H]⁺ 827.1.

**Table 2: Additional examples of compound prepared using Example procedures 6-9**

| Compound | Structure | Mass spectra data | Example Procedure Used |
|---|---|---|---|
| 206 | 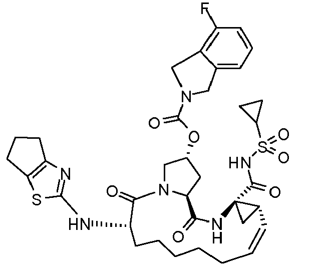 | (APCI-)m/z 753 (M-1) | 6 |
| 207 | 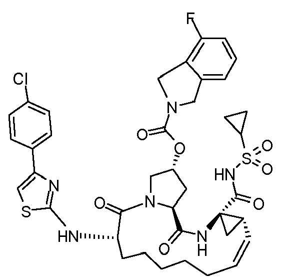 | (APCI-) m/z 823 (M-1) | 6 |
| 208 | 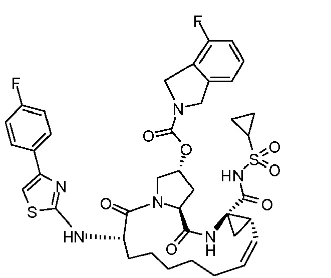 | (APCI-) m/z 807 (M-1) | 6 |
| 209 | 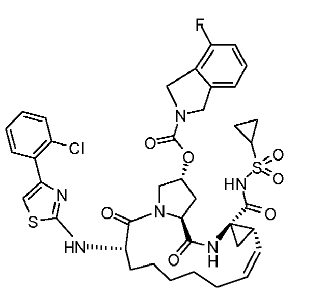 | (APCI-) m/z 823 (M-2) | 6 |
| 210 | 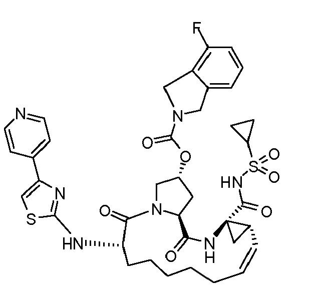 | (APCI-) m/z 790 (M-1) | 6 |
| 211 | 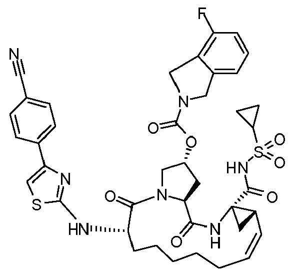 | (APCI-) m/z 814 (M-1) | 6 |
| 212 | 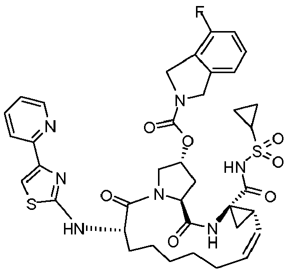 | (APCI-) m/z 790 (M-1) | 6 |
| 213 | 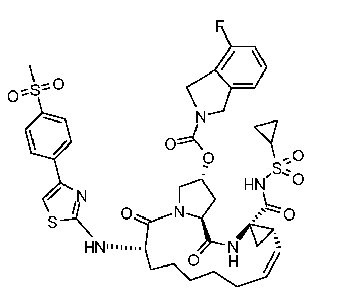 | (APCI-) m/z 868 (M-1) | 6 |
| 214 | 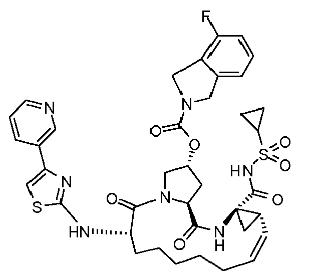 | (APCI-) m/z 790 (M-1) | 6 |
| 215 | 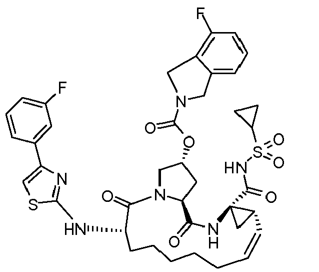 | (APCI-) m/z 808 (M-0) | 6 |
| 216 | 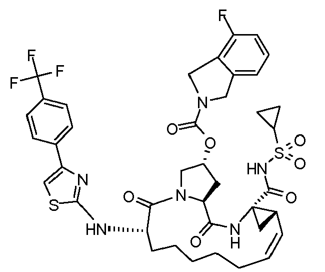 | (APCI-) m/z 857 (M-1) | 6 |
| 217 | 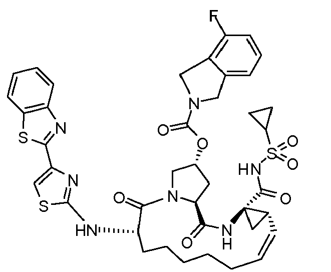 | (APCI-) m/z 846 (M-1) | 6 |
| 218 | 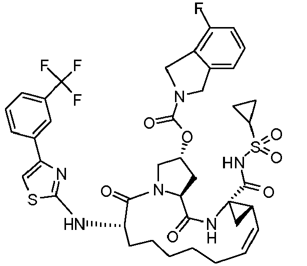 | (APCI-) m/z 858 (M-0) | 6 |
| 219 | 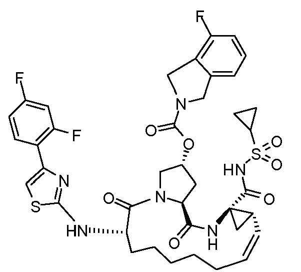 | (APCI-) m/z 825 (M-1) | 6 |
| 220 | 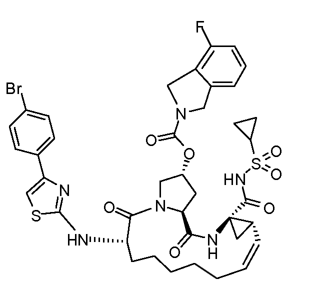 | (APCI-) m/z 869 (M-0) | 6 |
| 221 | 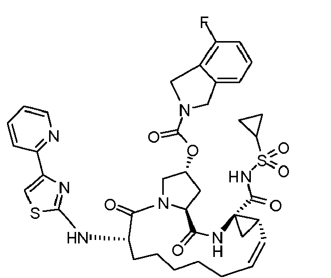 | (APCI-) m/z 790 (M-1) | 6 |
| 222 | 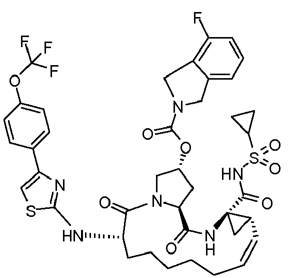 | (APCI-) m/z 873 (M-0) | 6 |
| 223 | 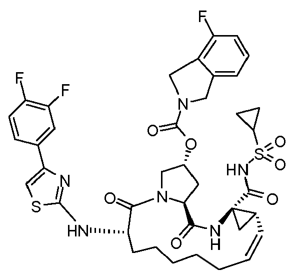 | (APCI-) m/z 825 (M-1) | 6 |
| 224 | 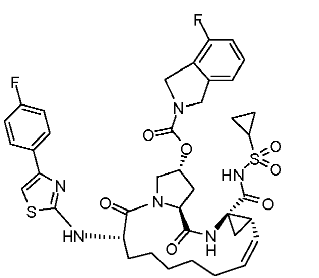 | (APCI-) m/z 807 (M-1) | 6 |
| 225 | 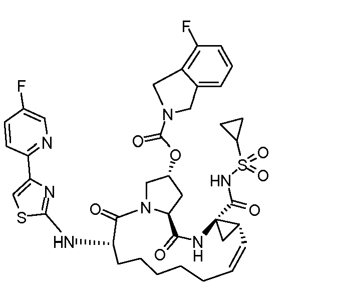 | (APCI-) m/z 808 (M-1) | 6 |
| 226 | 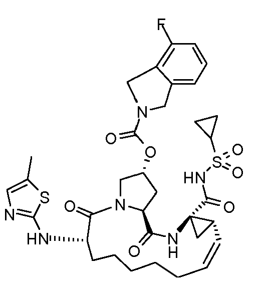 | (APCI-) m/z 727 (M-1) | 6 |
| 227 | 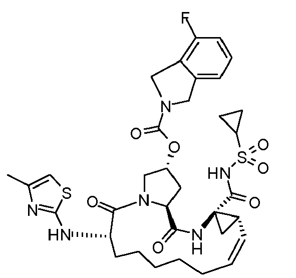 | (APCI-) m/z 727 (M-1) | 6 |
| 228 | 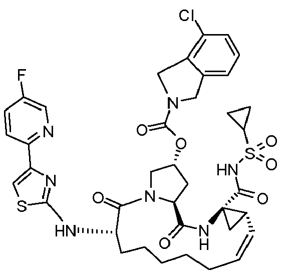 | (APCI-) m/z 824 (M-2) | 6 |
| 229 | 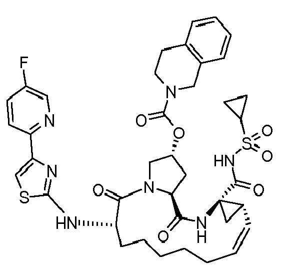 | (APCI-) m/z 804 (M-1) | 6 |
| 230 | 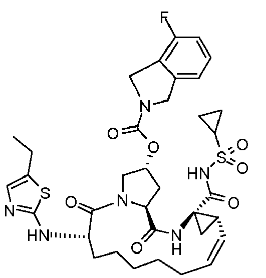 | (APCI-) m/z 741 (M-1) | 6 |
| 231 | 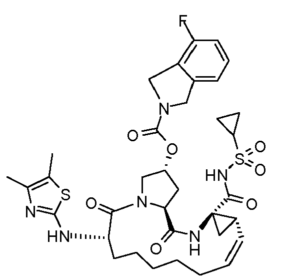 | (APCI-) m/z 741 (M-1) | 6 |
| 232 | 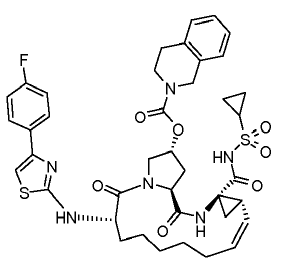 | (APCI-) m/z 803 (M-1) | 6 |
| 233 | 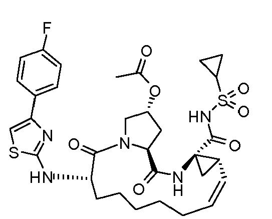 | (APCI-) m/z 686 (M-1) | 8 |
| 235 | 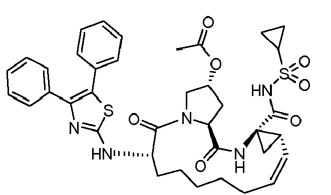 | (APCI-) m/z 744 (M-1) | 6 |
| 239 | 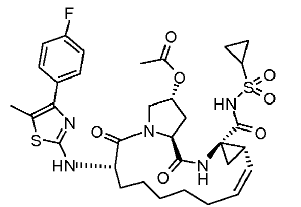 | (APCI-) m/z 700 (M-1) | 8 |
| 241 | 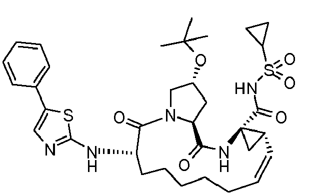 | (APCI-) m/z 682 (M-1) | 7 |
| 242 | 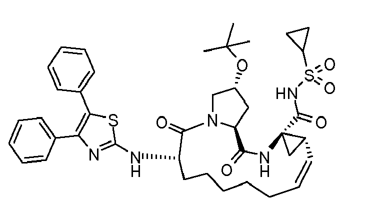 | (APCI-) m/z 759 (M-0) | 7 |
| 243 | 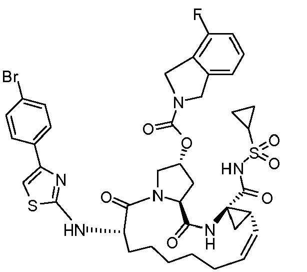 | (APCI-) m/z 869 (M-0) | 6 |

### Example 11

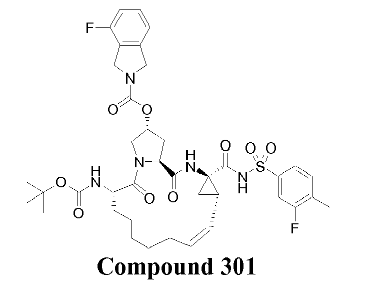

(2*R*,6*S*, 13a*S*, 14a*R*, 16a*S*,*Z*)-6-(tert-butoxycarbonylamino)-14a-(3-fluoro-4-methylphenylsulfonylcarbamoyl)-5,16-dioxo-1, 2, 3, 5, 6, 7, 8, 9, 10, 11, 13a, 14, 14a, 15, 16, 16a-hexadecahydrocyclopropa[e]pyrrolo[1,2-a][1,4]diazacyclopentadecin-2-yl 4-fluoroisoindoline-2-carboxylate, **Compound 301,** was prepared as shown in the following scheme:

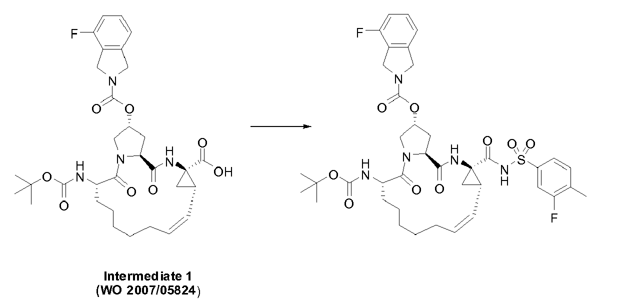

**Intermediate 1** (50 mg, 0.080 mmol), was dissolved in 0.4 mL anhydrous THF, followed by addition of 1,1'-carbonyldiimidazole (14 mg, 0.088 mmol) in one portion, and the reaction was stirred at room temperature for 3 hours, then benzenesulfonamide (18 mg, 0.12 mmol) was added, followed by addition of DBU (18 mg, 0.12 mmol), and the reaction was heated at 50 °C for 2h. After completion, the reaction mixture was directly purified by reverse-phase column chromatography (eluent = 5% to 85% MeCN in water), giving the (2*R*,6*S*, 13a*S*, 14a*R*, 16a*S*,*Z*)-6-(tert-butoxycarbonylamino)-14a-(3-fluoro-4-methylphenylsulfonylcarbamoyl)-5,16-dioxo-1,2,3,5,6,7,8,9,10,11,13 a,14,14a,15,16,16a-hexadecahydrocyclopropa[e]pyrrolo[1,2-a][1,4]diazacyclopentadecin-2-yl 4-fluoroisoindoline-2-carboxylate, **Compound 301** as a white solid (38 mg, 60 % yield).

### Example 12

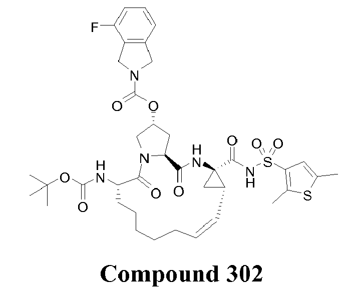

(2R,6S, 13a*S*, 14a*R*, 16a*S*,*Z*)-6-(tert-butoxycarbonylamino)-14a-(2,5-dimethylthiophen-3-ylsulfonylcarbamoyl)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 302,** was prepared as shown in the following scheme:

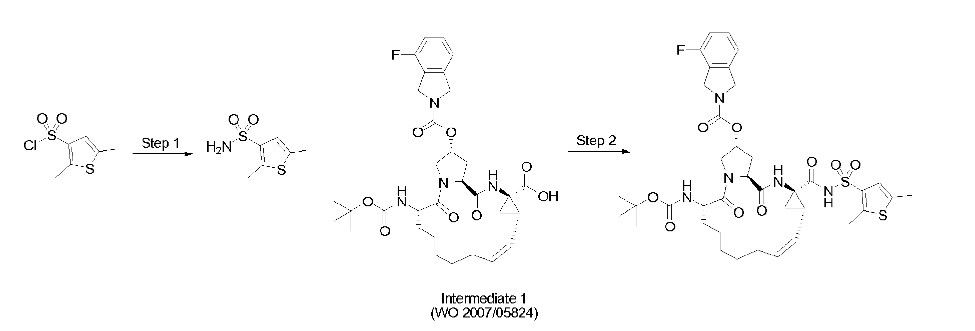

Step 1: Anhydrous THF (20 mL) was cooled to 0 °C and was saturated with anhydrous ammonia gas using a gas dispersion tube with bubbling for 10 minutes. To the solution was added 2,5-dimethylthiophene-3-sulfonyl chloride (530 mg, 2.5 mmol) in anhydrous THF (2.5 mL) dropwise over 15 minutes (immediate white ppt formed during addition). After addition, the reaction mixture was stirred at 0 °C for 1h and was purged with dry nitrogen for 10 minutes. The purged mixture was diluted with an equal volume of hexanes, treated with activated carbon and filtered through a Celite plug capped with MgSO₄ layer. The solution was concentrated to give a white solid. The solid was dissolved in dry Et₂O and filtered through a Celite pad capped with MgSO₄ layer. The Et₂O solution was concentrated to give 2,5-dimethylthiophene-3-sufonamide as a white solid (449 mg, 94%). ¹H NMR (CDCl₃) δ 2.39 (s, 3H), 2.63 (s, 3H), 4.85 (br s, 2H), 6.94 (s, 1H).

Step 2: To a solution of carbonyl diimidazole (19.5 mg, 0.12 mmol) in dry THF (1.0 mL) was added **Intermediate 1** and the mixture stirred at room temperature for 15h under a nitrogen atmosphere. Dimethylthiophene-3-sufonamide sulfonamide (23.0 mg, 0.12 mmol) and DBU (22.9, 0.15 mmol) were sequentially added and mixture stirred at 60 °C for 7h. The mixture was cooled to room temperature and the THF was evaporated. The residue was treated Et₂O (2 mL) and 1M HCl (3 mL) and the biphasic mixture was stirred until both layers were homogeneous. The Et₂O layer was removed and the remaining aqueous layer was extracted with Et₂O. The combined Et₂O extracts were washed with H₂O and saturated aqueous NaCl. The solution was dried over MgSO₄ and filtered through a silica gel plug capped with a MgSO₄ layer (EtOAc elution). The solution was concentrated to give the crude product as a white solid which was purified on a SiO₂ column (CH₂Cl₂, 25%, 50%, 100% EtOAc/hexanes step-gradient elution) yielding pure (2*R*,6*S*,13a*S*,14a*R*,16a*S*,*Z*)-6-(tert-butoxycarbonylamino)-14a-(2,5-dimethylthiophen-3-ylsulfonylcarbamoyl)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 302,** 46 mg, 57%. MS (apci negative) 800.4 (M-1).

### Example 13

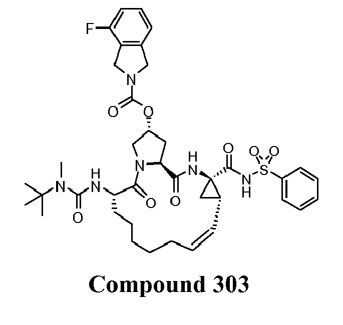

The titled compound, (2*R*,6*S*,13a*S*,14a*R*,16a*S*,*Z*)-6-(3-tert-butyl-3-methylureido)-5,16-dioxo-14a-(phenylsulfonylcarbamoyl)-1,2,3,5,6,7,8,9,10,11,13 a,14,14a,15,16,16a-hexadecahydrocyclopropa[e]pyrrolo[1,2-a][1,4]diazacyclopentadecin-2-yl 4-fluoroisoindoline-2-carboxylate, **Compound 303,** was prepared as shown in the following scheme:

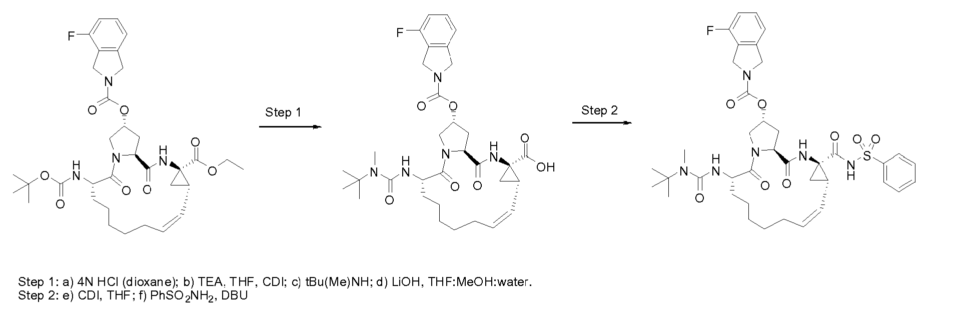

### Step 1: Synthesis of (2R,6S,13aS,14aR,16aS,Z)-6-(3-tert-butyl-3-methylureido)-2-(4-fluoroisoindoline-2-carbonyloxy)-5,16-dioxo-1,2,3,5,6,7,8,9,10,11,13a,14,14a,15,16,16a-hexadecahydrocyclopropa[e]pyrrolo[1,2-a][1,4]diazacyclopentadecine-14a-carboxylic acid.

a) The macrocyclic ester, (2*R*,6*S*,13a*S*,14a*R*,16a*S*,*Z*)-ethyl 6-(tert-butoxycarbonylamino)-2-(4-fluoroisoindoline-2-carbonyloxy)-5,16-dioxo-1,2,3,5,6,7,8,9,10,11,13a,14,14a,15,16,16a-hexadecahydrocyclopropa[e]pyrrolo[1,2-a][1,4]diazacyclopentadecine-14a-carboxylate (1.9 g, 2.89 mmol), was first deprotected with 4N HCl (dioxane) (20 mL) at room temperature for 4h.
b) After removal of solvent, the white solid residue was dissolved in 30 mL THF and treated with triethylamine (1.21 mL, 8.68 mmol). White suspension upon triethyl amine treatment.
c) To the free-based amine suspension was added 1,1'-carbonyldiimidazole (0.704 g, 4.34 mmol) in one portion at room temperature and stirred for 4h, followed by addition of methyl-tert-butyl amine (1.73 mL, 14.5 mmol) in one portion. After stirring at room temperature for overnight, the reacdtion was concentrated down. The thick residue was re-suspended in 150 mL EtOAc and washed with 1N HCl (2 × 100 mL), water and brine (100 mL each), and dried (Na₂SO₄), yielding the fairly clean crude product as 1.85 g white foamy solid.
d) The crude product from the previous step (1.85 g, 2.76 mmol) was dissolved in a mixture solvent of THF/MeOH/water (2:2:1 v/v, 27 mL) followed by addition of LiOH·H₂O (0.348 g, 8.29 mmol) in one portion. The reaction was stirred at room temperature for overnight. After removal of solvents, the solid residue was redissolved in 150 mL water and washed with ethyl ether (100 mL). The aqueous layer was then acidified with 1N HCl to pH ∼ 2, and extracted with EtOAc (3 × 100 mL). The combined organic layers was washed with water, brine, and dried (Na₂SO₄). Removal of solvent gave the desired product as a white glassy solid, 1.58 g. It was sufficiently pure to be used directly in the next coupling step without further purification.

### Step 2: Synthesis of (2R,6S,13aS,14aR,16aS,Z)-6-(3-tert-butyl-3-methylureido)-5,16-dioxo-14a-(phenylsulfonylcarbamoyl)-1,2,3,5,6,7,8,9,10,11,13a,14,14a,15,16,16a-hexadecahydrocyclopropa[e]pyrrolo[1,2-a] [1,4] diazacyclopentadecin-2-yl 4-fluoroisoindoline-2-carboxylate, Compound 303.

The crude product from Step 1, (50 mg, 0.078 mmol), was dissolved in 0.4 mL DriSolve THF, followed by addition of 1,1'-carbonyldiimidazole (14 mg, 0.086 mmol) in one portion, and the reaction was stirred at room temperature for 3h. Then benzenesulfonamide (18 mg, 0.12 mmol) was added, followed by addition of DBU (18 mg, 0.12 mmol), and the reacdtion was heated at 50 °C for 2h. After completion, the reaction mixture was directly purified by reverse-phase column chromatography (eluent = 5 to 85% MeCN in water), giving of (2*R*,6*S*,13a*S*,14a*R*,16a*S*,*Z*)-6-(3-tert-butyl-3-methylureido)-5,16-dioxo-14a-(phenylsulfonylcarbamoyl)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 303,** as a white solid (25 mg, 41 % yield).

### Example 14

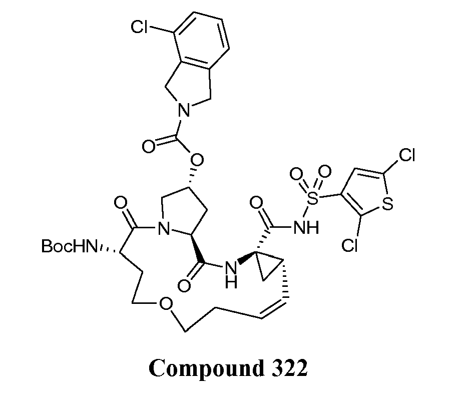

(2*R*,6*S*, 13a*S*, 14a*R*, 16a*S*,*Z*)-6-(tert-butoxycarbonylamino)-14a-(2,5-dichlorothiophen-3-ylsulfonylcarbamoyl)-5,16-dioxo-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate, **Compound 322,** was prepared as shown in the following scheme:

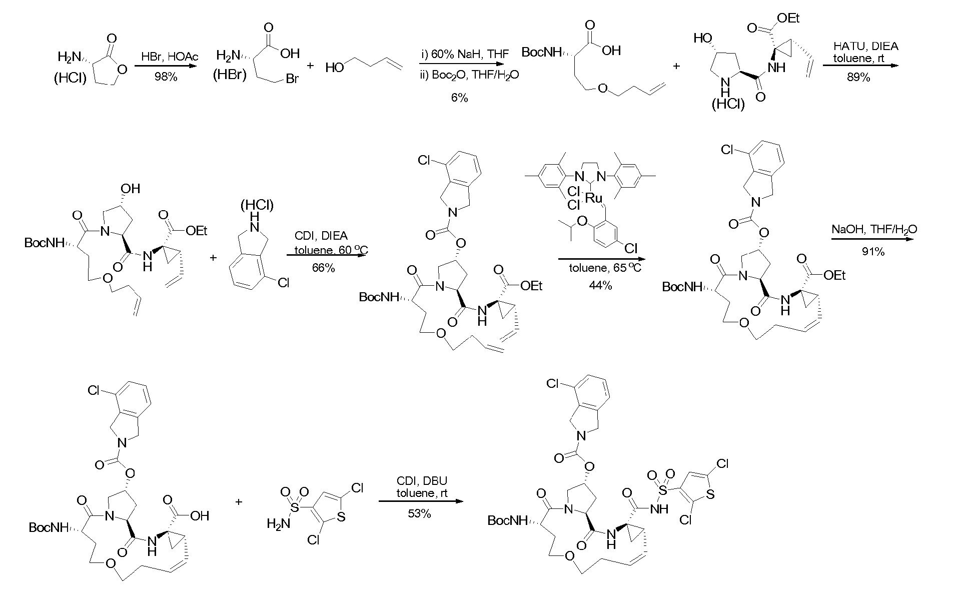

### Step 1: Synthesis of (S)-2-amino-4-bromobutanoic acid hydrobromide

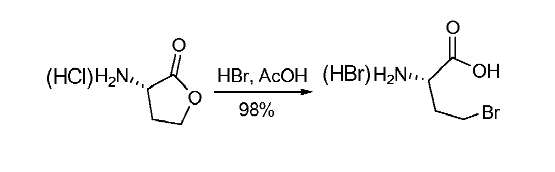

(*S*)-3-aminodihydrofuran-2(3*H*)-one hydrochloride (10.30 g, 74.87 mmol) in 58 mL of 30% w/w HBr in AcOH was stirred at 65 °C for 30 hours. The solvent was removed under reduced pressure and the resulted solid was suspended in MTBE (200 mL) and stirred for 30 minutes. The solid was collected by filtration and washed with MTBE (200 mL) and dried to give (*S*)-2-amino-4-bromobutanoic acid hydrobromide as white solid (19.33 g, 98%). ¹H NMR (400 MHz, DMSO-*d*⁶) δ 8.37 (s, 3H), 4.01 (m, 1H), 3.65 (m, 2H), 2.33 (m, 2H).

### Step 2: Synthesis of (S)-4-(but-3-enyloxy)-2-(tert-butoxycarbonylamino)-butanoic acid

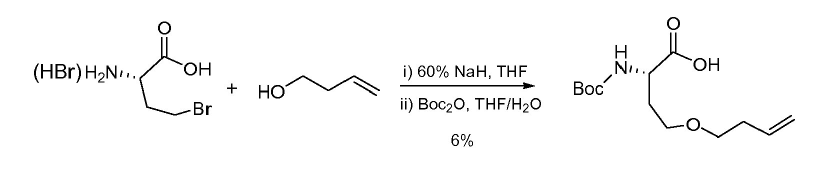

But-3-en-1-ol (98.2 mL, 1140 mmol) in THF (50 mL) was added NaH (27.4 g, 685 mmol) potion wise. When the hydrogen gas emission stopped, (*S*)-2-amino-4-bromobutanoic acid hydrobromide (15 g, 57 mmol) was added in one portion. The reaction was stirred at room temperature for 3 days. Water (100 mL) was added and all solvent was removed. Water (200 mL) was added and extracted with ethyl ether (400 mL). The aqueous layer was acidified to pH=3 and extracted with EtOAc (2 x 200 mL), dried over sodium sulfate. After removal of solvent, the residue was purified by chromatography (hexane:Ethyl acetate=3:1) to give (*S*)-4-(but-3-enyloxy)-2-(*tert*-butoxycarbonylamino)-butanoic acid as pale yellow oil (1.0 g, 6%). MS: Calcd.: 273; Found: [M-H]⁺ 272. ¹H NMR (400 MHz, DMSO-*d*⁶) δ 12.44 (s, 1H), 7.01 (d, *J*=8.0 Hz, 1H), 5.81 (m, 1H), 5.03 (m, 2H), 3.97 (m, 1H), 3.41 (m, 4H), 2.25 (m, 2H), 1.88 (m, 1H), 1.73 (m, 1H), 1.38 (s, 9H).

### Step 3: Synthesis of (1R,2S)-ethyl 1-((2S,4R)-1-((S)-4-(but-enyloxy)-2-(tert-butoxycarbonylamino)butanoyl)-4-hydroxypyrrolidine-2-carboxamido)-2-vinylcyclopropanecarboxylate

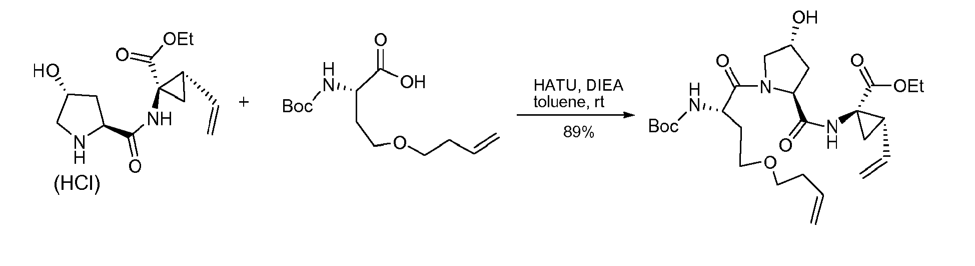

(1*R*,2*S*)-ethyl-1-((2*S*,4*R*)-4-hydroxypyrrolidine-2-carboxamido)-2-vinylcyclopropanecarboxylate hydrochloride salt (WO2005095403) (1.21 g, 3.84 mmol), (*S*)-4-(but-3-enyloxy)-2-(*tert*-butoxycarbonylamino)-butanoic acid (1.00 g, 3.66 mmol) and 2-(1*H*-7-azabenzotriazol-1-yl)-1,1,3,3-tetramethyl uronium hexafluorophosphate (1.53 g, 4.03 mmol) in toluene (18 mL) and MeCN (2 mL) was added diisopropylethylamine (1.28 mL, 4.03 mmol) at 0 °C. The reaction warmed to room temperature and stirred at room temperature for 1 hours. Ethyl acetate (30 mL) and water (20 mL) was added. The organic layer was separated and washed with brine, dried over sodium sulfate. After removal of solvent, the residue was purified by chromatography (Ethyl acetate) to give (1*R*,2*S*)-ethyl 1-((2*S*,4*R*)-1-((*S*)-4-(but-enyloxy)-2-(*tert*-butoxycarbonylamino)butanoyl)-4-hydroxypyrrolidine-2-carboxamido)-2-vinylcyclopropanecarboxylate as white wax solid (1.7 g, 89%). MS: Calcd.: 523; Found: [M+H]⁺524.

### Step 4: Synthesis of (3R,5S)-1-((S)-4-(but-3-enyloxy)-2-(tert-butoxycarbonylamino)butanoyl)-5-((1R,2S)-1-(ethoxycarbonyl)-2-vinylcyclopropylcarbamoyl)pyrrolidin-3-yl 4-chloroisoindoline-2-carboxylate

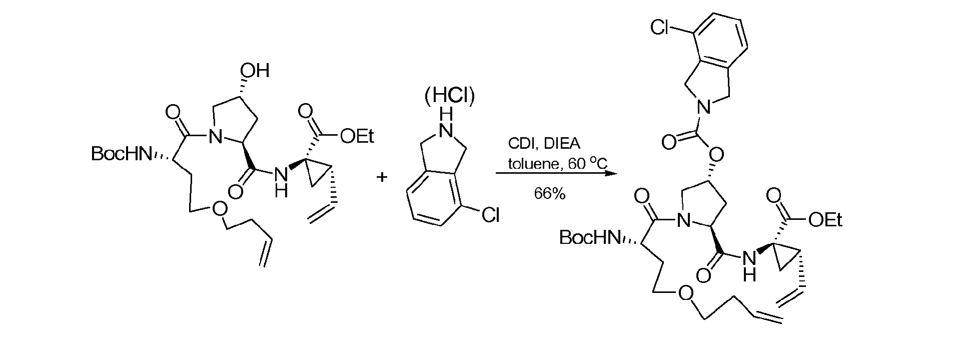

(1*R*,2*S*)-ethyl 1-((2*S*,4*R*)-1-((*S*)-4-(but-enyloxy)-2-(*tert-*butoxycarbonylamino)butanoyl)-4-hydroxypyrrolidine-2-carboxamido)-2-vinylcyclopropanecarboxylate (0.55 g, 1.1 mmol) in THF (20 mL) was added 1,1'-carbonyldiimidazole (0.204 g, 1.26 mmol) in one portion. The reaction was stirred at room temperature for 3 hours. To the reaction was then added the N-ethyl-N-isopropylpropan-2-amine (0.92 mL, 5.3 mmol), followed by 4-chloroisoindoline hydrochloride (0.258 g, 1.37 mmol). The reaction was stirred at 50 °C for 18 hours. The solvent was removed. The residue was partitioned between ethyl acetate (20 mL) and saturated sodium bicarbonate solution. The organic layer was separated and dried over sodium sulfate. After removal of solvent, the residue was purified by chromatography (Hexane:Ethyl acetate=1:3) to give (3*R*,5*S*)-1-((S)-4-(but-3-enyloxy)-2-(tert-butoxycarbonylamino)butanoyl)-5-((1*R*,2*S*)-1-(ethoxycarbonyl)-2-vinylcyclopropylcarbamoyl)pyrrolidin-3-yl 4-chloroisoindoline-2-carboxylate as white solid (0.49 g, 66%). MS: Calcd.: 702.3; Found: [M+H]⁺ 703.1.

### Step 5: Synthesis of (2R,6S,13aS,14aR,16aSZ)-ethyl 6-(tert-butoxycarbonylamino)-2-(4-chloroisoindoline-2-carbonyloxy)-5,16-dioxo-2,3,5,6,7,8,10,11,13a,14,14a,15,16,16a-tetradecahydro-1H-cyclopropa(j)pyrrolo[1,2-f][1,6,9]oxadiazacyclopentadecine-14a-carboxylate

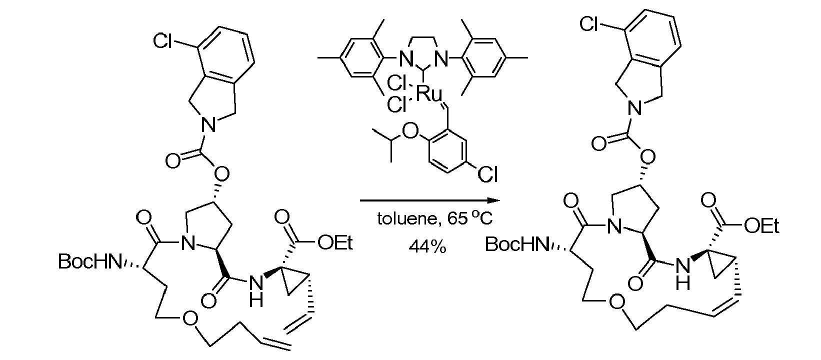

(3*R*,5*S*)-1-((*S*)-4-(but-3-enyloxy)-2-(tert-butoxycarbonylamino)butanoyl)-5-((1*R*,2*S*)-1-(ethoxycarbonyl)-2-vinylcyclopropylcarbamoyl)pyrrolidin-3-yl 4-chloroisoindoline-2-carboxylate (0.49 g, 0.70 mmol) in toluene (130 mL) was degassed by bubbling a stream of nitrogen through the reaction for 1 hour at room temperature. (5-chloro-2-isopropoxybenzylidene)(1,3-dimesitylimidazolidin-2-yl)ruthenium(V) chloride (0.0090 g, 0.014 mmol) was added to the mixture and the mixture was heated to 68 °C (oil bath) and stirred at this temperature for 3 hours. After removal of solvent, the residue was purified by chromatography (Ethyl acetate) to give (2*R*,6*S*,13a*S*,14a*R*,16a*S*,*Z*)-ethyl 6-(*tert-*butoxycarbonylamino)-2-(4-chloroisoindoline-2-carbonyloxy)-5,16-dioxo-2,3,5,6,7,8,10,11,13a,14,14a,15,16,16a-tetradecahydro-1*H*-cyclopropa(*j*)pyrrolo[1,2-*f*][1,6,9]oxadiazacyclopentadecine-14a-carboxylate as off white solid (0.21 g, 44%). MS: Calcd.: 674.3; Found: [M+H]⁺ 675.0.

### Step 6: Synthesis of (2R,6S,13aS,14aR,16aS,Z)-6-(tert-butoxycarbonylamino)-2-(4-chloroisoindoline-2-carbonyloxy)-5,16-dioxo-2,3,5,6,7,8,10,11,13a,14,14a,15,16,16a-tetradecahydro-1H-cyclopropa(j)pyrrolo[1,2-f][1,6,9]oxadiazacyclopentadecine-14a-carboxylic acid

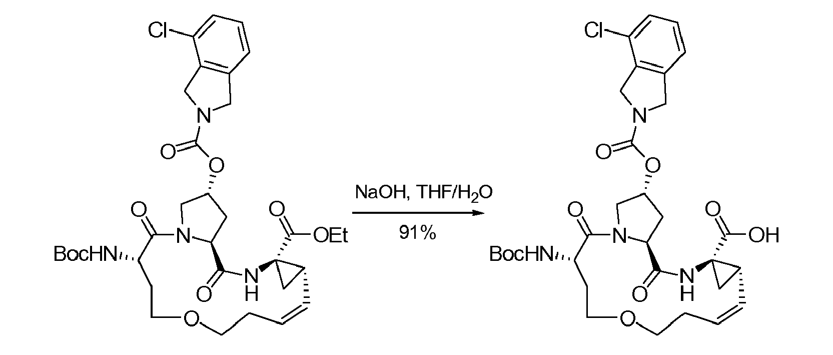

(2*R*,6*S*,13a*S*,14a*R*,16a*S*,*Z*)-ethyl 6-(*tert*-butoxycarbonylamino)-2-(4-chloroisoindoline-2-carbonyloxy)-5,16-dioxo-2,3,5,6,7,8,10,11,13a,14,14a,15,16,16a-tetradecahydro-1*H*-cyclopropa(*j*)pyrrolo[1,2-*f*][1,6,9]oxadiazacyclopentadecine-14a-carboxylate (0.205 g, 0.304 mmol) in THF (2 mL) was added 0.4 N NaOH solution (1.90 mL, 0.76 mmol) in H₂O. The reaction was stirred at room temperature for 3 days. Water (5 mL) and ether (15 mL) was added. The aqueous layer was separated and acidified by saturated potassium hydrogen sulfate solution to pH=2∼3. The aqueous layer was extracted with EtOAc (2x15 mL), washed with brine and dried over sodium sulfate. After removal of solvent, it (2*R*,6*S*, 13a*S*,14a*R*,16a*S*,*Z*)-6-(*tert*-butoxycarbonylamino)-2-(4-chloroisoindoline-2-carbonyloxy)-5,16-dioxo-2,3,5,6,7,8,10,11,13a,14,14a,15,16,16a-tetradecahydro-1*H-*cyclopropa(*j*)pyrrolo[1,2-*f*][1,6,9]oxadiazacyclopentadecine-14a-carboxylic acid as white solid (0.179 g, 91%). MS: Calcd.: 646.2; Found: [M+H]⁺ 647.0.

### Step 7: Synthesis of (2R,6S,13aS,14aR,16aS,Z)-6-(tert-butoxycarbonylamino)-14a-(2,5-dichlorothiophen-3-ylsulfonylcarbamoyl)-5,16-dioxo-2,3,5,6,7,8,10,11,13a,14,14a,15,16,16a-tetradecahydro-1H-cyclopropa[j]pyrrolo[1,2-f][1,6,9]oxadiazacyclopentadecin-2-yl 4-chloroisoindoline-2-carboxylate

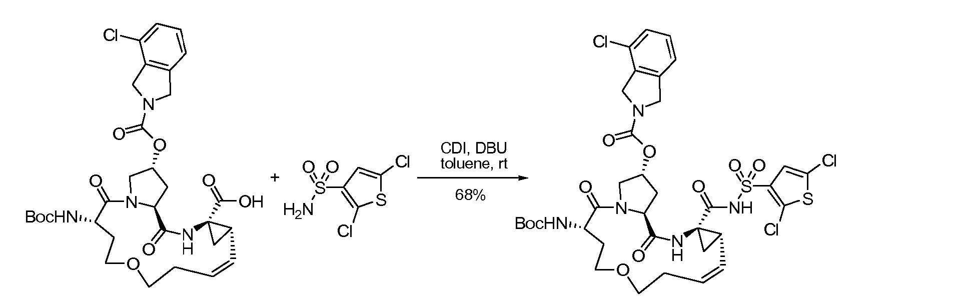

(2*R*,6*S*,13a*S*,14a*R*,16a*S*,*Z*)-6-(*tert*-butoxycarbonylamino)-2-(4-chloroisoindoline-2-carbonyloxy)-5,16-dioxo-2,3,5,6,7,8,10,11,13a,14,14a,15,16,16a-tetradecahydro-1*H-*cyclopropa(*j*)pyrrolo[1,2-*f*][1,6,9]oxadiazacyclopentadecine-14a-carboxylic acid (0.030 g, 0.046 mmol) in toluene (3 mL) was added 1,1'-carbonyldiimidazole (0.011 g, 0.065 mmol) in room temperature. The reaction was stirred at 60 °C for 3 hours. 2,5-dichlorothiophene-3-sulfonamide (0.019 g, 0.083 mmol) was added, followed by addition of DBU (0.012 mL, 0.083 mmol). The reaction was then stirred at room temperature for 17 hours. Water (5 mL) was added and acidified with saturated potassium hydrogen sulfate until pH=2∼3. The mixture was extracted with ethyl acetate (20 mL), washed with brine and dried over sodium sulfate. After removal of solvent, the residue was purified by chromatography (Ethyl acetate) to give (2*R*,6*S*,13a*S*,14a*R*, 16a*S*,Z)-6-(tert-butoxycarbonylamino)-14a-(2,5-dichlorothiophen-3-ylsulfonylcarbamoyl)-5,16-dioxo-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate, **Compound 322,** as white solid (0.021 g, 53%). MS: Calcd.: 859.1; Found: [M+H]⁺ 860.0.

### Example 15

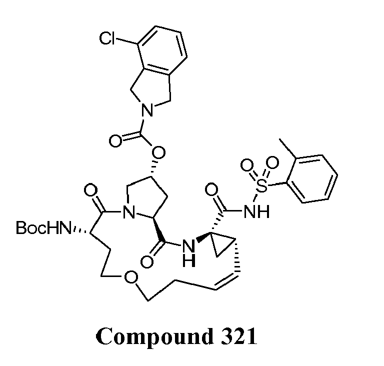

The titled compound, (2*R*,6*S*,13a*S*,14a*R*,16a*S*,*Z*)-6-(tert-butoxycarbonylamino)-5,16-dioxo-14a-(o-tolylsulfonylcarbamoyl)-2,3,5,6,7,8,10,11,13 a,14,14a,15,16,16a-tetradecahydro-1H-cyclopropa[*j*]pyrrolo[1,2-*f*][1,6,9]oxadiazacyclopentadecin-2-yl 4-chloroisoindoline-2-carboxylate, was prepared in a similar fashion as described for Example 14, **Compound 321**, except that 2-methylbenzenesulfonamide was used in lieu of 2,5-dichlorothiophene-3-sulfonamide. MS: Calcd.: 799.3; Found: [M+H]⁺799.7.

### Example 16

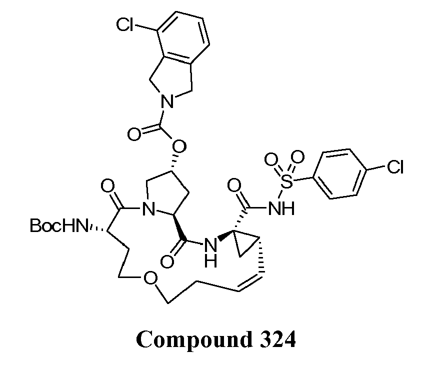

The titled compound, (2*R*,6*S*,13a*S*,14a*R*,16a*S*,*Z*)-6-(tert-butoxycarbonylamino)-14a-(4-chlorophenylsulfonylcarbamoyl)-5,16-dioxo-2,3,5,6,7,8,10,11,13a,14,14a,15,16,16a-tetradecahydro-1H-cyclopropa[*j*]pyrrolo[1,2-*f*][1,6,9]oxadiazacyclopentadecin-2-yl 4-chloroisoindoline-2-carboxylate, **Compound 324**, was prepared in a similar fashion as described for **Example 14, Compound 322**, except that 4-chlorobenzenesulfonamide was used in lieu of 2,5-dichlorothiophene-3-sulfonamide. MS: Calcd.: 819.2; Found: [M-H]⁺ 818.2.

### Example 17

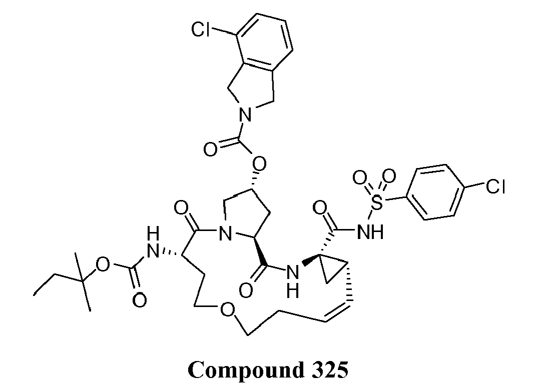

The titled compound, (2*R*,6*S*,13a*S*,14a*R*,16a*S*,*Z*)-14a-(4-chlorophenylsulfonylcarbamoyl)-5,16-dioxo-6-(tert-pentyloxycarbonylamino)-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate, **Compound 325**, was prepared as shown in the following scheme:

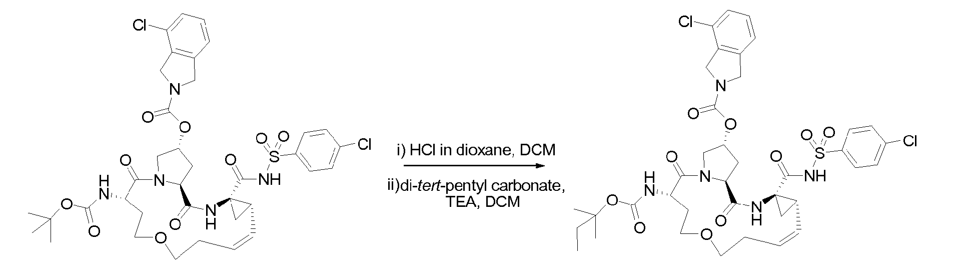

(2*R*,6*S*,13a*S*,14a*R*,16a*S*,*Z*)-6-(tert-butoxycarbonylamino)-14a-(4-chlorophenylsulfonylcarbamoyl)-5,16-dioxo-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate (0.075 g, 0.091 mmol) in DCM (4 mL) was added HCl in dioxane (0.183 mL, 0.73 mmol). The reaction was stirred at room temperature for 25 h. The solvent was removed to give (2*R*,6*S*,13a*S*,14a*R*,16a*S*,*Z*)-6-amino-14a-(4-chlorophenylsulfonylcarbamoyl)-5,16-dioxo-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate hydrochloride as white solid (0.069 g, 99.7%).

(2*R*,6*S*,13a*S*,14a*R*,16a*S*,*Z*)-6-amino-14a-(4-chlorophenylsulfonylcarbamoyl)-5,16-dioxo-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate hydrochloride (0.069 g, 0.091 mmol) and di-tert-pentyl carbonate (0.034 g, 0.14 mmol) in DCM (4 mL) was added triethylamine (d = 0.726 g/mL) (0.038 mL, 0.27 mmol). The reaction was stirred at room temperature for hours. Water (5 mL) was added and acidified with saturated potassium hydrogen sulfate until pH=2∼3. The mixture was extracted with ethyl acetate (20 mL), washed with brine and dried over sodium sulfate. After removal of solvent, the residue was purified by chromatography (Ethyl acetate) to give (2*R*,6*S*,13a*S*,14a*R*,16a*S*,*Z*)-14a-(4-chlorophenylsulfonylcarbamoyl)-5,16-dioxo-6-(tert-pentyloxycarbonylamino)-2,3,5,6,7,8,10,11,13a,14,14a,15,16,16a-tetradecahydro-1*H*-cyclopropa[*j*]pyrrolo[1,2-*f*][1,6,9]oxadiazacyclopentadecin-2-yl 4-chloroisoindoline-2-carboxylate, **Compound 325**, as white solid (0.052 g, 68%). MS: Calcd.: 833.2; Found: [M-H]⁺ 832.3.

**Table 3: Additional examples of compound prepared using Example Procedures 11-17.**

| Compound | Structure | mass spectral data | Example Procedure Used |
|---|---|---|---|
| 305 | 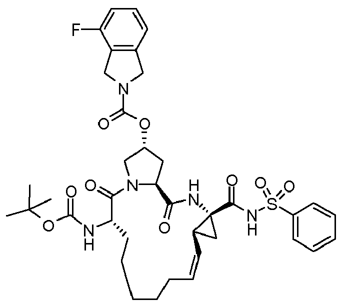 | (APCI-) m/z 766.4 (M-1) | 11 |
| 306 | 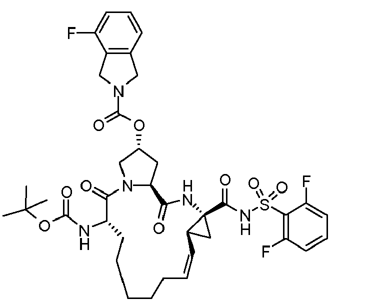 | (APCI-) m/z 802.3 (M-1) | 11 |
| 307 | 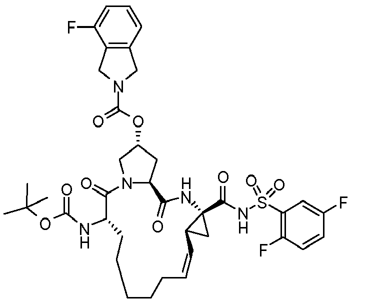 | (APCI-) m/z 802.7 (M-1) | 11 |
| 308 | 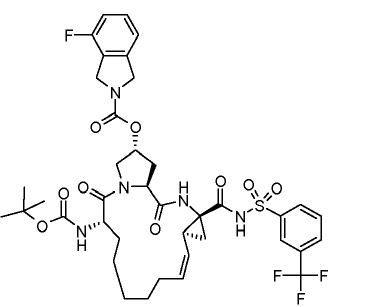 | (APCI-) m/z 834.3 (M-1) | 11 |
| 309 | 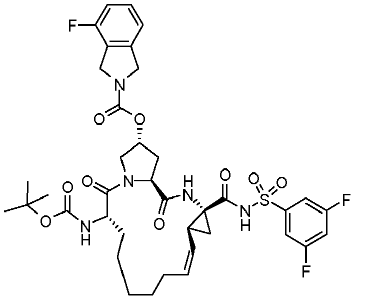 | (APCI-) m/z 802.7 (M-1) | 11 |
| 310 | 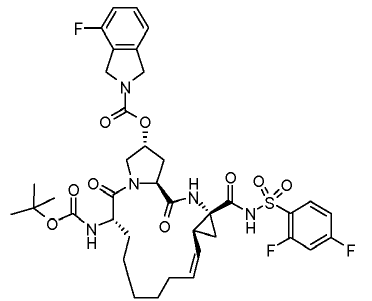 | (APCI-) m/z 802.4 (M-1) | 11 |
| 311 | 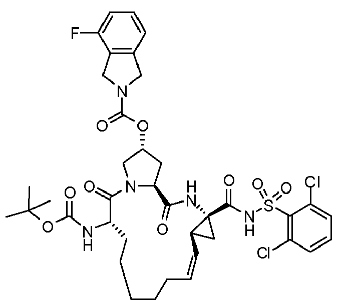 | (APCI-) m/z 834.2 (M-2) | 11 |
| 312 | 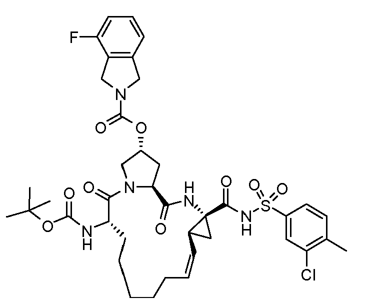 | (APCI-) m/z 814.4 (M-2) | 11 |
| 313 | 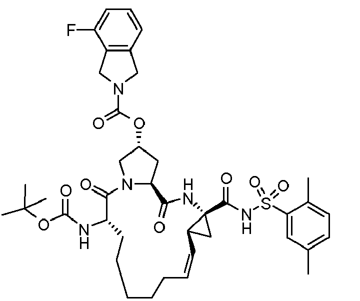 | (APCI-) m/z 794.4 (M-1) | 11 |
| 314 | 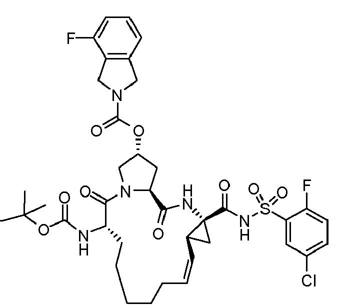 | (APCI-) m/z 818.3 (M-2) | 11 |
| 315 | 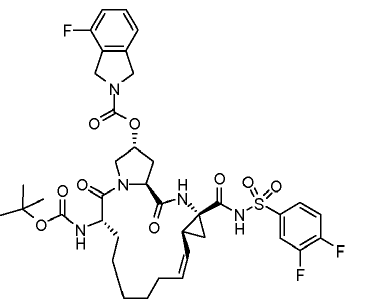 | (APCI-) m/z 802.3 (M-1) | 11 |
| 316 | 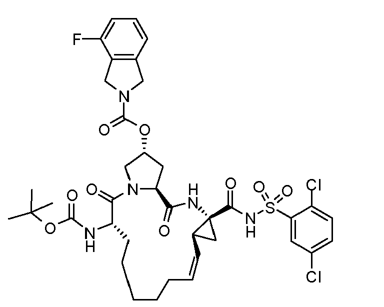 | (APCI-) m/z 834.4 (M-2) | 11 |
| 317 | 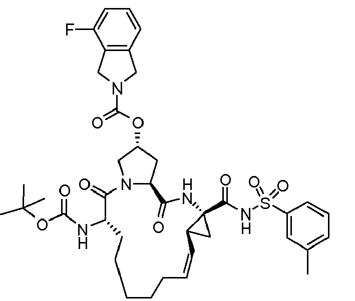 | (APCI-) m/z 780.4 (M-1) | 11 |
| 318 | 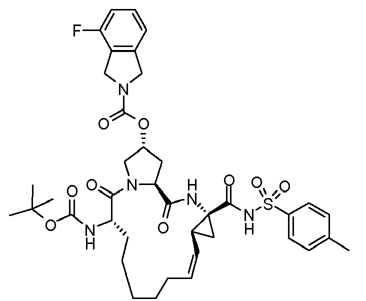 | (APCI-) m/z 780.4 (M-1) | 11 |
| 319 | 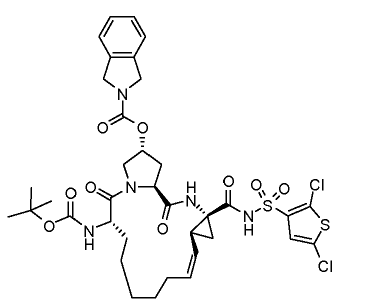 | (APCI-) m/z 822.3 (M-2) | 11 |
| 320 | 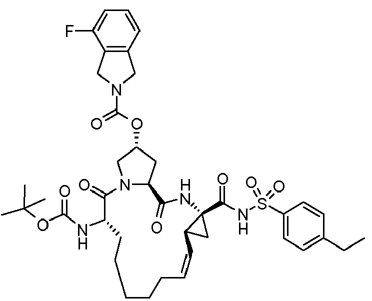 | (APCI-) m/z 794.4 (M-1) | 11 |
| 321 | 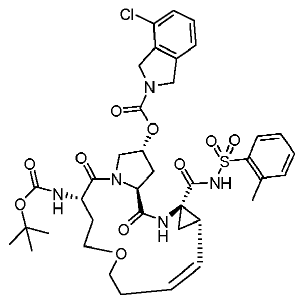 | (APCI-) m/z 199.7 (M+1) | 11 |
| 322 | 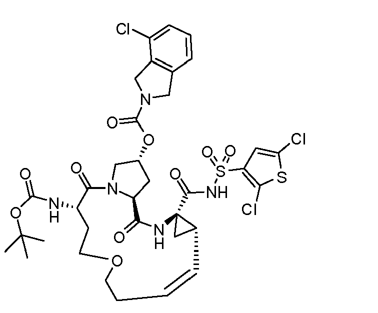 | (APCI-) m/z 861.8 (M+1) | 12 |
| 323 | 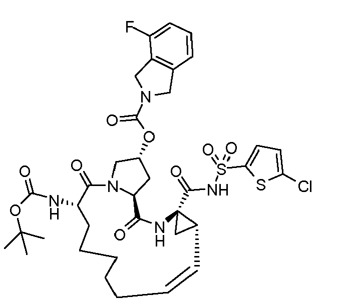 | (APCI-) m/z 807.9 (M+1) | 12 |
| 324 | 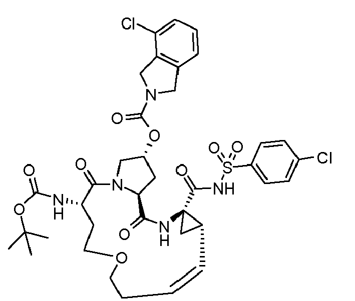 | (APCI-) m/z 818.2 (M-1) | 11 |
| 325 | 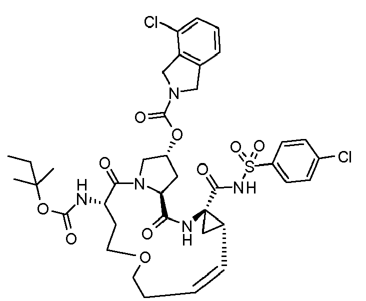 | (APCI-) m/z 832.3 (M-1) | 13 then 22 |
| 326 | 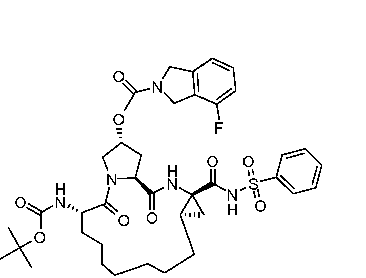 | (APCI-) m/z 670.3 (M-Boc+1) | 14 |
| 327 | 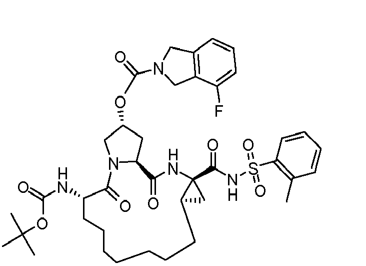 | (APCI-) m/z 684.3 (M-Boc+1) | 14 |
| 328 | 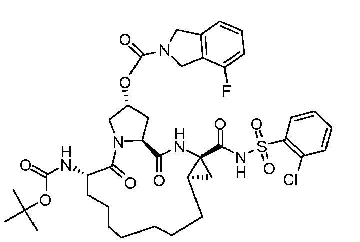 | (APCI-) m/z 704.2 (M-Boc+1) | 14 |
| 329 | 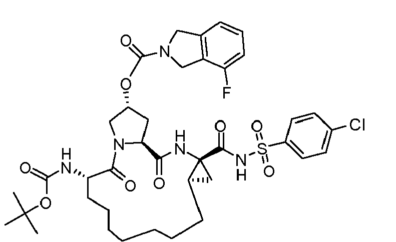 | (APCI-) m/z 704.2 (M-Boc+1) | 14 |
| 330 | 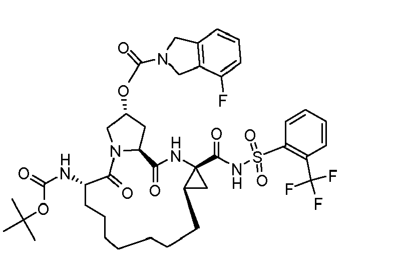 | (APCI-) m/z 738.3 (M-Boc+1) | 14 |
| 331 | 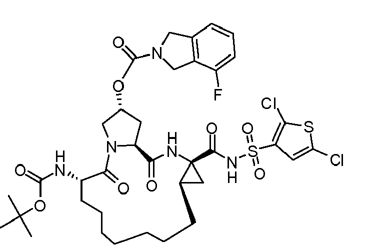 | (APCI-) m/z 744.2 (M-Boc+1) | 14 |
| 332 | 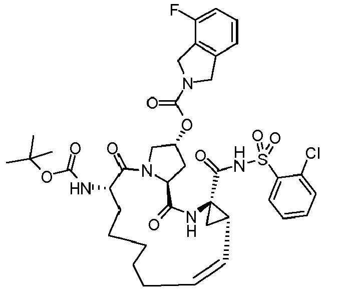 | (APCI+) m/z 702 (M-Boc) | 11 |
| 333 | 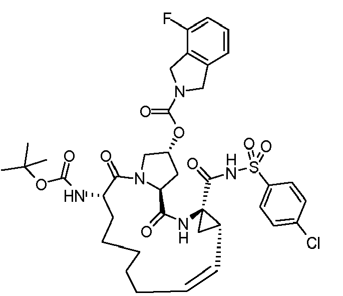 | (APCI+) m/z 702 (M-Boc) | 11 |
| 334 | 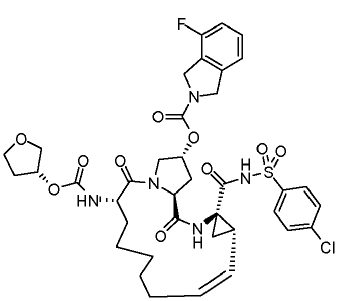 | (APCI-) m/z 814 (M-2) | 11 then 14 |
| 335 | 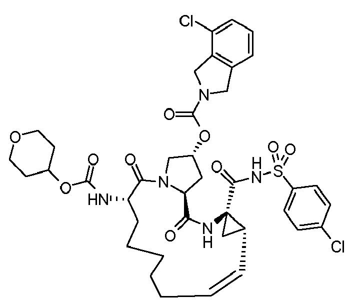 | (APCI-) m/z 844 (M-2) | 11 then 13 |
| 336 | 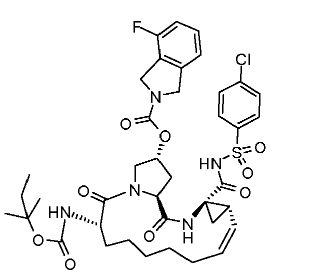 | (APCI-) m/z 814 (M-2) | 11 then 22 |
| 337 | 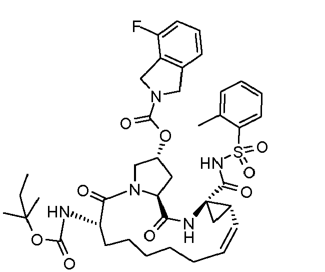 | (APCI-) m/z 794 (M-1) | 11 then 22 |
| 338 | 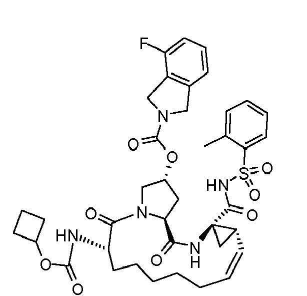 | (APCI-) m/z 778 (M-1) | 11 then 23 |
| 339 | 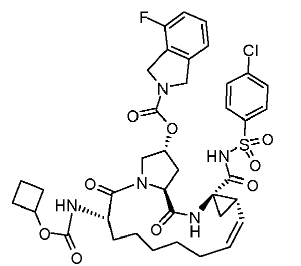 | (APCI-) m/z 798 (M-2) | 11 then 23 |
| 340 | 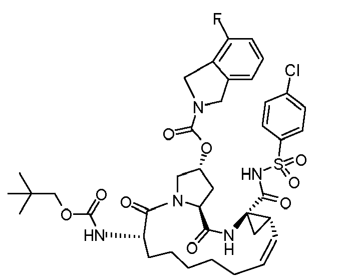 | (APCI-) m/z 814 (M-2) | 11 then 23 |
| 341 | 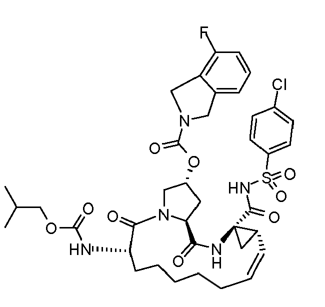 | (APCI-) m/z 800 (M-2) | 11 then 21 |
| 342 | 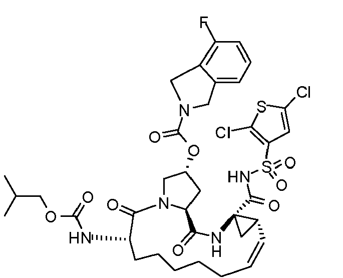 | (APCI-) m/z 840 (M-2) | 12 then 21 |
| 343 | 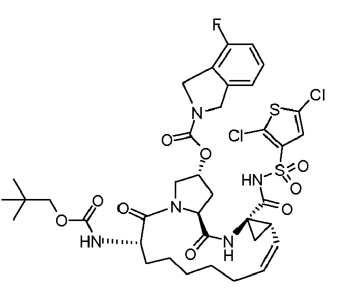 | (APCI-) m/z 854 (M-2) | 12 then 23 |
| 344 | 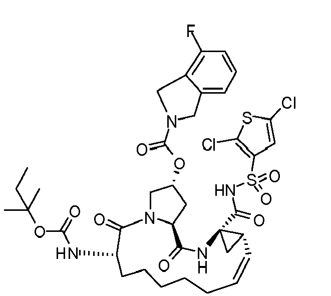 | (APCI-) m/z 854 (M-2) | 12 then 22 |
| 345 | 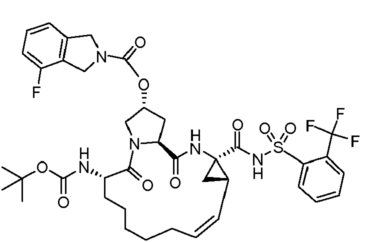 | (APCI -) m/z 834.4 (M-1) | 11 |
| 346 | 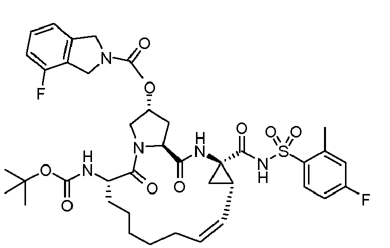 | (APCI -) m/z 798.4 (M-1) | 11 |
| 347 | 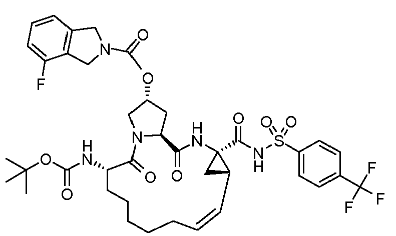 | (APCI -) m/z 834.4 (M-1) | 11 |
| 348 | 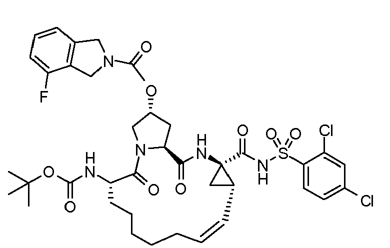 | (APCI -) m/z 834.3 (M-1) | 11 |
| 349 | 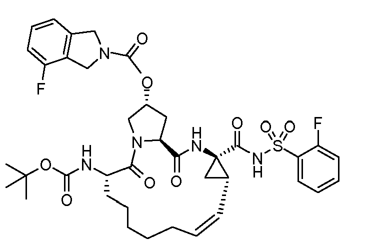 | (APCI -) m/z 784.4 (M-1) | 11 |
| 350 | 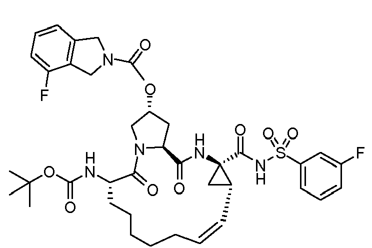 | (APCI -) m/z 784.4 (M-1) | 11 |
| 351 | 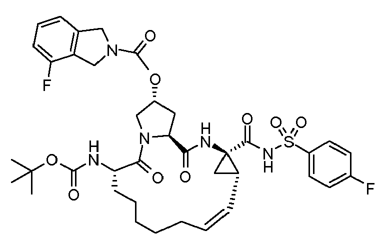 | (APCI -) m/z 784.4 (M-1) | 11 |
| 352 | 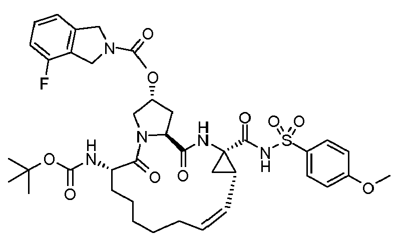 | (APCI -) m/z 796.4 (M-1) | 11 |
| 353 | 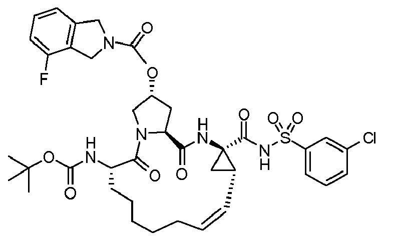 | (APCI -) m/z 800.4 (M-1) | 11 |
| 354 | 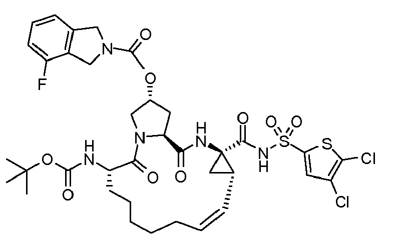 | (APCI -) m/z 840.3 (M-1) | 12 |
| 355 | 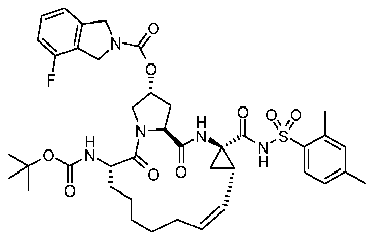 | (APCI -) m/z 794.4 (M-1) | 11 |
| 356 | 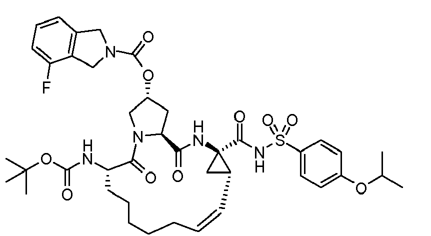 | (APCI -) m/z 824.4 (M-1) | 11 |
| 357 | 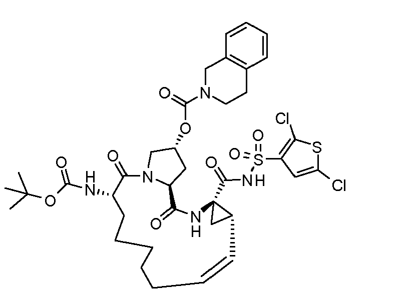 | (APCI+) m/z 738.1 (M-Boc) | 12 |
| 358 | 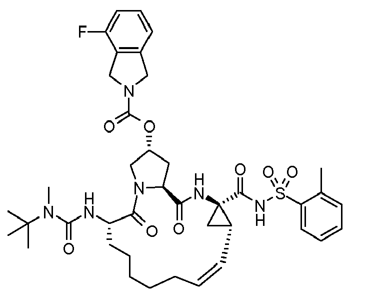 | (APCI+) m/z 796.2 (MH+) | 13 |
| 359 | 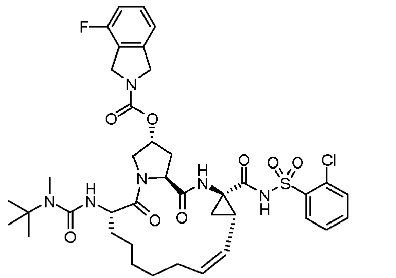 | (APCI+) m/z 816.2 (MH+) | 13 |
| 360 | 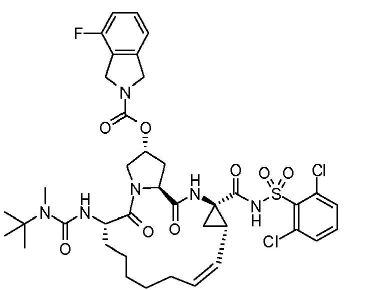 | (APCI+) m/z 849.3 (M) | 13 |
| 361 | 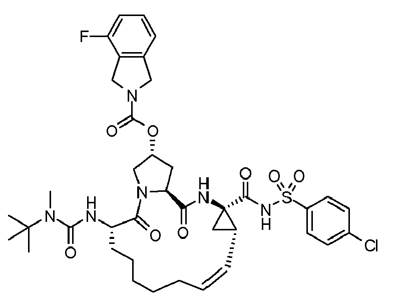 | (APCI+) m/z 815.2 (M) | 13 |
| 362 | 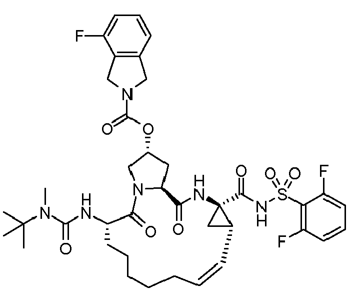 | (APCI+) m/z 818.2 (MH+) | 13 |
| 363 | 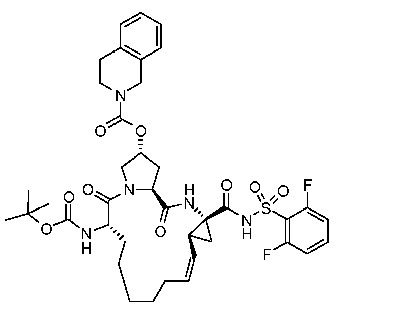 | (APCI-) m/z 798.4 (M-1) | 11 |
| 364 | 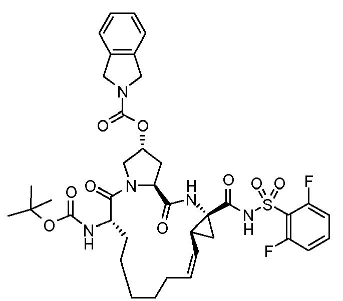 | (APCI-) m/z 784.2 (M-1) | 11 |

### Example 18:

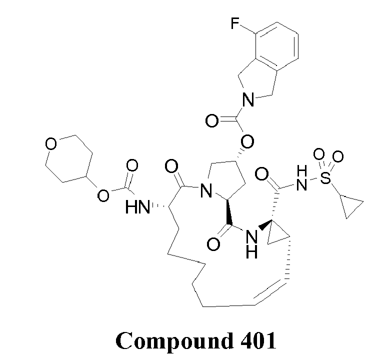

(2*R*,6*S*,13a*S*,14a*R*,16a*S*,*Z*)-14a-(cyclopropylsulfonylcarbamoyl)-5,16-dioxo-6-((tetrahydro-2*H*-pyran-4-yloxy)carbonylamino)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 401**, was prepared as follows:

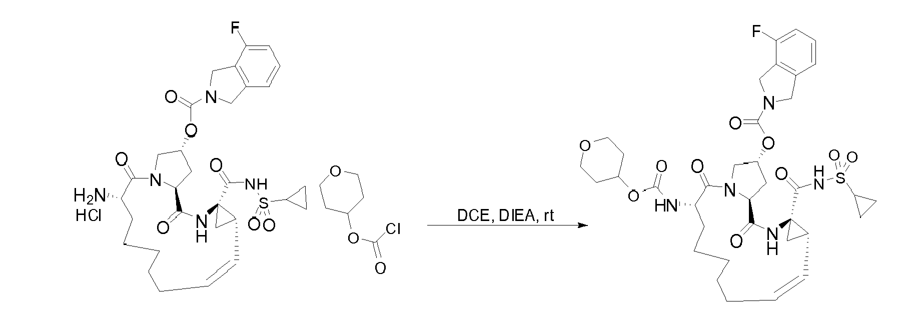

To an anhydrous DCE solution (1.5 mL) of the macrocyclic amine hydrochloride salt (synthesis of which is described in other parts of this application document) (200 mg, 0.229 mmol), was added diisopropylethylamine (0.209 mL, 1.20 mmol) and the mixture cooled on an ice-water bath. Tetrahydro-2H-pyran-4-yl carbonochloridate (synthesis of which is described in the following sections) (74 mg, 0.45 mmol) was added dropwise and the resulting mixture allowed to warm to room temperature. After stirring at room temperature for 16 h, the reaction mixture was diluted with EtOAc (10 mL) and the solution washed with IN HCl, water and brine, then dried over MgSO₄, filtered and concentrated. The residue was purified by preparative TLC (eluent = 3% MeOH in DCM), to provide (2*R*,6*S*,13a*S*,14a*R*,16a*S*,*Z*)-14a-(cyclopropylsulfonylcarbamoyl)-5,16-dioxo-6-((tetrahydro-2*H*-pyran-4-yloxy)carbonylamino)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 401**, as a cream colored solid (14.7 mg, 64 % yield). LCMS (APCI-) m/z 758 (M-1).

### Example 19:

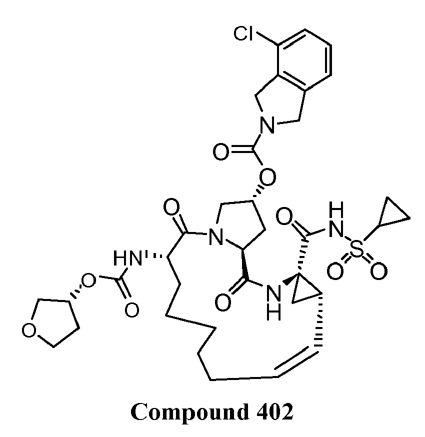

(2*R*,6*S*,13a*S*,14a*R*,16a*S*,*Z*)-14a-(cyclopropylsulfonylcarbamoyl)5,16-dioxo-6-(((*R*)-tetrahydrofuran-3-yloxy)carbonylamino)-1,2,3,5,6,7,8,9,10,11,13a,14,14a,15,16,16a-hexadecahydrocyclopropa[e]pyrrolo[1,2-a][1,4]diazacyclopentadecin-2-yl 4-chloroisoindoline-2-carboxylate, **Compound 402**, was prepared as follows:

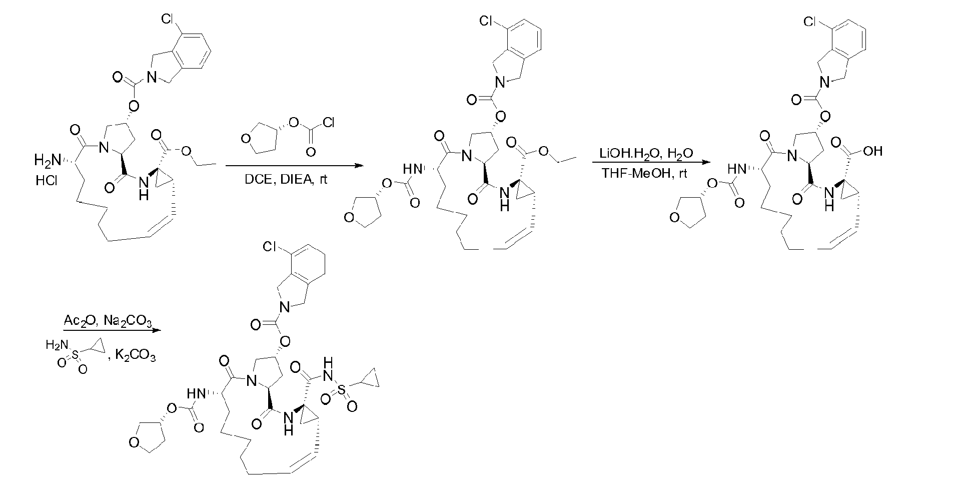

(*R*)-Tetrahydrofuran-3-yl carbonochloridate (synthesis of which is described in the following sections) (74 mg, 0.49 mmol) was added dropwise to an ice-water bath cooled solution of the macrocyclic amine hydrochloride ethyl ester salt (synthesis of which is described in other parts of this application document) (200 mg, 0.328 mmol) and diisopropylethylamine (0.229 mL, 1.31 mmol) in anhydrous DCE (1.5 mL) and allowed to warm to room temperature. After stirring for 16 h, the reaction mixture was diluted with EtOAc (10 mL), washed with 1N HCl and brine, then dried over MgSO₄, filtered and concentrated. The crude oil was then stirred in anhydrous THF (2 mL) and MeOH (1 mL) and to this solution was added a solution of LiOH-H₂O (41 mg, 0.98 mmol) in water (1 mL) and the mixture stirred at room temperature for 16 h. The mixture was diluted with EtOAc (10 mL) and washed with 1N HCl, water and brine, then dried over MgSO₄, filtered and concentrated to provide (2*R*,6*S*,13a*S*,14a*R*,16a*S*,*Z*)-14a-(cyclopropylsulfonylcarbamoyl)5,16-dioxo-6-(((*R*)-tetrahydrofuran-3-yloxy)carbonylamino)-1,2,3,5,6,7,8,9,10,11,13a,14,14a,15,16,16a-hexadecahydrocyclopropa[e]pyrrolo[11,2-a][1,4]diazacyclopentadecin-2-yl 4-chloroisoindoline-2-carboxylate, **Compound 402**, as a tan colored foam. LCMS (APCI+) m/z 659 (M⁺).

A solution of the macrocyclic carboxylic acid (167 mg, 0.253 mmol) in anhydrous THF (2.5 mL) was treated with acetic anyhydride (0.03 mL, 0.32 mmol) and Na₂CO₃ (81 mg, 0.76 mmol) and the resulting mixture heated at 40 °C for 16 h. K₂CO₃ (175 mg, 1.27 mmol) was then added and the mixture stirred at 40 °C for 30 minutes, followed by addition of cyclopropanesulfonamide (46 mg, 0.38 mmol). The reaction temperature was increased to 60°C and the mixture stirred for 16 h. The mixture was cooled to room temperature, diluted with EtOAc (10 mL) and water (5 mL) and stirred for 10 minutes and the layers separated. The organic layer was then washed with IN HCl, water and brine, then dried over MgSO₄, filtered and concentrated. The residue was purified by preparative TLC (eluent = 3% MeOH in DCM), yielding the titled product as a white solid (83 mg, 43 % yield). LCMS (APCI+) m/z 762 (M⁺).

The syntheses of the chloroformate derivatives that are used in the aforementioned carbamate formation step can be exemplified with the preparation of tetrahydro-2*H-*pyran-4-yl carbonochloridate as shown below:

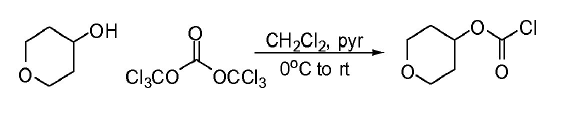

To a solution of tetrahydro-2*H*-pyran-4-ol (1.77 g, 17 mmol) in anhydrous CH₂Cl₂ (50 mL) cooled in an ice-water bath, was added dropwise a solution triphosgene (2.02 g, 6.79 mmol) and pyridine (1.37 mL, 17 mmol) in anhydrous CH₂Cl₂ (30 mL). The mixture was allowed to warm to room temperature and stirred for 2 h. The solvent was then evaporated at room temperature and the residue re-suspended in EtOAc (100 mL) and stirred for 30 minutes. The suspension was filtered and the solvent removed at 30 °C yielding the titled product as a straw colored liquid (2.41 g, 86 % yield). ¹H NMR (400 MHz, CDCl₃) δ 5.0-5.1 (m, 1 H), 3.9-4.0 (m, 2 H), 3.5-3.6 (m, 2 H), 2.0-2.1 (m, 2 H), 1.7-1.88 (m, 2 H). The liquid was of sufficient purity and was directly used for the next carbamate formation step without further purification.

By a similar fashion (*R*)-tetrahydrofuran-3-yl carbonochloridate was also prepared: 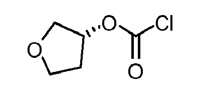 clear colorless liquid, (3 g, 88 % yield). ¹H NMR (400 MHz, CDCl₃) δ 3.84-4.02 (m, 5 H), 2.14-2.3 (m, 2 H).

### Example 20

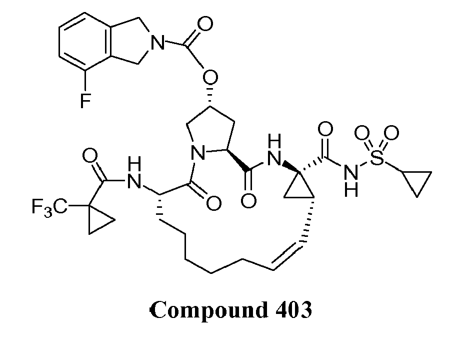

(2*R*,6*S*,13a*S*,14a*R*,16a*S*,*Z*)-14a-(cyclopropylsulfonylcarbamoyl)-5,16-dioxo-6-(1-(trifluoromethyl)cyclopropanecarboxamido)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 403**, was prepared as follows:

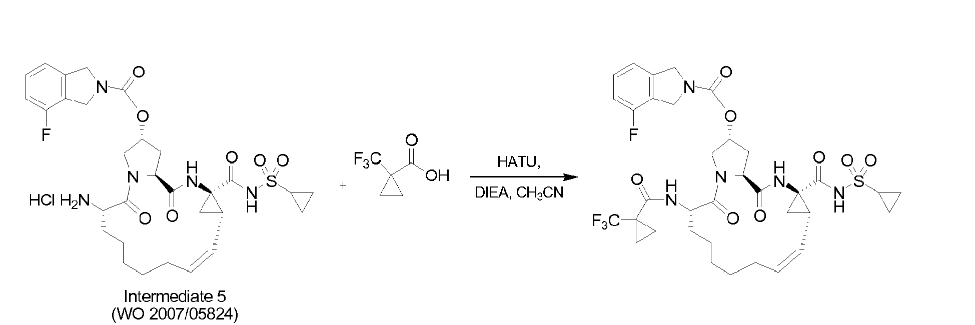

To a MeCN solution (0.20 mL) of the **Intrermediate 5** (35 mg, 0.052 mmol), 2-(1*H-*7-azabenzotriazol-1-yl)-1,1,3,3-tetramethyl uronium hexafluorophosphate (29.9 mg, 0.078 mmol) and 1-(trifluoromethyl)cyclopropanecarboxylic acid (12 mg, 0.078 mmol) was added diisopropylethylamine (27 µL, 0.16 mmol) at room temperature. After stirring at room temperature for 16h, the reaction mixture was directly purified by column chromatography to yield the (2*R*,6*S*,13a*S*,14a*R*,16a*S*,*Z*)-14a-(cyclopropylsulfonylcarbamoyl)-5,16-dioxo-6-(1-(trifluoromethyl)cyclopropanecarboxamido)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 403**, as a white solid (19 mg, 49 % yield). LCMS (APCI+) m/z 768.2 (MH⁺).

### Example 21

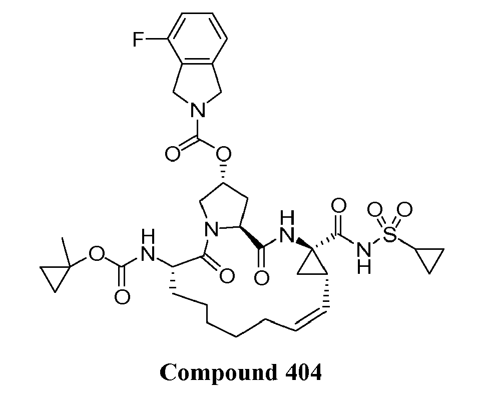

(2*R*,6*S*,13a*S*,14a*R*,16a*S*,*Z*)-14a-(cyclopropylsulfonylcarbamoyl)-6-((1-methylcyclopropoxy)carbonylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 404**, was prepared as follows:

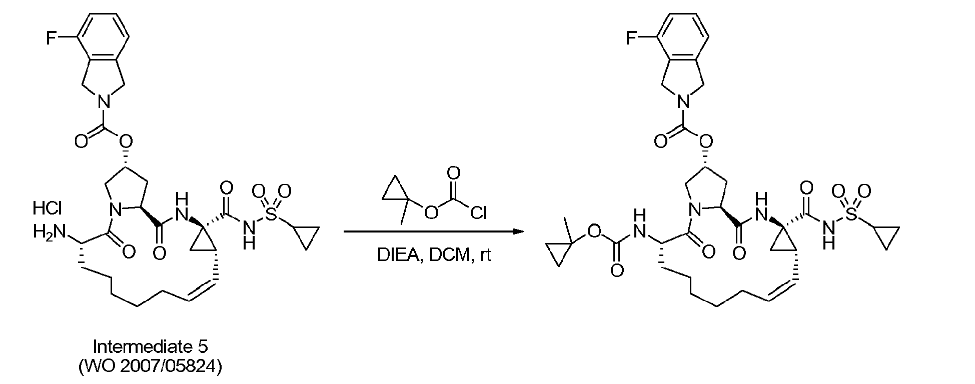

A DCM solution of the 1-methylcyclopropyl carbonochloridate (synthesis of which is described in the following sections) (0.10 g, 0.75 mmol) was added dropwise to a solution of the macrocyclic amine hydrochloride salt (synthesis of which is described in other parts of this application document) (0.1 g, 0.15 mmol), followed by additon of diisopropylethylamine (0.13 mL, 0.75 mmol) dropwise at room temperature. After stirring for overnight, the reaction mixture was diluted with EtOAc, washed with IN HCl and brine, then dried over Na₂SO₄. The crude oil was treated with reverse-phase column chromatography (eluent = 5 to 85% MeCN in water), yielding the final product as a white glassy solid. LCMS (APCI+) m/z 730.1 (MH⁺).

The syntheses of alpha-substituted cyclopropyl alcohols and their chloroformate derivatives that are used in the aforementioned carbamate formation step can be exemplified with the preparation of 1-methylcyclopropyl carbonochloridate and 1-methylcyclopropanol as shown below: 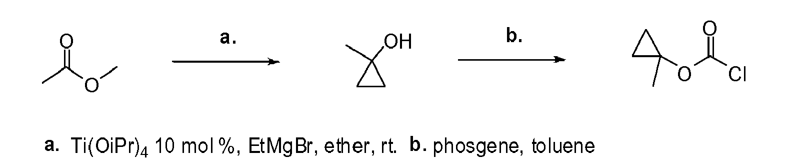
a. To a stirred solution of methyl acetate (1.98 mL, 25.0 mmol) and Ti(OiPr)₄ (0.754 mL, 2.50 mmol) in ethyl ether (80 mL) was added ethylmagnesium bromide (17.5 mL, 52.4 mmol) in ethyl ether (total volume 60 mL) slowly over 1 h at room temperature, and the stirring was continued for 10 minutes. For workup, the reaction mixture was poured into ice-cold 10% aq H₂SO₄ (250 mL) and the aqueous layer was extracted with ethyl ether (3 × 100 mL). The combined organic layers was washed with water, brine (100 mL each) and dried over Na₂SO₄ and the solvent removed. The 1-methylcyclopropanol product was obtained as a clear colorless liquid through distillation.
b. A 20 % toluene solution of phosgene (7.2 mL, 14 mmol) was cooled in an ice bath, followed by addition of 1-methylcyclopropanol (0.3 g, 1.4 mmol) in toluene (2 mL) dropwise. Ice bath was removed and the reaction was stirred at room temperature for overnight. The reaction was concentrated down, redissolved in DCM and concentrated down again. This solution containing 1-methylcyclopropyl carbonochloridate was directly used for the next carbamate formation step without further purification.

By a similar fashion, the following alpha-substituted cyclopropyl alcohols were prepared-substituting methyl acetate in step **a**. above with the corresponding alkyl esters: 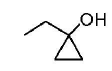 Ethylcyclopropanol, clear colorless liquid, b.p. = 30-33 °C (30 mbar). 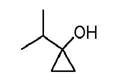 Isopropylcyclopropanol, clear colorless liquid, b.p. = 35-37 °C (28 mbar). 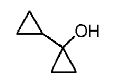 Bi(cyclopropan)-1-ol, clear colorless liquid, b.p. = 48-50 °C (28 mbar). 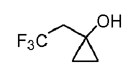 1-(2,2,2-trifluoroethyl)cyclopropanol, clear colorless liquid, b.p. = 35 °C (40 mbar).

### Example 22

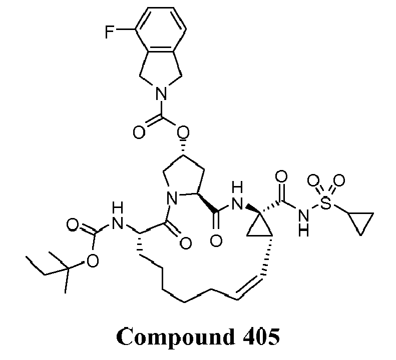

The titled compound, (2*R*,6*S*,13a*S*,14a*R*,16a*S*,*Z*)-14a-(cyclopropylsulfonylcarbamoyl)-5,16-dioxo-6-(tert-pentyloxycarbonylamino)-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 405**, was prepared as follows:

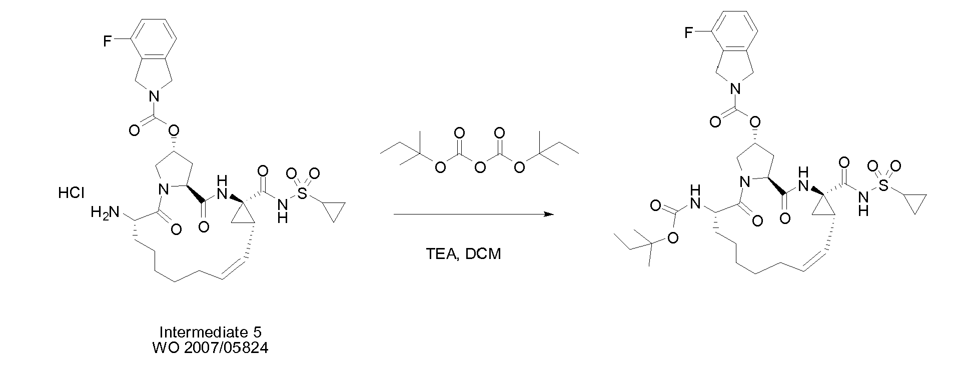

Intermediate 5 (200 mg, 0.299 mmol), triethyl amine (d = 0.726 g/mL) (0.17 mL, 1.2 mmol), di-tert-pentyl dicarbonate (0.15 mL, 0.60 mmol) in 2 mL of dichloromethane where stirred at room temperature for 15 min. The reaction was concentrated *in vacuo* and purified by reverse phase chromatography (Biotage SP4) to provide (2*R*,6*S*,13a*S*,14a*R*,16a*S*,*Z*)-14a-(cyclopropylsulfonylcarbamoyl)-5,16-dioxo-6-(tert-pentyloxycarbonylamino)-1,2,3,5,6,7,8,9,10,11,13a, 14,14a, 15,16,16a-hexadecahydrocyclopropa[e]pyrrolo[1,2-a][1,4]diazacyclopentadecin-2-yl 4-fluoroisoindoline-2-carboxylate, **Compound 405**, (126 mg, 0.169 mmol, 56% yield) as a white solid. LCMS (APCI-) m/z 744.4 (MH⁻).

### Example 23

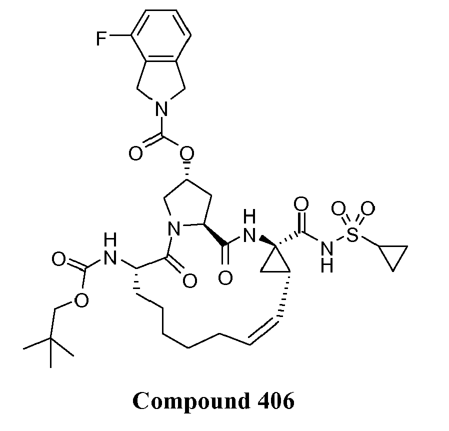

(2*R*,6*S*,13a*S*,14a*R*,16a*S*,*Z*)-14a-(cyclopropylsulfonylcarbamoyl)-6-(neopentyloxycarbonylamino)-5,16-dioxo-1,2,3,5,6,7,8,9,10,11,13a, 14,14a, 15,16,16a-hexadecahydrocyclopropa[e]pyrrolo[1,2-a][1,4]diazacyclopentadecin-2-yl 4-fluoroisoindoline-2-carboxylate, **Compound 406**, was prepared as follows:

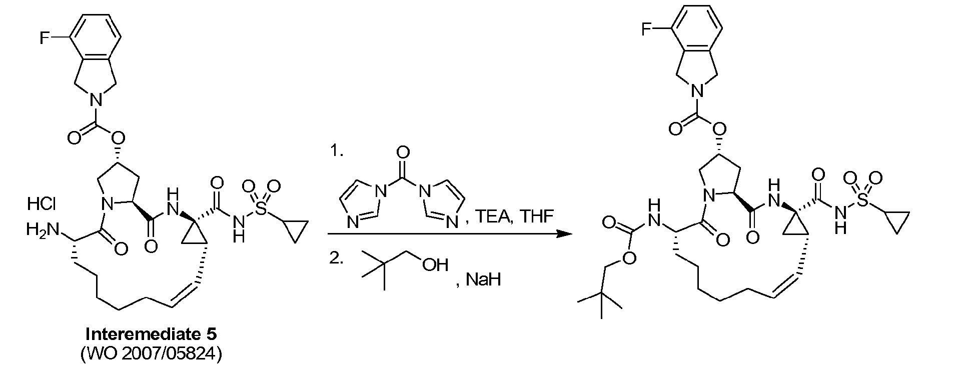

To **Intermediate 5** (165 mg, 0.247 mmol) in 1.5 mL THF was added triethylamine (0.17 mL, 1.2 mmol) followed by 1,1'-carbonyldiimidazole (52 mg, 0.32 mmol), 2,2-dimethylpropan-1-ol (435 mg, 4.94 mmol) and 60% NaH in mineral oil (24 mg, 0.74 mmol). The reaction was stirred at room temperature for 80 min, the was concentrated and purified by reverse phase chromatography (Biotage SP4) to provide (2*R*,6*S*,13a*S*,14a*R*,16a*S*,*Z*)-14a-(cyclopropylsulfonylcarbamoyl)-6-(neopentyloxycarbonylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 406**, (90 mg, 0.12 mmol, 49 % yield). LCMS (APCI-) m/z 745.2 (MH⁻).

### Example 24

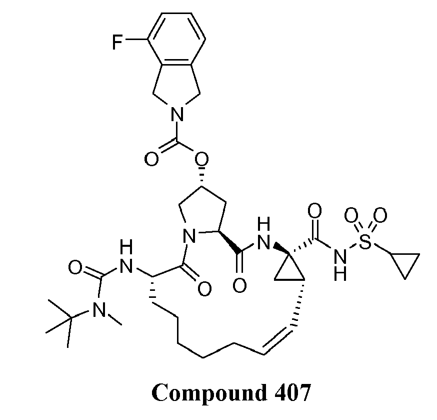

(2*R*,6*S*,13a*S*,14a*R*,16a*S*,*Z*)-6-(3-tert-butyl-3-methylureido)-14a-(cyclopropylsulfonylcarbamoyl)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 407**, was prepared as follows:

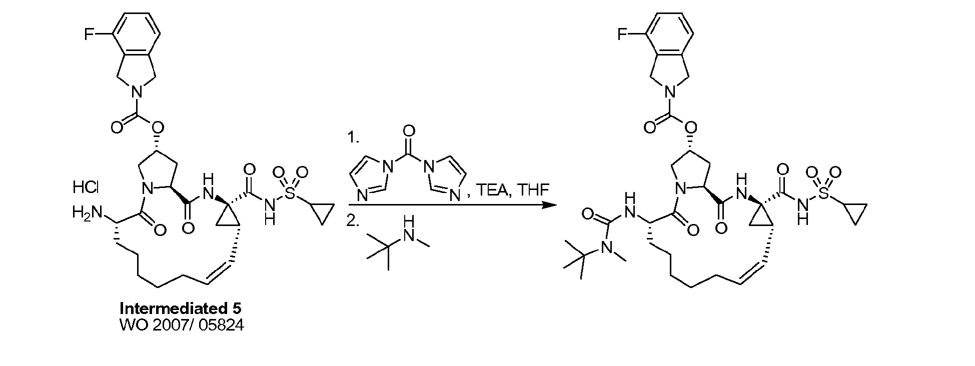

**Intermediate 5** (60 mg, 0.090 mmol), triethylamine (0.063 mL, 0.45 mmol) and 1,1'-carbonyldiimidazole (29 mg, 0.18 mmol) in 1.0 mL THF were stirred at room temperature for 2 h. To this mixture was added N,2-dimethylpropan-2-amine (0.086 mL, 0.72 mmol) and stirred at room temperature overnight. The reaction was then concentrated and purified by reverse phase chromatography to provide (2*R*,6*S*,13a*S*,14a*R*,16a*S*,*Z*)-6-(3-tert-butyl-3-methylureido)-14a-(cyclopropylsulfonylcarbamoyl)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 407**, (23 mg, 0.031 mmol, 35 % yield) as a white solid.

### Example 25

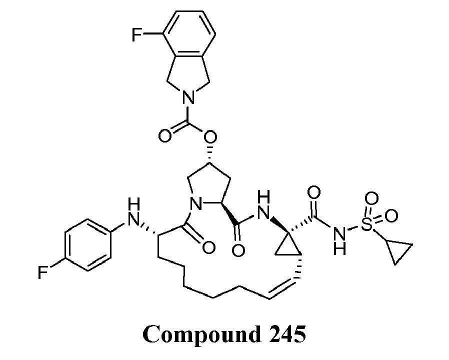

(2*R*,6*S*,13a*S*,14a*R*,16a*S*,*Z*)-14a-(cyclopropylsulfonylcarbamoyl)-6-(4-fluorophenylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 245**, was prepared as shown in the following scheme:

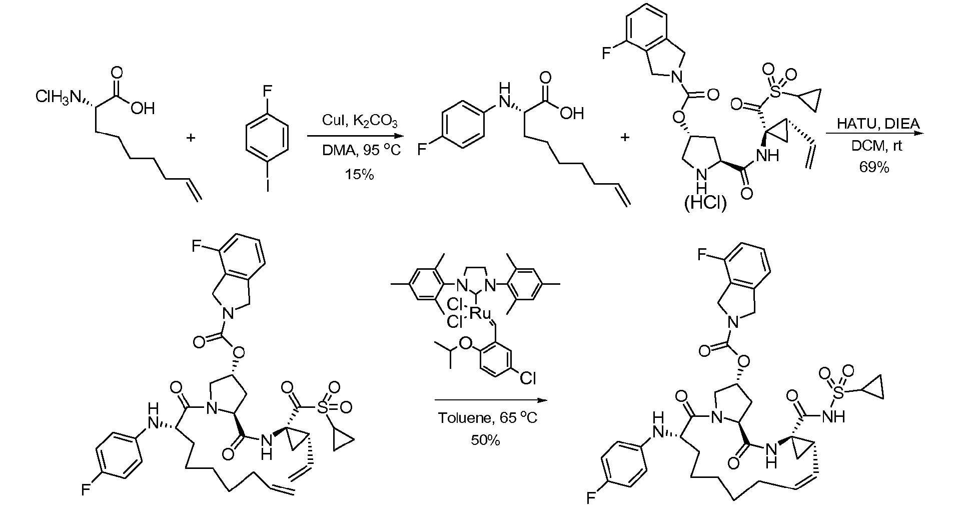

### Step 1: Synthesis of (S)-2-(4-fluorophenylamino)non-8-enoic acid

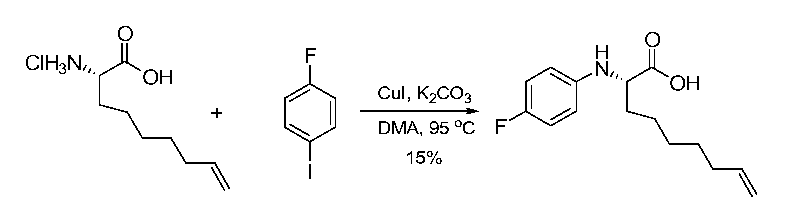

(*S*)-2-aminonon-8-enoic acid hydrochloride (0.300 g, 1.44 mmol), K₂CO₃ (0.299 g, 2.17 mmol), copper(I) iodide (0.028 g, 0.144 mmol) and 1-ffuoro-4-iodobenzene (0.18 mL, 1.59 mmol) in DMA (4 mL) was heated at 90 °C for 24 h. Water (10 mL) and Et₂O (20 mL) was added. The aqueous phase was isolated and acidified with saturated KHSO₄ to pH=3∼4 and extracted with EtOAc:Et₂O=1:1 (40 mL), dried over sodium sulfate. After removal of solvent, the residue was purified by chromatography (hexane:Ethyl acetate=2:1) to give (*S*)-2-(4-fluorophenylamino)non-8-enoic acid as pale yellow oil (0.058 g, 15%). MS: Calcd.: 265.2; Found: [M+H]⁺ 266.1.

### Step 2: Synthesis of (3R,5S)-5-((1R,2S)-1-(cyclopropylsulfonylcarbonyl)-2-vinylcyclopropylcarbamoyl)-1-((S)-2-(4-fluorophenylamino)non-8-enoyl)pyrrolidin-3-yl 4-fluoroisoindoline-2-carboxylate

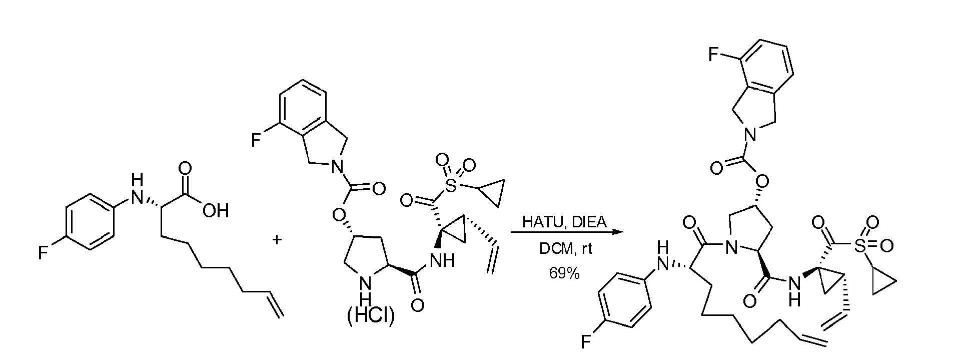

(3*R*,5*S*)-5-((1*R*,2*S*)-1-(cyclopropylsulfonylcarbonyl)-2-vinylcyclopropylcarbamoyl)pyrrolidin-3-yl 4-fluoroisoindoline-2-carboxylate hydrochloride (0.127 g, 0.235 mmol) (WO2007015824A2), (*S*)-2-(4-fluorophenylamino)non-8-enoic acid (0.052 g, 0.20 mmol) and HATU (0.075 g, 0.20 mmol) in DCM (5 mL) was added DIEA (d = 0.742 g/mL) (0.034 mL, 0.20 mmol). The reaction was stirred at rt for 20 h. Water (5 mL) was added and acidified with saturated KHSO₄ until pH=3∼4. The mixture was extracted with DCM (20 mL), washed with brine, dried over sodium sulfate. After removal of solvent, the residue was purified by chromatography (Ethyl acetate) to give (3*R*,5*S*)-5-((1*R*,2*S*)-1-(cyclopropylsulfonylcarbonyl)-2-vinylcyclopropylcarbamoyl)-1-((*S*)-2-(4-fluorophenylamino)non-8-enoyl)pyrrolidin-3-yl 4-fluoroisoindoline-2-carboxylate as white solid (0.102 g, 69%). MS: Calcd.: 753.3; Found: [M+H]⁺ 754.2.

### Step 3: Synthesis of (2R,6S,13aS,14aR,16aS,Z)-14a-(cyclopropylsulfonylcarbamoyl)-6-(4-fluorophenylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate (447)

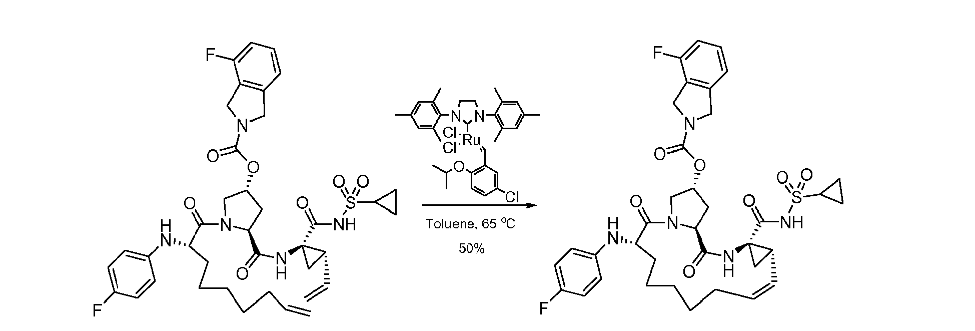

(3*R*,5*S*)-5-(((1*R*,2*S*)-1-(cyclopropylsulfonylcarbamoyl)-2-vinylcyclopropyl)carbamoyl)-1-((*S*)-2-(4-fluorophenylamino)non-8-enoyl)pyrrolidin-3-yl 4-fluoroisoindoline-2-carboxylate (0.100 g, 0.133 mmol) in toluene (130 mL) (0.0010 M) was degassed by bubbling a stream of N₂ through the reaction for 1 h at rt. (5-chloro-2-isopropoxybenzylidene)(1,3-dimesitylimidazolidin-2-yl)ruthenium(V) chloride (0.0018 g, 0.0027 mmol) was added to the mixture and the mixture was heated to 68 °C (oil bath) and stirred at this temperature for 1 h. The solvent was removed. After removal of solvent, the residue was purified by chromatography to give (2*R*,6*S*,13a*S*,14a*R*,16a*S*,*Z*)-14a-(cyclopropylsulfonylcarbamoyl)-6-(4-ffuorophenylamino)-5,16-dioxo-1,2,3,5,6,7,8,9,10,11,13a,14,14a,15,16,16a-hexadecahydrocyclopropa[e]pyrrolo[1,2-a][1,4]diazacyclopentadecin-2-yl 4-fluoroisoindoline-2-carboxylate, **Compound 447**, as a white solid (0.048 g, 50%). MS: Calcd.: 725.3; Found: [M-H]⁺ 724.4.

**Table 4: Additional examples of compound prepared using Example Procedures 18-25**

| Compound | Structure | Mass spectral data | Example Procedure Used |
|---|---|---|---|
| 408 | 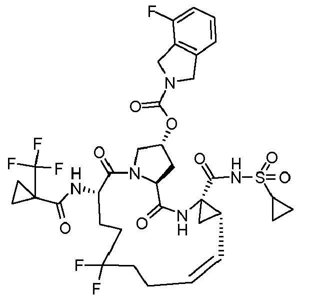 | (APCI-) m/z 804.2 (M+1) | 20 |
| 409 | 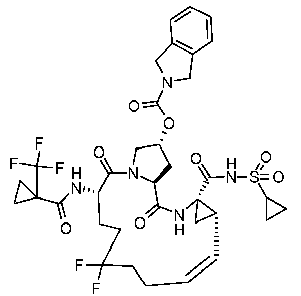 | (APCI-) m/z 786.2 (M+1) | 20 |
| 410 | 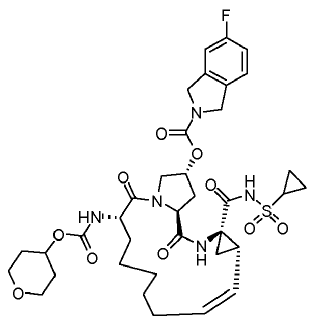 | (APCI-) m/z 758.2 (M+1) | 18 |
| 411 | 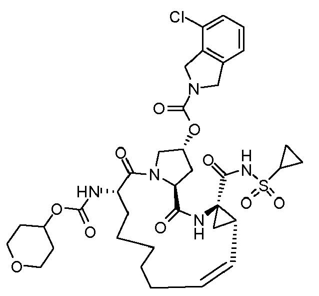 | (APCI-) m/z 775.2 (M+1) | 18 |
| 412 | 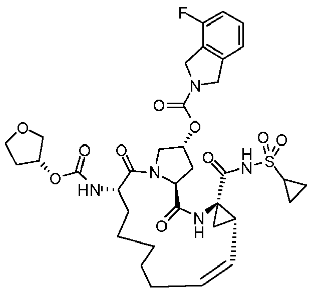 | (APCI-) m/z 744 (M-1) | 19 |
| 413 | 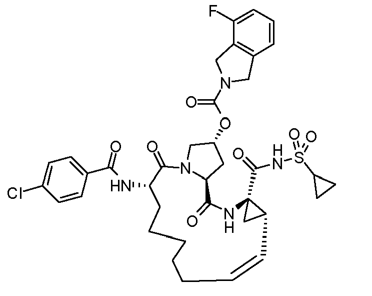 | (APCI-) m/z 768 (M-2) | 20 |
| 414 | 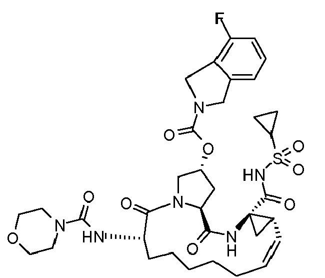 | (APCI-) m/z 743 (M-1) | 24 |
| 415 | 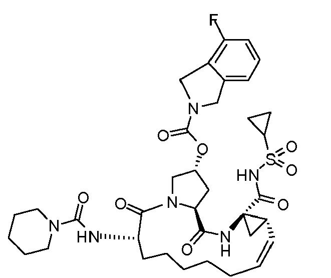 | (APCI-) m/z 741 (M-1) | 24 |
| 416 | 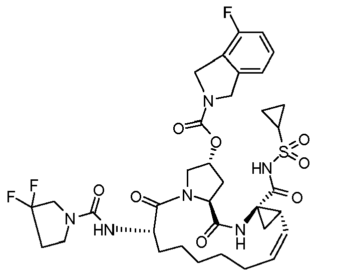 | (APCI-) m/z 763 (M-1) | 24 |
| 417 | 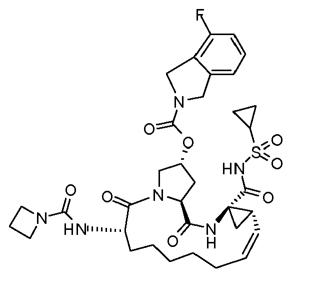 | (APCI-) m/z 713 (M-1) | 24 |
| 418 | 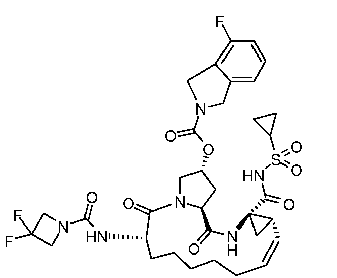 | (APCI-) m/z 749 (M-1) | 24 |
| 419 | 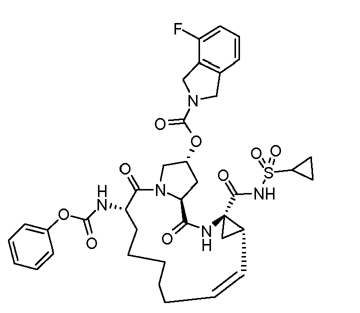 | (APCI-) m/z 750 (M-1) | |
| 420 | 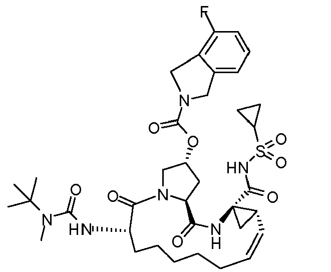 | (APCI-) m/z 743 (M-1) | 24 |
| 421 | 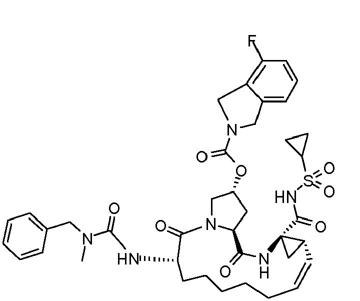 | (APCI-) m/z 777 (M-1) | 24 |
| 422 | 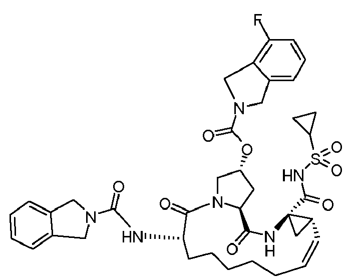 | (APCI-) m/z 775 (M-1) | 24 |
| 423 | 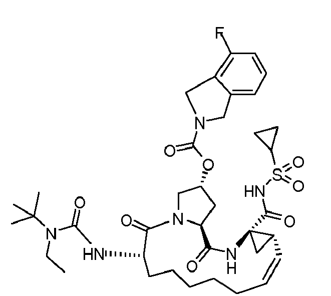 | (APCI-) m/z 757 (M-1) | 24 |
| 424 | 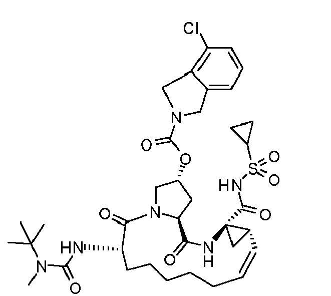 | (APCI-) m/z 759 (M-2) | 24 |
| 425 | 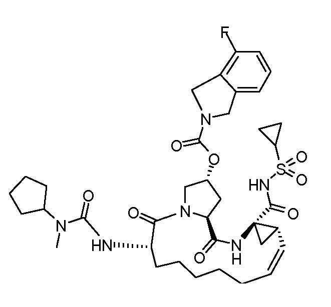 | (APCI-) m/z 755 (M-1) | 24 |
| 426 | 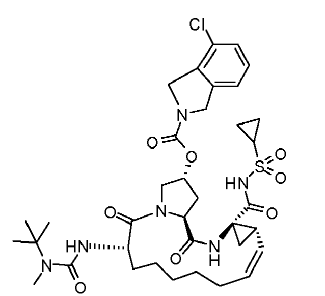 | (APCI-) m/z 759 (M-2) | 24 |
| 427 | 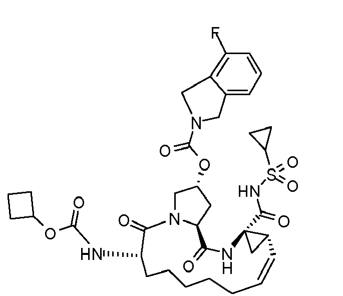 | (APCI-) m/z 728 (M-1) | 21 |
| 428 | 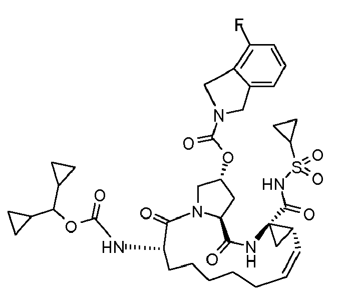 | (APCI-) m/z 768 (M-1) | 23 |
| 429 | 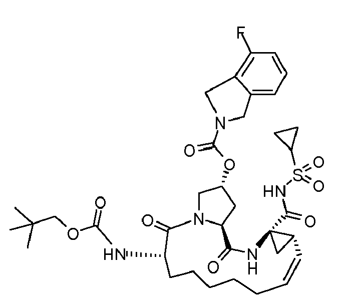 | (APCI-) m/z 745 (M-0) | 23 |
| 430 | 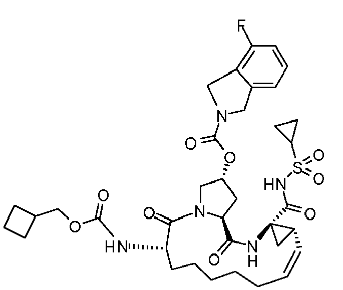 | (APCI-) m/z 743 (M-0) | 23 |
| 431 | 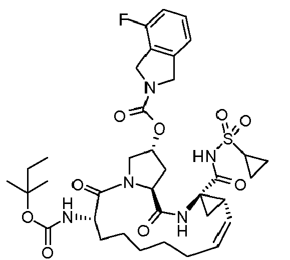 | (APCI-) m/z 744 (M-1) | 22 |
| 432 | 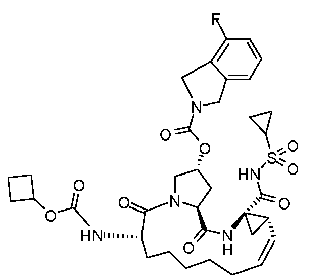 | (APCI-) m/z 728 (M-1) | 23 |
| 433 | 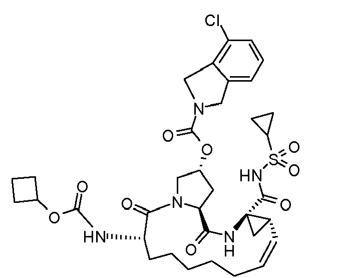 | (APCI-) m/z 745 (M-1) | 23 |
| 434 | 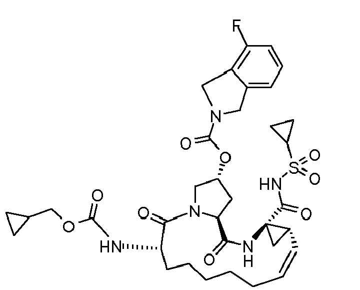 | (APCI-) m/z 728 (M-1) | 23 |
| 435 | 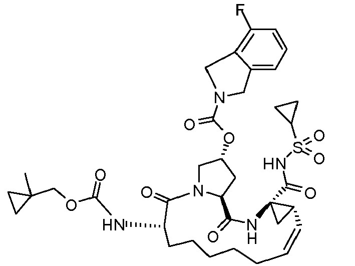 | (APCI-) m/z 742 (M-1) | 23 |
| 436 | 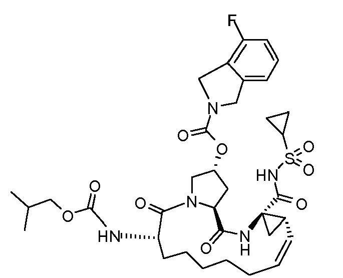 | (APCI-) m/z 730 (M-1) | 21 |
| 437 | 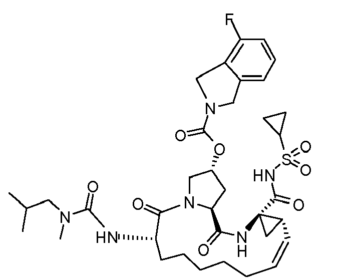 | (APCI-) m/z 743 (M-1) | 24 |
| 438 | 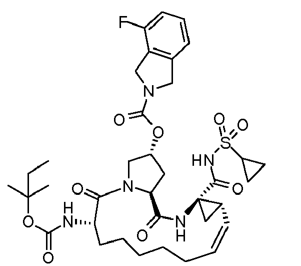 | (APCI-) m/z 744 (M-1) | 22 |
| 439 | 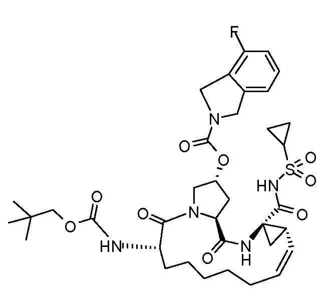 | (APCI-) m/z 744 (M-1) | 23 |
| 440 | 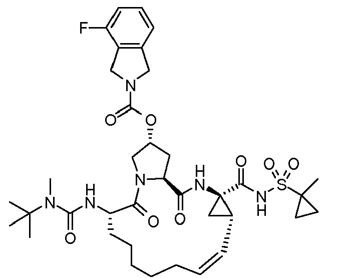 | (APCI+) m/z 759.4 (MH+) | 24 |
| 441 | 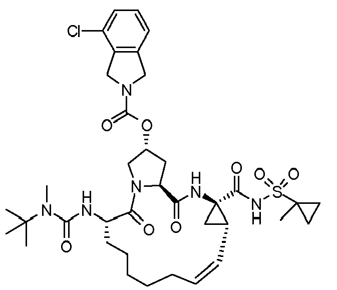 | (APCI+) m/z 775.2 (MH+) | 24 |
| 442 | 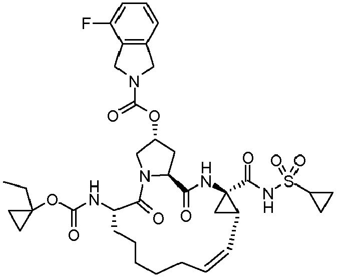 | (APCI+) m/z 743.3 (M) | 21 |
| 443 | 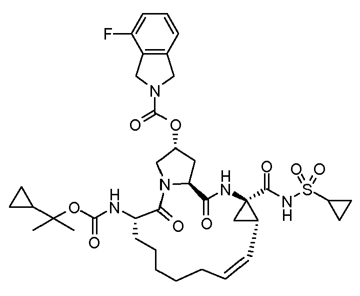 | (APCI+) m/z 757.3 (M) | 21 |
| 444 | 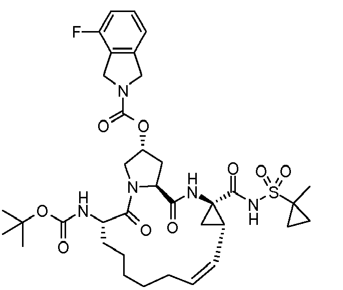 | (APCI+) m/z 646.3 (MH-Boc) | 21 |
| 445 | 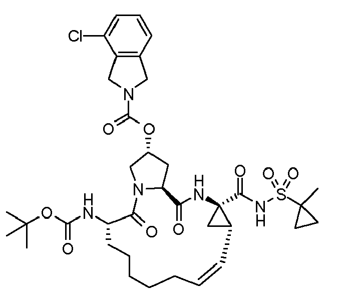 | (APCI-) m/z 760.4 (M-1) | 21 |
| 446 | 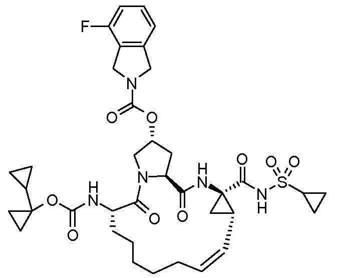 | (APCI+) m/z 756.3 (MH+) | 21 |

### Example 26

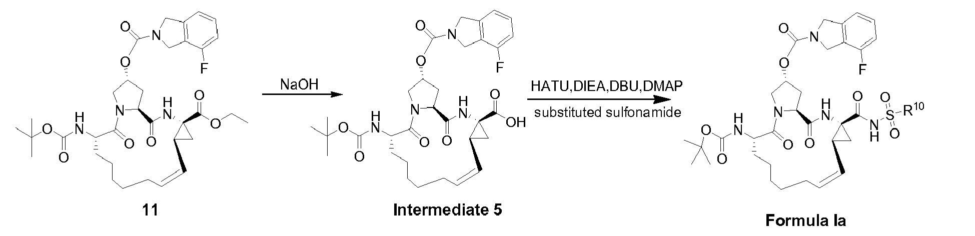

### General Procedure for the Preparation of Formula Ia

Compound 11 (1 eq.) was dissolved in toluene (5% w/w). The obtained solution was concentrated to 50% by reduced vacuum at 30∼35 °C. Charged ethanol to dilute the solution to 8%, concentrated to 50% by vacuum at 30∼35 °C. Aqueous solution of NaOH (12 eq. 20%) was added to the 50% solution of intermediate 5 in ethanol through 1 h, stirred at 5∼10 °C for 4∼5 h. Conc. HCl was added at 5∼10 °C for 1h until the pH was 3-4. Ethyl acetate (30 eq.) and H₂O (52 eq.) were added, and stirred further for 30 min. The solution was filtrated and the wet cake was washed two times with water (16 eq.) and two times with MTBE (0.1 eq.). Dried over vacuum at room temperature to give intermediate 5 as white solid, which was used without further purification.

To a solution of intermediate 5 (1 eq.) in dry DMF (0.05 mol/L for intermediate 5) was added HATU (2 eq.) and DIEA (4 eq.) under N₂ atmosphere, and stirred for 1 h. The sulfonamide (2 eq.), DMAP (4 eq.) and DBU (4 eq.) was then added and the mixture was stirred at room temperature overnight. The reactions were monitored by LCMS, and when found to be completed diluted with ethyl acetate washed with AcONa buffer, 5% aqueous sodium bicarbonate and brine, dried over MgSO₄, concentrated and purified by prep. HPLC to give a compound of formula **Ia**.

Different compounds of formula **Ia** (with different R¹⁰) were prepared using intermediate 5 and substituted sulfonamides (R¹⁰-sulfonamides) as described above. The phenylsulfonamide and substituted phenylsulfonamides used herein were commercially available. The substituted benzylsulfonamides were prepared using the method described in U.S. Patent No. 6,350,764. The substituted pyridylsulfonamides were prepared using the method described in U.S. Patent No. 5,866,568. The substituted furansulfonamides were prepared using the method described in U.S. Patent No. 6,342,610. The substituted thiophenesulfonamides were prepared using the method described in J. Med. Chem.1992, 35, 3012-3016. The benzofuransulfonamide was prepared using the method described in J. Med. Chem.1997, 40, 2276-2286.

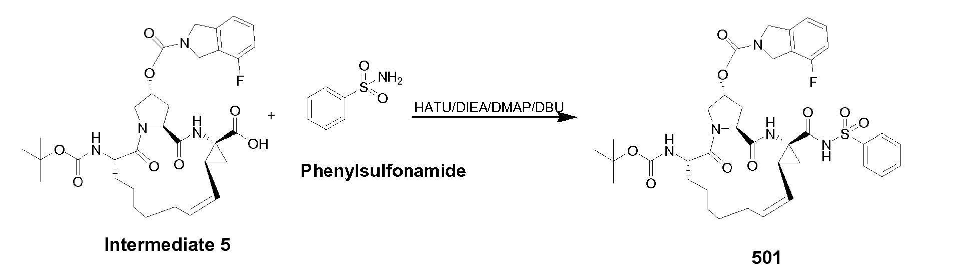

Compound 501 was formed using the scheme above as a white solide (43% yield). MS-ESI: m/z=768 [M+1]⁺.

### Example 27

### Synthesis of new sulfonamides

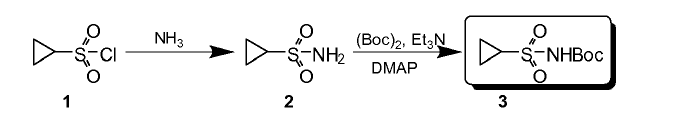

Preparation of compound **3**: compound **1** (10g, 71mmol) was dissolved in dry DCM (150ml) under nitrogen. The resulting solution was bubbled through NH₃ for 15 minutes at -78°C, then it was allowed to rise to room temperature and stirred for 3 h. Before filtration, the filtrate was concentrated to provide compound **2** as white solid 8.2 g (yield 95%).

Compound **2** (4g, 33 mmol) was suspended in dry DCM (100 mL) containing Et₃N (5 mL, 36.3 mmol) and DMAP (0.4g , 3.3 mmol). A solution of (Boc)₂O (8.3g, 38 mmol) in dry DCM (50 mL) was added dropwise with stirring over 5 min. after 5h, the solution was concentrated in vacuo and the residue treated with EtOAc (300mL) and 1N HCl. The EtOAc layer was washed successively with water (40 mLl) and brine (40 mL), dried (Na₂SO₄), and concentrated to leave a solid. Heating with hexane (30 mL), cooling to room temperature, and filtration provide compound **3** as a white solid 6.8 g (yield 93%).

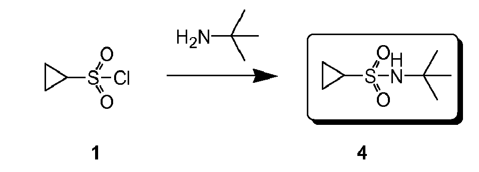

Preparation of compound **4**: tert-Butylamine (42.8 mol, 3.12g, 4.5 mL) was dissolved in THF (40 mL). The solution was cooled to -20 °C and compound **1** (21.4 mmol, 3g, 2.6 mL) was added slowly. The reaction mixture was allowed to warm to rt and stirred for 24 h. The mixture was filtered, and the filtrate concentrated in vacuo. The residue was dissolved in DCM (100 mL), washed with 1 N HCl (20 mL), H₂O (20 mL) and brine (20 mL) before being dried over Na₂SO₄. The solution was filtered and concentrated *in vacuo* to give a slightly yellow solid, which was crystallized from hexane to afford compound 4 as a white solid 3.6g (yield 95%).

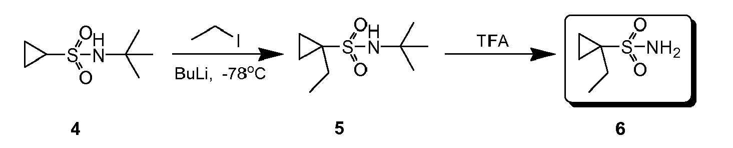

Preparation of compound 6: To a solution of compound **4** (1 g, 5.64 mmol) in dry THF (30 mL) under N₂ was added a solution of n-BuLi (2.5 M in hexane, 5.64 mL, 14.1 mmol) at -78 °C. The reaction mixture was allowed to warm to rt over a period of 2.5 h and then was recooled to -78 °C. The EtI (1.32 g, 8.46 mmol) was added and the reaction mixture was allowed to warm to -10°C over a period of 3 h. The mixture was quenched with a solution of sat. NH₄Cl (20 mL) and extracted with EtOAc (30 x 3 mL). The organic extract was washed with brine (20 mL), dried over MgSO₄, filtered and concentrated *in vacuo* to give a yellow oil which was purified by silica gel to provide compound **5** as a yellow solid 0.58 g (50% yield).

To a solution of compound **5** (0.58g, 2.82 mmol) in dry DCM (15 mL) was added TFA (15 mL). The solution was stirred overnight, then the resulting solution was concentrated *in vacuo* and the residue was treated with hot hexane (5 mL), crystallized to give compound **6** as a white solid 0.4 g (93% yield).

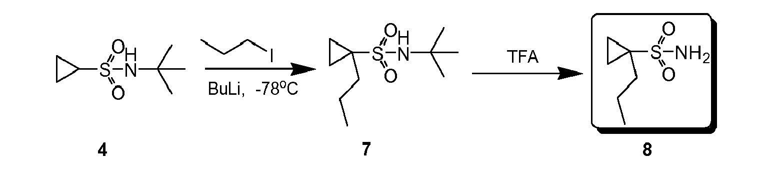

Preparation of compound 8: The compound **7** was prepared following similar general procedure for preparing the compound **5** and the yield was 65%. The compound **8** was prepared following similar general procedure for preparing the compound **6** and the yield was 92%.

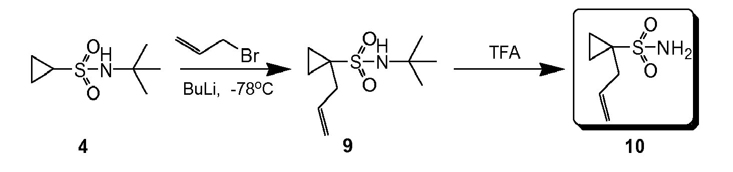

Preparation of compound 10: The compound **9** was prepared following similar general procedure for preparing the compound **5** and the yield was 56%. The compound **10** was prepared following similar general procedure for preparing the compound **6** and the yield was 90%.

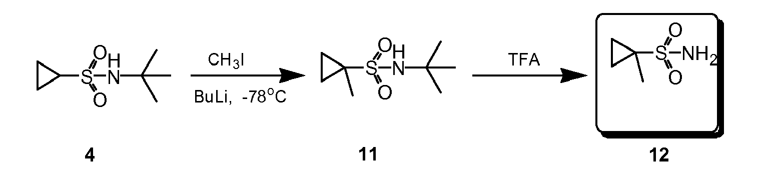

Preparation of compound 12: The compound **11** was prepared following similar general procedure for preparing the compound **5** and the yield was 70%. The compound **12** was prepared following similar general procedure for preparing the compound **6** and the yield was 90%.

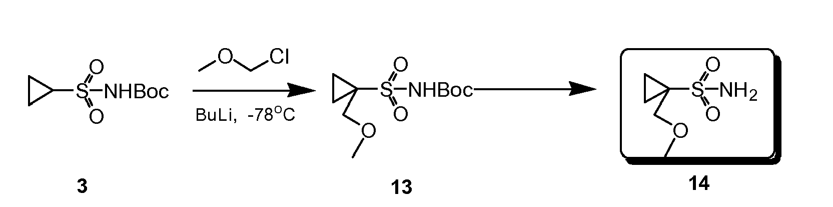

Preparation of compound 14: To a solution of compound **3** (1g 4.5 mmol) dissolved in THF (30 mL) cooled to -78 °C, was added n-butyllithium (4.5 mL, 11.25 mmol, 2.5M in hexane) and the reaction mixture was stirred for 1 h. To this solution was added a neat solution of chloromethyl methyl ether (0.4 mL, 5.24 mmol), and the mixture was slowly allowed to warm to room temperature overnight. The solution pH was adjusted to 3 using 1N aqueous HCl and was then extracted with ethyl acetate (4 x 50 mL portions). The combined extracts were dried (Na₂SO₄), filtered, and concentrated to afford crude product which was purified by silica gel to provide compound 4 as a yellow solid 0.83 g (70% yield).

The compound **14** was prepared following similar general procedure for preparing the compound **6,** and the yield was 94%.

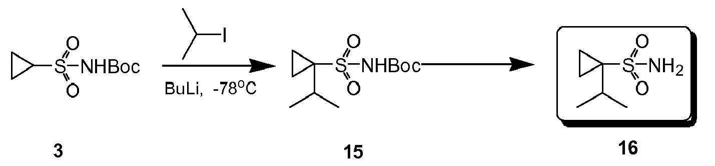

Preparation of compound 16: The compound **15** was prepared following similar general procedure for preparing the compound **11** and the yield was 30%. The compound **16** was prepared following similar general procedure for preparing the compound **6** and the yield was 93%.

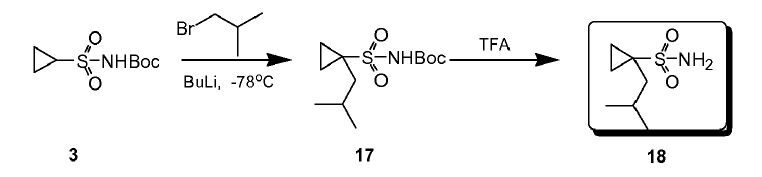

Preparation of compound 18: The compound **17** was prepared following similar general procedure for preparing the compound **11** and the yield was 30%. The compound **18** was prepared following similar general procedure for preparing the compound **6** and the yield was 93%.

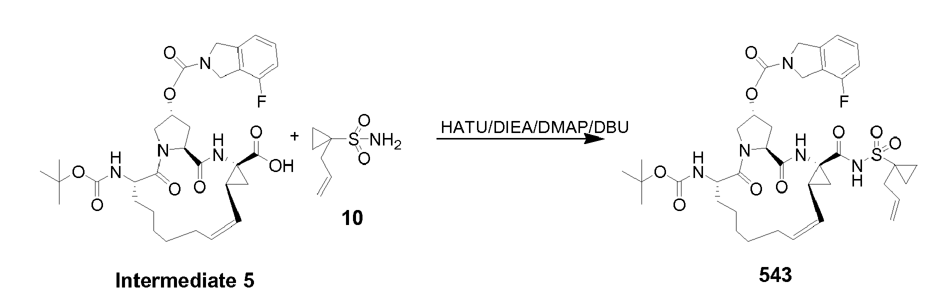

Preparation of compound **543:** To a solution of Intermediate 5 (150 mg, 0.24 mmol) in dry DMF (5 mL) was added HATU (120 mg, 0.316 mmol), DIEA (0.15 mL, 0.96 mmol) under N₂ atmosphere, and the mixture was allowed to stir at room temperature for 1.5 h.. Then sulfonamide (77 mg, 0.48 mmol), DMAP (120 mg, 0.96 mmol) and DBU (0.14 mL, 0.96 mmol) were added, and the mixture was stirred at room overnight. The reaction was quenched by adding EtOAc (20 mL), and washed with aqueous NaOAc buffer (pH 4, 2×15 mL), 5% aqueous NaHCO₃ (15 mL) and brine (20 mL). The organic layer was dried (Na₂SO₄), filtered, and concentrated to get a residue, which was purified by Prep-HPLC to give **compound 543** as white solid, 55 mg (yield 30%). MS (ESI) m/e (M+H⁺) 772.

### Example 28:

### General Procedure for the Preparation of Formula Ib

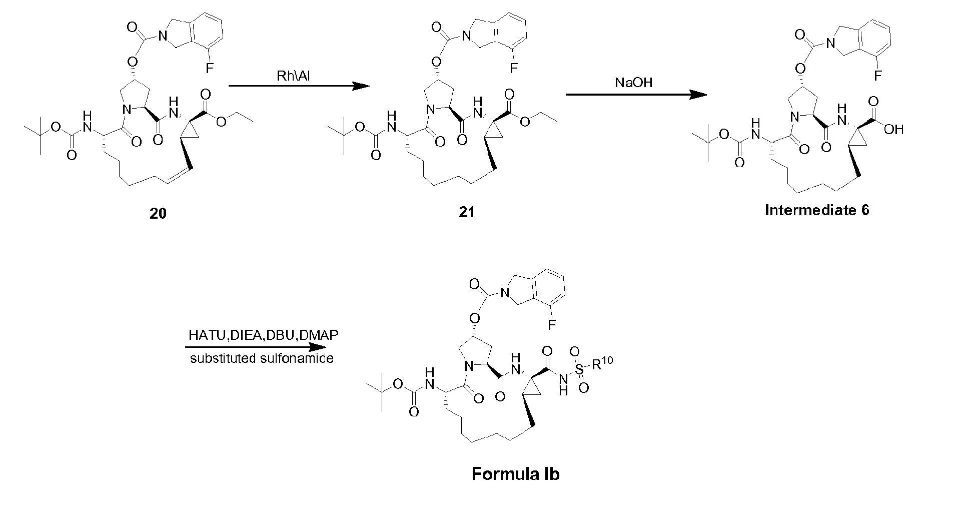

Compound 20 (1 eq) and Rh/Al (5%) (0.1 eq) in ethyl acetate (0.5% for compound 1 ,w/v %) was charged with 1 atm of hydrogen and stirred for 16hr. Catalyst was removed by filtration. H₂O (20 eq of weight for compound 20) and saturated potassium hydrogen sulfate (10 eq of weight for compound 20) was added into the filtration and stirred for 10 min. the organic phase was separated and dried over sodium sulfate. After removal of solvent, the residue was purified by Prep-HPLC to give compound 21.

Preparation of **Intermediate 6:** Compound 21 (1 eq.) was dissolved in toluene (5% w/w%). The obtained solution was concentrated to 50% by reduced vacuum at 30∼35 °C. Charged ethanol to dilute the solution to 8%, concentrated to 50% by vacuum at 30∼35 °C. Aqueous solution of NaOH (12 eq. 20%) was added to the 50% solution of compound 1 in ethanol through 1h, stirred at 5∼10 °C for 4∼5 h. Conc. HCl was added at 5∼10 °C for 1h until the pH was 3-4. Ethyl acetate (30 eq.) and H₂O (52 eq.) were added , stirred for further 30 min. Filtrated and the wet cake was washed two times with water (16 eq.) and two times with MTBE (0.1 eq.). Dried over the vacuum at room temperature to give Intermediate 6 as white solid, which was used without further purification.

Preparation of Formula Ib: To a solution of Intermediate 6 (1 eq.) in dry DMF (0.05 mol/L for Intermediate 6) was added HATU (2 eq.), DIEA (4 eq.) under N₂ atmosphere. Before the addition of substituted sulfonamide (2 eq.), DMAP (4 eq.) and DBU (4 eq.), it was allowed to stirred at room temperature for 1h. It was stirred at room temperature over night. The reactions were monitored by LCMS, and when found to be completed, the mixture was diluted with ethyl acetate and washed with AcONa buffer, 5% aqueous sodium bicarbonate and brine, and dried over MgSO₄, concentrated and purified by prep. HPLC to give a compound of Forumula Ib.

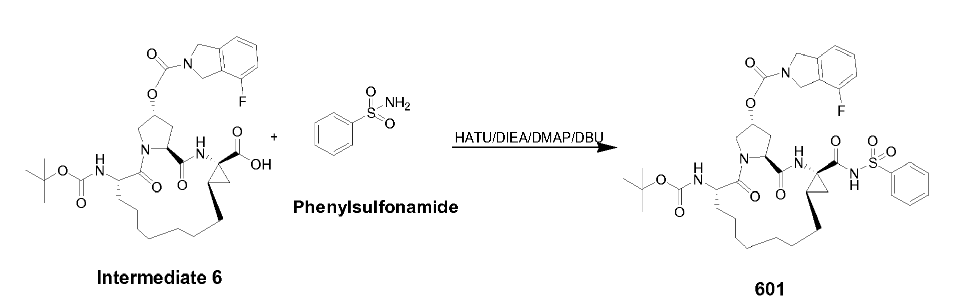

Compound 601 was prepared using the general method described above by reacting Intermediate 6 with phenylsulfonamide. White solid of compound 601 (52% yield) was formed. MS-ESI: m/z=770[M+1]⁺.

**Table 5: Additional examples of compound prepared using Example Procedures 26 and 28**

| Compound | Structure | Mass Spectral Data | Example Procedure Used |
|---|---|---|---|
| 503 | 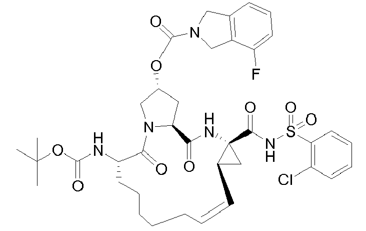 | MS-ESI: m/z=802 [M+1]⁺ | 26 |
| 504 | 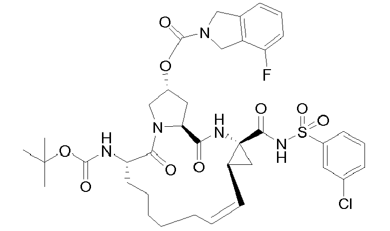 | MS-ESI: m/z=802 [M+1]⁺ | 26 |
| 505 | 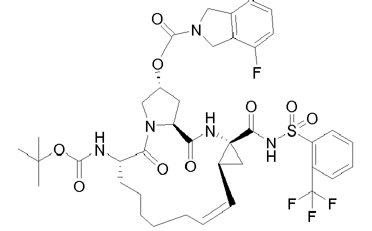 | MS-ESI: m/z=836 [M+1]⁺ | 26 |
| 507 | 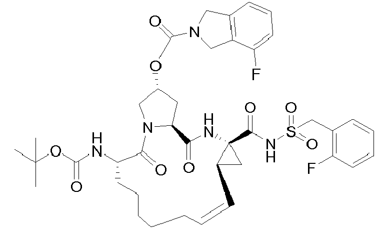 | MS-ESI: m/z=800 [M+1]⁺ | 26 |
| 508 | 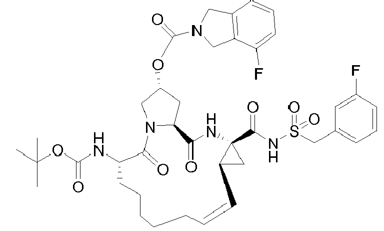 | MS-ESI: m/z=800 [M+1]⁺ | 26 |
| 509 | 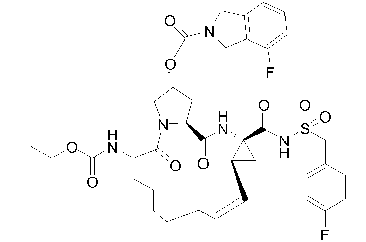 | MS-ESI: m/z=800 [M+1]⁺ | 26 |
| 510 | 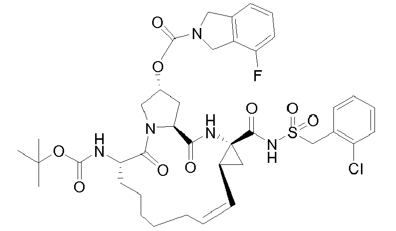 | MS-ESI: m/z=816 [M+1]⁺ | 26 |
| 511 | 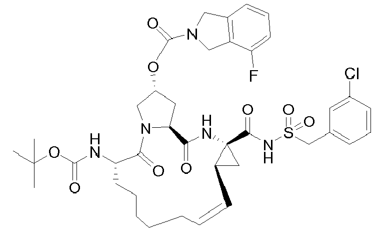 | MS-ESI: m/z=816 [M+1]⁺ | 26 |
| 512 | 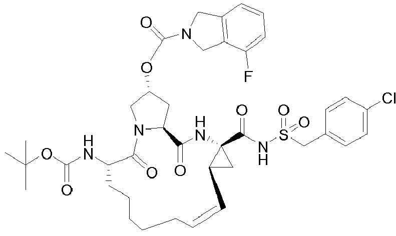 | MS-ESI: m/z=816 [M+1]⁺ | 26 |
| 513 | 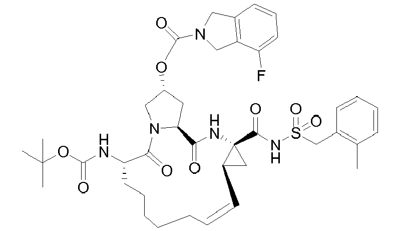 | MS-ESI: m/z=796 [M+1]⁺ | 26 |
| 514 | 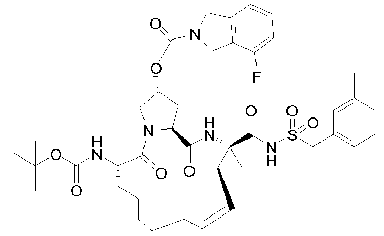 | MS-ESI: m/z=796 [M+1]⁺ | 26 |
| 515 | 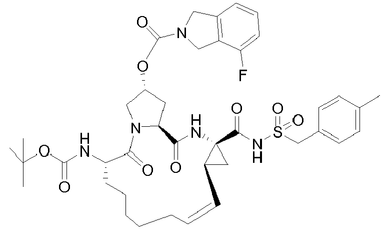 | MS-ESI: m/z=796 [M+1]⁺ | 26 |
| 516 | 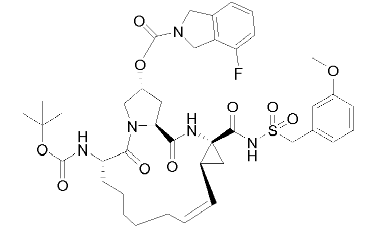 | MS-ESI: m/z=812 [M+1]⁺ | 26 |
| 517 | 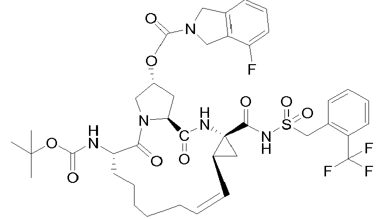 | MS-ESI: m/z=853 [M+1]⁺ | 26 |
| 518 | 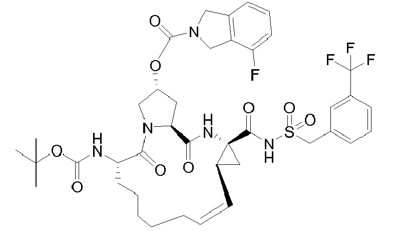 | MS-ESI: m/z=850 [M+1]⁺ | 26 |
| 519 | 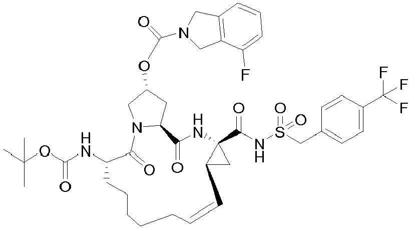 | MS-ESI: m/z=850 [M+1]⁺ | 26 |
| 520 | 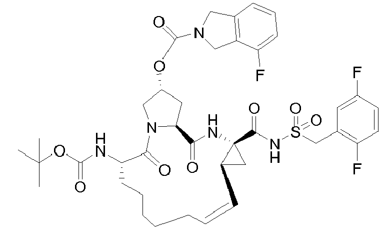 | MS-ESI: m/z=818 [M+1]⁺ | 26 |
| 521 | 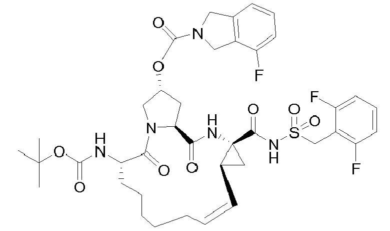 | MS-ESI: m/z=818 [M+1]⁺ | 26 |
| 522 | 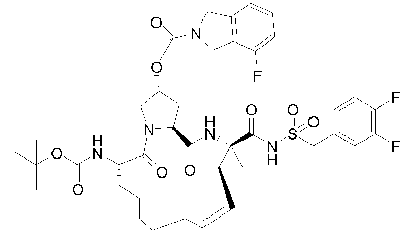 | MS-ESI: m/z=818 [M+1]⁺ | 26 |
| 523 | 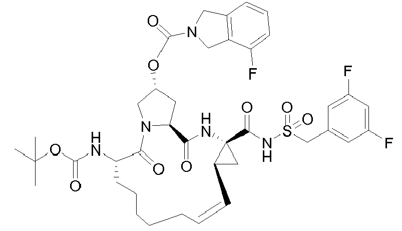 | MS-ESI: m/z=818 [M+1]⁺ | 26 |
| 524 | 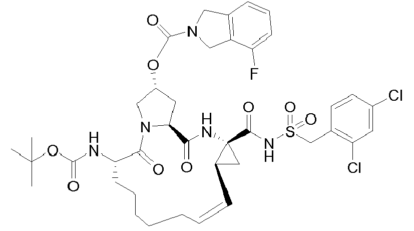 | MS-ESI: m/z=850 [M+1]⁺ | 26 |
| 525 | 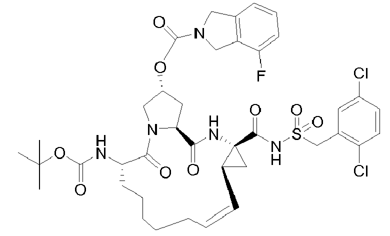 | MS-ESI: m/z=850 [M+1]⁺ | 26 |
| 526 | 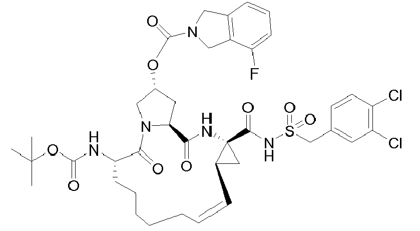 | MS-ESI: m/z=850 [M+1]⁺ | 26 |
| 527 | 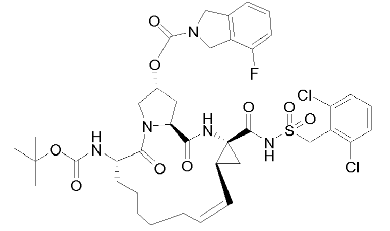 | MS-ESI: m/z=850 [M+1]⁺ | 26 |
| 528 | 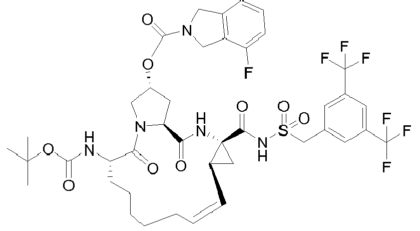 | MS-ESI: m/z=918 [M+1]⁺ | 26 |
| 529 | 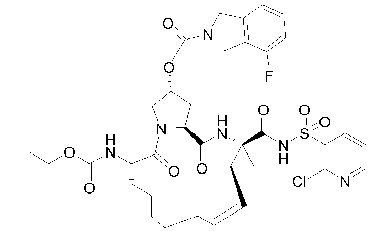 | MS-ESI: m/z=803 [M+1]⁺ | 26 |
| 530 | 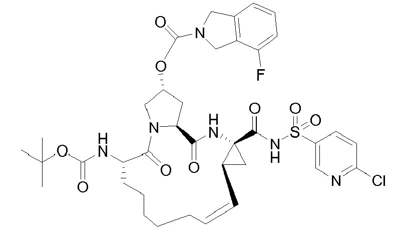 | MS-ESI: m/z=803 [M+1]⁺ | 26 |
| 531 | 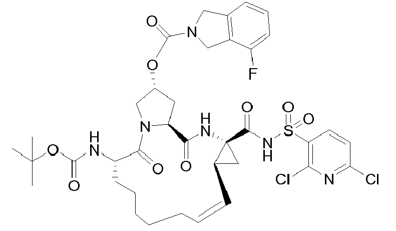 | MS-ESI: m/z=837 [M+1]⁺ | 26 |
| 532 | 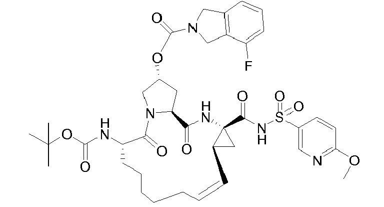 | MS-ESI: m/z=799[M+ 1]⁺ | 26 |
| 533 | 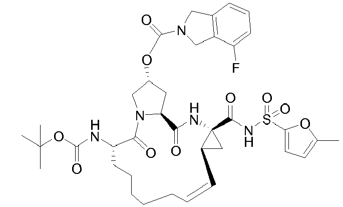 | MS-ESI: m/z=772 [M+1]⁺ | 26 |
| 534 | 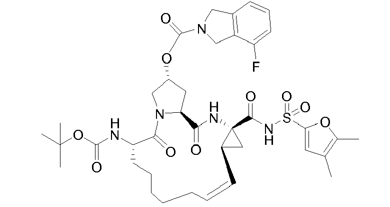 | MS-ESI: m/z=786 [M+1]⁺ | 26 |
| 535 | 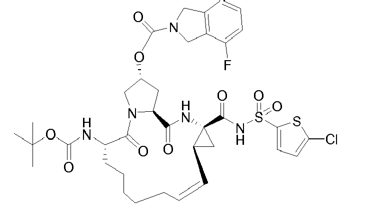 | MS-ESI: m/z=808 [M+1]⁺ | 26 |
| 536 | 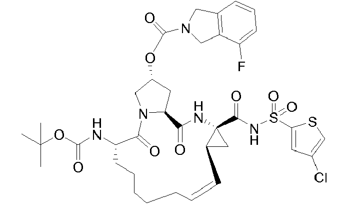 | MS-ESI: m/z=808 [M+1]⁺ | 26 |
| 537 | 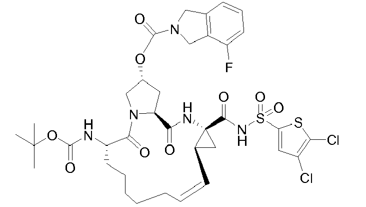 | MS-ESI: m/z=842 [M+1]⁺ | 26 |
| 538 | 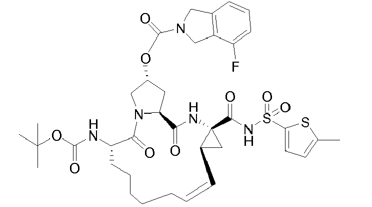 | MS-ESI: m/z=788 [M+1]⁺ | 26 |
| 539 | 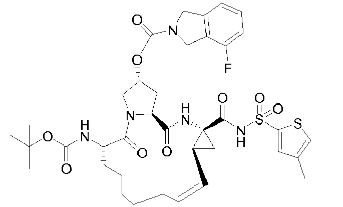 | MS-ESI: m/z=788 [M+1]⁺ | 26 |
| 540 | 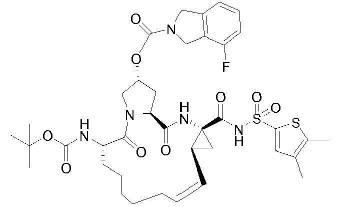 | MS-ESI: m/z=802 [M+1]⁺ | 26 |
| 541 | 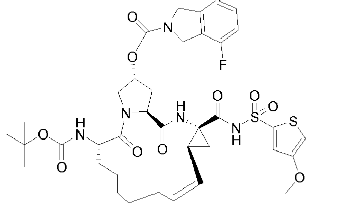 | MS-ESI: m/z=804 [M+1]⁺ | 26 |
| 542 | 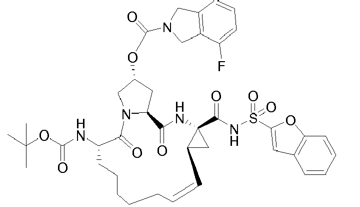 | MS-ESI: m/z=808 [M+1]⁺ | 26 |
| 544 | 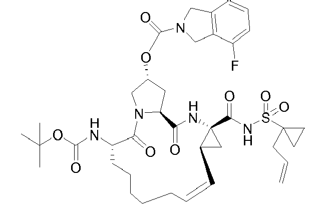 | MS (ESI) m/e (M+H⁺) 746.3 | 27 |
| 545 | 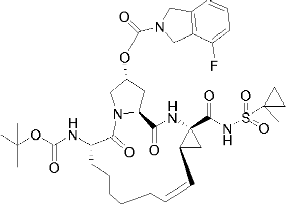 | MS (ESI) m/e (M+H⁺) 760.3 | 27 |
| 546 | 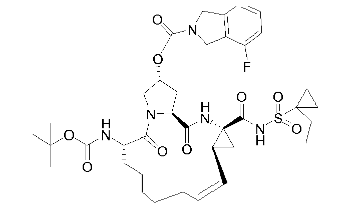 | MS (ESI) m/e (M+H⁺) 774.4 | 27 |
| 547 | 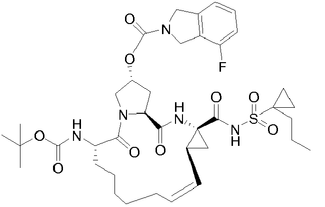 | MS (ESI) m/e (M+H⁺) 776.3 | 27 |
| 548 | 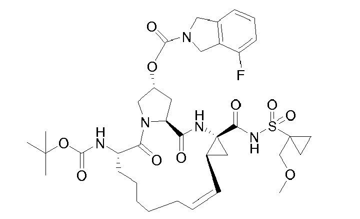 | MS (ESI) m/e (M+H⁺) 788.4 | 27 |
| 549 | 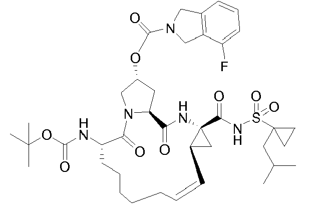 | MS-ESI: m/z=774.4 [M+1]⁺ | 27 |
| 550 | 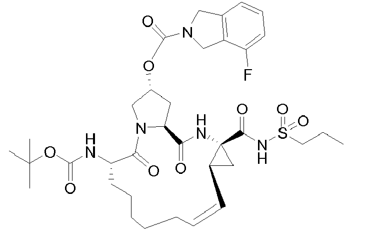 | MS-ESI: m/z=734.3 [M+1]⁺ | 27 |
| 551 | 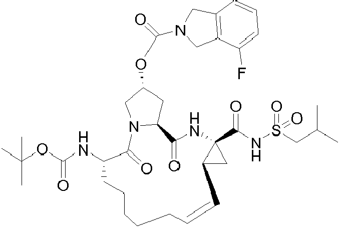 | MS-ESI: m/z=748.3 [M+1]⁺ | 27 |
| 602 | 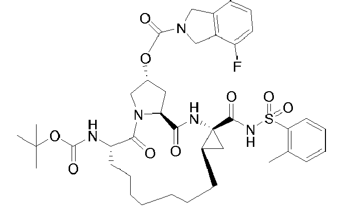 | MS-ESI: m/z=784 [M+1]⁺ | 28 |
| 603 | 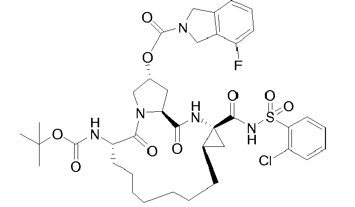 | MS-ESI: m/z=804 [M+1]⁺ | 28 |
| 604 | 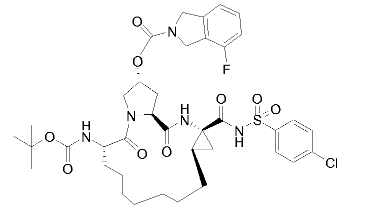 | MS-ESI: m/z=804 [M+1]⁺ | 28 |
| 605 | 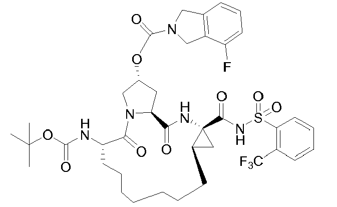 | MS-ESI: m/z=838 [M+1]⁺ | 28 |
| 606 | 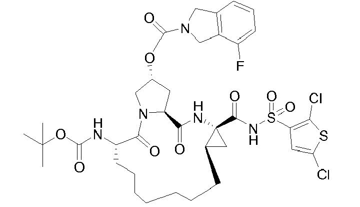 | MS-ESI: m/z=844 [M+1]⁺ | 28 |
| 607 | 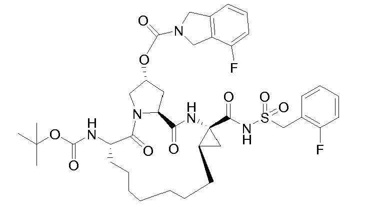 | MS-ESI: m/z=802 [M+1]⁺ | 28 |
| 608 | 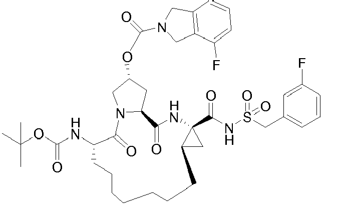 | MS-ESI: m/z=802 [M+1]⁺ | 28 |
| 609 | 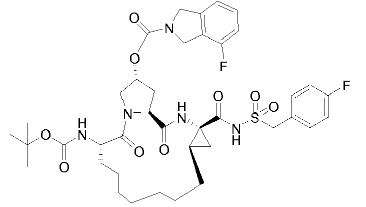 | MS-ESI: m/z=802 [M+1]⁺ | 28 |
| 610 | 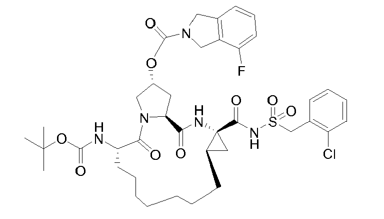 | MS-ESI: m/z=818 [M+1]⁺ | 28 |
| 611 | 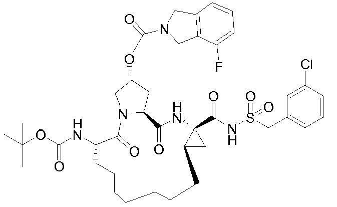 | MS-ESI: m/z=818 [M+1]⁺ | 28 |
| 612 | 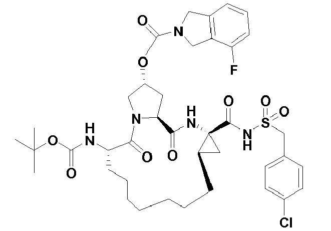 | MS-ESI: m/z=818 [M+1]⁺ | 28 |
| 613 | 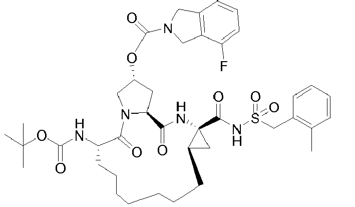 | MS-ESI: m/z=798 [M+1]⁺ | 28 |
| 614 | 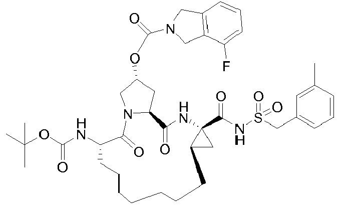 | MS-ESI: m/z=798 [M+1]⁺ | 28 |
| 615 | 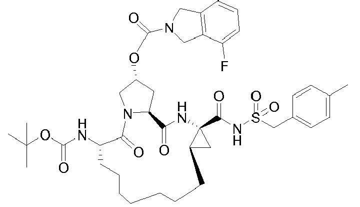 | MS-ESI: m/z=798 [M+1]⁺ | 28 |
| 616 | 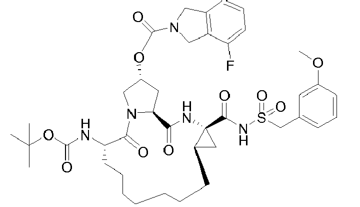 | MS-ESI: m/z=814 [M+1]⁺ | 28 |
| 617 | 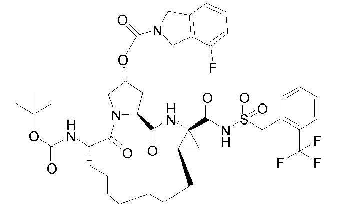 | MS-ESI: m/z=852 [M+1]⁺ | 28 |
| 618 | 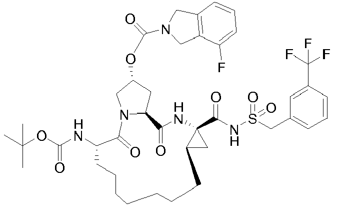 | MS-ESI: m/z=852 [M+1]⁺ | 28 |
| 619 | 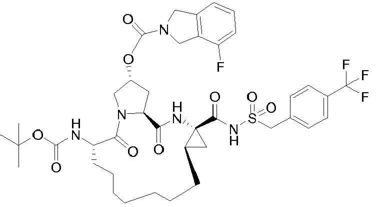 | MS-ESI: m/z=852 [M+1]⁺ | 28 |
| 620 | 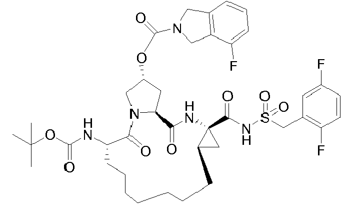 | MS-ESI: m/z=820 [M+1]⁺ | 28 |
| 621 | 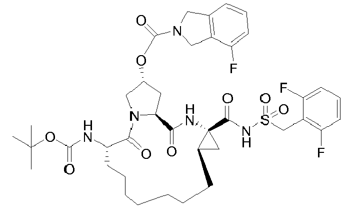 | MS-ESI: m/z=820 [M+1]⁺ | 28 |
| 622 | 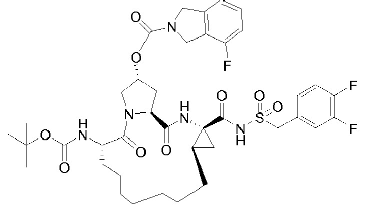 | MS-ESI: m/z=820 [M+1]⁺ | 28 |
| 623 | 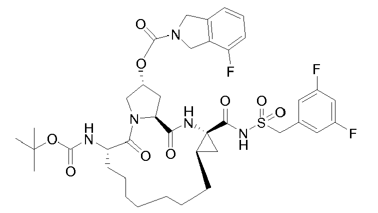 | MS-ESI: m/z=820 [M+1]⁺ | 28 |
| 624 | 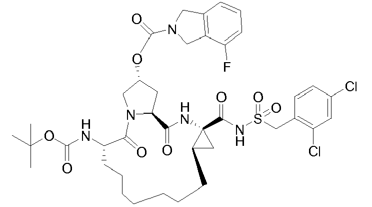 | MS-ESI: m/z=852 [M+1]⁺ | 28 |
| 625 | 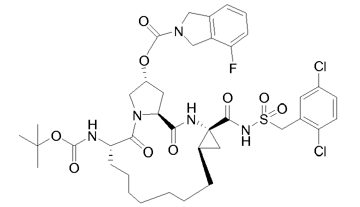 | MS-ESI: m/z=852 [M+1]⁺ | 28 |
| 626 | 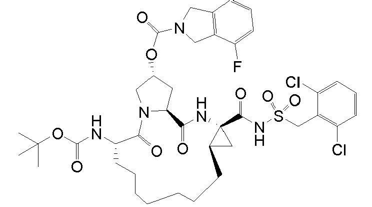 | MS-ESI: m/z=852 [M+1]⁺ | 28 |
| 627 | 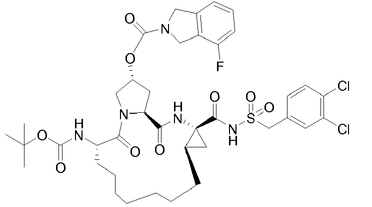 | MS-ESI: m/z=852 [M+1]⁺ | 28 |
| 628 | 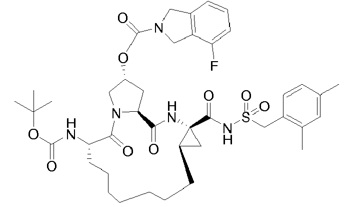 | MS-ESI: m/z=813 [M+1]⁺ | 28 |
| 629 | 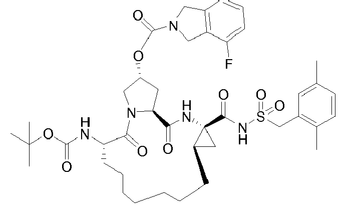 | MS-ESI: m/z=812 [M+1]⁺ | 28 |
| 630 | 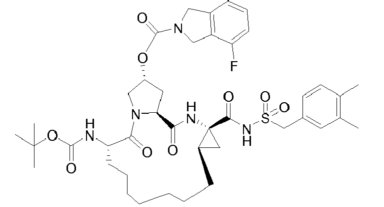 | MS-ESI: m/z=812 [M+1]⁺ | 28 |
| 631 | 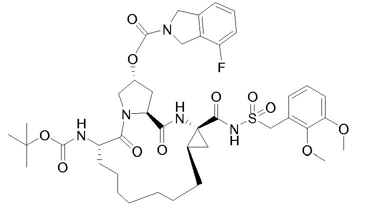 | MS-ESI: m/z=844 [M+1]⁺ | 28 |
| 632 | 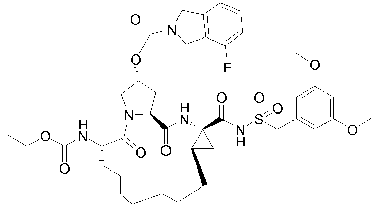 | MS-ESI: m/z=843 [M+1]⁺ | 28 |
| 633 | 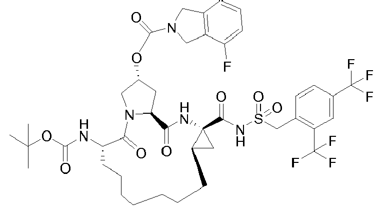 | MS-ESI: m/z=920 [M+1]⁺ | 28 |
| 634 | 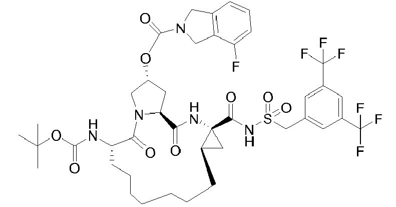 | MS-ESI: m/z=920 [M+1]⁺ | 28 |
| 635 | 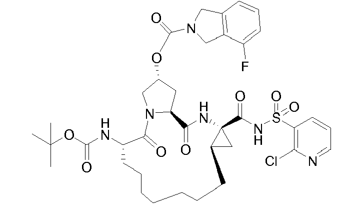 | MS-ESI: m/z=805 [M+1]⁺ | 28 |
| 636 | 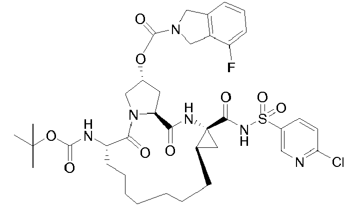 | MS-ESI: m/z=805 [M+1]⁺ | 28 |
| 637 | 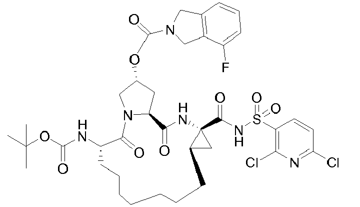 | MS-ESI: m/z=839.2 [M+1]⁺ | 28 |
| 638 | 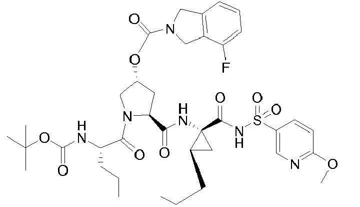 | MS-ESI: m/z=801 [M+1]⁺ | 28 |
| 639 | 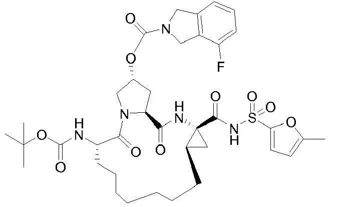 | MS-ESI: m/z=744 [M+1]⁺ | 28 |
| 640 | 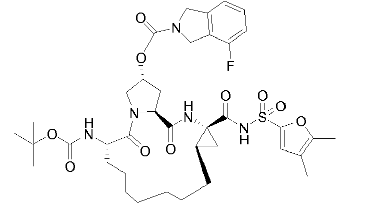 | MS-ESI: m/z=788 [M+1]⁺ | 28 |
| 641 | 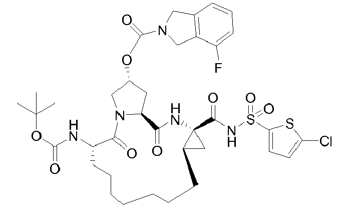 | MS-ESI: m/z=810 [M+1]⁺ | 28 |
| 642 | 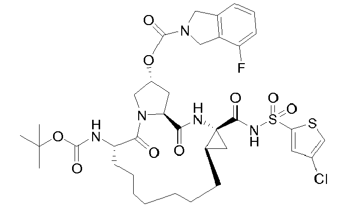 | MS-ESI: m/z=810 [M+1]⁺ | 28 |
| 643 | 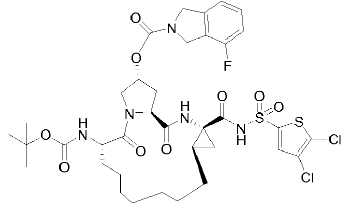 | MS-ESI: m/z=845 [M+1]⁺ | 28 |
| 644 | 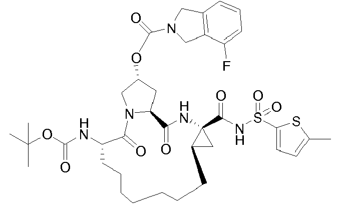 | MS-ESI: m/z=790 [M+1]⁺ | 28 |
| 645 | 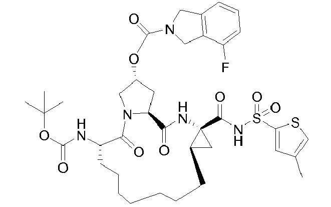 | MS-ESI: m/z=791 [M+1]⁺ | 28 |
| 646 | 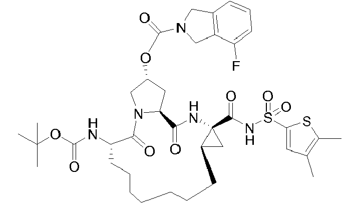 | MS-ESI: m/z=804 [M+1]⁺ | 28 |
| 647 | 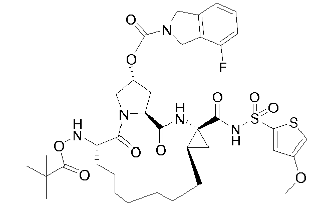 | MS-ESI: m/z=806 [M+1]⁺ | 28 |
| 648 | 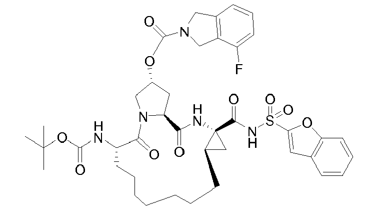 | MS-ESI: m/z=810 [M+1]⁺ | 28 |
| 649 | 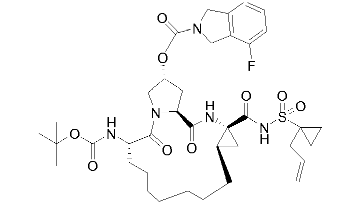 | MS (ESI) m/e (M+H⁺) 774.4 | 28 |
| 650 | 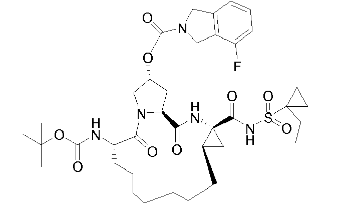 | MS (ESI) m/e (M+H⁺) 762.4 | 28 |
| 651 | 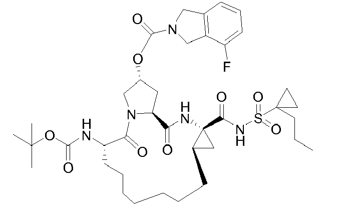 | MS (ESI) m/e (M+H⁺) 776.4 | 28 |
| 652 | 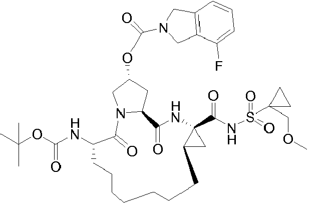 | MS (ESI) m/e (M+H⁺) 778.3 | 28 |
| 653 | 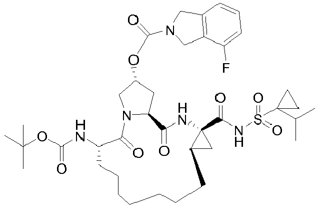 | MS (ESI) m/e (M+H⁺) 776.4 | 28 |
| 654 | 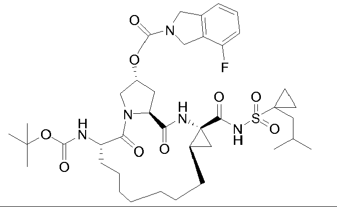 | MS (ESI) m/e (M+H⁺) 790.4 | 28 |
| 655 | 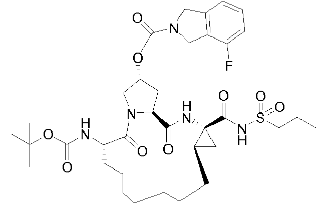 | MS (ESI) m/e (M+H⁺) 736.3 | 28 |
| 656 | 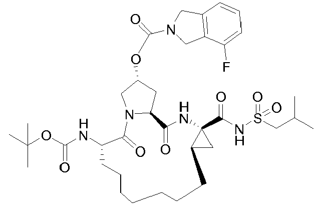 | MS (ESI) m/e (M+H⁺) 750.3 | 28 |

### Example 29

### General Procedure for the Preparation of Formula Ic

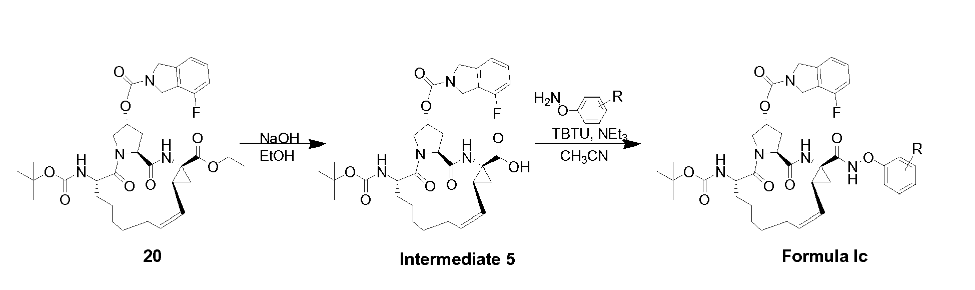

Compound 20 (1 eq.) was dissolved in toluene (5 w/w%). The obtained solution was concentrated to 50% by reduced vacuum at 30∼35 °C. Charge ethanol to dilute the solution to 8%, concentrated to 50% by vacuum at 30∼35 °C. Aqueous solution of NaOH (12 eq. 20%) was added to the 50% solution of compound 20 in ethanol through 1h, stirred at 5∼10 °C for 4∼5 h. Conc. HCl was added at 5∼10 °C for 1h until the pH was 3-4. Ethyl acetate (30 eq.) and H₂O (52 eq.) were added , stirred for further 30 min. Filtrated and the wet cake was washed two times with water (16 eq.) and two times with MTBE (0.1 eq.). Dried over the vacuum at room temperature to give **Intermediate 5** as white solid which was used without further purification.

Preparation of Formula Ic: To a solution of **Intermediate 5** (1 eq.) in dry CH₃CN (0.05 mol/L for Intermediate 5) was added substituted hydroxylamine (2 eq.), NEt₃ (4 eq.) under N₂ atmosphere. Before the addition of TBTU (2 eq.), it was allowed to stirred at room temperature for 0.5 h. It was stirred at room temperature for another 1 h after adding TBTU. The reactions were monitored by LCMS, and when found to be completed, it was diluted with ethyl acetate washed with HCl (2 *N*), 5% aqueous sodium bicarbonate and brine, dried over Na₂SO₄, concentrated and purified by *prep-HPLC* to give a compound of Formula 1c.

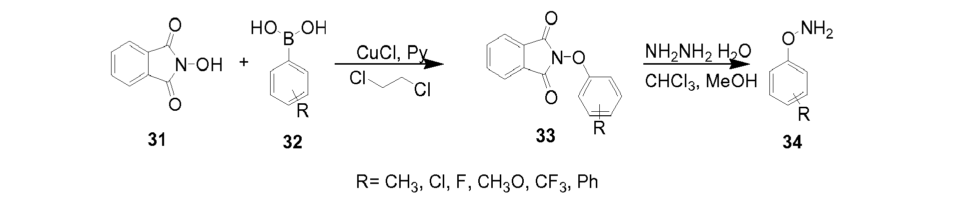

Preparation of substituted hydroxylamine: A flask was charged with N-hydroxyphthalimide (1 mmol), CuCl (1 mmol), freshly activated 4 Å molecular sieves (∼250 mg), and R-substituted phenylboronic acid (2.0 mmol). 1,2-Dichloroethane (5 mL) was added followed by pyridine (90 µL, 1.1 mmol), resulting in a light brown suspension. The cap was loosely applied such that the reaction suspension was open to air and stirred at room temperature until completion was detected by analytical RP-HPLC (the mixture turned from brown to emerald green as the reaction proceeded). Upon completion (∼48 h), the mixture was adsorbed onto silica gel and concentrated to a powder. Flash chromatographic purification over silica (25% EtOAc in hexanes) afforded *N*-aryloxyphthalimide **33** as a white solid.

Preparation of aryloxyamines **(34):** Hydrazine monohydrate (0.40 mL, 8.2 mmol) was added slowly to a solution of *N*-aryloxyphthalimide **33** (652 mg, 2.73 mmol) in 10% MeOH in CHCl₃ (25 mL) and the reaction was stirred at room temperature. Upon completion (TLC monitoring, 12 h) a white precipitate appeared (the phthalizine) in a colorless reaction solution. The reaction mixture was passed through a plug of silica gel, washing with 30% EtOAc in hexane. Removal of the EtOAc/hexanes produced a slightly pale yellow oil.

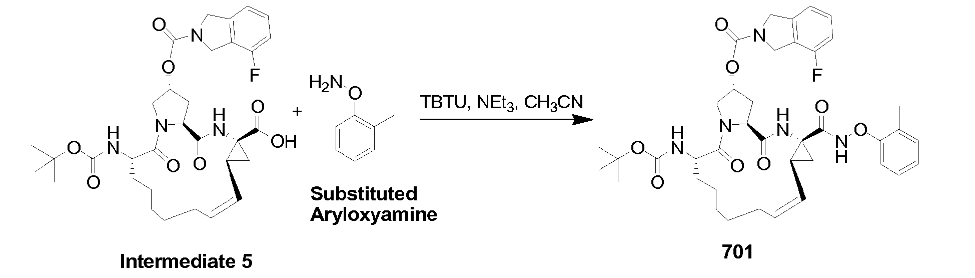

Compound 701 was prepared using the method described above by reacting Intermediate 5 with and substituted aryloxyamine. White solid (37% yield) was formed. MS-ESI: m/z=734 [M+1]⁺.

### Example 30

### General Procedure for the Preparation of Formula Id

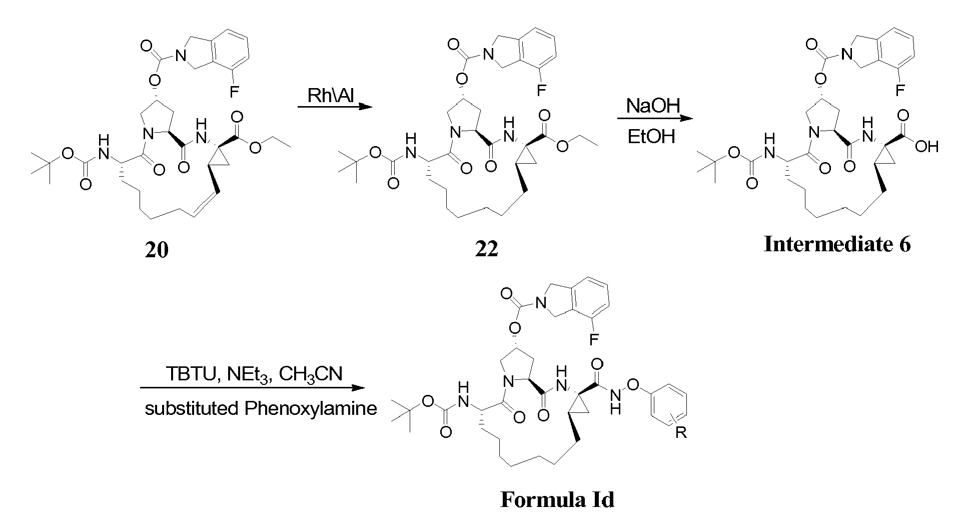

Compound 20 (1. eq) and Rh/Al (5%) (0.1 eq) in ethyl acetate (0.5% for compound 1, w/v%) was charged with 1 atm of hydrogen and stirred for 16 h. Catalyst was removed by filtration. H₂O (20 eq of weight for compound 20) and saturated potassium hydrogen sulfate (10 eq of weight for compound 20) was added into the filtration and stirred for 10 min. the organic phase was separated and dried over sodium sulfate. After removal of solvent, the residue was purified by Prep-HPLC.

Preparation of **Intermediate 6:** compound 22 (1 eq.) was dissolved in toluene (5 w/w%). The obtained solution was concentrated to 50% by reduced vacuum at 30∼35 °C. Ethanol was added to dilute the solution to 8%, and the solution was concentrated to 50% by vacuum at 30∼35 °C. Aqueous solution of NaOH (12 eq. 20%) was added to the 50% solution of compound 22 in ethanol through 1h, stirred at 5∼10 °C for 4∼5 h. Conc. HCl was added at 5∼10 °C for 1h until the pH was 3-4. Ethyl acetate (30 eq.) and H₂O (52 eq.) were added, stirred for further 30 min. The mixture was filtrated and the wet cake was washed two times with water (16 eq.) and two times with MTBE (0.1 eq.). Dried over the vacuum at room temperature to give **Intermediate 6** as white solid, which was used without further purification.

Preparation of Formula **Id:** To a solution of **Intermediate 6** (1 eq.) in dry CH₃CN (0.05 mol/L for Intermediate 6) was added substituted aryloxylamine (2 eq.), NEt₃ (4 eq.) under N₂ atmosphere, and allowed to stirred at room temperature for 0.5 h. After the addition of TBTU (2 eq.), it was stirred at room temperature for another 1 h. The reactions were monitored by LCMS, and when found to be completed diluted with ethyl acetate washed with HCl (2 *N*), 5% aqueous sodium bicarbonate and brine, dried over Na₂SO₄, concentrated and purified by prep-HPLC to give a compound of Formula **Id.**

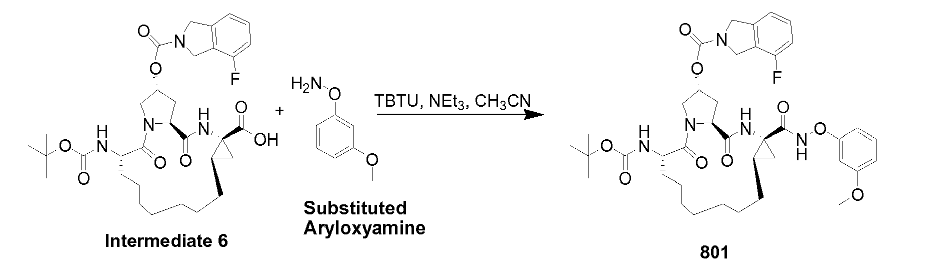

Compound 801 was prepared using the method described above by reacting Intermediate 6 and substituted aryloxyamine. White solid (32% yield) was formed. MS-ESI: m/z=752 [M+1]⁺.

### Example 31

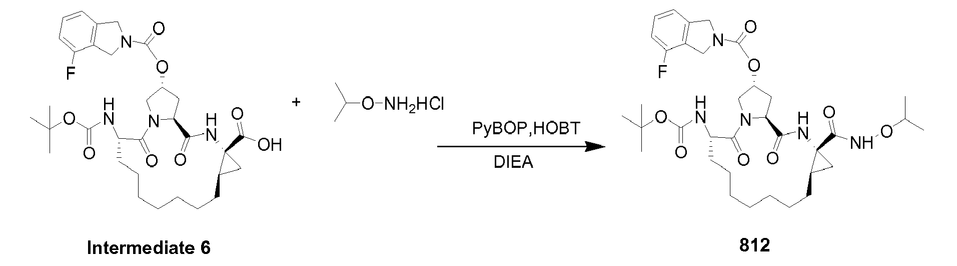

To a solution of Intermediate 6 (150 mg, 0.24 mmol) in 5 m of dry DMF was added PyBOP (185 mg, 0.36 mmol), HOBT (54.6 mg, 0.36 mmol) at room temperature. The resulting mixture was stirred at same temperature for 2h. Then O-phenylhydroxylamine-hydrochloride (66.1 mg, 0.60 mmol) and DIEA (138.2 mg, 1.0 mmol) were added to the mixture and stirred overnight at rt. The reaction was quenched by adding water (20 mL), extract by ethyl acetate (3 x 15mL), combined organic layers was washed with brine, dried over Na₂SO₄, concentrated to get a residue, which was purified by Prep-HPLC to give **compound 812** as white solid 46.7 mg (yield 28.3 %). MS (ESI) m/e (M+H⁺) 688.3.

**Table 6: Additional examples of compound prepared using Example Procedures 29-31**

| Compound | Structure | Mass Spectral Data | Example Procedure Used |
|---|---|---|---|
| 702 | 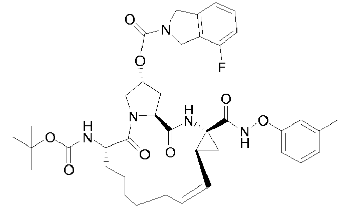 | MS-ESI: m/z=734 [M+1]⁺ | 29 |
| 703 | 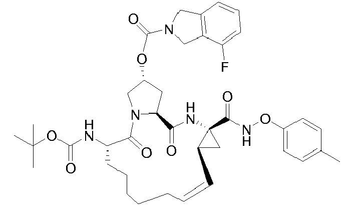 | MS-ESI: m/z=738 [M+1]⁺ | 29 |
| 704 | 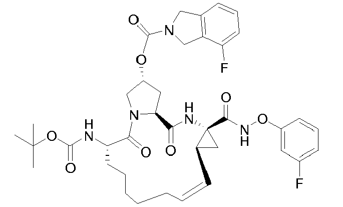 | MS-ESI: m/z=738 [M+1]⁺ | 29 |
| 705 | 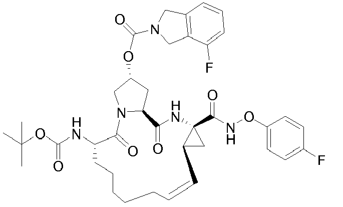 | MS-ESI: m/z=738 [M+1]⁺ | 29 |
| 706 | 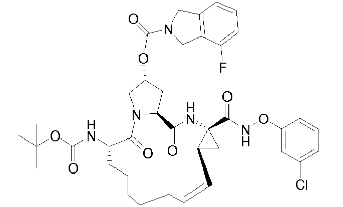 | MS-ESI: m/z=754 [M+1]⁺ | 29 |
| 707 | 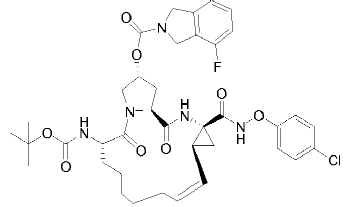 | MS-ESI: m/z=754 [M+1]⁺ | 29 |
| 708 | 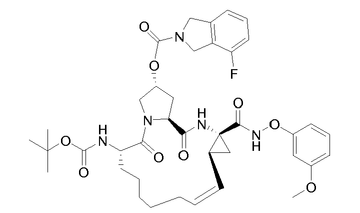 | MS-ESI: m/z=750 [M+1]⁺ | 29 |
| 709 | 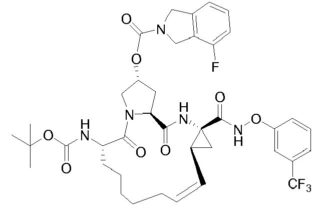 | MS-ESI: m/z=788 [M+1]⁺ | 29 |
| 710 | 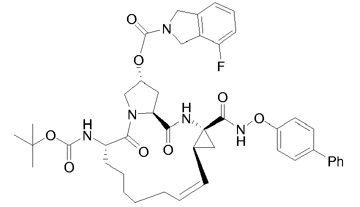 | MS-ESI: m/z=796 [M+1]⁺ | 29 |
| 711 | 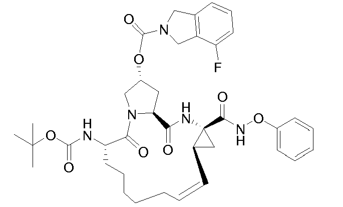 | MS-ESI: m/z=720 [M+1]⁺ | 29 |
| 712 | 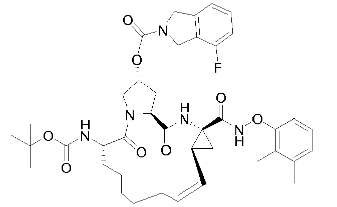 | MS-ESI: m/z=748 [M+1]⁺ | 29 |
| 713 | 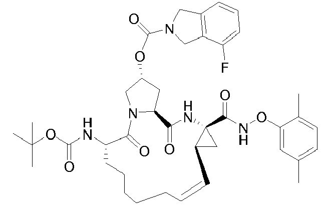 | MS-ESI: m/z=748 [M+1]⁺ | 29 |
| 714 | 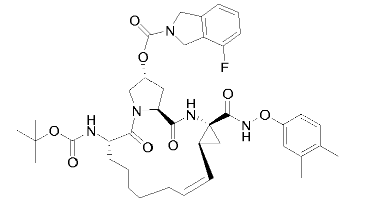 | MS-ESI: m/z=748 [M+1]⁺ | 29 |
| 715 | 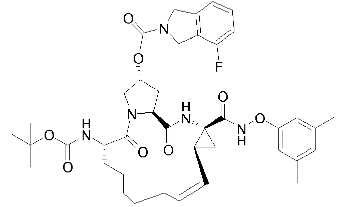 | MS-ESI: m/z=748 [M+1]⁺ | 29 |
| 716 | 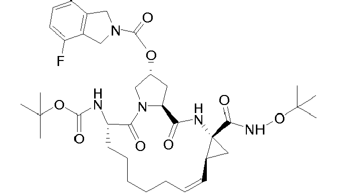 | MS (ESI) m/e (M+H⁺) 700.3 | 29 |
| 717 | 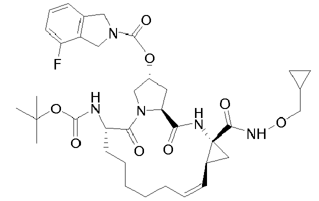 | MS (ESI) m/e (M+H⁺) 698.3 | 29 |
| 718 | 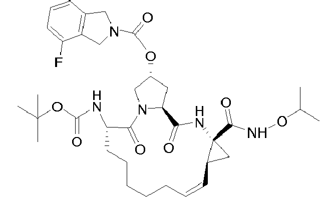 | MS (ESI) m/e (M+H⁺) 686.3 | 29 |
| 719 | 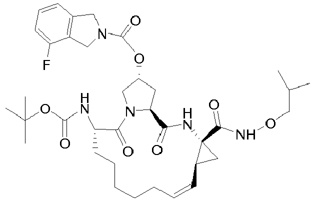 | MS (ESI) m/e (M+H+) 700.2 | 29 |
| 720 | 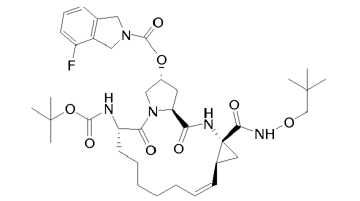 | MS (ES) m/e (M+H⁺) 714.3 | 29 |
| 802 | 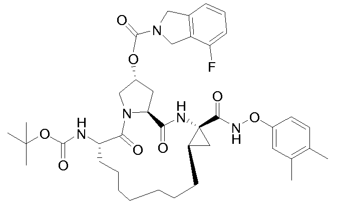 | MS-ESI: m/z=750 [M+1]⁺ | 30 |
| 803 | 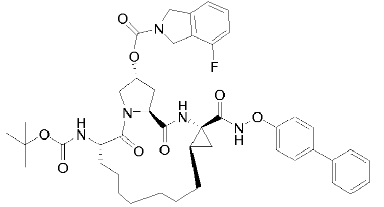 | MS-ESI: m/z=798 [M+1]⁺ | 30 |
| 804 | 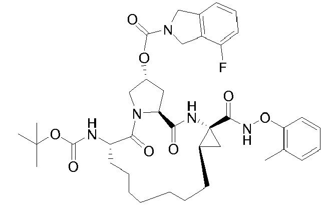 | MS-ESI: m/z=736 [M+1]⁺ | 30 |
| 805 | 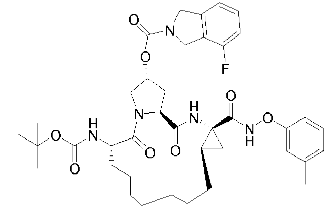 | MS-ESI: m/z=736 [M+1]⁺ | 30 |
| 806 | 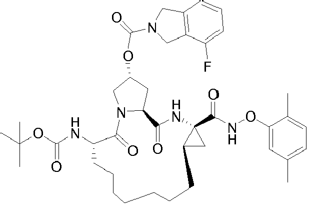 | MS-ESI: m/z=750 [M+1]⁺ | 30 |
| 807 | 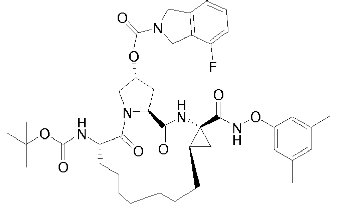 | MS-ESI: m/z=750 [M+1]⁺ | 30 |
| 808 | 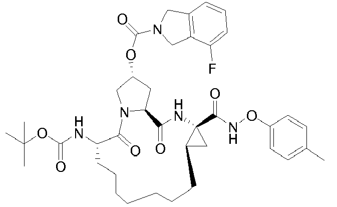 | MS-ESI: m/z=736 [M+1]⁺ | 30 |
| 809 | 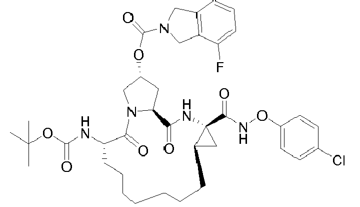 | MS-ESI: m/z=756 [M+1]⁺ | 30 |
| 810 | 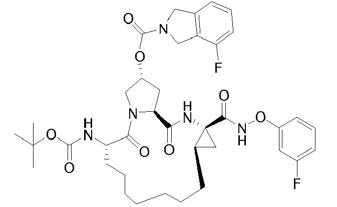 | MS-ESI: m/z=740 [M+1]⁺ | 30 |
| 811 | 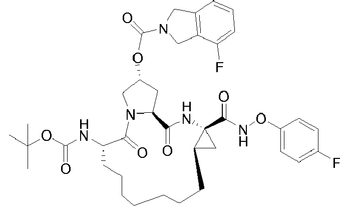 | MS-ESI: m/z=740 [M+1]⁺ | 30 |
| 813 | 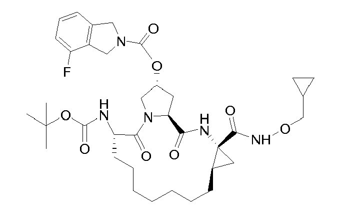 | MS (ESI) m/e (M+H⁺) 700.2 | 31 |
| 814 | 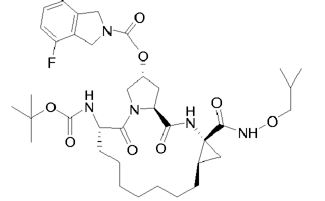 | MS (ESI) m/e (M+H⁺) 702.3 | 31 |
| 815 | 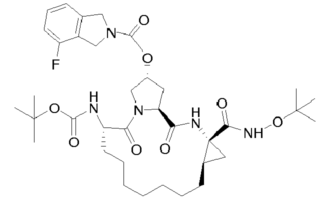 | MS (ESI) m/e (M+H⁺) 702.2 | 31 |

### Example 32:

### General Procedure for preparing cyclopropane Ring Acids

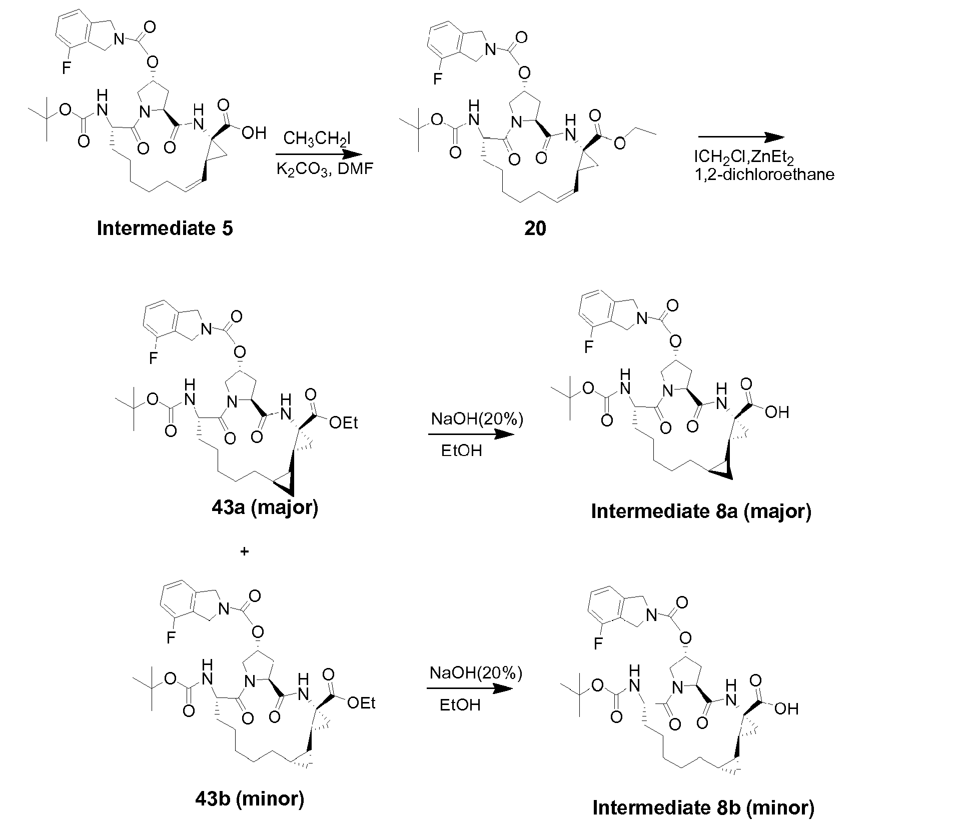

A mixture of **Intermediate 5** (1 g, 5.6 mmol) in DMF (35 mL) in presence of K₂CO₃ (0.86 g, 6.25 mmol) was stirred at room temperature under nitrogen atmosphere. Iodoethane (2.0 g, 12.5 mmol) was dropped into the mixture system. The reaction mixture was diluted with water after stirring for 5h, extracted with ethyl acetate. Compound 20 was obtained after the oil layer dried, filtered and evaporated to give pure white solid and the yield was 88.5 %. MS (ESI) m/e (M+H⁺) 657.2.

Procedure for cyclization: A mixture of diethylzinc (1.25 g, 10.3 mmol) in dichloromethane (10 mL) was cooled to -5 °C. The reaction mixture was stirred for 25 min after chloroiodomethane dropped into the system. Compound 20 (0.9 g , 1.4 mmol) dissoloved in dichloromethane (7.5 mL) was added in one portion at -5 °C, and stirred for 1h under this condition and then stirred for 4∼5h at 5∼10 °C. The resulting mixture was charged with NH₄Cl(aq), and extracted with DCM, dried, concentrated, purified by prep-HPLC. Two isomers **43a** and **43b** were obtained and their yields were 31.4 % and 6.3 % respectively. They have the same mass, MS (ESI) m/e (M+H⁺) 671.1.

Procedure for synthesis of **Intermediate 8a** and **8b:** A mixture of compound **3** (470 mg, 0.37 mol) in ethanol (6 mL) was cooled to 5∼10 °C, and sodium hydroxide (1.5 mL, 6.3 mol/L) was dropped into the system. The resulting mixture was stirred for 4∼5 h at 5∼10 °C, and monitored by LCMS. Charged conc. HCl into the mixture at 5∼10 °C until PH=3∼4. Intermediate 8a (major) and 8b (minor) were obtained after the mixture was extracted with ethyl acetate, dryed, concentrated and purified by prep-HPLC. MS (ESI) m/e (M+H⁺) 643.1.

### Example 33:

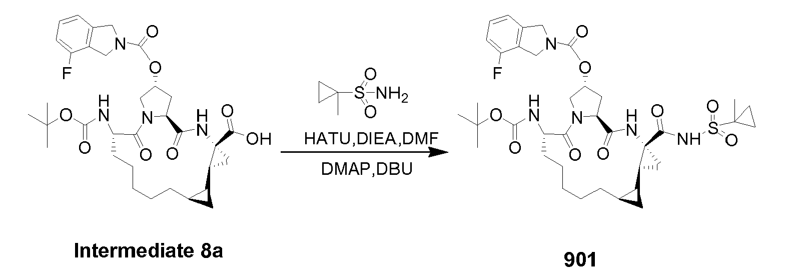

To a solution of **Intermediate 8a** (100 mg, 0.16 mmol) in 4 mL of dry DMF was added HATU (118 mg, 0.3 mmol), DIEA (0.11 mL, 0.6 mmol) at 20°C. The resulting mixture was stirred at same temperature for 1h. After the addition of methylcyclopropanyl sulfonamide (85.6 mg, 0.6mmol), DMAP (76.1 mg, 0.6 mmol), and DBU (0.1 mL, 0.6 mmol), the resulting mixture was stirred overnight at 20°C. The reaction was quenched by adding EtOAc (20mL), and washed with aqueous NaOAc buffer (pH 4, 2×15 mL), 5% aqueous NaHCO₃ (15 mL) and brine (20 mL). The organic layer was dried (Na₂SO₄), filtered, and concentrated to get a residue, which was purified by Prep-HPLC to give compound **901** as white solid, 14.6 mg (yield 12.4%). MS (ESI) m/e (M+H⁺) 760.3

### Example 34:

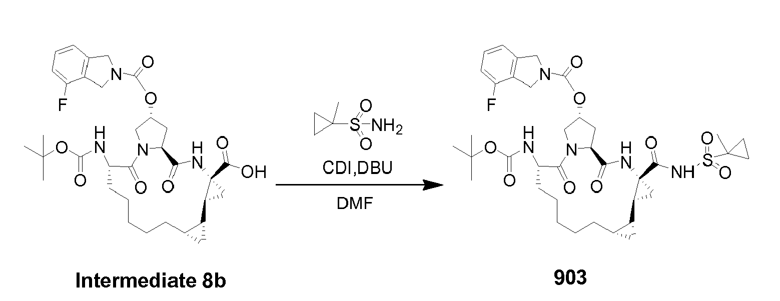

To a solution of **Intermediate 8b** (50 mg, 0.078 mmol) in dry DCM (3 mL) was added CDI (55 mg, 0.34 mmol) at 25°C. The resulting mixture was stirred at same temperature for 1h before the addition of methylcyclopropanyl sulfonamide (15.8 mg, 0.117 mmol) and DBU (0.018 mL, 0.117 mmol). The resulting mixture was stirred overnight at 25°C. That concentrated to get a residue, which was purified by Prep-HPLC to give compound **903** as white solid, 10.6 mg (yield 17.9%). MS (ESI) m/e (M+H⁺) 760.2.

### Example 35:

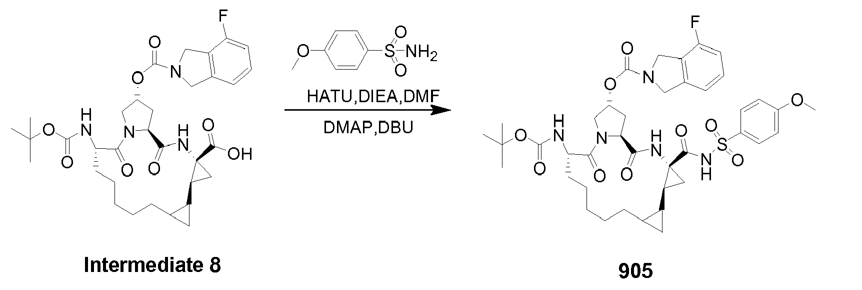

Compound 905 was prepared according to the experimental procedure of Example 26. The pure product was isolated as a white solid. Yield =30.3%. MS (ESI) m/e (M+H⁺) 782.3.

### Example 35:

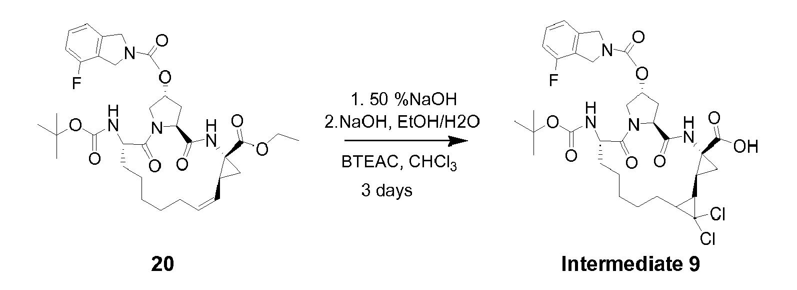

Aqueous NaOH (50%, 30 mL) was added in several portions to a cooled solution of compound 20 (5 g, 7.6 mmol) and BTEAc( benzyl triethyl ammonium chloride) (1.2 g, 6.2 mmol) in chloroform (30 mL) at 0 to 5 °C. The vessel was sealed and allowed to stir for 3 days at ambient temperature. The reaction mixture was diluted with H₂O and extracted with DCM for three times. The combined organics was concentrated in vacuo and purified by P-HPLC (acidic column) to give 0.65 g of compound ester. NaOH (0.3 g, 7.5 mmol) was added the solution of the ester ( 0.3 g, 0.4 mmol) in 10 mL of EtOH and 3 mL of H₂O and stirred for 20 h at rt.

The reaction mixture was concentrated in vacuo and acidified to PH = 3-4 with diluted HCl at 0 °C and extracted with ethyl acetate. The organics was dried over Na₂SO4 and concentrated to afford 0.2 g of **Intermediate 9** as a white solid (yield 11.6%). ¹H NMR (400 MHz, DMSO-*d*₆) δ 8.59 (s, 1H), 7.31 (q, J=7.6Hz), 7.16 (d, J = 7.6 Hz, 1H), 7.06 (t, 8.4 Hz, 1H), 6.97 (br, 1H). 5.23 (s, 1H) 4.33 (br, 1H), 4.17 (t, J = 7.6 Hz), 3.85 (br, 1H), 2.2 (br, 1H), 2.08 (m, 1H), 1.96 (t, J=08.8 Hz) 1.62 (br, 4 H), 1.48-1.22 (br, 11 H), 1.05 (d, 14.8 Hz, 9 H). LC-MS: purity: 96.2%, MS : m/e 733 (M+Na⁺), 611 ( M-Boc+H).

**Table 7: Additional examples of compound prepared using Example Procedures 33 and 35**

| Compound | Structure | Mass Spectral Data | Example Procedure Used |
|---|---|---|---|
| 902 | 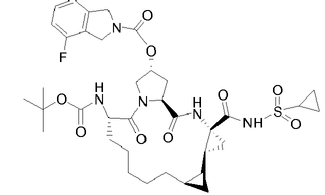 | MS-ESI: m/z=746.1 [M+1]⁺ | 33 |
| 904 | 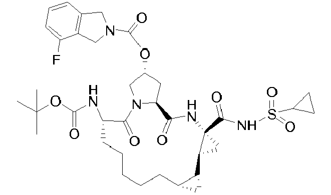 | MS (ESI) m/e (M+H⁺) 746.2 | 34 |
| 906 | 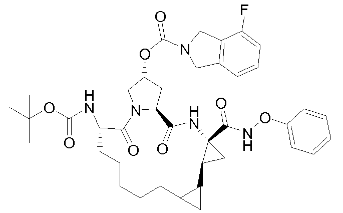 | MS (ESI) m/e (M+H⁺) 734.2 | 35 |

### Example 36:

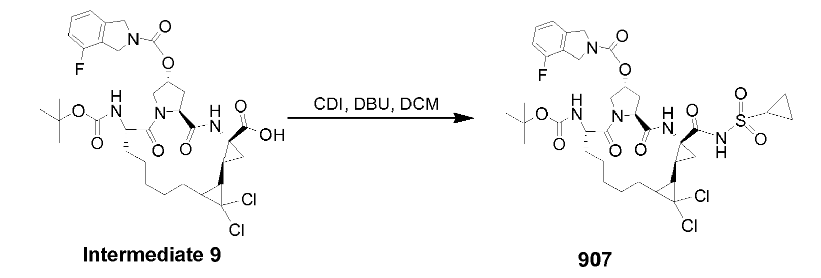

To the solution of **Intermediate 9** (70 mg, 0.1 mmol.) in dichloromethane (5 mL) was added CDI (32 mg, 2 mmol). The resulting mixture was stirred at room temperature for 1 h, then cyclopropyl sulfonamide (30 mg, 0.25 mmol.) and DBU (0.1 mL, 6 eq) was added, the resulting mixture was stirred at room temperature for another 12 h and the reaction was monitored by LCMS. After completion of the reaction, the solvent was removed and the crude was purified by P-HPLC to give the pure compound as the white solid **(907).** ¹H NMR (400 MHz, DMSO-*d*₆) δ: 10.79 (s, 1H), 7.28 ( m, 1H), 7.06 (d, J =7.2 Hz, 1H), 6.96 (q, J = 8.4 Hz, 1H), 6.85 (d, J= 4.4Hz, 1H), 6.23 ( br, 1H), 5.4 (s, 1H), 5.05 (br, 1H), 4.78-4.54 (m, 4H), 4.52-4.85 (m, 2 H), 4.19 (br, 1H), 3.84 ( d, J= 9.6Hz, 1 H), 2.97 (m, 1H), 2.51 ( m, 1H), 2.35 (m, 1H), 1.97 (m, 1H), 1.9 -1.65 (m, 3H), 1.68- 1.3 (m, 13H), 1.27 ( d, 10.4 Hz, 9 H), 1.18 ( m, 1H), 1.01 (m, 1H) Yield= 22%. Lc-Ms: purity 96.9%, MS: m/z = 714 [M-Boc+1]⁺, 836.1 (M+Na).

### Example 37:

**Table 8: Examples of NS3-NS4 activity**

| Compound | EC₅₀ | IC₅₀ | Compound | EC₅₀ | IC₅₀ |
|---|---|---|---|---|---|
| 101 | D | D | 106 | C | D |
| 102 | D | D | 107 | B | D |
| 103 | D | D | 108 | D | D |
| 104 | B | D | 109 | B | D |
| 105 | A | D | 110 | D | D |
| | | | | | |

| Compound | EC₅₀ | IC₅₀ | Compound | EC₅₀ | IC₅₀ |
|---|---|---|---|---|---|
| 201 | D | D | 221 | D | D |
| 202 | C | D | 222 | D | D |
| 203 | B | D | 223 | D | D |
| 204 | D | D | 224 | D | D |
| 205 | D | D | 225 | D | D |
| 206 | C | D | 226 | D | D |
| 207 | D | D | 227 | D | D |
| 208 | D | D | 228 | D | D |
| 209 | D | D | 229 | D | D |
| 210 | D | D | 230 | D | D |
| 211 | D | D | 231 | D | D |
| 212 | D | D | 232 | D | D |
| 213 | D | D | 233 | C | D |
| 214 | D | D | 235 | C | D |
| 215 | D | D | 239 | C | D |
| 216 | D | D | 241 | C | D |
| 217 | D | D | 242 | B | D |
| 218 | D | D | 243 | D | D |
| 219 | D | D | 246 | D | C |
| 220 | D | D | | | |
| | | | | | |

| Compound | EC₅₀ | IC₅₀ | Compound | EC₅₀ | IC₅₀ |
|---|---|---|---|---|---|
| 301 | D | D | 333 | D | D |
| 302 | D | D | 334 | D | D |
| 303 | D | D | 335 | C | D |
| 304 | D | D | 336 | D | D |
| 305 | D | D | 337 | D | D |
| 306 | C | D | 338 | D | D |
| 306 | C | D | 339 | D | D |
| 307 | D | D | 340 | D | D |
| 308 | D | D | 341 | D | D |
| 309 | D | D | 342 | D | D |
| 310 | D | D | 343 | D | D |
| 311 | C | D | 344 | D | D |
| 312 | C | D | 345 | D | D |
| 313 | D | D | 346 | D | D |
| 314 | D | D | 347 | D | D |
| 315 | D | D | 348 | D | D |
| 316 | D | D | 349 | D | D |
| 317 | D | D | 350 | D | D |
| 318 | D | D | 351 | D | D |
| 319 | D | D | 352 | D | D |
| 320 | D | D | 353 | D | D |
| 321 | D | D | 354 | C | D |
| 322 | D | D | 355 | D | D |
| 323 | D | D | 356 | D | D |
| 324 | D | D | 357 | C | C |
| 325 | D | D | 358 | D | D |
| 326 | D | D | 359 | D | D |
| 327 | D | D | 360 | C | D |
| 328 | D | D | 361 | D | D |
| 329 | D | D | 362 | C | D |
| 330 | D | D | 363 | B | D |
| 331 | D | D | 364 | C | D |
| 332 | D | D | | | |
| | | | | | |

| Compound | EC₅₀ | IC₅₀ | Compound | EC₅₀ | IC₅₀ |
|---|---|---|---|---|---|
| 401 | D | D | 425 | D | D |
| 402 | D | D | 426 | D | D |
| 403 | D | D | 427 | D | D |
| 404 | D | D | 428 | C | D |
| 405 | D | D | 429 | D | D |
| 406 | D | D | 430 | D | D |
| 407 | D | D | 431 | D | D |
| 408 | D | D | 432 | D | D |
| 409 | D | D | 433 | D | D |
| 410 | C | D | 434 | D | D |
| 411 | D | D | 435 | D | D |
| 412 | D | D | 436 | D | D |
| 413 | D | D | 437 | D | D |
| 414 | C | D | 438 | C | D |
| 415 | D | D | 439 | D | D |
| 416 | C | D | 440 | D | D |
| 417 | D | D | 441 | D | D |
| 418 | D | D | 442 | D | D |
| 419 | C | D | 443 | D | D |
| 420 | D | D | 444 | D | D |
| 421 | D | D | 445 | D | D |
| 422 | C | D | 446 | D | D |
| 423 | D | D | 447 | D | D |
| 424 | D | D | | | |
| | | | | | |

| Compound | EC₅₀ | IC₅₀ | Compound | EC₅₀ | IC₅₀ |
|---|---|---|---|---|---|
| 501 | D | D | 528 | A | D |
| 503 | n.a. | n.a. | 529 | D | D |
| 504 | n.a. | n.a. | 530 | n.a. | D |
| 505 | n.a. | n.a. | 531 | n.a. | D |
| 507 | D | D | 532 | n.a. | D |
| 508 | C | D | 533 | D | D |
| 509 | C | D | 534 | C | D |
| 510 | D | D | 535 | n.a. | D |
| 511 | D | D | 536 | D | D |
| 512 | D | D | 537 | C | D |
| 513 | n.a. | D | 538 | D | D |
| 514 | C | D | 539 | D | D |
| 515 | D | D | 540 | n.a. | D |
| 516 | n.a. | D | 541 | D | D |
| 517 | C | D | 542 | C | D |
| 518 | C | D | 543 | D | D |
| 519 | C | D | 544 | D | D |
| 520 | D | D | 545 | D | D |
| 521 | D | D | 546 | D | D |
| 522 | C | D | 547 | D | D |
| 523 | C | D | 548 | D | D |
| 524 | C | D | 549 | D | D |
| 525 | C | D | 550 | D | D |
| 526 | C | D | 551 | D | D |
| 527 | D | D | | | |
| | | | | | |

| Compound | EC₅₀ | IC₅₀ | Compound | EC₅₀ | IC₅₀ |
|---|---|---|---|---|---|
| 601 | D | D | 629 | D | D |
| 602 | D | D | 630 | n.a. | D |
| 603 | D | D | 631 | n.a. | D |
| 604 | D | D | 632 | C | D |
| 605 | D | D | 633 | n.a. | D |
| 606 | D | D | 634 | n.a. | D |
| 607 | n.a. | D | 635 | n.a. | D |
| 608 | n.a. | D | 636 | n.a. | D |
| 609 | n.a. | D | 637 | C | D |
| 610 | n.a. | D | 638 | D | D |
| 611 | n.a. | D | 639 | C | D |
| 612 | n.a. | D | 640 | n.a. | D |
| 613 | D | D | 641 | n.a. | D |
| 614 | n.a. | D | 642 | n.a. | D |
| 615 | n.a. | D | 643 | n.a. | D |
| 616 | n.a. | D | 644 | n.a. | D |
| 617 | n.a. | D | 645 | D | D |
| 618 | n.a. | D | 646 | n.a. | D |
| 619 | n.a. | D | 647 | n.a. | D |
| 620 | n.a. | D | 648 | C | D |
| 621 | n.a. | D | 649 | D | D |
| 622 | n.a. | D | 650 | D | D |
| 623 | n.a. | D | 651 | D | D |
| 624 | n.a. | D | 652 | D | D |
| 625 | n.a. | D | 653 | D | D |
| 626 | n.a. | D | 654 | D | D |
| 627 | n.a. | D | 655 | D | D |
| 628 | n.a. | D | 656 | D | D |
| | | | | | |

| Compound | EC₅₀ | IC₅₀ | Compound | EC₅₀ | IC₅₀ |
|---|---|---|---|---|---|
| 701 | n.a. | D | 711 | B | D |
| 702 | B | D | 712 | B | D |
| 703 | B | D | 713 | B | D |
| 704 | B | D | 714 | B | D |
| 705 | n.a. | D | 715 | B | D |
| 706 | n.a. | D | 716 | n.a. | D |
| 707 | B | D | 717 | n.a. | D |
| 708 | B | D | 718 | D | D |
| 709 | B | D | 719 | D | D |
| 710 | B | D | 720 | D | D |
| | | | | | |

| Compound | EC₅₀ | IC₅₀ | Compound | EC₅₀ | IC₅₀ |
|---|---|---|---|---|---|
| 801 | B | D | 809 | B | D |
| 802 | B | D | 810 | B | D |
| 803 | B | D | 811 | B | D |
| 804 | n.a. | D | 812 | D | D |
| 805 | B | D | 813 | D | D |
| 806 | B | D | 814 | D | D |
| 807 | B | D | 815 | D | D |
| 808 | B | D | | | |
| 901 | D | D | 905 | B | D |
| 902 | D | D | 906 | n.a. | D |
| 903 | D | D | 907 | n.a. | C |
| 904 | D | D | | | |

| | | | | | |
|---|---|---|---|---|---|
| A indicates an EC₅₀ or IC₅₀ between 10 and 50 µM B indicates an EC₅₀ or IC₅₀ between 1 and 10 µM C indicates an EC₅₀ or IC₅₀ between 0.1 and 1 µM D indicates an EC₅₀ or IC₅₀ of less than 0.1 µM | | | | | |

### PREPARATION OF PRECIRSORS OF COMPOUND 100

### Example 38:

### Synthesis of Compound 2-A

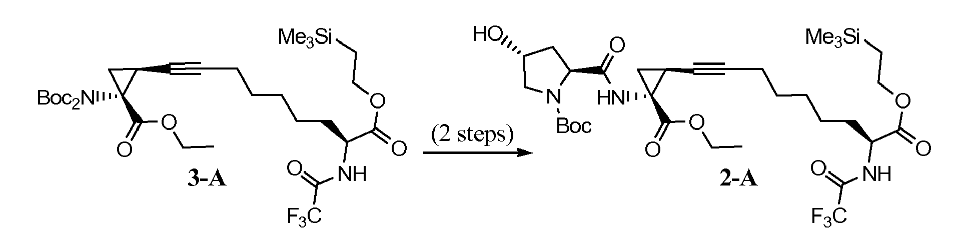

Compound **3-A** (0.510 g, 0.74 mmol) was treated with HCl in dioxane (4M, 6 mL) and the resulting mixture was then stirred for 3 h. The solvent was removed then DCM was added to the residue and co-evaporated, and then repeated. The residue was placed under high vacuum for 2 h to remove residual solvents. The resulting hydrochloride salt was dissolved in DMF (3 mL) and Boc-L-hydroxyproline (0.185 g, 0.80 mmol) was added to the stirring mixture. The mixture was cooled to 0°C and *O*-(7-Azabenzotriazol-1-yl)-*N,N,N',N'-*tetramethyluronium hexafluorophosphate (HATU, 0.304 g, 0.8 mmol) and diisopropylethylamine (DIEA, 140 µL) were added. After stirring 30 min at 0°C additional DIEA (280 µL) was added and the cooling bath was removed. The mixture was stirred at rt over night and then the solvent was evaporated to afford a residue. The residue was dissolved in ethyl acetate. The organic solution was subsequently washed with 1 N sulfuric acid, 1 N sodium bicarbonate solution and brine (2 x each), then dried over (Na₂SO₄). The solid was removed by filtration and the solvent removed under reduced pressure. Compound **2-A** was obtained as a yellow residue (0.336 g, 64% yield) after purification by flash chromatography (20 g silica; ethyl acetate).

### Example 39:

### Synthesis of Compound 1-D

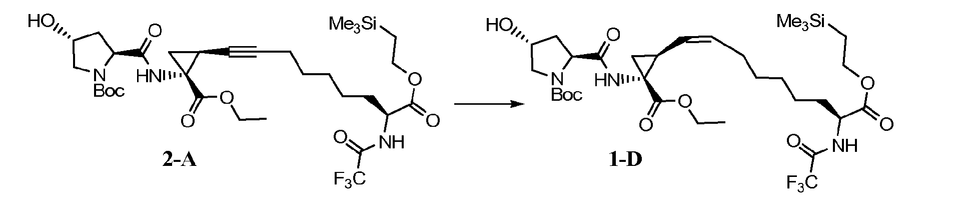

### Procedure:

Compound **2-A** (1.68 g, 2.38 mmol) was dissolved in ethyl acetate (1.2 L) with 5% Pd/BaSO₄ (1.68 g) and then quinoline (10 % in THF, 400 µL) was added under an inert atmosphere. The heterogeneous mixture was placed under hydrogen (1 bar) and stirred at rt for 6 hours. The mixture was then placed under an inert atmosphere and then the catalyst was removed by filtration, rinsing with ethyl acetate. The combined filtrates were washed with 1 N hydrochloric acid (2 x), water and brine. The organic layer was dried over Na₂SO₄, filtered and the solvent was removed under reduced pressure. Compound **1-D** was obtained as a yellow residue (1.7 g, quantitative yield). Note: the reaction concentration was found to be important, because compound **2-A** is strongly adsorbed on the 5% Pd/BaSO₄ catalyst. Careful reaction monitoring (LC-MS) is required to ensure complete conversion and to avoid over-hydrogenation. The quinoline is substantially removed by the aqueous work-up.

### Example 40:

### Synthesis of Compound 1-E

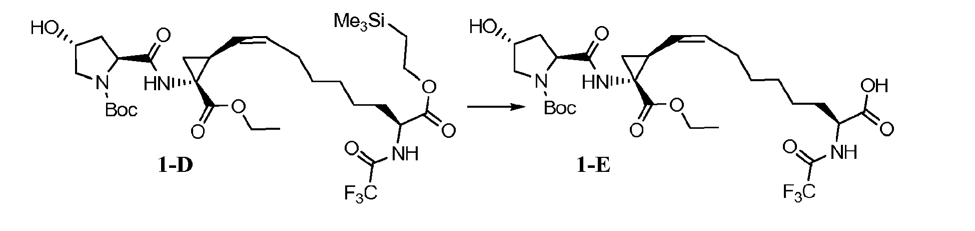

### Reaction:

| | **Material** | **Amount** | **mmol** | **Mw** | **equiv** |
|---|---|---|---|---|---|
| **A** | Compound **1-D** | 1.70 g | 2.38 | 707.87 | 1 |
| **B** | TBAF 1M in THF | 4.76 ml | 4.76 | | 2 |
| **C** | THF. | 50 ml | | | |

### Procedure:

Compound **1-D** (1.70 g, 2.38 mmol) was dissolved in THF (50 mL) and the resulting solution was cooled to 0 °C. To the cooled solution was added tetrabutylammonium fluoride (TBAF, 1 M in THF, 4.76 mL) at a rate of around 1 mL/min (TBAF was added within 5 min). The resulting mixture was slowly warmed to rt and stirred for 20 hours. After 20 h the mixture was diluted with ethyl acetate (200 mL). The organic solution was washed with water, 1 N hydrochloric acid (50 mL), water and brine. The organic layer was dried over Na₂SO₄, the solid was removed by filtration and the solvent was removed under vacuum to give the product **1-E** in quantitative yield as a yellow foam.

| | Amount | Yield |
|---|---|---|
| Theoretical | 1.44 g | 100% |
| Isolated | 1.44 g | **quant.** |

### Example 41:

### Synthesis of Compound 1-A

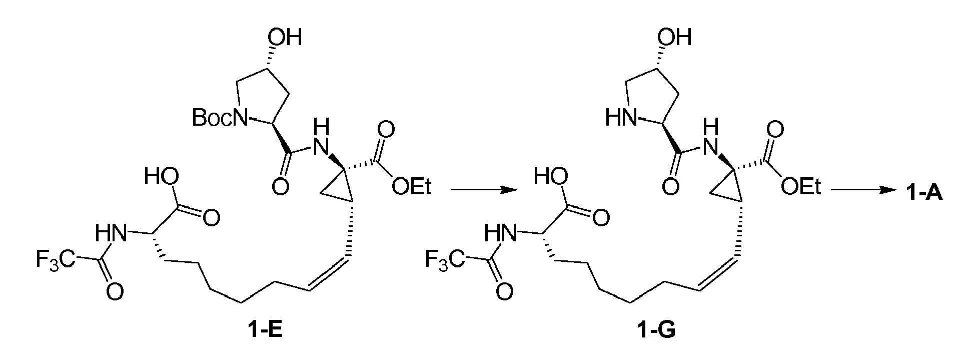

**Reaction:**

| | **Material** | **Amount** | **mmol** | **Mw** | **Equiv.** |
|---|---|---|---|---|---|
| **A** | Compound **1-E** | 180 mg | 0.296 | 607.63 | 1 |
| **B** | HCl in dioxane 4M | 3 ml | | | |
| **C** | HATU | 570 mg | 1.5 | 380.4 | 5 |
| **D** | DMF | 150 mL+ 150 mL | | | |
| **E** | DIEA (d=0.755) | 77 µL +20 µL | 0.3 + 3 | 129.25 | 1.5 + 10 |

### Procedure:

### Cleavage of Boc-group

Compound **1-E** was stirred with HCl in dioxane for 2 h at rt, then evaporated to dryness to afford the hydrochloride salt of compound **1-G,** the salt was further dried under vacuum for 20 min.

### Cyclization

The hydrochloride salt of compound **1-G** was dissolved in DMF (150 mL) together with DIEA (77 µL). This solution was added dropwise within 5 h to a stirred solution of HATU (570 mg) and DIEA (520 µL) in DMF (150 mL). The mixture was stirred for 60 h and then the solvent was evaporated to afford the crude product **1-A** as a residue. The residue was dissolved in ethyl acetate and washed with 1 N sulfuric acid, 1 N sodium bicarbonate solution and brine, then dried (Na₂SO₄) and the solvent was removed under reduced pressure to afford **1-A** (104 mg) as a pale yellow residue that still contained a byproduct (m/z = 208) after flash chromatography (15 g silica; ethyl acetate). From NMR a yield of 73 mg was calculated.

### Alternative Synthesis of Compound 1-A

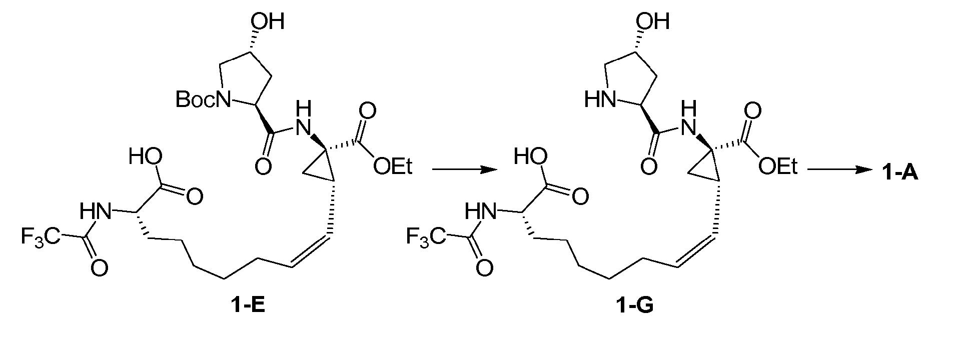

### Reaction:

| | **Material** | **Amount** | **mmol** | **Mw** | **Equiv.** |
|---|---|---|---|---|---|
| **A** | Compound **1-E** | 600 mg | 0.988 | 607 | 1 |
| **B** | HCl in dioxane 4M | 10 mL | | | |
| **C** | EDAC | 947 mg | 4.94 | 191.71 | 5 |
| **D** | DMF | 1000 mL | | | |
| **E** | DIEA (d=0.755) | 0.25 mL + 0.25 mL | 1.48 + 1.48 | 129.25 | 1.5 + 1.5 |

### Procedure:

### Cleavage of Boc-group

Compound **1-E** was stirred with HCl in dioxane for 2 h at rt, then evaporated to dryness to afford the hydrochloride salt of compound **1-G,** the salt was further dried under vacuum for 20 min.

### Cyclization

The hydrochloride salt of compound **1-G** was dissolved in DMF (500 mL) together with DIEA (250 µL). This solution was added dropwise within 5 h to a stirred solution of EDAC (0.947 g) and DIEA (250 µL) in DMF (500 mL). The mixture was stirred for 20 h, then evaporated. The residue was dissolved in ethyl acetate and washed with 1 N hydrochloric acid, 1 N sodium bicarbonate solution and brine, then dried (Na₂SO₄) and evaporated to give 273 mg of compound **1-A** as a brown foam. The product compound **1-A** (0.126 g, 26% yield) was obtained as a white foam after flash chromatography (50 g silica; ethyl acetate).

### Alternative Synthesis of Compound 1-A

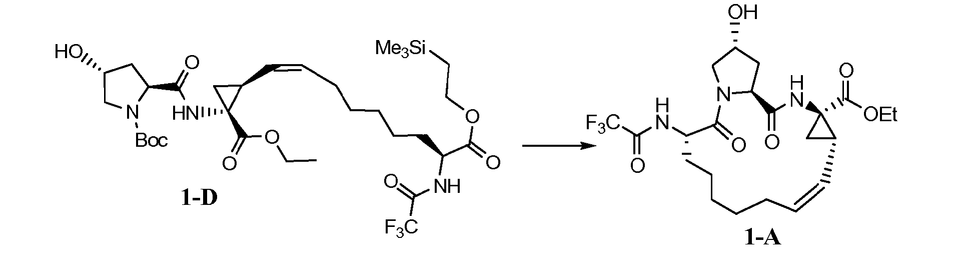

### Reaction:

| | **Material** | **Amount** | **µmol** | **Mw** | **equiv** |
|---|---|---|---|---|---|
| | **Cleavage of TMSE-ester** | | | | |
| **A** | Compound **1-D** | 294 mg | 416 | 707.87 | 1 |
| **B** | TBAF in THF 1M | 832 µL | 832 | | 2 |
| **C** | THF | 4 mL | | | |
| | **Formation of PFP-ester** | | | | |
| **D** | Pentafluorophenol | 383 mg | 2080 | 184.06 | 5 |
| **E** | EDAC | 96 mg | 500 | 191.71 | 1.2 |
| **F** | DMAP | 10 mg | 80 | 122.17 | 0.2 |
| **G** | DCM | 10 mL | | | |
| | **Cleavage of Boc-group** | | | | |
| **H** | HCl in dioxane 4M | 3 mL | | | |
| | **Cyclization** | | | | |
| **I** | CHCl₃ | 5 + 10 mL | | | |
| **J** | 1 N aq. NaHCO₃ | 5 mL | | | |

### Procedure:

### Cleavage of TMSE-ester

Compound **1-D** was dissolved in THF (4 mL) and cooled to 0 °C then TBAF (1 M in THF) was added within 1 min. The resulting mixture was stirred for 20 hours and warmed slowly to rt. Ethyl acetate (30 mL) was added. The mixture was subsequently washed with water, 0.1 N hydrochloric acid (5 mL), water and brine, then dried (Na₂SO₄) and evaporated to give the free acid of formula **1-E** which was used for the next step without further purification after 1 h additional drying under vacuum.

### Formation of PFP-ester

The compound of formula **1-E** was dissolved in DCM (10 mL) together with Pentafluorophenol and DMAP. The solution was cooled to -20 °C and then EDAC was added in a single portion. The mixture was slowly allowed to warm to rt over night. The solvent was removed under reduced pressure and the PFP-ester **1-F** was further dried under vacuum for 1h. The PFP-ester **1-F** product was used without further purification in the next step.

### Cleavage of Boc-group

To the PFP-ester **1-F** was added HCl in dioxane (3 mL). The mixture was stirred at rt for 3 h. The solvent was removed under reduced pressure, then co-evaporated with chloroform (2 x) and further dried under vacuum for 1 h. The resultant amine was directly used in the next step.

### Cyclization

The amine from the Boc cleavage step was dissolved in chloroform (50 mL) and added dropwise (1 h) into a vigorously stirred mixture of chloroform (100 mL) and 1 N sodium bicarbonate. The mixture was stirred for 4 additional hours. The organic and aqueous layers were separated and the aqueous layer was extracted with chloroform (2 x). The combined organic layers were evaporated. The residue was dissolved in ethyl acetate and washed with 1 N sulfuric acid, water, 1 N sodium bicarbonate solution and brine, then dried (Na₂SO₄) and evaporated. The macrocyclic product **1-A** (0.020 g, 10% yield) was obtained as a colorless resin after flash chromatography (15 g silica; ethyl acetate).

### Example 42:

### Synthesis of Compound 2-B

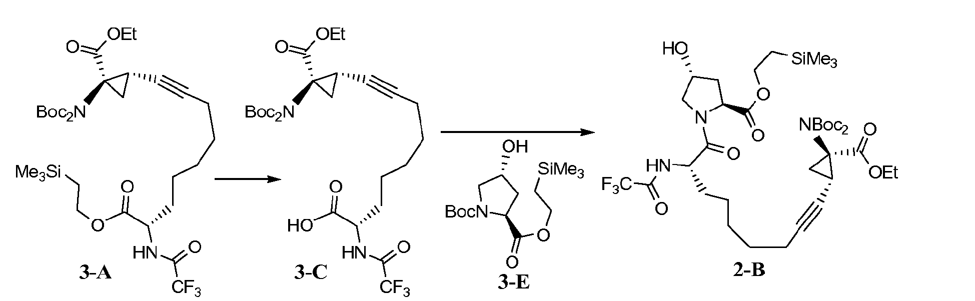

### Reaction:

| | **Material** | **Amount** | **mmol** | **Mw** | **equiv** |
|---|---|---|---|---|---|
| **A** | Compound **3-A** | 560 mg | 0.808 | 692.85 | 1 |
| **B** | HCl in dioxane 4M | 5 mL | 20 | | |
| **C** | Compound **3-E** | 295 mg | 0.890 | 331.49 | 1.1 |
| **D** | DIEA (d. 0.755) | 460 µL (160 + 300) | 2.7 | 129.25 | 3.3 |
| **E** | HATU | 308 mg | 0.810 | 380.4 | 1 |
| **F** | DMF | 5 mL | | | |
| **G** | DIEA (d. 0.755) | 300 µL | 1.8 | 129.25 | 3.3 |
| **H** | TBAF in THF (1M) | 1.62 mL | 1.62 | | 2 |
| **I** | THF | 10 mL | | | |

### Procedure:

Compound **3-A** was dissolved in THF (10 mL) and cooled to 0 °C then TBAF (1M in THF) was added within 2 min. The resulting mixture was stirred for 20 hours and warmed slowly to rt. The mixture was diluted with Ethyl acetate (100 mL) and subsequently washed with 1 N sulfuric acid, water and brine, and dried over Na₂SO₄. The organic solvent was removed under reduced pressure to give the corresponding free acid **3-C** in quantitative yield (480 mg) as a yellow resin.

In a separate flask, HCl in dioxane was added to **3-E.** The mixture was stirred for 3 h. The solvent was removed under reduced pressure then co-evaporated with DCM (2 x) and dried under vacuum for 1 h to afford the hydrochloride salt of **3-F.** To the resulting hydrochloride salt **3-F** were added the free acid **3-C,** prepared above, DIEA (160 µL) and DMF (5 mL). The solution was cooled to 0 °C and then HATU and DIEA (300 µL) were added. After 30 min additional DIEA (300 µL) was added and the mixture was stirred over night at rt. The solvent was then removed under reduced pressure to afford crude **2-B** which was subsequently dissolved in ethyl acetate. The ethyl acetate solution was washed with 1 N sulfuric acid, 1 N sodium bicarbonate solution and brine (2 x each), then dried over Na₂SO₄ and evaporated. The product **2-B** (350 mg, 54 % yield) was obtained as a yellow resin after flash chromatography (20 g silica; ethyl acetate).

### Example 43:

### Synthesis of Compound 1-H

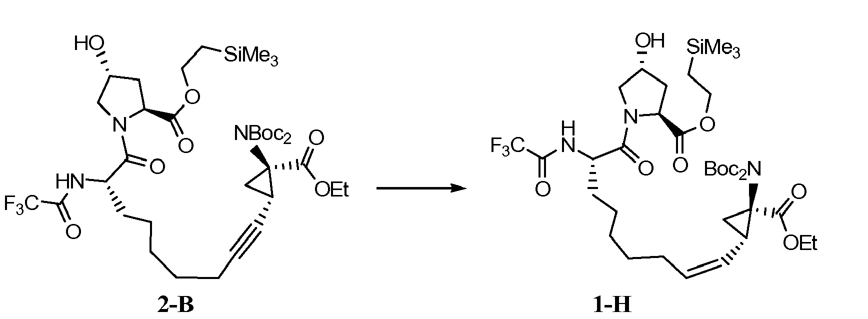

### Reaction:

| | **Material** | **Amount** | **µmol** | **Mw** | **equiv** |
|---|---|---|---|---|---|
| **A** | Compound **2-B** | 340 mg | 62 | 805.97 | 1 |
| **B** | Pd/BaSO₄ 5 % | 340 mg | | | 100 w% |
| **C** | quinoline 10 % in THF | 350 µL | | | |
| **D** | Ethyl acetate | 1.2L | | | |

### Procedure:

Compound **2-B** was hydrogenated using 5% Pd/BaSO₄ and quinoline (10 % in THF) in ethyl acetate at 1 bar hydrogen pressure and rt for 6 hours. The catalyst was removed by filtration and washed with ethyl acetate. The combined filtrates were washed with 1 N hydrochloric acid (2 x), water and brine, then dried over Na₂SO₄ and evaporated. The alkene **1-H** (0.345 g, quantitative yield) was obtained as a yellow resin.

Note: The dilution was found to be important, because the starting material is strongly adsorbed on the catalyst. Careful reaction monitoring (LC-MS) is required to ensure complete conversion and to avoid over-hydrogenation. The quinoline is widely removed by the aqueous work-up.

### Example 44:

### Synthesis of Compound 6-B

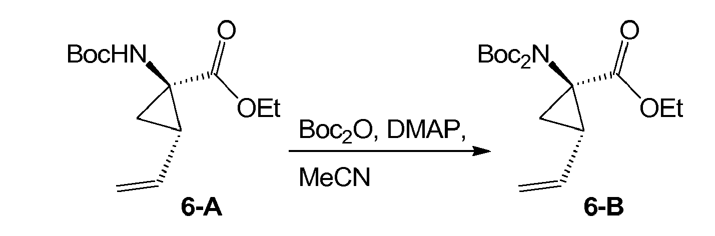

### Reaction:

| | **Material** | **Amount** | **mmol** | **Mw** | **equiv** |
|---|---|---|---|---|---|
| **A** | Compound **6-A** | 51 g | 200 | 255.32 | 1 |
| **B** | Boc₂O | 131 g | 600 | 218.25 | 3 |
| **C** | DMAP | 4.9 g | 40 | 122.17 | 0.2 |
| **D** | CH₃CN | 600 mL | | | |

### Procedure:

Compound **6-A** was dissolved in acetonitrile (500 mL) and then DMAP was added to the yellow solution. A solution of Boc₂O in acetonitrile (100 mL) was added slowly and carefully within 40 minutes. A steady evaporation of gases took place and the solution darkened to brown. The mixture was heated to 50 °C for 20 h. When the conversion was found to be complete (monitored by TLC), the volatiles were evaporated. The residue was dissolved in ethyl acetate. The organic solvent, containing the dissolved residue, was washed with water and brine, then dried over MgSO₄ and the solvent evaporated. The product **6-B** was obtained as a brown oil that still contained most of the added DMAP

Note: For a successful next reaction to compound **6-C** it is essential not to remove the DMAP. The crude product **6-B** can be used as isolated.

### Example 45:

### Synthesis of Compound 6-C

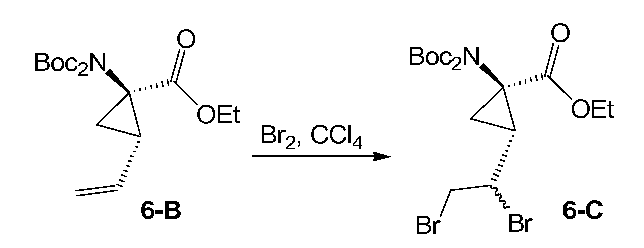

### Reaction:

| | **Material** | **Amount** | **mmol** | **Mw** | **equiv** |
|---|---|---|---|---|---|
| **A** | Compound **6-B** crude (∼85%) | 9.50g | 22.7 | 355.43 | 1 |
| **B** | Br₂ (dissolved in CCl₄; mol/L) | 1 22.7 mL | 22.7 | 159.81 | 1 |
| **C** | CCl₄ | 140 mL | | | |

### Procedure:

The crude compound **6-B** was dissolved in CCl₄ (140 mL) and cooled to 0°C. A solution of bromine in CCl₄ was added dropwise within 80 min. The conversion was not fully complete (monitored by ¹H-NMR). Additional 5 mL of Br₂-solution were added within 30 min and stirred for additional 20 minutes at 0°C. The solution was diluted with DCM (200 mL) and aqueous sodium thiosulfate (160 mL, 0.1 M) and the resulting mixture stirred for 20 min. The layers were separated. The organic layer was washed with water and brine, then dried over MgSO₄ and evaporated. The crude dibromide **6-B** was crystallized from ethanol/water (20 mL/dropwise addition of some mL) at 0°C. The beige crystals were washed with cold water, then dried under vacuum over night.

Note: The reaction was best monitored with proton NMR. The mother liquor still contained product, but the purification was not optimized further. The dibromide **6-B** (9.34 g, 80% yield) was obtained as a mixture of epimers.

### Example 46:

### Synthesis of Compound 6-D

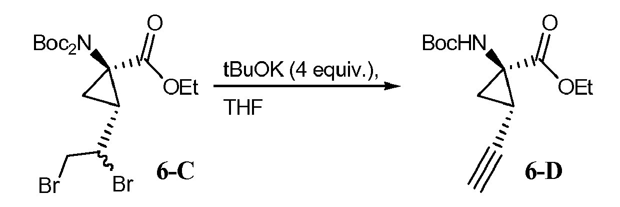

### Reaction:

| | **Material** | **Amount** | **mmol** | **Mw** | **equiv** |
|---|---|---|---|---|---|
| **A** | Compound **6-C** | 12.0 g | 23.5 | 515.24 | 1 |
| **B** | tBuOK in THF 1M | 94 mL | 94 | 112.22 | 4 |
| **C** | THF | 120 mL | | | |

### Procedure:

The dibromide **6-C** was dissolved in THF (120 mL). The slightly turbid solution was cooled to 0°C and then a solution of KOtBu (94 mL, 4 equiv. in THF) was added in one portion. The conversion was complete after 30 min as indicated by ¹H-NMR. The mixture was stirred for an additional 40 min (time for NMR measurement). Acetic acid (5.8 mL) was added and then the volatiles were evaporated (water bath at 35 °C). The residue was dissolved in TBME (600 mL) and subsequently washed with water (300 mL) and brine, dried over MgSO₄ and evaporated. The crude product **6-D** was dissolved in small amount of DCM and filtered over a pad of silica using heptane / ethyl acetate (2/1) as eluent. The product 6-D (5.47 g, 92 % yield) was obtained as a brown oil.

Note: It is also possible to add the starting material to the base. Four equivalents were found to be essential to achieve a defined conversion.

### Example 47:

### Synthesis of Compound 6-D

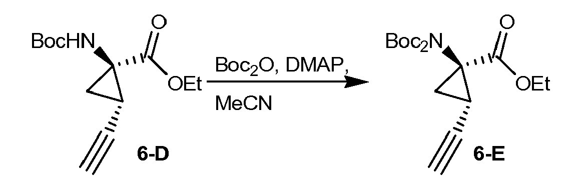

### Reaction:

| | **Material** | **Amount** | **mmol** | **Mw** | **equiv** |
|---|---|---|---|---|---|
| **A** | Compound **6-D** | 5.1 g | 20.1 | 253.3 | 1 |
| **B** | Boc₂O | 13.7 g | 60.3 | 218.25 | 3 |
| **C** | DMAP | 0.73 g | 6 | 122.17 | 0.3 |
| **D** | CH₃CN | 120 mL | | | |

### Procedure:

Compound **6-D** was dissolved in acetonitrile (120 mL) then DMAP (0.73 g, 6 mmol) was added to the yellow solution. Subsequently, Boc₂O (13.7 g, 60 mmol) was added in small portions carefully within 5 minutes. A steady evolution of gas took place and the solution darkened to brown. The mixture was heated to 50°C for 20 h. When the conversion was found to be complete (monitored by ¹H-NMR), the volatiles were evaporated and the residue dried under for 1 h. The crude product **6-E** was purified by flash chromatography (200 g silica) using heptane / ethyl acetate (3/1) as eluent. The product **6-E** (6.6 g, 93 % yield) was obtained as a yellow oil.

### Example 48:

### Synthesis of Compound 4-BB

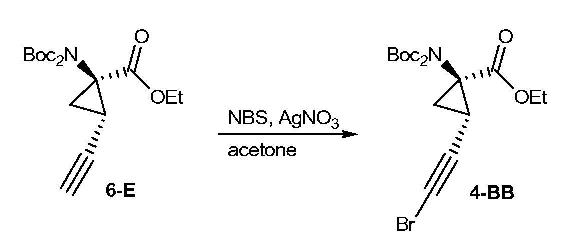

### Reaction:

| | **Material** | **Amount** | **mmol** | **Mw** | **equiv** |
|---|---|---|---|---|---|
| **A** | Compound **6-E** | 10.6 g | 30.0 | 353.42 | 1 |
| **B** | NBS | 5.34 g | 30.0 | 177.99 | 1 |
| **C** | AgNO₃ | 510 mg | 3 | 169.87 | 0.1 |
| **D** | acetone | 300 mL | | | |

### Procedure:

To a solution of compound **6-E** in acetone at 20°C were added AgNO₃ (0.510 g, 3 mmol) and NBS (5.34 g, 30 mmol). The mixture was stirred in the dark for 90 min. After complete conversion (monitored by ¹H-NMR) the solvent was evaporated (water bath temperature < 40°C). A mixture of diethylether/heptane (9/1, 50 mL) was added to the residue and kept at 4°C for 1 h. The solids were filtered off and washed with a few mL of diethylether/heptane (9/1). The filtrate was evaporated to half the volume and kept at 4°C for additional 2 h. Again, the precipitate was removed by filtration and washed with ether/heptane (1/1). The filtrate was evaporated (water bath temperature < 40°C) to a afford the bromide **4-BB** (13.1 g, quant.) as a yellow oil which was used directly in the coupling reaction.

Note: The bromide **4-BB** was stored only a couple of hours under argon in the fridge because it seemed to be rather labile to heat.

### Example 49:

### Synthesis of Compound 5-B

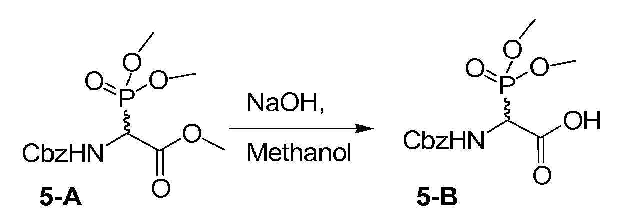

### Reaction:

| | **Material** | **Amount** | **mmol** | **Mw** | **equiv** |
|---|---|---|---|---|---|
| **A** | Cbz-Phosgly-OMe **(5-A)** | 66.2g | 200 | 331.26 | 1 |
| | **B** 1 N sodium hydroxide | 200 mL | 200 | | 1 |
| **C** | MeOH / water 9/1 | 400 mL | | | |

### Procedure:

Compound **5-A** was dissolved in MeOH/water (9/1, 400 mL) at rt then 1 N sodium hydroxide was added dropwise within 1 h and the mixture was stirred for additional 30 min. Upon complete conversion the methanol was removed under reduced pressure. The remaining aqueous solution was acidified with 1 N sulfuric acid to pH ∼ 1 and extracted with ethyl acetate (3 x). The combined organic layers were washed with brine, then dried over MgSO₄ and evaporated. The white solid carboxylic acid **5-B** (63.1 g, 99% yield) was dried under vacuum.

### Example 50:

### Synthesis of Compound 5-C

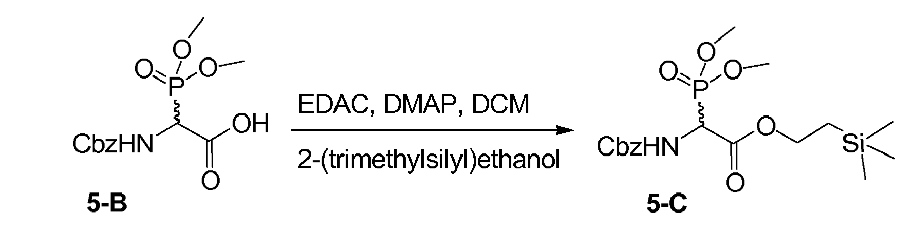

### Reaction:

| | **Material** | **Amount** | **mmol** | **Mw** | **equiv** |
|---|---|---|---|---|---|
| **A** | Cbz-Phosgly-OH (5-B) | 30.0 g | 94.6 | 317.24 | 1 |
| **B** | Trimethylsilyl-ethanol (d = 0.825) | 14.3 mL | 100 | 118.25 | 1.06 |
| **C** | DMAP | 1.22 g | 9.5 | 122.17 | 0.1 |
| **D** | EDAC | 19.2 | 100 | 191.70 | 1.06 |
| **E** | DCM | 200 mL | | | |

### Procedure:

A mixture of Cbz-Phosgly-OH **(5-B),** Trimethylsilyl-ethanol (14.3 mL, 100 mmol) and DMAP (1.22 g, 9.5 mmol) in DCM (200 mL) was cooled to -15°C then EDAC (19.2 g, 100 mmol) was added in one portion. The mixture was allowed to warm to rt within 5 h and showed complete conversion (HPLC, LC-MS). The volatiles were evaporated and the crude ester **5-C** was dissolved in ethyl acetate. The ethyl acetate solution was subsequently washed with 1 N sulfuric acid, 1 N sodium bicarbonate solution and brine (2 x each), then dried over Na₂SO₄ and evaporated to give the silyl ester **5-C** (37.4 g, 95 % yield) as a colorless oil.

### Example 51:

### Synthesis of Compound 5-D

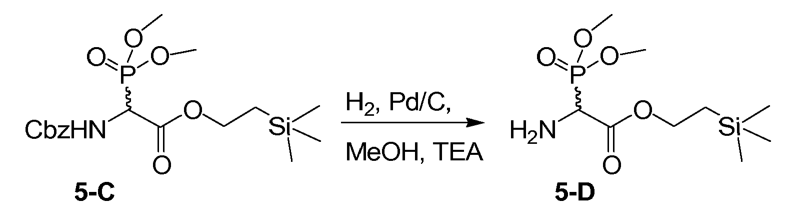

### Reaction:

| | **Material** | **Amount** | **mmol** | **Mw** | **equiv** |
|---|---|---|---|---|---|
| **A** | Cbz-Phosgly-OTMSE **(5-C)** | 27.5 g | 66 | 417.48 | 1 |
| **B** | MeOH | 300 mL | | | |
| **C** | Triethylamine | 0.7 g | 6.9 | 101.19 | 0.1 |
| **D** | 10% Pd/C | 0.7 g | | | ∼2.5% |
| **E** | H₂ | | | | |

### Procedure:

Cbz-Phosgly-OTMSE (5-C, 27.5 g) was dissolved in MeOH (300 mL) and hydrogenated for 30 min using 10% Pd/C as the catalyst at 1 bar H₂ in the presence of TEA at rt. The catalyst was removed by filtration with rinsing with MeOH. The filtrates were evaporated to dryness. The product 5-D (18.4 g, 99% yield) was obtained as a pale yellow oil and was stored at -18°C under argon.

### Example 52:

### Synthesis of Compound 5-E

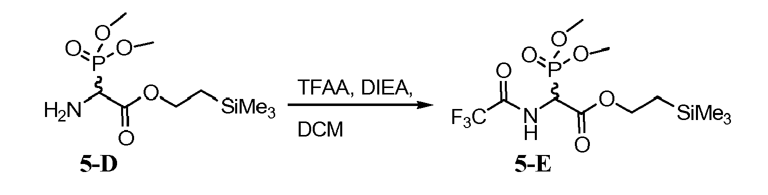

### Reaction:

| | **Material** | **Amount** | **mmol** | **Mw** | **equiv** |
|---|---|---|---|---|---|
| **A** | H-PhosGly-OTMSE**(5-D)** | 18.41 g | 65 | 283.34 | 1 |
| **B** | DIEA d = 0.755 | 12.3 mL | 71.5 | 129.25 | 1.1 |
| **C** | TFAA d=1.511 | 9.0mL | 65 | 210.03 | 1 |
| **D** | CH₂Cl₂ | 300 mL | | | |

### Procedure:

H-PhosGly-OTMSE **(5-D,** 18.41 g, 65 mmol) was dissolved in DCM (300 mL) and cooled to 0°C then DIEA (12.3 mL, 71.5 mmol) was added followed by dropwise addition of TFAA (9.0 mL, 65 mmol) within 20 min. The mixture was stirred for one hour at 0°C, then over night at rt. The solvent was evaporated and the residue was dissolved in ethyl acetate and washed with water (3 x). The combined aqueous layers were extracted with ethyl acetate. The combined organic layers were washed with brine, then dried over MgSO₄ and evaporated to afford the product **5-E** (22.0 g, 89 % yield) as a white crystalline solid.

### Example 53:

### Synthesis of 5-chloropentanal

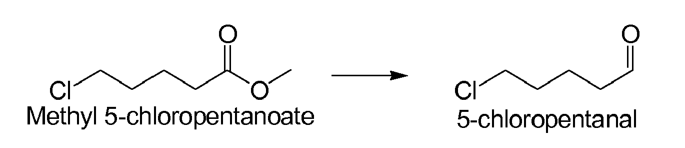

### Reaction:

| | **Material** | **Amount** | **mmol** | **Mw** | **equiv** |
|---|---|---|---|---|---|
| **A** | Methyl-5-Cl-pentanoate | 3.5 mL | 23.6 | 150.61 | 1 |
| **B** | DIBALH 1 mol/L in toluene | 30 mL | 30 | (142.22) | 1.27 |
| **C** | toluene | 130 mL | | | |

### Procedure:

A solution of Methyl 5-chloropentanoate in toluene (130 mL) was cooled to -78°C then DIBALH ( 30 mL, 30 mmol) was added dropwise within 1 h. After three hours of additional stirring at -78°C the reaction was quenched by dropwise addition of 6 N hydrochloric acid (50 mL). The mixture was allowed to warm to rt. The layers were separated and then organic layer was washed with water (2 x), dried (Na₂SO₄) and partially evaporated. The 5-chloropentanal was isolated as a clear colorless liquid (49% by NMR) in toluene. The product solution was stored under argon at 4°C and used in the next step without further purification.

Note: Lower yield on scale up (to 50 %), keep temperature below -60°C until quench. Product solutions of varying concentrations were obtained in different experiments.

### Example 54:

### Synthesis of Compound 5-F

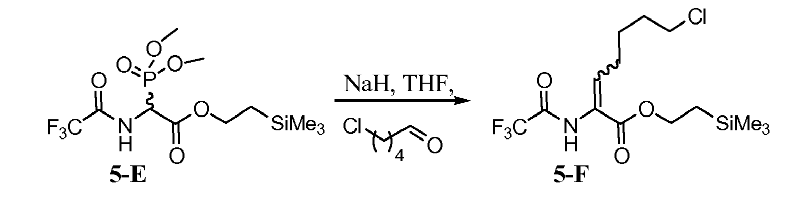

### Reaction:

| | **Material** | **Amount** | **mmol** | **Mw** | **equiv** |
|---|---|---|---|---|---|
| **A** | Compound **5-E** | 4.89 g | 13 | 379.35 | 1 |
| **B** | NaH (60 %) | (520 mg) | 13 | 24 | 1 |
| **C** | 5-Chloropentanal 16% in toluene | (9.80 g) | 13 | 120.58 | 1 |
| **D** | THF | 160 mL | | | |

### Procedure:

A solution of compound **5-E** in THF (50 mL) was cooled to 0°C. Sodium hydride (520 mg, 60%, 13 mmol) was added in small portions within 15 min. After stirring for 30 min, a solution of 5-Chloropentanal (16% in toluene) in THF (30 mL) was added dropwise in 20 min. The mixture was allowed to warm slowly to rt within 3 h. The volatiles were evaporated and the residue dissolved in ethyl acetate. The ethyl acetate solution was washed with water (2 x) and brine, then dried (MgSO₄) and evaporated to give α,β-unsaturated ester **5-F** as a yellow oil. This crude product contained (Z):(E) ∼ 5:1 (NMR) and was purified by flash chromatography (180 g silica) using heptane / ethyl acetate (4/1) as eluent to afford the purified α,β-unsaturated ester **5-F** (4.70 g, 97 % yield).

Note: It is not necessary to separate the double bond isomers for the next step.

### Example 55:

### Synthesis of Compound 5-G

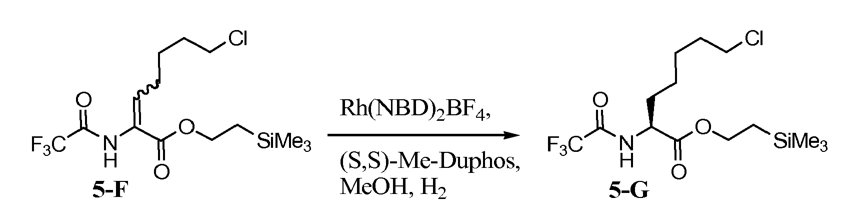

### Reaction:

| | **Material** | **Amount** | **mmol** | **Mw** | **equiv** |
|---|---|---|---|---|---|
| **A** | Compound **5-F** | 9.7 g | 26 | 373.88 | 1 |
| **B** | Rh(NBD)₂BF₄ | 48.3 mg | 0.13 | 374.00 | 0.005 |
| **C** | (S,S)-Me-duphos | 41.7 mg | 0.136 | 306.37 | 0.0053 |
| **D** | MeOH puriss p.a. | 75 mL | | | |

### Procedure:

The α,β-unsaturated ester **5-F** was hydrogenated in MeOH (1 bar of H₂) in the presence of Rh(NBD)₂BF₄ and (S,S)-Me-Duphos at 25°C for 21 h, after which the conversion was fully complete. GC analysis showed an enantiomeric excess of > 99 % for the desired (*S*)-enantiomer. The mixture was filtered through a pad of HyFlo. The solvent was evaporated and the residue filtered over a pad of silica using ethyl acetate/heptane (1/1) to afford **5-G** (4.70 g, 97 % yield, > 99 % ee).

### Example 56:

### Synthesis of Compound 5-H

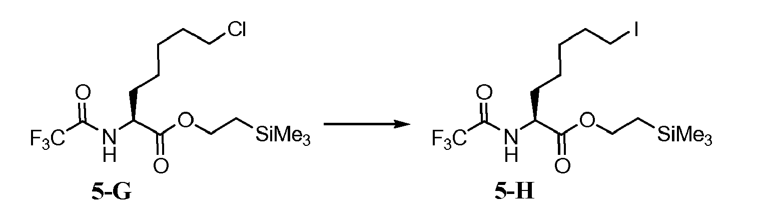

### Reaction:

| | **Material** | **Amount** | **mmol** | **Mw** | **equiv** |
|---|---|---|---|---|---|
| **A** | Compound **5-G** | 3.48 g | 9.1 | 375.89 | 1 |
| **B** | NaI | 4.1 g | 27.3 | 149.90 | 3 |
| **C** | Acetone | 20 mL | | | |

### Procedure:

Compound **5-G** and NaI (4.1 g, 27.3 mmol) were refluxed in acetone (20 mL) for 20 h (complete by ¹H NMR). The solvent was evaporated and then DCM (100 mL) was added to the residue. The solids were removed by filtration and washed with DCM. The combined filtrates were evaporated to afford the iodo compound **5-H** (4.02 g, 95 % yield) as pale brown oil.

### Example 57:

### Synthesis of Compound 3-A

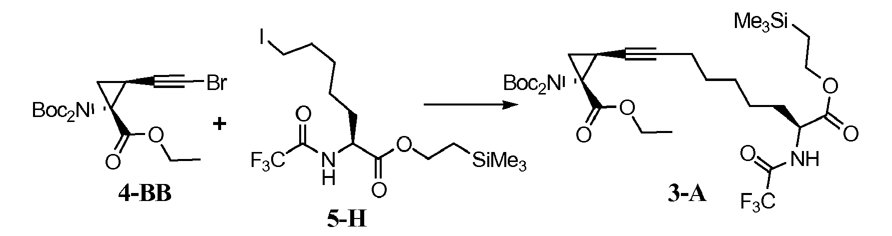

### Reaction:

| | **Material** | **Amount** | **mmol** | **Mw** | **equiv** |
|---|---|---|---|---|---|
| **A** | Compound **4-BB** | 13.1 g | 30.0 | 432.32 | 1 |
| **B** | Compound **5-H** | 14.0 g | 30.0 | 467.35 | 1 |
| **C** | Zn (dust) | 7.40 g | 113 | 65.39 | 3.75 |
| **D** | THF | | | | |
| **E** | 1,2-dibromo-ethane (d=2.18) | 430 µL | 5 | 187.89 | |
| **F** | TMS-Cl (d=0.859) | 220 µL | 1.72 | 108.64 | |
| **G** | CuCN | 2.68 g | 30.0 | 89.56 | |
| **H** | LiCl | 2.54 g | 60.0 | 42.38 | |

### Procedure:

Zinc dust was weighed in a 250 mL three-neck flask and dried repeatedly with heating (heat gun) under vacuum and argon alternately. After cooling to rt, THF (50 mL) and di-bromo-ethane were added and the mixture was heated to 80°C for 40 min. The mixture was cooled to rt and then TMS-Cl (trimethylsilyl chloride) was added. After vigorous stirring for 30 min, a solution of the iodide **5-H** (14.0 g, 30.0 mmol) in THF was added within 2 min. The resulting mixture was stirred for approx. 2 to 3 hours until the conversion to the Zn-organyl showed to be complete (monitored by ¹H NMR, MeOH quench of sample).

Note: If the conversion is not complete ultrasonication can be beneficial. Heating has to be avoided because Wurtz-coupling occurs

The solution of the Zn-organyl was used directly for the next step after careful decanting from the excess Zn or via transfer through a double-tip needle.

In parallel to the preparation described above, CuCN and LiCl are weighed in a 500 mL three-neck flask and dried under vacuum at 150°C for 2 h. After cooling to rt THF (75 mL) was added. The CuLi-complex dissolved after 10 min. The resulting CuLi-complex containing solution was cooled to -25°C. The solution of the Zn-organyl was added dropwise within 5 min. After stirring for 15 min at -20°C the mixture was cooled to -78°C.

A solution of freshly prepared bromo-acetylene **4-BB** (13.1 g, 30.0 mmol) in THF (30 mL) was added dropwise within 30 min. The mixture was allowed to warm to room temperature slowly over night.

The resulting clear brown mixture was diluted with ethyl acetate (1 L) and washed with water (2 x). Separation of the layers was difficult because of the formation of large amounts of inorganic solids. The combined aqueous phases were back extracted with ethyl acetate (3 x) The combined organic layers were washed with water and brine, then dried (MgSO₄) and evaporated to give **3-A** (crude, 25 g) as a brown oil. The latter was purified by filtration on 50 g of silica (heptane / ethyl acetate 1/1). All product containing fractions were collected and purified by flash chromatography (600 g of silica; heptane:ethyl acetate; 9/1 to 4/1). The product **3-A** (5.50 g, 26 % yield) was obtained as a yellow oil.

### Example 58:

### Synthesis of Compound 3-E

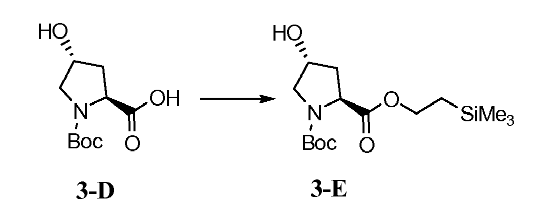

### Reaction:

| | **Material** | **Amount** | **mmol** | **Mw** | **equiv** |
|---|---|---|---|---|---|
| **A** | Boc-4-trans-hydroxy-(L)-proline **(3-D)** | 4.62 g | 20 | 231.25 | 1 |
| **B** | Trimethylsilyl-ethanol (d = 0.825) | 3.6 mL | 25 | 118.25 | 1.25 |
| **C** | DMAP | 305 mg | 2.5 | 122.17 | 0.125 |
| **D** | EDAC | 3.83 g | 20 | 191.70 | 1 |
| **E** | DCM | 20 mL | | | |
| **F** | CuCl | 20 mg | 0.2 | 99.00 | 0.1 |

### Procedure:

A mixture of Boc-4-trans-hydroxy-(L)-proline **(3-D),** Trimethylsilyl-ethanol (3.6 mL, 25 mmol), DMAP (0.305 g, 2.5 mmol) and CuCl (0.020 g, 0.2 mmol) in DCM (20 mL) was cooled to -15°C and stirred for 30 min. then EDAC (3.83 g, 20 mmol) was added in one portion. The mixture was allowed to warm to rt within 5 h and stirred further over night. The solvent was evaporated and the residue dissolved in ethyl acetate. The ethyl acetate solution was subsequently washed with 1 N sulfuric acid, 1 N sodium bicarbonate solution and brine (2 x each), then dried (Na₂SO₄) and evaporated. The product **3-E** (5.41 g, 86% yield) was obtained as a colorless oil after flash chromatography (200 g silica; heptane:ethyl acetate; 2/1).

### PHARMACOHINETIC STUDIES

### Example 59:

Interim, blinded data were used for Pharmacokinetic (PK) study relating to food effects. With the exception of the 400 mg cohort, eight subjects were studied at each dose (single doses of 100, 200, or 800 mg of the sodium salt of the compound 100, which may be referred to herein as TTMN-191, administered in the fasting state). In the 400 mg cohort, ten subjects received single 400 mg doses of ITMN-191 under both fasted and fed conditions with a 5-day washout period between dosing. In the 100 mg cohort, subjects were sampled within 1 hour before dosing and at 0.5, 1, 1.5, 2, 2.5, 3, 4, 6, 8, 12, 18, 24, 32 and 48 hours after dosing. For subsequent cohorts, subjects were sampled within 1 hour before dosing and at 0.25, 0.5, 1, 1.5, 2, 2.5, 3, 4, 6, 8, 12, 18, 24, and 32 hours after dosing. Nominal (not actual) sampling times were used for the analysis. PK samples were analyzed using liquid chromatography-tandem mass spectrometry (LC/MS/MS with API 4000 mass spectrometer) following solid-phase extraction. The upper and lower limits of quantitation (ULOQ and LLOQ) are 100 and 0.01 ng/mL, respectively.

Pharmacokinetic parameter estimates were calculated using non-compartmental methods (implemented using Microsoft Excel^{®}). Half-lives were determined by visual inspection of the log concentration-time plots to choose the terminal phase (minimum of three detectable observations). Using linear regression of the chosen terminal phase observations, elimination rate constants were calculated as the negative slope of the regression line and half-life as the natural log of 2 divided by the elimination rate constant.

The compartmental model was constructed to fit the PK data from the 400 mg, fed cohort only. Candidate PK models were fit to plasma data using weighted, non-linear regression implemented in Adapt 5. (1, 2) Each subjects' data were fit separately for a total of 10 fitted profiles. Model discrimination was accomplished according to the "Rule of Parsimony" based on Akaike's Information Criterion. (3, 4)

Fitted parameters were used to compute predicted steady-state AUC₀₋₂₄ and integrated average steady-state concentrations (Cavg) in plasma and in liver. Three hypothetical liver to plasma ratios (LPR) were considered given the variability seen in various animal species: 8 to 1, 30 to 1, and 100 to 1. The LPR seen in rats was approximately 8:1, LPR in dogs was approximately 25-40:1 and LPR in monkeys was approximately 80-125:1. As was seen in the animal studies to date, it was assumed that plasma and liver concentration-time profiles are approximately parallel.

Using ADAPT 5, simulated plasma profiles were generated using individual subject fitted parameters and two different dosing regimens, 800 mg Q12H and 500 mg Q8H, dosed to steady-state. In addition to simulated concentrations, percent of the dosing interval in which predicted plasma and liver concentrations remained above the EC90 (14.1 nM or 10.6 ng/mL) and the ratio of predicted trough concentrations in the liver to the EC90 were also calculated.

Summary statistics for the non-compartmental PK parameter estimates, stratified by cohort are provided in Table 9 below. Based on AUC_{0-inf}, the pharmacokinetics of ITMN-191 appear linear over the dose range of 100-800 mg. The influence of the administration of doses under fed conditions appears to be threefold: 1) a delay in the time to maximal drug concentrations (Tmax); 2) a decrease in the maximal drug concentration (Cmax), and 3) an increase in the AUC_{0-inf}. The median percentage change in AUC_{0-inf} between the fasted and fed conditions was 26% and 8 of 10 subjects had an increase in their AUC_{0-inf} under fed conditions. This difference is enough to deem the fasted and fed states not equivalent with absorption being significantly improved under fed conditions.

**Table 9: Median (25^{th}-75^{th} percentile) for selected ITMN-191 PK parameters, stratified by dosing cohort**

| Dose | N | AUC_{0-inf} (ng•hr/mL) | Cmax (ng/mL) | Tmax (hr) | T_{1/2} (hr) |
|---|---|---|---|---|---|
| 100 mg | 8 | 20.4 (17.3-26.6) | 14.7 (13.2-34.9) | 0.75 (0.5-1.25) | 1.78 (1.61-2.24) |
| 200 mg | 8 | 36.8 (28.4-45.1) | 30.2 (25.0-52.5) | 0.75 (0.5-1.13) | 1.49 (1.42-1.63) |
| 400 mg (Fasted) | 10 | 81.0 (66.7-95.8) | 63.8 (45.6-95.1) | 1 (0.5-1.88) | 1.53 (1.34-1.65) |
| 400 mg (Fed) | 10 | 113 (77.9-170) | 56.6 (31.0-98.4) | 2.5 (1.25-3.75) | 1.74 (1.40-2.08) |
| 800 mg | 8 | 194 (140-289) | 216 (126-414) | 0.5 (0.5-0.5) | 1.91 (1.77-1.99) |

The most robust fit to the data was obtained using a three-compartment model (one absorptive, two distributive) with first order absorption and elimination. Examination of the raw concentration versus time plots indicated that a simple, one-phase, first-order absorption model might not be completely adequate to fit the data, thus up to three absorption phases were allowed for each subject. No *a priori* assumptions regarding the number of phases were made; the fit of the observed data and the AIC were used to guide the selection of a one, two, or three-phase absorption.

Overall, excellent fits of the data were obtained. The r² values for fitted versus observed data for the individual profiles ranged from 0.966 to 1.00. Summary statistics for selected compartmental PK parameters are provided in Table 10 below. Two subjects required only one absorption phase, five required two absorption phases and three required three absorption phases for an adequate fit.

**Table 10: Summary statistics for compartmental PK parameters for the 400 mg (fed) cohort (n=10)**

| | CLt/F | Vc/F | Vss/F | T_{1/2,λz} |
|---|---|---|---|---|
| | (L/hr) | (L) | (L) | (hr) |
| Median | 3800 | 1975 | 2948 | 1.53 |
| 25^{th} percentile | 2301 | 1235 | 2464 | 0.971 |
| 75^{th} percentile | 5306 | 4429 | 4936 | 2.21 |

PK simulations predict little accumulation at steady-state. Summary statistics for steady-state AUC₀₋₂₄ are provided in Table 11, stratified by total daily dose.

**Table 11: Median (25^{th} - 75^{th} percentile) predicted AUC₀₋₂₄ for various multiple dose regimens (n=10)**

| Regimen | Steady-State AUC₀₋₂₄ |
|---|---|
| | (ng-hr/mL) |
| 100 mg Q12H | 53.5 (37.7 - 86.9) |
| 200 mg Q12H | 107 (75.4 - 174) |
| 400 mg Q12H | 214 (151 - 174) |
| 500 mg Q8H | 402 (283 - 652) |
| 800 mg Q12H | 428 (302 - 695) |

By simulating plasma concentration profiles at steady-state, it is possible to predict ITMN-191 concentrations in liver and then index these to viral sensitivity. Summary statistics for the percentage of a dosing interval for which predicted liver concentrations remain above the EC90 (%Time>EC90) and the ratio of predicted trough concentration in the liver to the EC90 (Ctrough,liver/EC90) are provided in Table 12.

**Table 12: Median (25^{th} - 75^{th} percentile) predicted TTMN-191 pharmacodynamic index values (n=10)**

| Regimen | LPR | %Time>EC90 | Ctrough,liver/EC90 |
|---|---|---|---|
| | 8:1 | 63.8 (52.7 - 79.1) | 0.334 (0.0201 - 0.481) |
| 800 mg Q12H | 30:1 | 94.8 (68.1 - 100) | 1.25 (0.0755 - 1.80) |
| | 100:1 | 100 (82.2 - 100) | 4.18 (0.251 - 6.01) |
| | 8:1 | 82.4 (70.9 - 100) | 0.861 (0.220 - 2.70) |
| 500 mg Q8H | 30:1 | 100 (94.0 - 100) | 3.23 (0.824 - 10.1) |
| | 100:1 | 100 (100 - 100) | 10.8 (2.75 - 33.8) |

The predicted Cavg were also examined. Even at the lower regimens considered, these were high, compared to the EC90. For example, the 25^{th}, 50^{th} and 75^{th} percentiles for Cavg/EC90 predicted for a regimen of 200 mg Q12H, were 2.3, 3.3 and 5.3 (for an LPR of 8:1), 9, 12 and 20 (for an LPR of 30:1) and were 29, 41 and 66 (for an LPR of 100:1).

Since the terminal half-life is short (1-2 hours in most subjects), the accumulation associated with multiple doses, at either an 8 or 12 hour dose interval, is predicted to be negligible. Thus, estimates of exposure (AUC_{0-inf} and Cmax) after administration of single doses are close approximations of the exposure seen after administration of multiple doses.

### Example 60

Ten subjects received single 1600 mg doses of ITMN-191 under both fasted and fed conditions with a 5-day washout period between dosing. For each condition, subjects were sampled within 1 hour before dosing and at 0.25, 0.5, 1, 1.5, 2, 2.5, 3, 4, 6, 8, 12, 18, 24, and 32 hours after dosing. Pharmacokinetic parameter estimates were calculated using non-compartmental methods. Half-life (t1/2) was determined by visual inspection of the log concentration-time plots to choose the terminal phase (minimum of three detectable observations). Using linear regression of the chosen terminal phase observations, elimination rate constants were calculated as the negative slope and half-life as the natural log of 2 divided by the elimination rate constant.

Results for the 1600 mg cohort are shown in Table 13 below. Boxplots of AUC_{0-inf}, with individual estimates overlaid, are provided in Figure 1. The influence of the administration of doses under fed conditions appears to be threefold: 1) a delay in the time to maximal drug concentrations (Tmax); 2) a decrease in the maximal drug concentration (Cmax), and 3) an increase in the area under the concentration-time curve (AUC_{0-inf}). The median percentage change in AUC_{0-inf} between the fasted and fed conditions was 54% and 10 of 10 subjects had an increase in their AUC_{0-inf} under fed conditions. This difference is enough to deem the fasted and fed states not equivalent with absorption being improved under fed conditions.

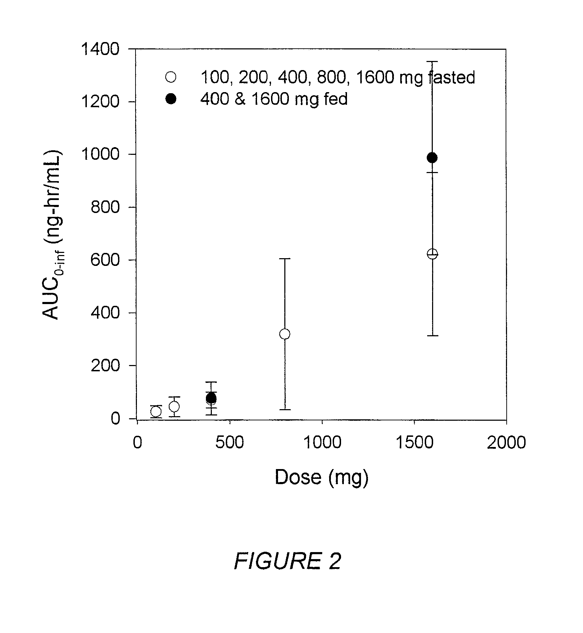

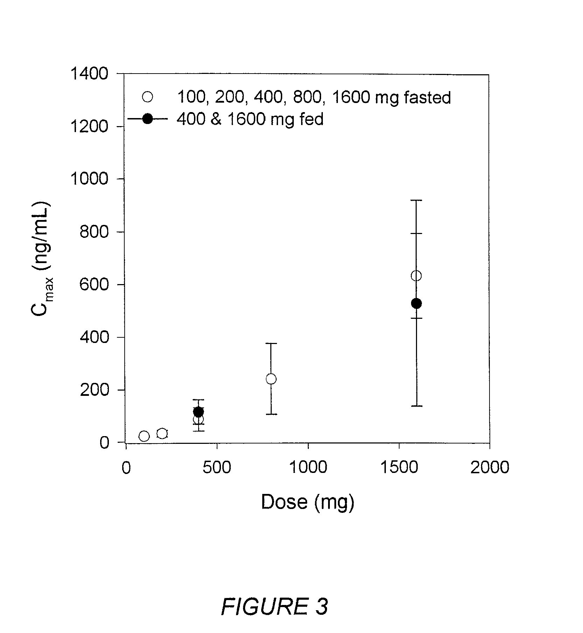

Additionally, the linearity in pharmacokinetics that had been seen over a dose range of 100-800 mg (see Example 59) is no longer present. When comparing the median AUC_{0-inf} and Cmax values for the 800 mg cohort (194 ng·hr/mL and 216 ng/mL, respectively) and the 1600 mg fasted cohort (737 and 685, respectively), one sees a 3-4 fold increase, not the 2-fold increase that would ordinarily be expected. Figures 2 and 3 display the mean AUC_{0-inf} and Cmax values at various dose levels under both fed and fasted conditions, and the similar trend was observed.

The fact that this is seen with both AUC_{0-inf} and Cmax suggests that the non-linearity is due to a bioavailability process (for example, saturable first-pass metabolism) as opposed to a clearance mechanism.

**Table 13: Non-Compartmental Pharmacokinetic Parameter Estimates for the 1600 mg Cohort**

| Fasted | | | | | Fed | | | |
|---|---|---|---|---|---|---|---|---|
| Subject | Tmax (hr) | Cmax (ng/m) | AUC_{0-inf} (ng•hr/m) | t1/2 (hr) | Tmax (hr) | Cmax (ng/m) | AUC_{0-inf} (ng•hr/m) | t1/2 (hr) |
| BB | 0.5 | 909 | 652 | 1.69 | 0.5 | 346 | 806 | 1.82 |
| CQ | 0.5 | 822 | 720 | 3.57 | 0.5 | 760 | 1133 | 2.70 |
| EJ | 2.5 | 442 | 779 | 4.02 | 1.5 | 493 | 1174 | 3.48 |
| HZ | 0.5 | 892 | 890 | 3.10 | 1.5 | 1510 | 1895 | 3.00 |
| IK | 0.5 | 225 | 360 | 2.30 | 2.5 | 381 | 583 | 2.01 |
| RF | 0.5 | 548 | 529 | 1.89 | 4 | 199 | 939 | 2.65 |
| SC | 1 | 214 | 507 | 1.89 | 4 | 100 | 583 | 1.80 |
| SU | 0.5 | 1000 | 907 | 2.16 | 2 | 444 | 1116 | 3.35 |
| ZL | 0.5 | 860 | 809 | 2.27 | 1 | 592 | 1225 | 2.16 |
| ZN | 0.5 | 305 | 753 | 1.91 | 4 | 454 | 1209 | 2.63 |
| | | | | | | | | |
| Mean | | 622 | 691 | 2.48 | | 528 | 1066 | 2.56 |
| SD | | 309 | 178 | 0.802 | | 392 | 380 | 0.605 |
| | | | | | | | | |
| Median | 0.5 | 685 | 737 | 2.21 | 1.75 | 449 | 1125 | 2.64 |
| Minimum | 0.5 | 214 | 360 | 1.69 | 0.5 | 100 | 583 | 1.80 |
| Maximum | 2.5 | 1000 | 907 | 4.02 | 4 | 1510 | 1895 | 3.48 |
| Geometric Mean | | 538.2 | 667 | 2.38 | | 422 | 1007 | 2.50 |
| Harmonic Mean | | 452.8 | 639 | 2.29 | | 326 | 949 | 2.43 |

### Conclusion

Potent small molecule inhibitors of the HCV NS3 protease have been developed.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. A compound having the structure of Formula I: 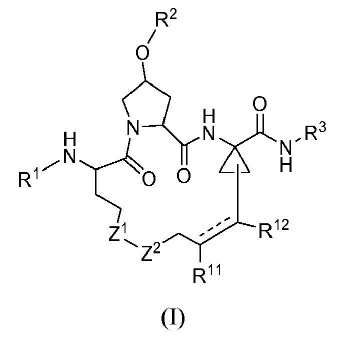 or a pharmaceutically acceptable salt or prodrug thereof wherein:
R¹ is selected from the group consisting of substituted aryl, substituted heteroaryl, - C(O)OR⁴, -C(O)NR⁵R⁶, -C(O)R⁷, and 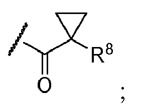
R² is 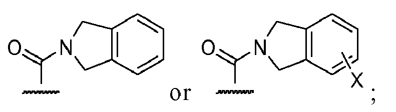
R³ is selected from the group consisting of -OR⁹ and -SO₂R¹⁰;
R⁴ is selected from the group consisting of alkyl, heterocyclyl, and aryl;
R⁵ is alkyl and R⁶ is selected from the group consisting of alkyl and aralkyl, or R⁵ together with R⁶ form an optionally substituted heterocyclyl or optionally substituted heteroaryl;
R⁷ is phenyl substituted one or more times with halogen;
R⁸ is selected from the group consisting of -CF₃ and methyl;
R⁹ is selected from the group consisting of hydrogen, optionally substituted alkyl, optionally substituted aryl, and optionally substituted aralkyl;
R¹⁰ is selected from the group consisting of alkyl optionally substituted with alkoxy or alkenyl, optionally substituted aryl, optionally substituted aralkyl, substituted heteroaryl, and 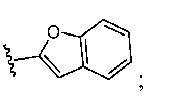
R¹¹ and R¹² are each hydrogen or together with the carbon atoms to which they are attached form an optionally substituted cycloalkyl;
X is halogen and is present 1 to 4 times; and
Z¹ and Z² are independently selected from the group consisting of -CH₂-, -CF₂-, and -O- provided that at least one of Z¹ and Z² is -CH₂-;
provided that if R¹¹ and R¹² are each hydrogen and R² is 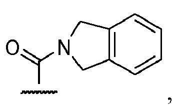 then R³ is -SO₂-phenyl disubstituted with halogen or -SO₂-thiophene disubstituted with halogen, or R¹ is 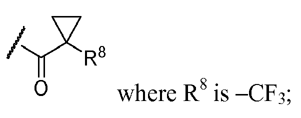
provided that if R¹¹ and R¹² are each hydrogen, R² is 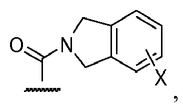 and R³ is - SO₂-cyclopropyl, then R¹ is not -C(O)O-t-butyl, -C(O)O-haloalkyl, or -C(O)O-cyclopentyl;
provided that if R¹¹ and R¹² are each hydrogen, R² is 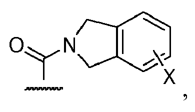 and R³ is substituted-SO₂-heteroaryl, then R¹ is not -C(O)O-cyclopentyl;
provided that if R¹¹ and R¹² are each hydrogen, R² is 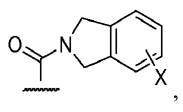 and R³ is - SO₂-alkyl substituted with alkoxy or alkenyl, then R¹ is -C(O)O-t-butyl and X is F; and
provided that the compound of formula (I) is not selected from the group consisting of:

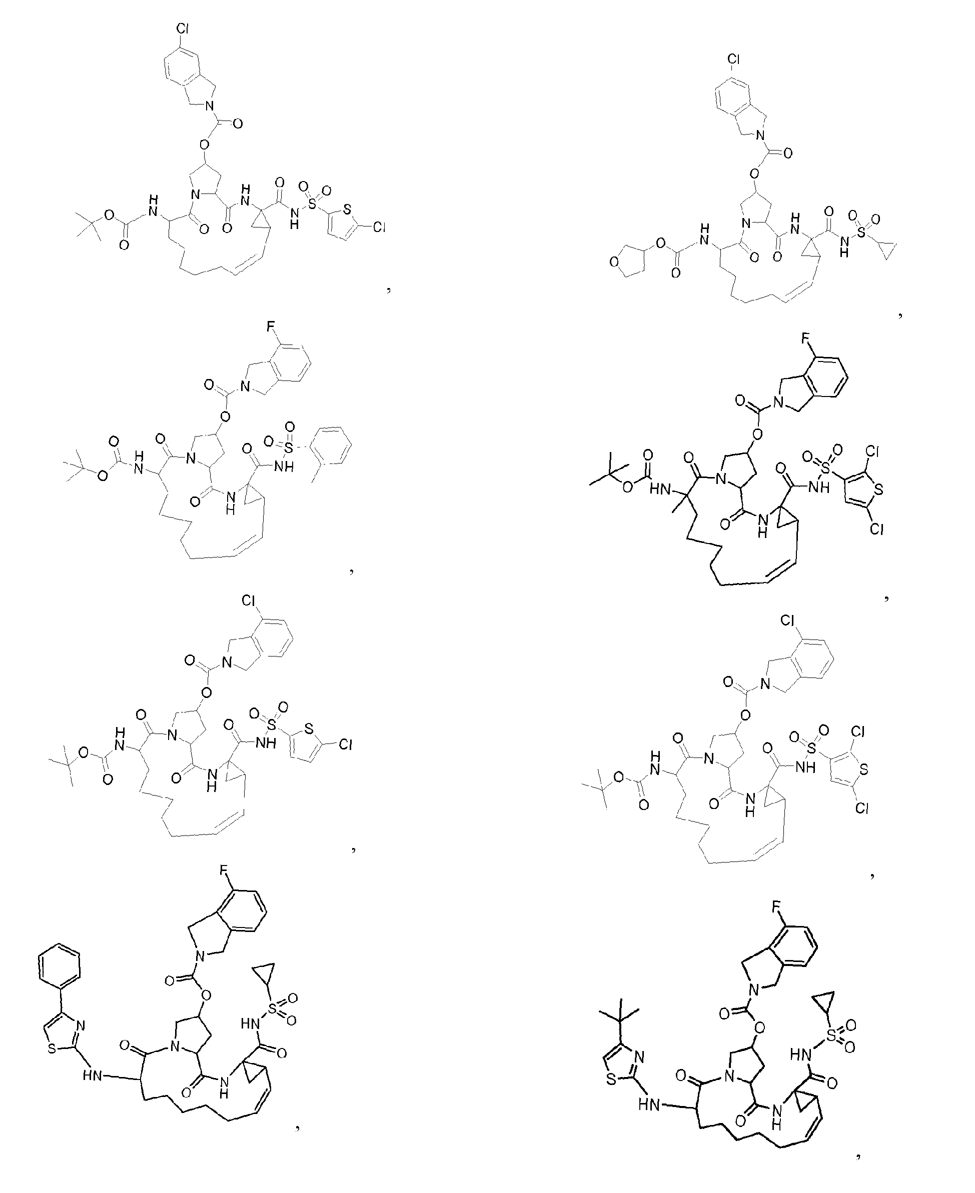

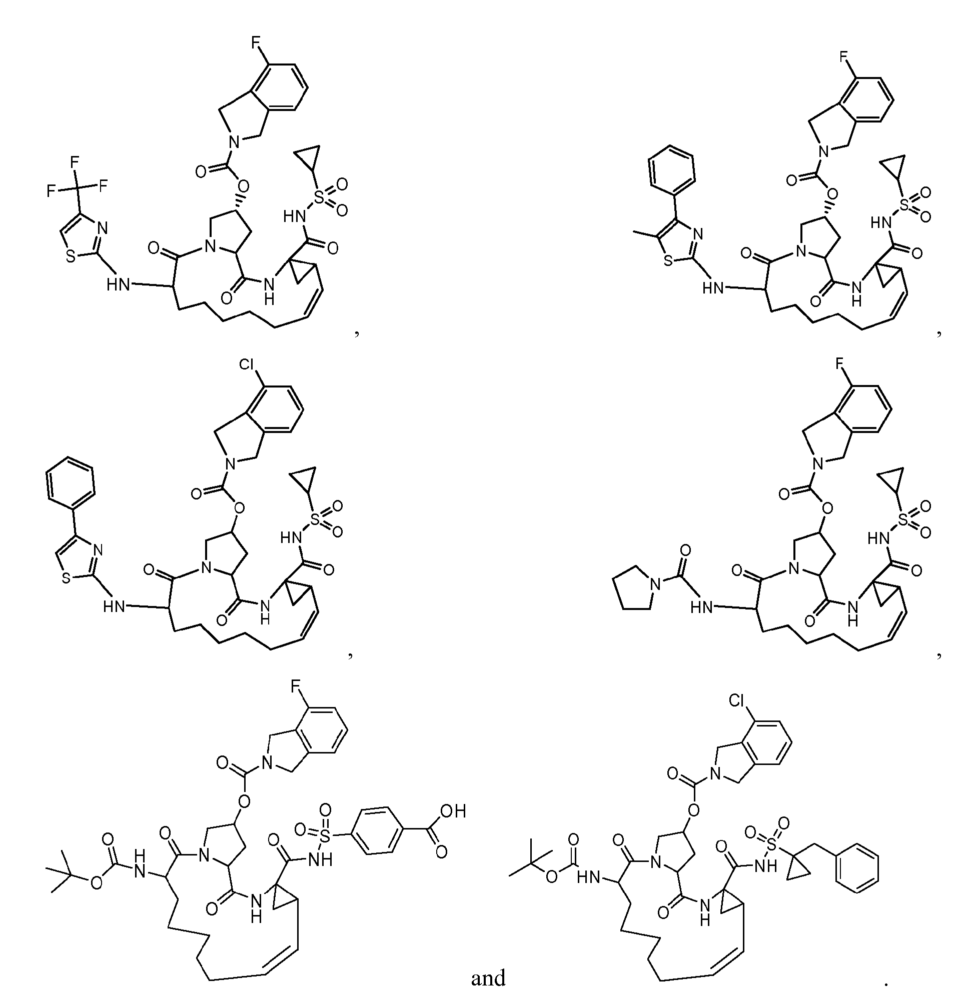

2. The compound of Claim 1, wherein R¹ is -C(O)OR⁴.

3. The compound of Claim 2, wherein R⁴ is selected from the group consisting of alkyl, tetrahydrofuranyl, tetrahydropyranyl, and phenyl.

4. The compound of Claim 2, wherein R⁴ is tert-butyl.

5. The compound of Claim 1, wherein R¹ has the structure: 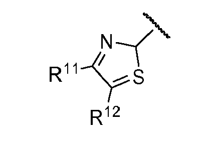 wherein R¹¹ and R¹² are independently selected from the group consisting of hydrogen, optionally substituted alkyl, optionally substituted aryl, and optionally substituted heteroaryl, or R¹¹ and R¹² together form a cycloalkyl, provided that at least one of R¹¹ and R¹² is not hydrogen.

6. The compound of Claim 5, wherein R¹¹ and R¹² are independently selected from the group consisting of hydrogen; alkyl; phenyl optionally substituted with one or more of halogen, -CN, -SO₂CH₃, -CF₃, and -OCF₃; pyridine optionally substituted with one or more halogen; and benzothiazole; or R¹¹ and R¹² together form a cyclopentyl, provided that at least one of R¹¹ and R¹² is not hydrogen.

7. The compound of Claim 1, wherein R¹ is -C(O)NR⁵R⁶.

8. The compound of Claim 7, wherein R⁵ is methyl and R⁶ is alkyl or benzyl.

9. The compound of Claim 7, wherein R⁵ together with R⁶ form an optionally substituted heterocyclyl or optionally substituted heteroaryl selected from the group consisting of N-morphlino, N-heterocyclyl optionally substituted with one or more halogen, and N-isoindolinyl.

10. The compound of Claim 1, wherein R¹ is

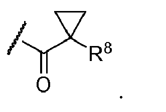

11. The compound of Claim 10, wherein R⁸ is -CF₃ or methyl.

12. The compound of Claim 1, wherein R¹ is phenyl substituted with one or more halogen.

13. The compound of Claim 1, wherein R¹ is -C(O)R⁷.

14. The compound of Claim 13, wherein R⁷ is selected from the group consisting of phenyl substituted with one or more halogen.

15. The compound of any one of Claims 1-14, wherein R³ is -OR⁹.

16. The compound of Claim 15, wherein R⁹ is selected from the group consisting of hydrogen, alkyl optionally substituted with hydroxy, phenyl, and benzyl optionally substituted with -CF₃.

17. The compound of any one of Claims 1-14, wherein R³ is -SO₂R¹⁰.

18. The compound of Claim 17, wherein R¹⁰ is selected from the group consisting of alkyl; phenyl optionally substituted with one or more of methyl, halogen, carboxy, CF₃, and alkoxy; and thiophene substituted with one or more of alkyl and halogen.

19. The compound of Claim 17, wherein R¹⁰ is cyclopropyl.

20. The compound of Claim 17, wherein:
R¹⁰ is selected from the group consisting of alkyl, optionally substituted aryl, optionally substituted aralkyl, and substituted heteroaryl;
R¹¹ and R¹² are each hydrogen; and
Z² is -CH₂-.

21. The compound of Claim 1 having a formula selected from the group consisting of the formulas of compound numbers 101-110, 201, 204, 206-228, 230, 231, 243, 245-246, 301-303, 305-356, 358-362, 364, 401-447, 501, 503-505, 507-551, 601-656, 701-720, 801-815 and 901-907 as described in the specification.

22. The compound of Claim 1 having a formula:

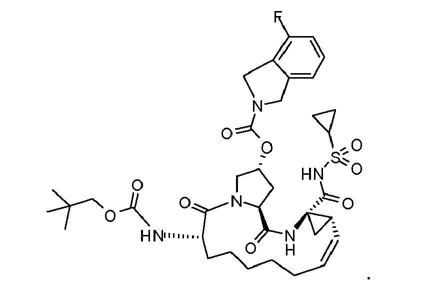

23. The compound of Claim 1 having a formula:

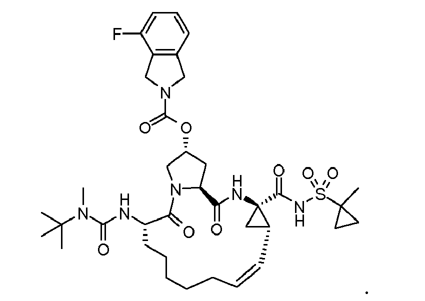

24. The compound of Claim 1 having a formula:

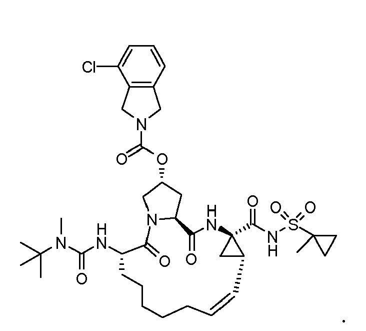

25. The compound of Claim 1 having a formula:

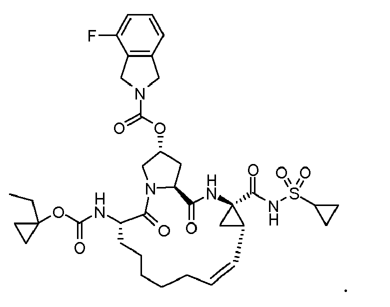

26. The compound of Claim 1 having a formula:

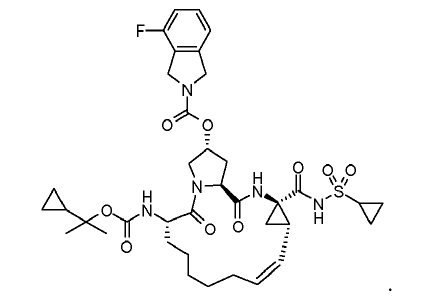

27. The compound of Claim 1 having a formula:

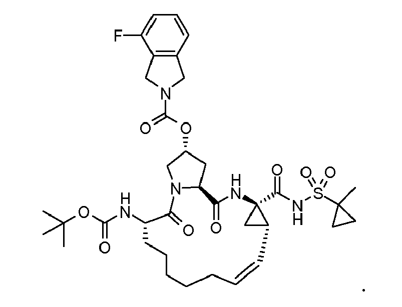

28. The compound of Claim 1 having a formula:

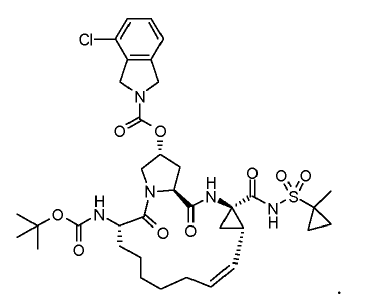

29. The compound of Claim 1, wherein R² is

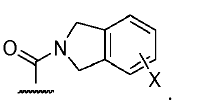

30. A pharmaceutical composition comprising a pharmaceutically acceptable excipient and a compound of any one of the preceding claims.

31. A method of inhibiting NS3/NS4 protease activity *in vitro* comprising contacting a NS3/NS4 protease with the compound of any one of Claims 1-29 or with the composition of Claim 30.

32. Use of a compound of any one of Claims 1-29 or the composition of Claim 30 in the preparation of a medicine for inhibiting NS3/NS4 protease activity.

33. Use of a compound of any one of Claims 1-29 or the composition of Claim 30 in the preparation of a medicine for treating a hepatitis C infection.

34. The use of Claim 33, wherein the medicine achieves a sustained viral response.

35. Use of a compound of any one of Claims 1-29 or the composition of Claim 30 in the preparation of a medicine for treating liver fibrosis.

36. Use of a compound of any one of Claims 1-29 or the composition of Claim 30 in the preparation of a medicine for increasing liver function in an individual having a hepatitis C virus infection.

37. The use of any one of Claim 32 to 36, wherein the medicine is manufactured for use in combination with an effective amount of a nucleoside analog.

38. The use of Claim 37, wherein the nucleoside analog is selected from ribavirin, levovirin, viramidine, an L-nucleoside, and isatoribine.

39. The use of any one of Claim 32 to 36, wherein the medicine is manufactured for use in combination with an effective amount of a human immunodeficiency virus 1 protease inhibitor.

40. The use of Claim 39, wherein the protease inhibitor is ritonavir.

41. The use of any one of Claim 32 to 36, wherein the medicine is manufactured for use in combination with an effective amount of an NS5B RNA-dependent RNA polymerase inhibitor.

42. The use of any one of Claim 32 to 36, wherein the medicine is manufactured for use in combination with an effective amount of interferon-gamma (IFN-γ).

43. The use of Claim 42, wherein the IFN-γ is suitable for administration subcutaneously in an amount of from about 10 µg to about 300 µg.

44. The use of any one of Claim 32 to 36, wherein the medicine is manufactured for use in combination with an effective amount of interferon-alpha (IFN-α).

45. The use of Claim 44, wherein the IFN-α is monoPEG-ylated consensus IFN-α and suitable for administration at a dosing interval of every 8 days to every 14 days.

46. The use of Claim 44, wherein the IFN-α is monoPEG-ylated consensus IFN-α and suitable for administration at a dosing interval of once every 7 days.

47. The use of Claim 44, wherein the IFN-α is INFERGEN consensus IFN-α.

48. The use of any one of Claim 32 to 36, wherein the medicine is manufactured for use in combination with an effective amount of an agent selected from 3'-azidothymidine, 2',3'-dideoxyinosine, 2',3'-dideoxycytidine, 2',3'-didehydro-2',3'-dideoxythymidine, combivir, abacavir, adefovir dipoxil, cidofovir, and an inosine monophosphate dehydrogenase inhibitor.

49. Use of an effective amount of compound 100, or a pharmaceutically acceptable salt, ester or prodrug thereof, in the preparation of a medicine for treating a hepatitis C virus (HCV) infection, when taken in conjunction with the consumption of food:

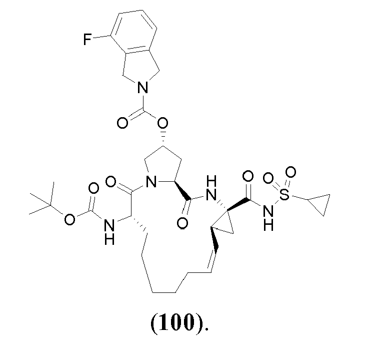

50. The use of Claim 49, wherein the medicine is manufactured to be taken orally.

51. The use of Claim 49 or Claim 50, wherein the consumption of food is effective to provide an area under the plasma concentration-time curve (AUC_{0-inf} after a single dose or AUC₀₋₂₄ at steady-state) for compound 100, or active metabolite thereof, that is greater than when the medicine is not undertaken in conjunction with the consumption of food.

52. The use of any one of Claims 49 to 51, wherein the consumption of food is undertaken substantially simultaneously as the medicine that comprises the compound 100, or the pharmaceutically acceptable salt, ester or prodrug thereof.

53. The use of any one of Claims 49 to 52, wherein the pharmaceutically acceptable salt is the sodium salt of compound 100.

54. Use of an effective amount of compound 100, or a pharmaceutically acceptable salt, ester or prodrug thereof, in the preparation of a medicine for treating a hepatitis C virus (HCV) infection: 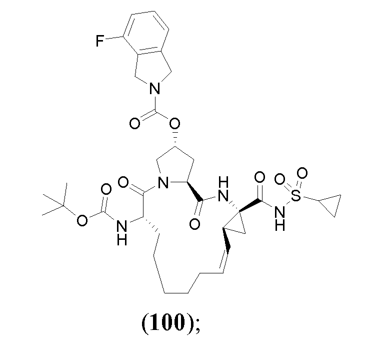 and wherein the medicine includes information that the compound 100, or the pharmaceutically acceptable salt, ester or prodrug thereof, should be accompanied by the consumption of food.

55. The use of Claim 54, wherein the medicine is manufactured to be taken orally.

56. The use of Claim 55, wherein the medicine comprises a pharmaceutically acceptable salt of compound 100.

57. The use of Claim 56, wherein the pharmaceutically acceptable salt of compound 100 is a sodium salt of compound 100.

58. A method of distributing an oral dosage form, comprising:
distributing a pharmaceutical composition, wherein the pharmaceutical composition comprises compound 100, or a pharmaceutically acceptable salt, ester or prodrug thereof: 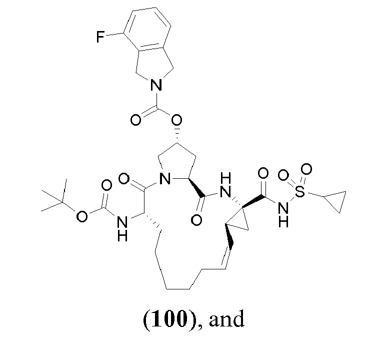
concomitantly distributing information, which information comprises that the administering of the pharmaceutical composition should be accompanied by the consumption of food.

59. The method of Claim 58, wherein the pharmaceutical composition comprises a pharmaceutically acceptable salt of compound 100.

60. The method of Claim 59, wherein the pharmaceutically acceptable salt of compound 100 is a sodium salt of compound 100.
